# EUROPEAN PATENT SPECIFICATION

(11) **EP 2 408 772 B1**
(45) Date of publication and mention of the grant of the patent: **01.07.2015**
(21) Application number: 10711443.1
(22) Date of filing: 19.03.2010
(51) Int. Cl.: C07D 471/04, A61K 31/437, A61P 25/28, A61P 35/00

(54) **COMPOUNDS**
VERBINDUNGEN
COMPOSÉS

(30) Priority: 19.03.2009 GB 0904746; 14.07.2009 GB 0912238; 28.01.2010 GB 201001418; 20.03.2009 US 162024 P
(43) Date of publication of application: 25.01.2012
(73) Proprietor: Medical Research Council Technology, London WC1H 9LT (GB)
(72) Inventor: MCIVER, Edward, Giles, London NW7 1AD (GB); SMILJANIC, Ela, London NW7 1AD (GB); HARDING, Denise, Jamilla, London NW7 1AD (GB); HOUGH, Joanne, London NW7 1AD (GB)
(74) Representative: Clyde-Watson, Zöe
(86) International application number: PCT/GB2010/000498
(87) International publication number: WO 2010/106333

(56) References cited:
- EP-A1- 1 772 454
- WO-A1-94/17062
- WO-A1-2006/077319
- WO-A1-2010/036632

## Description

The present invention relates to pyrazolopyridine compounds that are capable of inhibiting one or more kinases, more particularly, LRRK2. The compounds find applications in the treatment of a variety of disorders, including cancer and neurodegenerative diseases such as Parkinson's disease.

### BACKGROUND TO THE INVENTION

There has been much interest raised by the recent discovery that different autosomal dominant point mutations within the gene encoding for LRRK2 predispose humans to develop late-onset PD (OMIM accession number 609007), with a clinical appearance indistinguishable from idiopathic PD [1-3]. The genetic analysis undertaken to date indicates that mutations in LRRK2 are relatively frequent, not only accounting for 5-10% of familial PD, but also being found in a significant proportion of sporadic PD cases [4, 5]. Little is known about how LRRK2 is regulated in cells, what its physiological substrates are and how mutations cause or increase risk of PD.

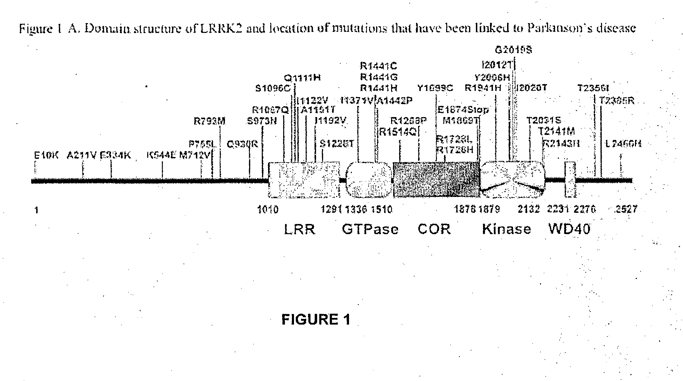

The domain structure of LRRK2 is shown in Figure 1, which also depicts the mutations that have thus far been reported in patients with PD. The defining feature of the LRRK2 enzyme is a Leucine Rich Repeat (LRR) motif (residues 1010-1291), a Ras-like small GTPase (residues 1336-1510), a region of high amino acid conservation that has been termed the C-terminal Of Ras of complex (COR) domain (residues 1511-1878), a protein kinase catalytic domain (residues 1879-2132) and a C-terminal WD40 motif (2231-2276) [6, 7]. The protein kinase domain of LRRK2 belongs to the tyrosine-like serine/threonine protein kinases and is most similar to the kinase RIP (Receptor Interacting Protein), which play key roles in innate immunity signalling pathways [8]. To date, almost 40 single amino acid substitution mutations have been linked to autosomal-dominant PD and the location of these mutations is illustrated in Figure 1A ([2, 3]). The most prevalent mutant form of LRRK2 accounting for approximately 6% of familial PD and 3% of sporadic PD cases in Europe, comprises an amino acid substitution of Gly2019 to a Ser residue. Gly2019 is located within the conserved DYG-Mg²⁺-binding motif, in subdomain-VII of the kinase domain [2]. Recent reports suggest that this mutation enhances the autophosphorylation of LRRK2, as well as its ability to phosphorylate myelin basic protein 2-3-fold [9, 10], a finding confirmed by the Applicant [11]. These observations suggest that over-activation of LRRK2 predisposes humans to develop PD, implying that drugs which inhibited LRRK2, could be utilised to halt progression or even perhaps reverse symptoms of some forms of PD.

The study of LRRK2 has been hampered by the difficulty in expressing active recombinant enzyme and by the lack of a robust quantitative assay. In work undertaken by th Applicant, an active recombinant fragment of LRRK2 containing the GTPase-COR and kinase domains encompassing residues 1326-2527 was expressed in 293 cells [11]. The more active G2019S mutant of this LRRK2 fragment was utilised in a KinasE Substrate TRacking and ELucidation (KESTREL) screen in an initial attempt to identify physiological substrates (reviewed in [14]). This led to the identification of a protein termed moesin, which was efficiently phosphorylated by LRRK2 in vitro [11]. Moesin is a member of the Ezrin/Radixin/Moesin (ERM) family of proteins which functions to anchor the actin cytoskeleton to the plasma membrane and plays an important role in regulating membrane structure and organization [15, 16]. It was found that LRRK2 phosphorylated moesin at Thr558 [11], a previously characterised physiologically relevant phosphorylation site [15, 16]. LRRK2 also phosphorylated ezrin and radixin at the equivalent Thr residue. Phosphorylation of ERM proteins at the residue equivalent to Thr558, opens up the structures of these proteins and enables them to interact with actin microfilaments at their C-terminal residues and phosphoihositides and plasma membrane proteins through an N-terminal FERM domain. These findings were utilised to develop a robust and quantitative assay for LRRK2, based upon the phosphorylation of moesin or a short peptide that encompasses the Thr558 residue of moesin which is also efficiently phosphorylated by LRRK2 [11]. These assays were further adapted to develop an improved assay based on the use of the Nictide peptide [17].

WO2006/077319 A1 discloses substituted pyrazolo pyridines for use as a medicament, in particular, as anticancer agents.

>

The present invention seeks to provide compounds that are capable of inhibiting one or more kinases, more particularly, LRRK, even more preferably LRRK2.

### STATEMENT OF INVENTION

A first aspect of the invention relates to a compound of formula I, or a pharmaceutically acceptable salt thereof, 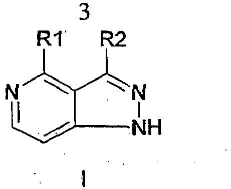 wherein:
R¹ is selected from:
   aryl;
   heteroaryl;
   C₄₋₇-heterocycloalkyl;
   -NHR³;
   fused aryl-C₄₋₇-heterocycloalkyl;
   -CONR⁴R⁵;
   -NHCOR⁶;
   -C₃₋₇-cycloalkyl;
   -NR³R⁶;
   OR³;
   OH;
   NR⁴R⁵; and
   -C₁₋₆ alkyl optionally substituted with a substituent selected from R¹¹ and a group A;
wherein said aryl, heteroaryl, fused aryl-C₄₋₇-heterocycloalkyl and C₄₋₇- heterocycloalkyl are each optionally substituted with one or more substituents selected from C₁₋₆-alkyl, C₃₋₇-cycloalkyl, heteroaryl, C₄₋₇-heterocycloalkyl, aryl and a group A, and said C₁₋₆-alkyl, C₃₋₇-cycloalkyl, heteroaryl; C₄₋₇-heterocycloalkyl, and aryl substituents are in turn each optionally substituted with one or more groups selected from R¹¹ and a group A;
R² is selected from hydrogen, aryl, C₁₋₆-alkyl, C₂₋₆-alkenyl, C₃₋₇-cycloalkyl, heteroaryl, C₄₋₇ heterocycloalkyl, fused aryl-C₄₋₇-heterocycloalkyl and halogen, wherein said C₁₋₆-alkyl, C₂₋₆-alkenyl, aryl, heteroaryl, fused aryl-C₄₋₇-heterocycloalkyl and C₄₋₇-heteropycloalkyl are each optionally substituted with one or more substituents selected from R¹¹ and A;
each R³ is selected from aryl, heteroaryl, C₄₋₇-heterocycloalkyl, C₃₋₇-cycloalkyl, fused aryl-C₄₋₇-heterocycloalkyl and C₁₋₆-alkyl, each of which is optionally substituted with one or more substituents selected from R¹¹ and A;
R⁴ and R⁵ are each independently selected from hydrogen, C₃₋₇-cycloalkyl, C₁₋₆-alkyl-C₃₋₇-cycloalkyl, aryl, heteroaryl, C₁₋₆-alkyl and a C₃₋₆-heterocycloalkyl ring optionally further containing one or more groups selected from oxygen, sulfur, nitrogen and CO, and optionally substituted by one or more R¹⁰ groups, wherein each C₁₋₆-alkyl, heteroaryl and aryl is optionally substituted by one or more substituents selected from C₁₋₆-alkyl, halogen, cyano, hydroxyl, aryl, halo-substituted aryl, heteroaryl, -NR⁸R⁹, -NR⁶R⁷, Nk⁷(CO)R⁶, -NR⁷COOR⁶, -NR⁷(SO₂)R⁶, -COOR⁶, -CONR⁸R⁹, OR⁶, -SO₂R⁶ and a C₃₋₆-heterocycloalkyl ring optionally further containing one or more groups selected from oxygen, sulfur, nitrogen and CO and optionally substituted by one or more or R¹⁰ groups; or
R⁴ and R⁵ together with the N to which they are attached form a C₃₋₆-heterocycloalkyl ring optionally further containing one or more groups selected from oxygen, sulfur, nitrogen and CO, wherein said C₃₋₆-heterocycloalkyl ring is saturated or unsaturated and is optionally substituted with one or more groups selected from A, NR⁸R⁹ and R¹⁰;
each R⁶ is independently selected from C₁₋₆-alkyl, C₃₋₇ cycloalkyl, C₄₋₇-heterocycloalkyl, aryl and heteroaryl, each of which is optionally substituted by one or more substituents selected from R¹⁰, R¹¹ and A;
each R⁷ is selected from hydrogen, C₁₋₆-alkyl and C₃₋₇-cycloalkyl, wherein said C₁₋₆-alkyl is optionally substituted by one or more halogens;
each of R⁸ and R⁹ is independently selected from hydrogen and C₁₋₆-alkyl, wherein said C₁₋₆-alkyl group is optionally substituted by one or more halogens; or
R⁸ and R⁹ together with the N to which they are attached form a C₄₋₆-heterocycloalkyl ring optionally further containing one or more heteroatoms selected from oxygen and sulfur, wherein said C₄₋₆-heterocycloalkyl ring is optionally substituted by one or more R¹⁰ groups; and each R¹⁰ is selected from C₃₋₇-cycloalkyl, aryl, heteroaryl, O-heteroaryl, aralkyl and C₁₋₆-alkyl, each of which is optionally substituted by one or more A groups, wherein where R¹⁰ is C₁₋₆-alkyl and two or more R¹⁰ groups are attached to the same carbon atom, the R¹⁰ groups may be linked to form a spiroalkyl group; and
each R¹¹ is independently selected from C₁₋₆-alkyl, C₃₋₇-cycloalkyl, C₁₋₆-alkyl-C₃₋₇-cycloalkyl, C₁₋₆-alkyl-heteroaryl, C₄₋₇-heterocycloalkyl, aryl and heteroaryl, each of which is optionally substituted with one or more substituents selected from A; and

A is selected from halogen, -NR⁴SO₂R⁵, -CN, -OR⁶, -NR⁴R⁵, -NR⁷R¹¹, hydroxyl, -CF₃, - CONR⁴R⁵, -NR⁴COR⁵, -NR⁷(CO)NR⁴R⁵, -NO₂, -CO₂H, -CO₂R⁶, -SO₂R⁶, -SO₂NR⁴R⁵, - NR⁴COR⁵ ,-NR⁴COOR⁵, C₁₋₆-alkyl, aryl and -COR⁶.

A second aspect of the invention relates to a pharmaceutical composition comprising at least one compound as described above and a pharmaceutically acceptable carrier, diluent or excipient.

A third aspect of the invention relates to a compound as described above for use in medicine.

A fourth aspect of the invention relates to a compound as described above for use in treating a disorder selected from cancer and neurodegenerative diseases such as Parkinson's Disease.

A fifth aspect of the invention relates to the use of a compound as described above in the preparation of a medicament for treating or preventing a disorder selected from cancer and neurodegenerative diseases such as Parkinson's Disease.

A sixth aspect of the invention relates to the use of a compound as described above in the preparation of a medicament for the prevention or treatment of a disorder caused by, associated with or accompanied by any abnormal kinase activity wherein the kinase is preferably LRRK, more preferably LRRK2.

A further aspect of the invention relates to the use of a compound as described above in an assay for identifying further candidate compounds capable of inhibition of a kinase, preferably LRRK, more preferably LRRK2.

A further aspect of the invention relates to a process for preparing a compound of formula I, said process comprising converting a compound of formula II into a compound of formula I:

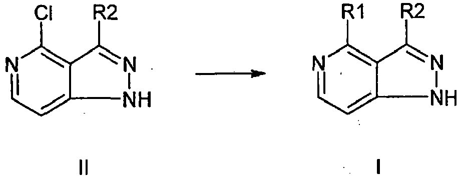

### DETAILED DESCRIPTION

The present invention relates to pyrazolopyridine compounds that are capable of inhibiting one or more kinases, more particularly LRRK, even more particularly LRRK2. Specifically, the invention relates to substituted pyrazolo[4,3-c]pyridine derivatives.

"Alkyl" is defined herein as a straight-chain or branched alkyl radical, for example, methyl, ethyl, propyl, isopropyl, butyl, isobutyl, tert-butyl, pentyl, hexyl.

"Cycloalkyl" is defined herein as a monocyclic alkyl ring, such as, cyclopropyl, cyclobutyl, cyclopentyl, cyclohexyl or cycloheptyl, or a fused bicyclic ring system such as norbomane.

"Halogen" is defined herein as chloro, fluoro, bromo or iodo.

"Heteroaryl" is defined herein as a monocyclic or bicyclic C₂₋₁₂ aromatic ring comprising one or more heteroatoms (that may be the same or different), such as oxygen, nitrogen or sulphur. Examples of suitable heteroaryl groups include thienyl, furanyl, pyrrolyl, pyridinyl, oxazolyl, thiazolyl, imidazolyl, pyrazolyl, isoxazolyl, isothiazolyl, oxadiazolyl, triazolyl, thiadiazolyl etc. and benzo derivatives thereof, such as benzofuranyl, benzothienyl, benzimidazolyl, indolyl, isoindolyl, indazolyl etc.; or pyridyl, pyrazinyl, pyrimidinyl, pyridazinyl, triazinyl etc. and benzo derivatives thereof, such as quinolinyl, isoquinolinyl, cinnolinyl, phthalazinyl, quinazolinyl, quinoxalinyl, naphthyridinyl etc.

"Heterocycloalkyl" refers to a cyclic aliphatic group containing one or more heteroatoms selected from nitrogen, oxygen and sulphur, which is optionally interrupted by one or more -(CO)- groups in the ring and/or which optionally contains one or more double bonds in the ring. Preferably, the heterocycloalkyl group is a C₃₋₇-heterocycloalkyl, more preferably a C₃₋₆-heterocycloalkyl. Alternatively, the heterocycloalkyl group is a C₄₋₇-heterocycloalkyl, more preferably a C₄₋₆-hetorocycloalkyl. Preferred heterocycloalkyl groups include, but are not limited to, piperazinyl, piperidinyl, morpholinyl, thiomorpholinyl, pyrrolidinyl, tetrahydrofuranyl and tetrahydropyranyl.

In one preferred embodiment of the invention, R² is selected from:
hydrogen;
halogen, more preferably bromine;
aryl optionally substituted by one or more substituents selected from R¹¹ and A;
C₁₋₆-alkyl optionally substituted by one or more substituents selected from R¹¹ and A;
C₂₋₆-alkenyl optionally substituted by one or more A substituents;
C₃₋₇-cycloalkyl;
heteroaryl optionally substituted by one or more substituents selected from R¹¹ and A;
C₄₋₇-heterocycloalkyl; and
fused aryl-C₄₋₇-heterocycloalkyl.

In one preferred embodiment of the invention, R² is selected from:
aryl optionally substituted by one or more substituents selected from -NR⁴COR⁵, -CONR⁴R⁵, OR⁶, halogen, optionally substituted C₁₋₆-alkyl, CN, C₄₋₇-heterocycloalkyl and heteroaryl;
C₁₋₆-alkyl optionally substituted by one or more substituents selected from -NR⁴COR⁵, - CONR⁴R⁵, -NR⁴R⁵, OR⁶, optionally substituted aryl, optionally substituted heteroaryl and C₄₋₇-heterocycloalkyl;
C₂₋₆-alkenyl optionally substituted by one or more -CONR⁴R⁵ substituents;
C₃₋₇-cycloalkyl;
heteroaryl optionally substituted by one or more substituents selected from C₄₋₇-heterocycloalkyl, C₁₋₆-alkyl, C₃₋₇-cycloalkyl, C₁₋₆-alkyl-C₃₋₇-cycloalkyl and OR⁶;
C₄₋₇-rheterocycloalkyl; and
fused aryi-C₄₋₇-rheterocycloalkyl.

In one preferred embodiment of the invention, R² is selected from:
a phenyl group optionally substituted by one or more substituents selected from -NHCO-C₁₋₆-alkyl, -CONHC₁₋₆-alkyl, CO-(N-morpholinyl), Cl, F, -OC₁₋₆-alkyl, -CONMe₂, OCF₃, CN, CF₃, C₁₋₆-alkyl-(A), N-morpholinyl and pyrazolyl;
a heteroaryl group selected from pyridinyl, quinolinyl, pyrazoyl, furanyl and pyrimidinyl, each of which may be optionally substituted by one or more substituents selected from C₁₋₆-alkyl, aralkyl, OC₁₋₆-alkyl, N-morpholinyl;
a C₁₋₆-alkyl group optionally substituted by one or more substituents selected from -CONR⁴R⁵, phenyl, pyridinyl and oxadiazolyl and piperidinyl, wherein said phenyl, pyridinyl and oxadiazolyl and piperidinyl groups are each optionally further substituted by one or more-NR⁴COR⁵, -CONR⁴R⁵, COR⁶, SO₂R⁶ or aryl groups.

In a more preferred embodiment of the invention, each -CONR⁴R⁵ group is independently selected from:
- CO(N-morpholinyl), -CO(N-piperidinyl), -CO(N-pyrrolidinyl), -CO-(N-piperazinyl), each of which may be optionally further substituted by one or more substituents selected from aryl, heteroaryl, -OR⁶, CF₃, aralkyl, -NR⁴COR⁵-CONR⁴R⁵, -NR⁴R⁵, halogen, C₁₋₆-alkyl; and
- CON(C₁₋6-alkyl)₂, CONH(C₁₋₆-alkyl), CON(C₁₋₆-alkyl)(aralkyl), CONH(C₃₋₇-cycloalkyl),-CONH(aryl), -CONH(heteroaryl), wherein said C₁₋₆-alkyl, aralkyl, aryl and heteroaryl, groups are each optionally further substituted by one or more R¹¹ or A groups.

In one preferred embodiment of the invention, R² is a C₁₋₆-alkyl group optionally substituted by one or more substituents selected from -NR⁴COR⁵, -CONR⁴R⁵, -NR⁴R⁵, OR⁶, C₄₋₇-heterocycloalkyl, heteroaryl and aryl, wherein said aryl group is optionally substituted by one or more substituents selected from -NR⁴COR⁵ and -CONR⁴R⁵.

In one preferred embodiment of the invention, R² is selected from -CH₂CH₂CO-NR⁴R⁵, C₁₋₆-alkyl, C₃₋₇ cycloalkyl and a heteroaryl selected from furanyl and pyrazolyl, wherein said furanyl and pyrazolyl groups may be optionally substituted by one or more substituents selected from C₁₋₆-alkyl, C₃₋₇-cycloalkyl and C₁₋₆-alkyl-C₃₋₇cycloalkyl.

In one preferred embodiment of the invention, R² is selected from Me, 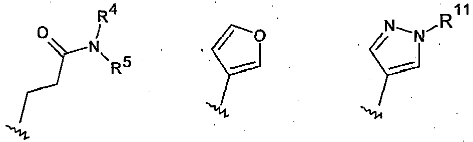 wherein R⁴ and R⁵ together with the N to which they are attached form a C₃₋₆-heterocycloalkyl ring optionally further containing one or more groups selected from oxygen, sulfur, nitrogen and CO, wherein said C₃₋₆-heterocycloalkyl ring is saturated or unsaturated and is optionally substituted with one or more groups selected from A, NR⁸R⁹ and R¹⁰. Even more preferably, R⁴ and R⁵ together with the N to which they are attached form a 6-membered heterocycloalkyl ring that is optionally substituted with one or more groups selected from A, NR⁸R⁹ and R¹⁰. More preferably, still, R⁴ and R⁵ together with the N to which they are attached form a saturated 6-membered ring (more preferably, a piperidinyl ring) that is optionally substituted with one or more groups selected from A, NR⁸R⁹ and R¹⁰.

In one highly preferred embodiment of the invention, R² is selected from Me,

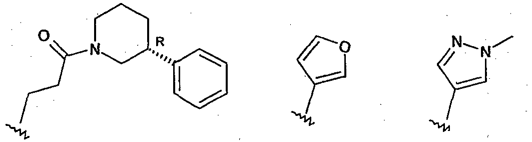

In one preferred embodiment of the invention, R² is an unsubstituted C₁₋₆-alkyl group, more preferably methyl.

In one preferred embodiment of the invention, R¹ is selected from:
-NHR³;
aryl;
heteroaryl;
C₄₋₇-heterocycloalkyl;
fused aryl-C₄₋₇-heterocycloalkyl;
-C₃₋₇-cycloalkyl;
-NR³R⁶;
OR³;
NR⁴R⁵; and
-C₁₋₆ alkyl optionally substituted with a substituent selected from R¹¹ and a group A;
wherein said aryl, heteroaryl, fused aryl-C₄₋₇-heterocycloalkyl and C₄₋₇- heterocycloalkyl are each optionally substituted with one or more substituents selected from C₁₋₆-alkyl, C₃₋₇-cycloalkyl, heteroaryl, C₄₋₇-heterocycloalkyl, aryl and a group A, and said C₁₋₆-alkyl, C₃₋₇-cycloalkyl, heteroaryl, C₄₋₇-heterocycloalkyl, and aryl substituents are in turn each optionally substituted with one or more groups selected from R¹¹ and a group A.

In one preferred embodiment of the invention, R¹ is -NHR³ and R³ is selected from:
C₁₋₆-alkyl, optionally substituted by one or more -OR⁶, NR⁴COR⁵, heteroaryl, aryl, C₄₋₇-heterocycloalkyl, and C₃₋₇-cycloalkyl groups, wherein said aryl and heteroaryl groups are each independently optionally further substituted by one for more groups selected from CF₃, halogen, C₁₋₆-alkyl, -OR⁶ and -NR*⁴*R⁵;
a phenyl group optionally substituted by one or more substituents selected from -OR⁶, NR⁴COR⁵, -CONR⁴R⁵, aryl, -NR⁴R⁵, C₁₋₆-alkyl-heteroaryl, heteroaryl, halogen, -SO₂R⁶, CN, CF₃, C₁₋₆-alkyl, -SO₂NR⁴R⁵, -NR⁴SO₂R⁵, wherein said C₁₋₆-alkyl, heteroaryl and aryl groups are each independently optionally further substituted by one or more groups selected from CN, CF₃, halogen, C₁₋₆-alkyl, -OR and -NR⁴R⁵;
a heteroaryl group optionally substituted by one or more substituents selected from aryl, C₁₋₆-alkyl, and -NR⁴R⁵, wherein said aryl group is optionally further substituted by one or more A groups;
a C₄₋₇-heterocycloalkyl optionally substituted by one or more -COR⁶ groups;
a C₃₋₇-cycloalkyl group optionally substituted by one or more halogen or C₁₋₆-alkyl groups.

In one preferred embodiment of the invention, R¹ is -NHR³, wherein R³ is selected from C₁₋₆-alkyl, C₃₋₇-cycloalkyl, C₄₋₇-heterocycloalkyl and aryl, each of which may be optionally substituted by one or more with one or more substituents selected from R¹¹ and A.

In one preferred embodiment of the invention, R¹ is -OR³, wherein R³ is selected from C₁₋₆-alkyl, C₃₋₇-cycloalkyl, C₄₋₇-heterocycloalkyl and aryl, each of which may be optionally substituted by one or more with one or more substituents selected from R¹¹ and A.

In one preferred embodiment of the invention, R¹ is -OR³, wherein R³ is C₁₋₆-alkyl, C₃₋₇-cycloalkyl or C₄₋₇-heterocycloalkyl, each of which may be optionally substituted by one or more A substituents. In one particularly preferred embodiment of the invention, R¹ is -O-C₃₋₇-cycloalkyl, more preferably, -O-cyclohexyl.

In one preferred embodiment of the invention, R¹ is aryl or heteroaryl, each of which may be optionally substituted by one or more with one or more substituents selected from R¹¹ and A.

In one preferred embodiment of the invention, R¹ is -NH-C₃₋₇-cycloalkyl or NH-C₄₋₇-heterocycloalkyl, each of which may be optionally substituted by one or more A substituents. Preferably, A is halogen or C₁₋₆-alkyl.

In one preferred embodiment of the invention, R³ is cyclohexyl or tetrahydropyranyl, each of which may be optionally substituted by one or more A substituents.

In one preferred embodiment of the invention, R¹ is selected from the following:

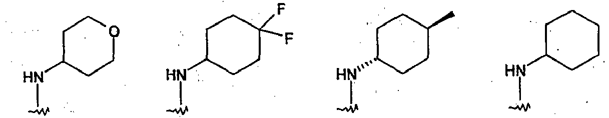

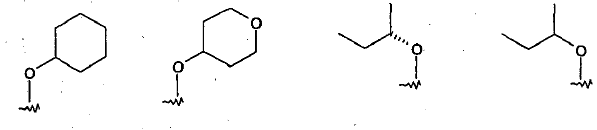

In one preferred embodiment of the invention, R¹ is -NH-cyclohexyl.

In one preferred embodiment of the invention, R¹ is -NHR³ and R² is an unsubstituted C₁₋₆-alkyl group, more preferably methyl.

In one preferred embodiment of the invention, R¹ is -NHR³ and R² is a C₁₋₆-alkyl group substituted by one or more -CONR⁴R⁵ groups.

In one preferred embodiment of the invention, R¹ is -NHR³ and R² is an aryl or heteroaryl group, each of which may be optionally substituted by one or more substituents selected from C₄₋₇-heterocycloalkyl, C₁₋₆-alkyl, C₃₋₇-cycloalkyl, C₁₋₆-alkyl-C₃₋₇-cycloalkyl and OR⁶.

In one preferred embodiment of the invention, R¹ is -OR³ and R² is a C₁₋₆-alkyl group, more preferably methyl.

In one preferred embodiment of the invention, R¹ is selected from: 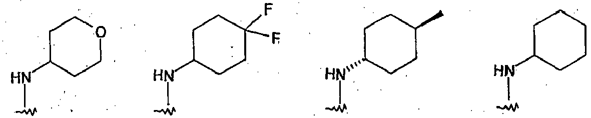 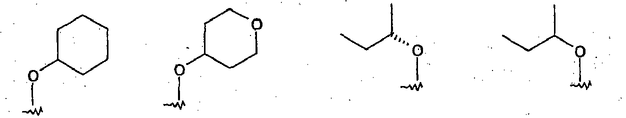 and R² is selected from 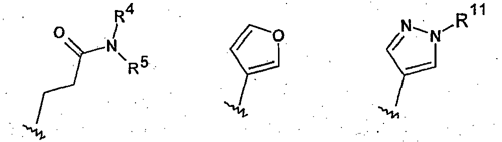 wherein R⁴ and R⁵ together with the N to which they are attached form a C₃₋₆-heterocycloalkyl ring optionally further containing one or more groups selected from oxygen, sulfur, nitrogen and CO, wherein said C₃₋₆-heterocycloalkyl ring is saturated or unsaturated and is optionally substituted with one or more groups selected from A, NR⁸R⁹ and R¹⁰. Even more preferably, R⁴ and R⁵ together with the N to which they are attached form a 6-membered heterocycloalkyl ring that is optionally substituted with one or more groups selected from A, NR⁸R⁹ and R¹⁰.

More preferably still, R⁴ and R⁵ together with the N to which they are attached form a saturated 6-membered ring (more preferably, a piperidinyl ring) that is optionally substituted with one or more groups selected from A, NR⁸R⁹ and R¹⁰.

More preferably, R¹ is as defined above, and R² is selected from Me,

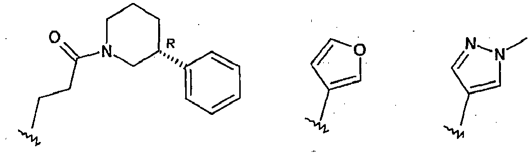

In one preferred embodiment of the invention:
R¹ is selected from aryl, heteroaryl, C₄₋₇-heterocycloalkyl, fused aryl-C₄₋₇-heterocycloalkyl and -NHR³, wherein said aryl, heteroaryl, fused aryl-C₄₋₇-heterocycloalkyl and C₄₋₇-heterocycloalkyl are each optionally substituted with one or more substituents selected from C₁₋₆-alkyl, C₃₋₇-cycloalkyl, heteroaryl, C₄₋₇-heterocycloalkyl, aryl and a group A, and said C₁₋₆-alkyl, C₃₋₇-cycloalkyl, heteroaryl, C₄₋₇-heterocycloalkyl, and aryl substituents are in turn each optionally substituted with one or more groups selected from R¹¹ and a group A; and
R² is selected from hydrogen, aryl, C₁₋₆-alkyl, C₃₋₇-cycloalkyl, heteroaryl, C₄₋₇ heterocycloalkyl and halogen, wherein said C₁₋₆-alkyl, aryl, heteroaryl and C₄₋₇-heterocycloalkyl are each optionally substituted with one or more substituents selected from R¹¹ and A.

In another preferred embodiment of the invention R² is a C₁₋₆-alkyl group optionally substituted with one or more substituents selected from R¹¹ and A.

In one preferred embodiment of the invention R¹ is selected from:
NH-R³, where R³ is selected from C₁₋₆-alkyl, morpholinyl, C₃₋₇-cycloalkyl, fused aryl-C₄₋₇-heterocycloalkyl, piperidinyl, tetrahydropyranyl, piperazinyl, phenyl, pyridinyl, indazolyl and pyrazolyl, each of which is optionally substituted by one or more substituents selected from R¹¹ and A; and
furyl, pyrazolyl and phenyl, each of which is optionally substituted by one or more substituents selected from R¹¹ and A.

In one preferred embodiment of the invention R¹ is selected from:
NH-C₁₋₆-alkyl, wherein said C₁₋₆-alkyl is optionally substituted by one or more substituents selected from OR⁶, OH, C₄₋₇ heterocycloalkyl, NR⁴R⁵, heteroaryl, C₃₋₇-cycloalkyl, phenyl,
wherein said phenyl group is optionally substituted by one or more halo groups, and said C₄₋₇ heterocycloalkyl group is optionally substituted by one or more C₁₋₆-alkyl groups;
NH-piperazinyl, wherein said piperazinyl is optionally substituted by one or more substituents selected from C₁₋₆-alkyl, aryl, C₁₋₆-alkyl-aryl and heteroaryl, each of which is optionally further substituted by one or more halo groups;
NH-morpholinyl;
NH-C₃₋₇-cycloalkyl, wherein said C₃₋₇-cycloalkyl is optionally substituted by one or more substituents selected from OH and halo;
NH-fused aryl-C₄₋₇-heterocycloalkyl, wherein said fused aryl-C₄₋₇-heterocycloalkyl is optionally substituted by one or more C₁₋₆-alkyl groups;
NH-piperidinyl, wherein said piperidinyl is optionally substituted by one or more C₁₋₆-alkyl groups;
NH-tetrahydropyranyl;
a furyl group;
a pyrazolyl group, optionally substituted by one or more C₁₋₆-alkyl groups;
NH-phenyl, wherein said phenyl is optionally substituted by one or more substituents selected from halo, CF₃, OH, OR⁶, NR⁴SO₂R⁵ NR⁴R⁵, C₄₋₇ heterocycloalkyl, CONR⁴R⁵ and -NR⁴COR⁵; NH-pyridinyl, wherein said pyridinyl is optionally substituted by one or more substituents selected from C₄₋₇ heterocycloalkyl and aryl, wherein said aryl group is optionally further substituted with one or more halo groups;
phenyl, optionally substituted by one or more substituents selected from halo, OR⁶,-NR⁴SO₂R⁵, CN, C₄₋₇ heterocycloalkyl and C₁₋₆-alkyl-NR⁴SO₂R⁵;
NH-indazolyl, wherein said indazolyl is optionally substituted by one or more C₁₋₆-alkyl groups; and
NH-pyrazolyl.

In one preferred embodiment of the invention R¹ is selected from:
NH-C₁₋₆-alkyl, wherein said C₁₋₆-alkyl is optionally substituted by one or more substituents selected from OMe, OH, tetrahydropyranyl, pyrrolidinyl, NEt₂, imidazolyl, cyclopropyl, cyclobutyl, cyclopentyl, cyclohexyl, phenyl, wherein said phenyl group is optionally substituted by one or more chloro groups, and said pyrrolidinyl group is optionally substituted by one or
more methyl groups;
NH-piperazinyl, wherein said piperazinyl is optionally substituted by one or more substituents selected from methyl, phenyl, CH₂-phenyl and pyridinyl, wherein the phenyl group is optionally further substituted by one or more F or Cl groups;
NH-morpholinyl;
NH-cyclopropyl, NH-cyclobutyl, NH-cyclopentyl and NH-cyclohexyl, wherein said cyclopropyl, cyclobutyl, cyclopentyl and cyclohexyl groups are optionally substituted by one or more substituents selected from OH and F;
NH-(1,2,3,4-tetrahydroisoquinolinyl), wherein said 1,2,3,4-tetrahydroisoquinolinyl group is optionally substituted by one or more methyl groups;
NH-piperidinyl, wherein said piperidinyl is optionally substituted by one or more methyl groups; NH-tetrahydropyranyl;
a furyl group;
a pyrazolyl group, optionally substituted by one or more methyl groups;
NH-phenyl, wherein said phenyl is optionally substituted by one or more substituents selected from F, Cl, Br, CF₃, OH, OEt, NHSO₂Me, NMe₂, morpholinyl, CONMe₂, CONH₂ and-NHCOMe;
NH-pyridinyl, wherein said pyridinyl is optionally substituted by one or more substituents selected from morpholinyl and phenyl wherein said phenyl group is optionally further substituted with one or more CN groups;
phenyl, optionally substituted by one or more substituents selected from F, Cl, One,-NHSO₂Me, CN, morpholinyl and CH₂-NHSO₂Me;
NH-indazolyl, wherein said indazolyl is optionally substituted by one or more methyl groups; and
NH-pyrazolyl.

In one highly preferred embodiment of the invention the compound of formula I is selected from the following:

| | | | |
|---|---|---|---|
| 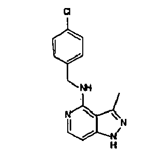 | Example 1 | 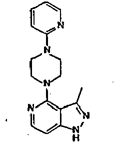 | Example 9 |
| 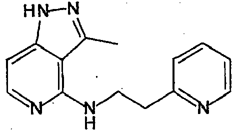 | Example 2 | 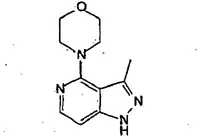 | Example 10 |
| 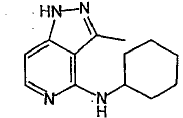 | Example 3 | 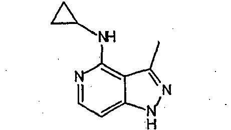 | Example 11 |
| 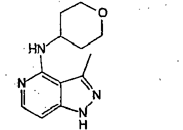 | Example 4 | 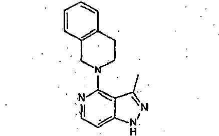 | Example 12 |
| 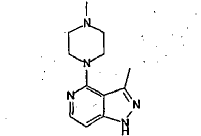 | Example 5 | 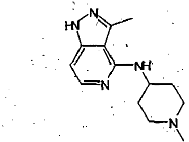 | Example 13 |
| 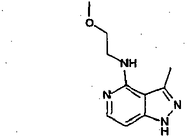 | Example 6 | 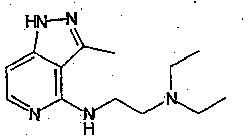 | Example 14 |
| 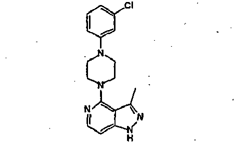 | Example 7 | 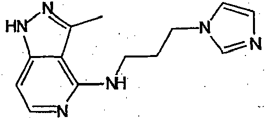 | Example 15 |
| 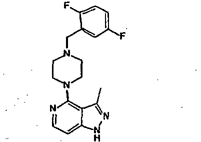 | Example 8 | 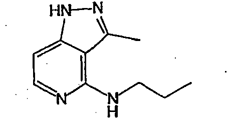 | Example 16 |
| 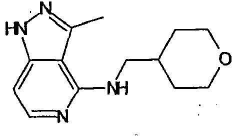 | Example 17 | 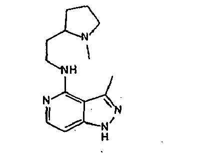 | Example 25 |
| 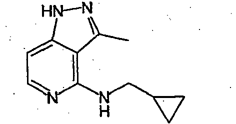 | Example 18 | 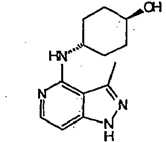 | Example 26 . |
| 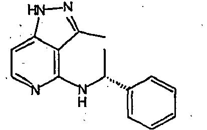 | Example 19 | 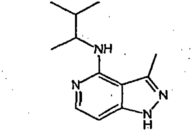 | Example 27 |
| 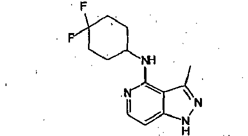 | Example 20 | 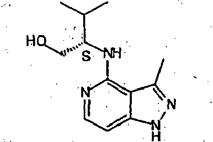 | Example 28 |
| 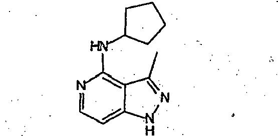 | Example 21 | 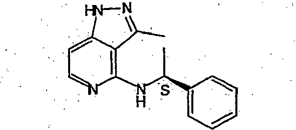 | Example 29 |
| 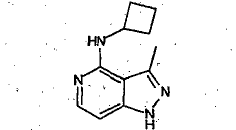 | Example 22 | 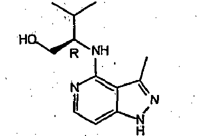 | Example 30 |
| 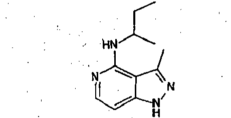 | Example 23 | 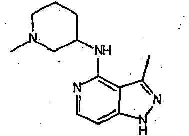 | Example 31 |
| 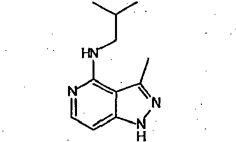 | Example 24 | 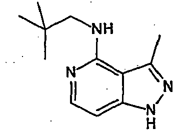 | Example 32 |
| 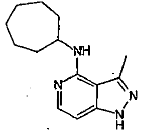 | Example 33 | 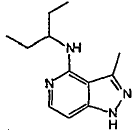 | Example 41 |
| 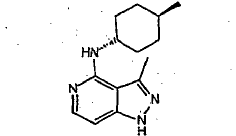 | Example 34 | 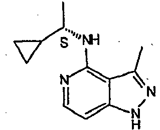 | Example 42 |
| 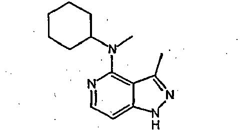 | Example 35 | 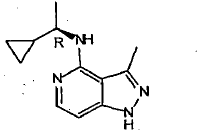 | Example 43. |
| 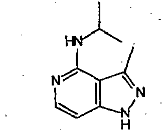 | Example 36 | 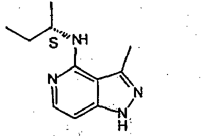 | Example 44 |
| 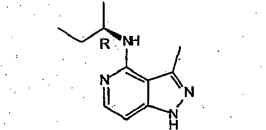 | Example 37 | 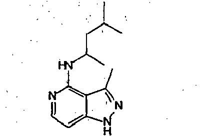 | Example 45 |
| 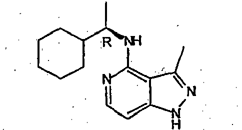 | Example 38 | 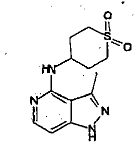 | Example 46 |
| 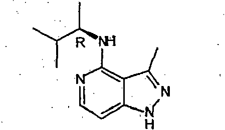 | Example 39 | 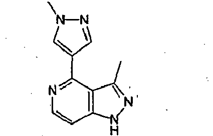 | Example 47 |
| 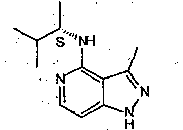 | Example 40 | 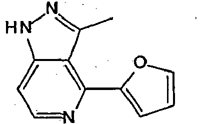 | Example 48 |
| 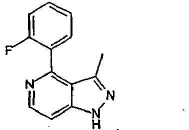 | Example 49 | 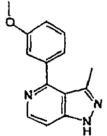 | Example 57 |
| 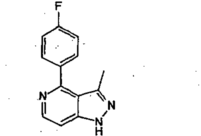 | Example 50 | 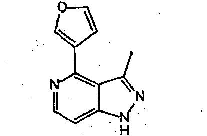 | Example 58 |
| 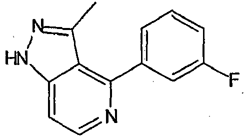 | Example 51 | 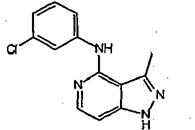 | Example 59 |
| 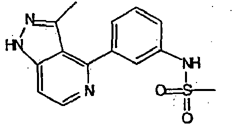 | Example 52 | 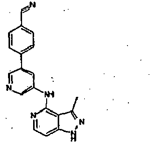 | Example 60 |
| 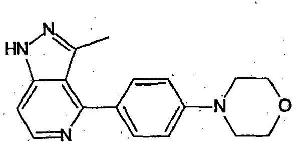 | Example 53 | 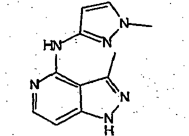 | Example 61 |
| 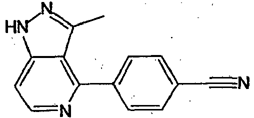 | Example 54 | 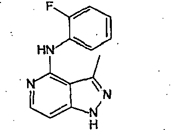 | Example 62 |
| 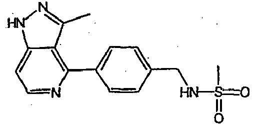 | Example 55 | 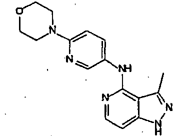 | Example 63 |
| 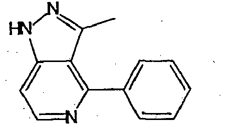 | Example 56 | 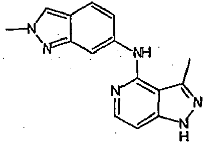 | Example 64 |
| 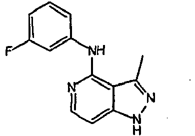 | Example 65 | 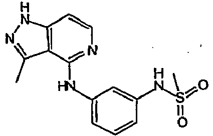 | Example 73 |
| 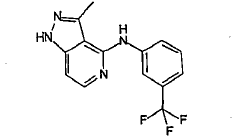 | Example 66 | 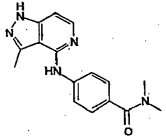 | Example 74 |
| 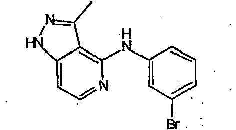 | Example 67 | 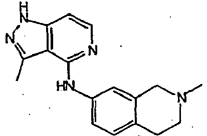 | Example 75 |
| 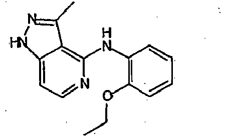 | Example 68 | 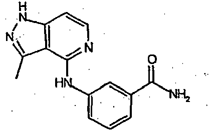 | Example 76 |
| 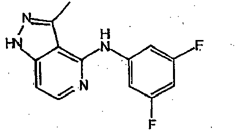 | Example 69 | 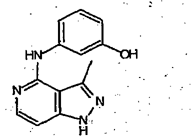 | Example 77 |
| 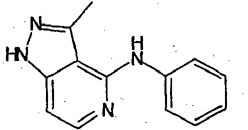 | Example 70 | 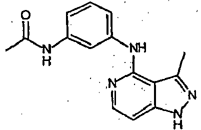 | Example 78 |
| 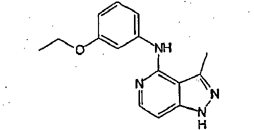 | Example 71 | 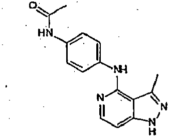 | Example 79 |
| 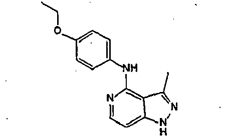 | Example 72 | 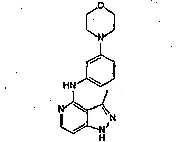 | Example 80 |
| 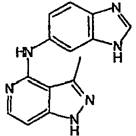 | Example 81 | 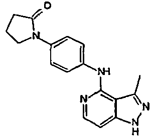 | Example 89 |
| 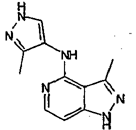 | Example 82 | 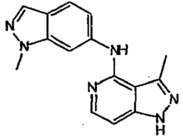 | Example 90 |
| 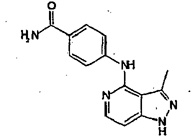 | Example 83 | 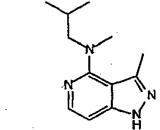 | Example 91 |
| 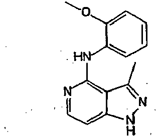 | Example 84 | 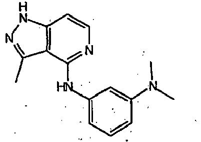 | Example 92 |
| 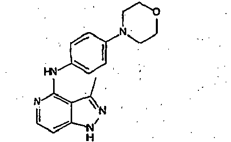 | Example 85 | 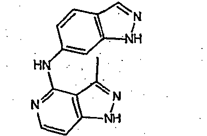 | Example 93 |
| 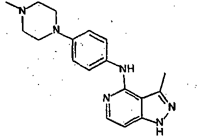 | Example 86 | 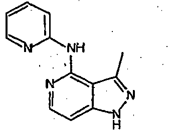 | Example 94 |
| 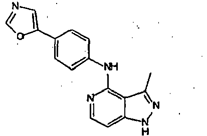 | Example 87 | 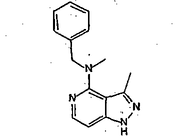 | Example 95 |
| 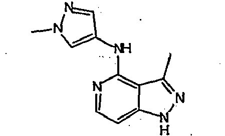 | Example 88 | 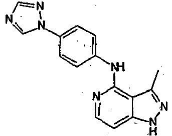 | Example 96 |
| 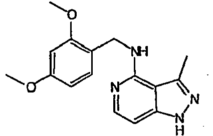 | Example 97 | 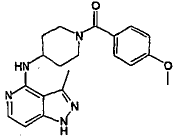 | Example 105 |
| 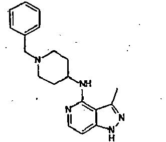 | Example 98 | 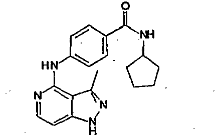 | Example 106 |
| 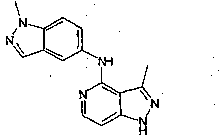 | Example 99 | 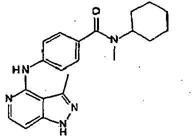 | Example 107 |
| 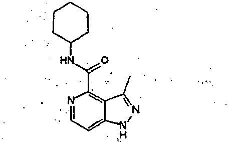 | Example 100 | 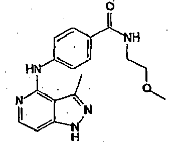 | Example 108 |
| 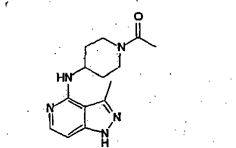 | Example 101 | 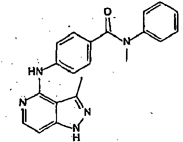 | Example 109 |
| 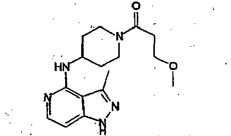 | Example 102 | 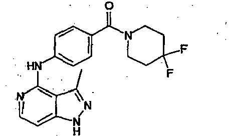 | Example 110 |
| 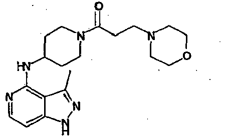 | Example 103 | 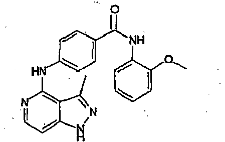 | Example 111 |
| 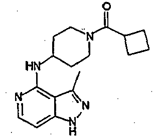 | Example 104 | 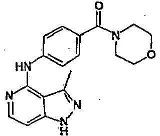 | Example 112 |
| 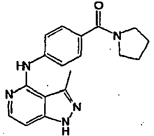 | Example 113 | 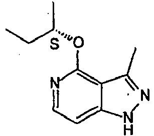 | Example 121 |
| 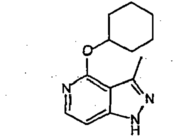 | Example 114 | 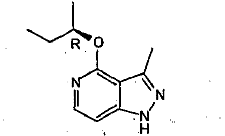 | Example 122 |
| 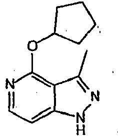 | Example 115 | 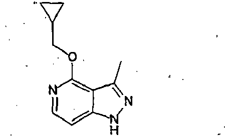 | Example 123 |
| 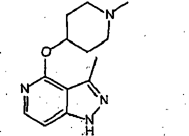 | Example 116 | 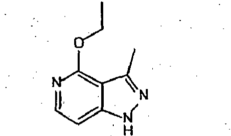 | Example 124 |
| 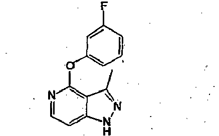 | Example 117 | 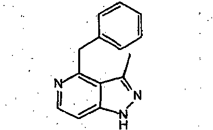 | Example 125 |
| 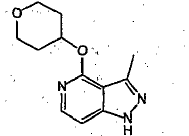 | Example 118 | 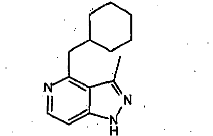 | Example 126 |
| 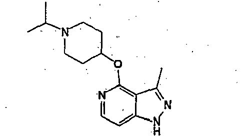 | Example 119 | 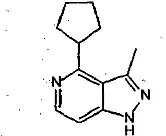 | Example 127 |
| 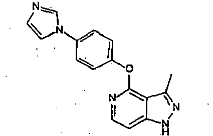 | Example 120 | 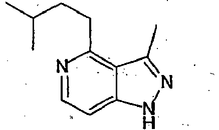 | Example 128 |
| 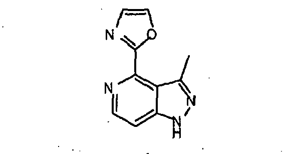 | Example 129 | 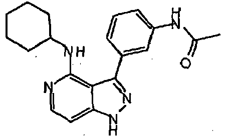 | Example 137 |
| 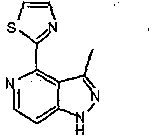 | Example 130 | 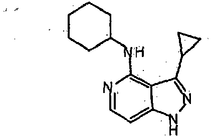 | Example 138 |
| 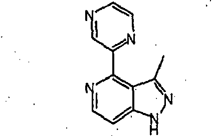 | Example 131 | 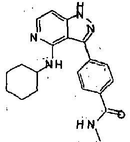 | Example 139 |
| | | 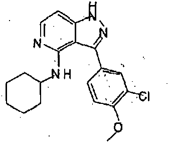 | Example 140 |
| | | 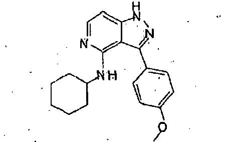 | Example 141 |
| 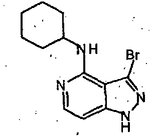 | Example 134 | 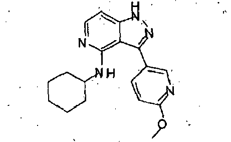 | Example 142 |
| 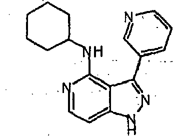 | Example 135 | 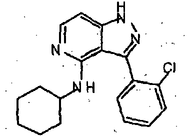 | Example 143 |
| 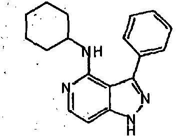 | Example 136 | 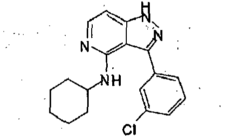 | Example 144 |
| 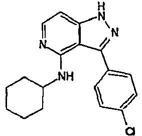 | Example 145 | 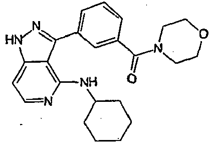 | Example 153 |
| 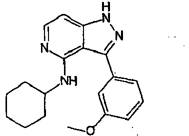 | Example 146 | 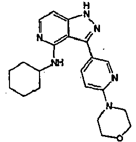 | Example 154 |
| 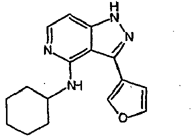 | Example 147 | 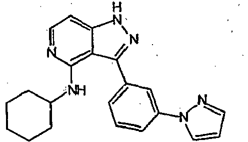 | Example 155 |
| 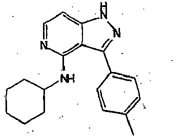 | Example 148 | 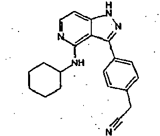 | Example 156 |
| 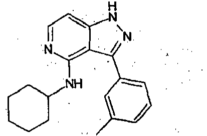 | Example 149 | 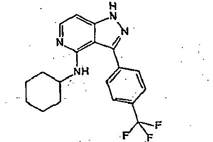 | Example 1 157 |
| 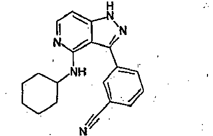 | Example 150 | 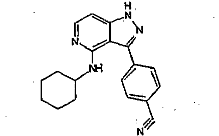 | Example 158 |
| 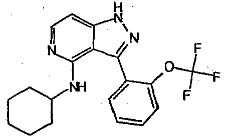 | Example 151 | 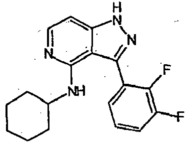 | Example 159 |
| 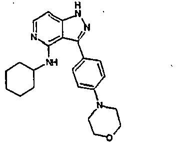 | Example 152 | 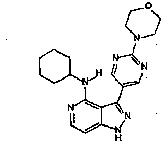 | Example 160 |
| 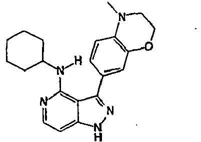 | Example 161 | 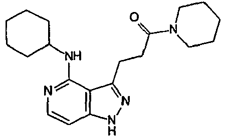 | Example 169 |
| 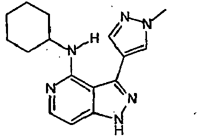 | Example 162 | 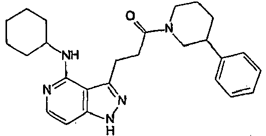 | Example 170 |
| 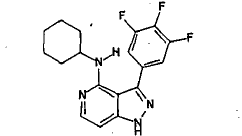 | Example 163 | 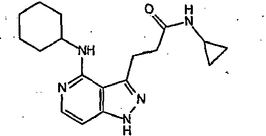 | Example 171 |
| 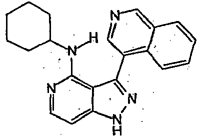 | Example 164 | 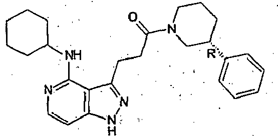 | Example 172 |
| 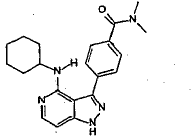 | Example 165 | 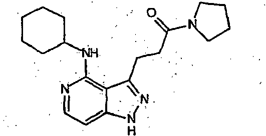 | Example 173 |
| 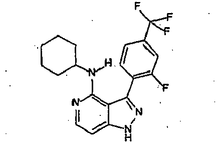 | Example 166 | 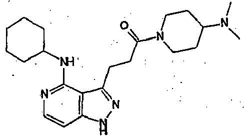 | Example 174 |
| 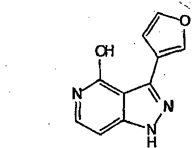 | Example 167 | 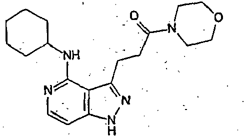 | Example 175 |
| 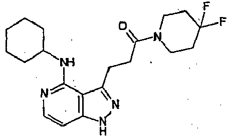 | Example 168 | 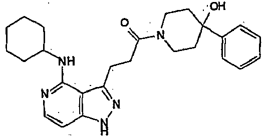 | Example 176 |
| 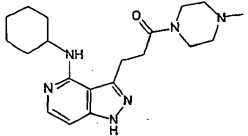 | Example 177 | 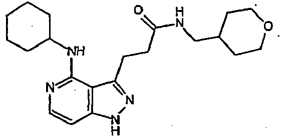 | Example 185 |
| 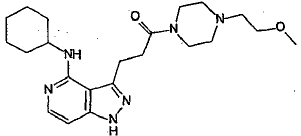 | Example 178 | 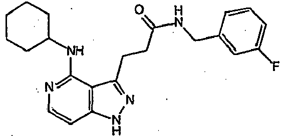 | Example 186 |
| 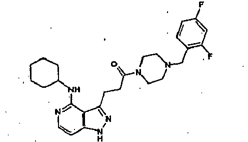 | Example 179 | 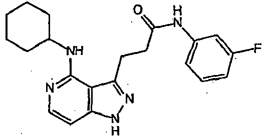 | Example 187 |
| 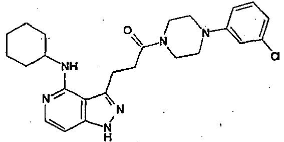 | Example 180 | 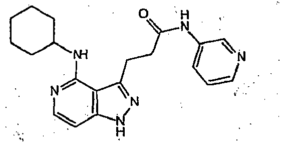 | Example 186 |
| 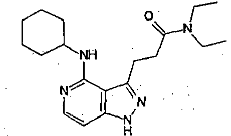 | Example 181 | 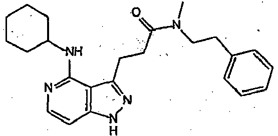 | Example 189 |
| 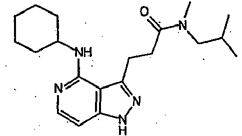 | Example 182 | 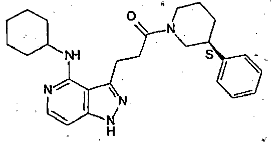 | Example 190 |
| 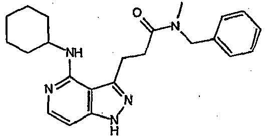 | Example 183 | 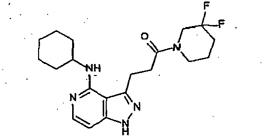 | Example 191 |
| 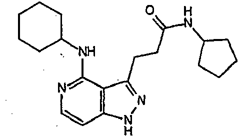 | Example 184 | 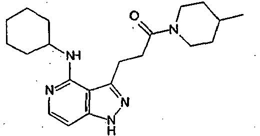 | Example 192 |
| 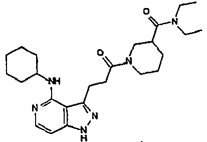 | Example 193 | 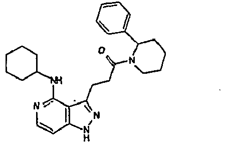 | Example 201 |
| 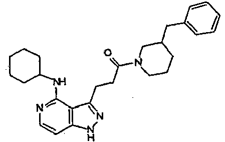 | Example 194 | 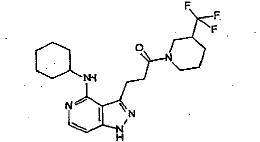 | Example 202 |
| 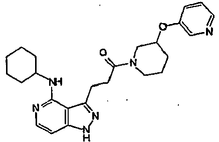 | Example 195 | 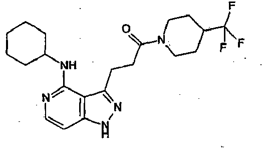 | Example 203 |
| 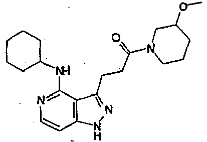 | Example 196 | 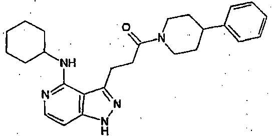 | Example 204 |
| 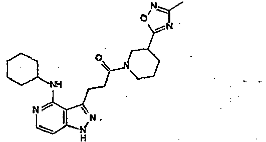 | Example 197 | 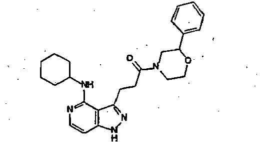 | Example 205 |
| 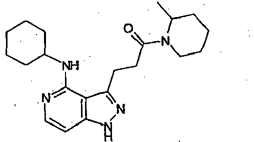 | Example 198 | 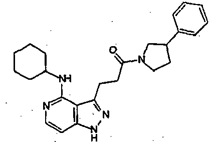 | Example 206 |
| 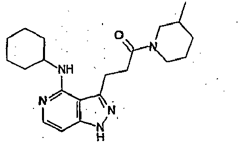 | Example 199 | 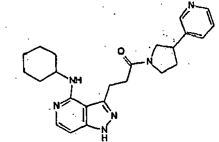 | Example 207 |
| 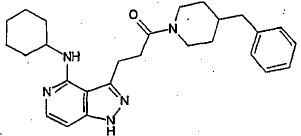 | Example 200 | 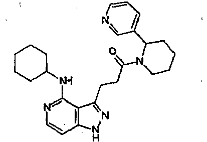 | Example 208 |
| 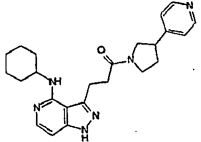 | Example 209 | 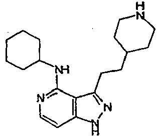 | Example 217 |
| 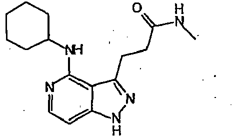 | Example 210 | 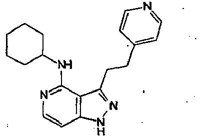 | Example 218 |
| 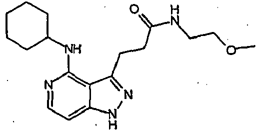 | Example 211 | 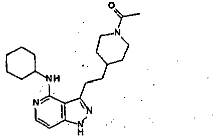 | Example 219 |
| 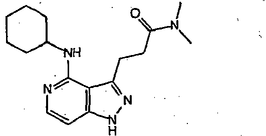 | Example 212 | 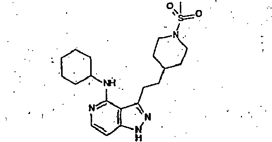 | Example 220 |
| 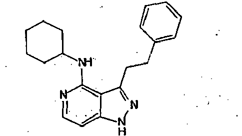 | Example 213 | 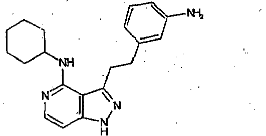 | Example 221 |
| 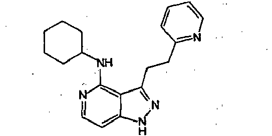 | Example 214 | 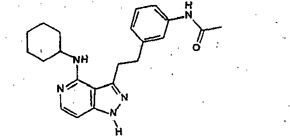 | Example 222 |
| 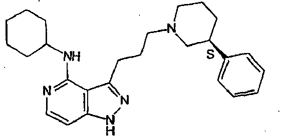 | Example 215 | 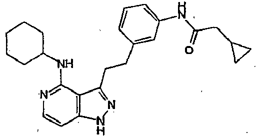 | Example 223 |
| 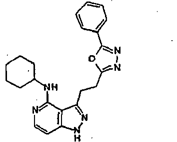 | Example 216 | 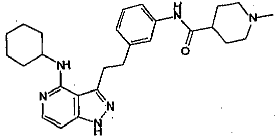 | Example 224 |
| 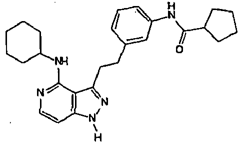 | Example 225 | | |
| 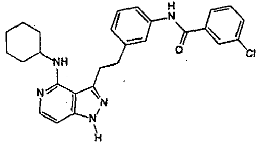 | Example 226 | | |
| 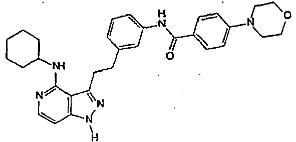 | Example 227 | | |
| 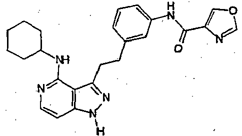 | Example 228 | | |
| 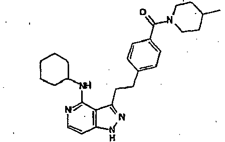 | Example 229 | | |
| 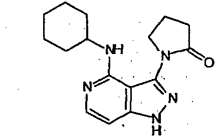 | Example 230 | | |
| 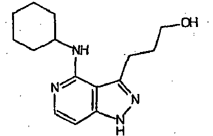 | Example 231 | | |
| 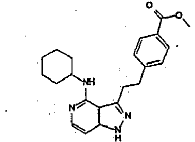 | Example 232 | | |
| 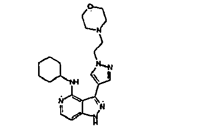 | Example 233 | 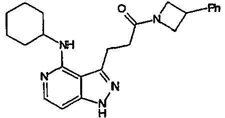 | Example 242 |
| 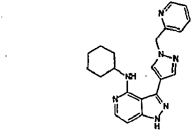 | Example 234 | 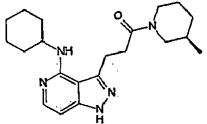 | Example 243 |
| 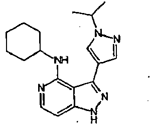 | Example 235 | 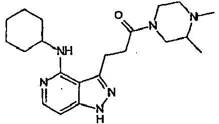 | Example 244 |
| 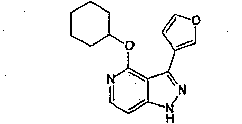 | Example 236 | 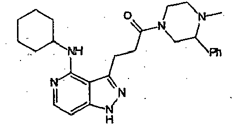 | Example 245 |
| 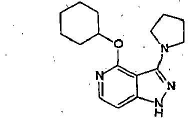 | Example 237 | 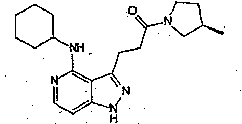 | Example 246 |
| 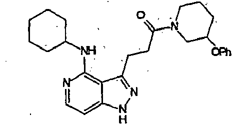 | Example 238 | 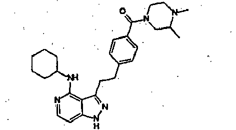 | Example 247 |
| 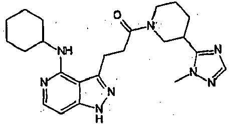 | Example 239 | 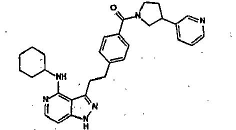 | Example 248 |
| 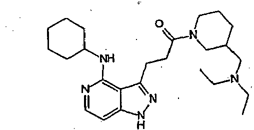 | Example 240 | 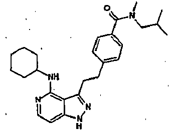 | Example 249 |
| 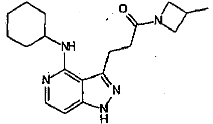 | Example 241 | 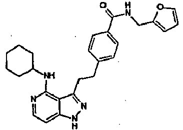 | Example 250 |
| 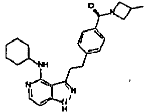 | Example 251 | 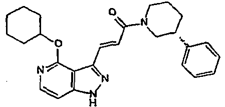 | Example 261 |
| 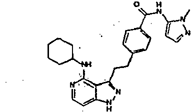 | Example 252 | 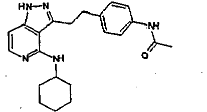 | Example 262 |
| 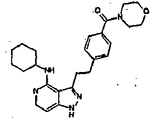 | Example 253 | 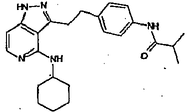 | Example 263 |
| 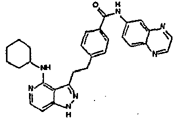 | Example 254 | 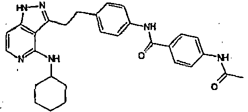 | Example 264 |
| 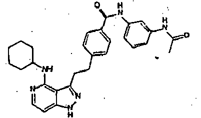 | Example 255 | 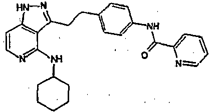 | Example 265 |
| 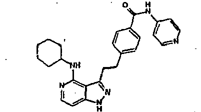 | Example 256 | 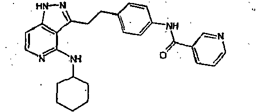 | Example 266 |
| 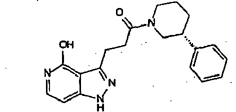 | Example 257 | 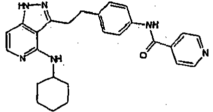 | Example 267 |
| 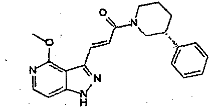 | Example 258 | 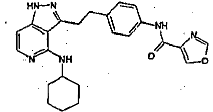 | Example 268 |
| 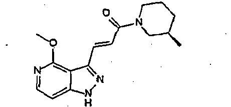 | Example 259 | 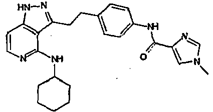 | Example 269 |
| 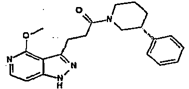 | Example 260 | | |
| 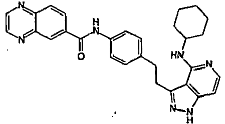 | Example 270 | 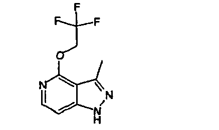 | Example 279 |
| 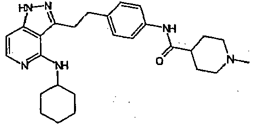 | Example 271 | 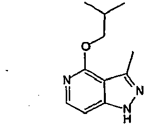 | Example 280 |
| 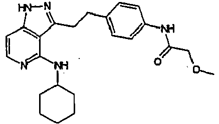 | Example 272 | 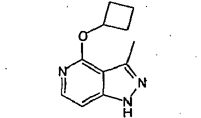 | Example 281 |
| 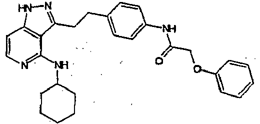 | Example 273 | 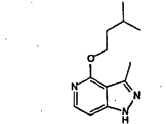 | Example 282 |
| 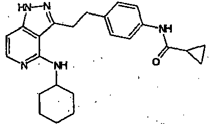 | Example 274 | 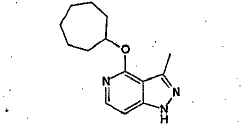 | Example 283 |
| 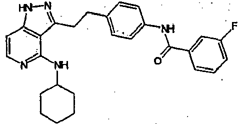 | Example 275 | 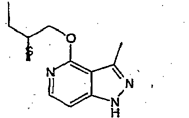 | Example 284 |
| 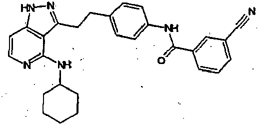 | Example 276 | 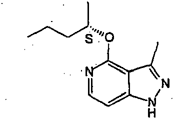 | Example 285 |
| 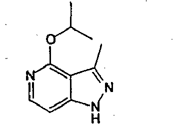 | Example 277 | 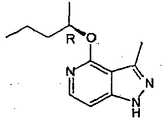 | Example 286 |
| 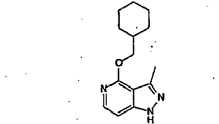 | Example 278 | 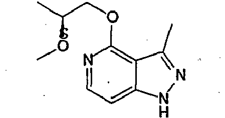 | Example 287 |
| 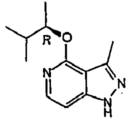 | Example 288 | 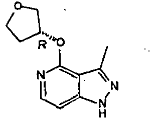 | Example 297 |
| 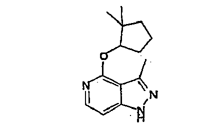 | Example 289 | 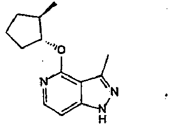 | Example 298 |
| 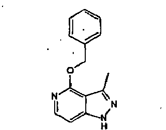 | Example 290 | 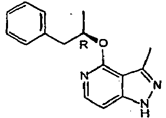 | Example 299 |
| 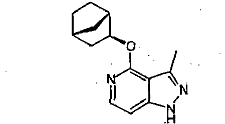 | Example 291 | 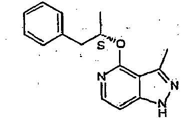 | Example 300 |
| 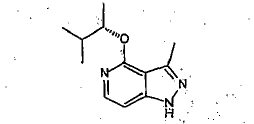 | Example 292 | 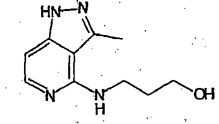 | Example 301 |
| 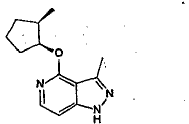 | Example 293 | 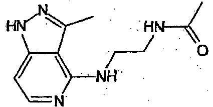 | Example 302 |
| 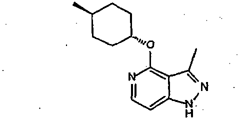 | Example 294 | 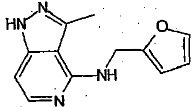 | Example 303 |
| 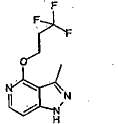 | Example 295 | 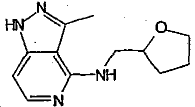 | Example 304 |
| 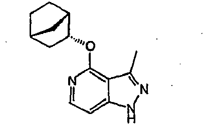 | Example 296 | 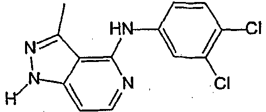 | Example 305 |
| 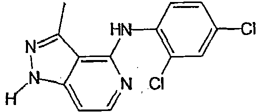 | Example 306 | 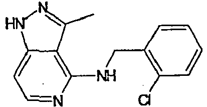 | Example 315 |
| 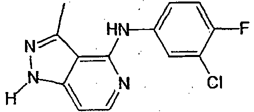 | Example 307 | 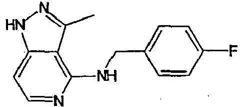 | Example 316 |
| 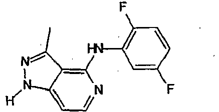 | Example 308 | 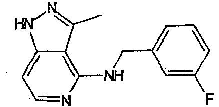 | Example 317 |
| 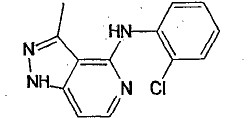 | Example 309 | 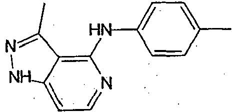 | Example 318 |
| 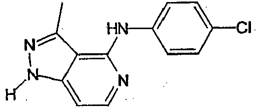 | Example 310 | 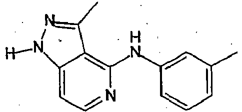 | Example 319 |
| 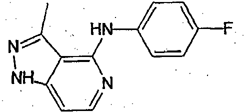 | Example 311 | 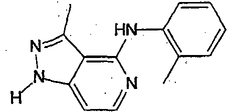 | Example 320 |
| 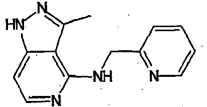 | Example 312 | 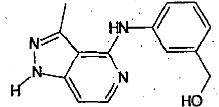 | Example 321 |
| 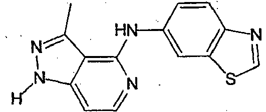 | Example 313 | 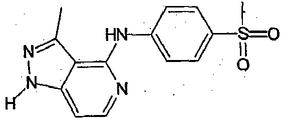 | Example 322 |
| 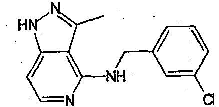 | Example 314 | 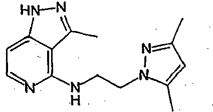 | Example 323 |
| 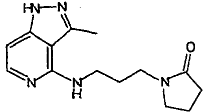 | Example 324 | 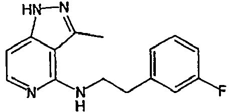 | Example 333 |
| 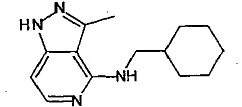 | Example 325 | 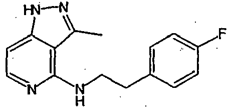 | Example 334 |
| 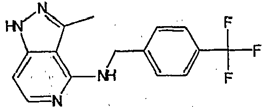 | Example 326 | 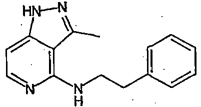 | Example 335 |
| 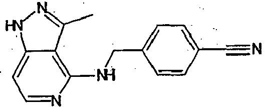 | Example 327 | 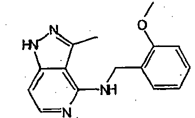 | Example 336 |
| 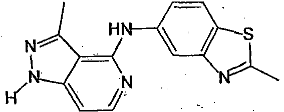 | Example 328 | 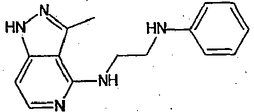 | Example 337 |
| 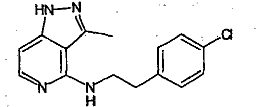 | Example 329 | 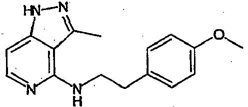 | Example 338 |
| 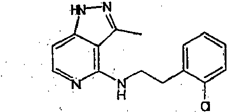 | Example 330 | 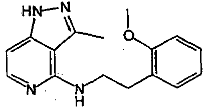 | Example 339 |
| 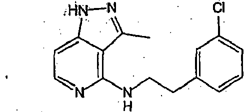 | Example 331 | 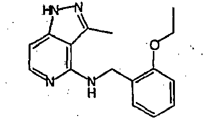 | Example 340 |
| 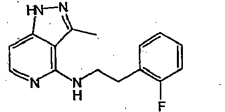 | Example 332 | 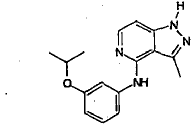 | Example 341 |
| 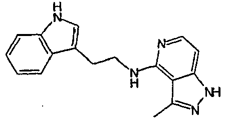 | Example 342 | 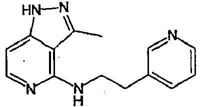 | Example 351 |
| 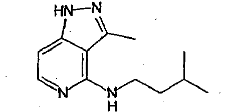 | Example 343 | 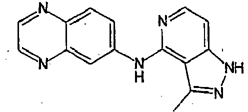 | Example 352 |
| 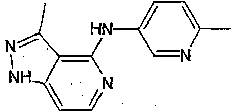 | Example 344 | 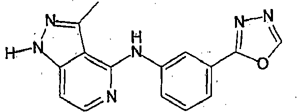 | Example 353 |
| 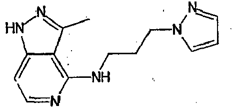 | Example 345 | 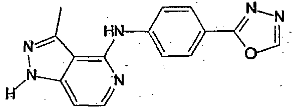 | Example 354 |
| 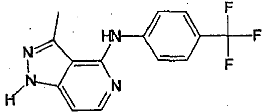 | Example 346 | 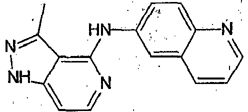 | Example 355 |
| 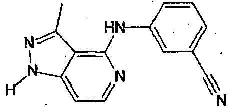 | Example 347 | 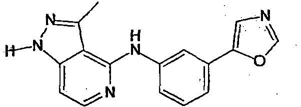 | Example 356 |
| 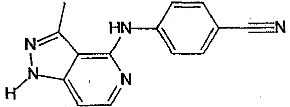 | Example 348 | 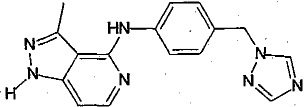 | Example 357 |
| 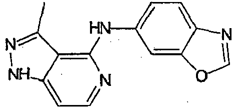 | Example 349 | 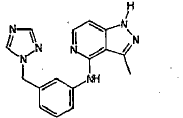 | Example 358 |
| 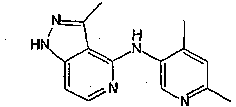 | Example 350 | 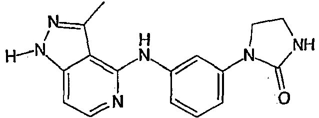 | Example 359 |
| 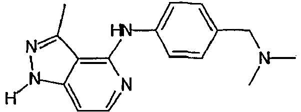 | Example 360 | 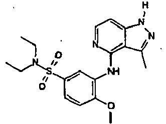 | Example 369 |
| 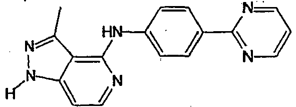 | Example 361 | 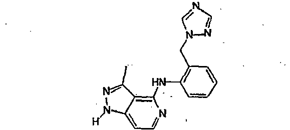 | Example 370 |
| 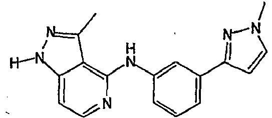 | Example 362 | 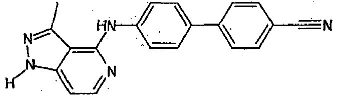 | Example 371 |
| 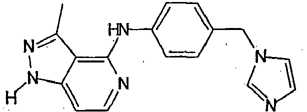 | Example 363 | 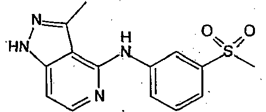 | Example 372 |
| 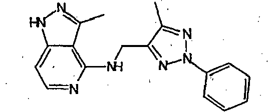 | Example 364 | 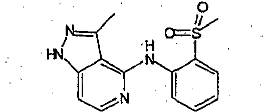 | Example 373 |
| 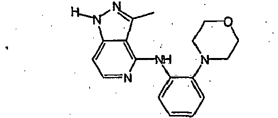 | Example 365 | 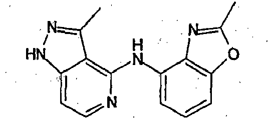 | Example 374 |
| 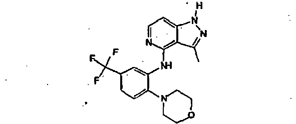 | Example 366 | 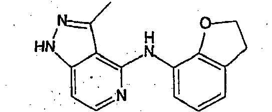 | Example 375 |
| 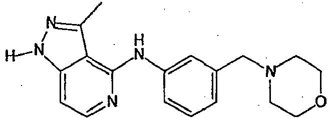 | Example 367 | 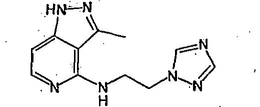 | Example 376 |
| 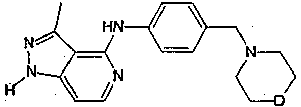 | Example 368 | 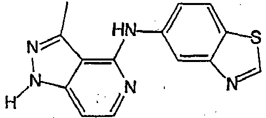 | Example 377 |
| 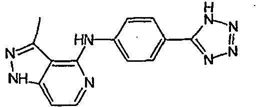 | Example 378 | 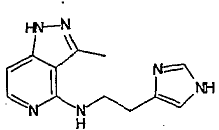 | Example 387 |
| 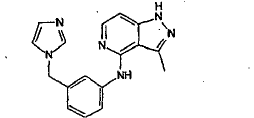 | Example 379 | 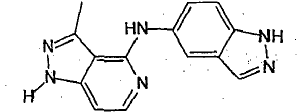 | Example 388 |
| 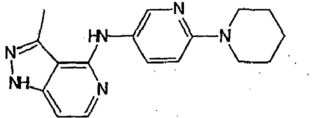 | Example 380 | 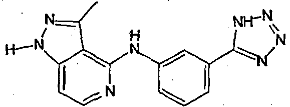 | Example 389 |
| 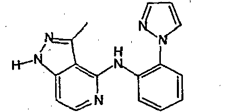 | Example 381 | 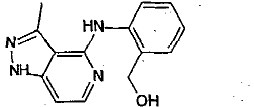 | Example 390 |
| 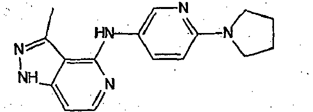 | Example 382 | 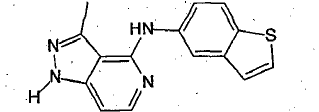 | Example 391 |
| 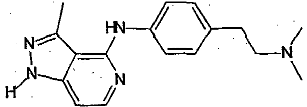 | Example 383 | 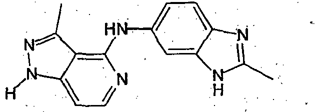 | Example 392 |
| 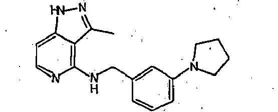 | Example 384 | | |
| 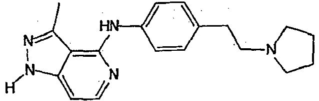 | Example 385 | | |
| 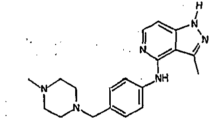 | Example 386 | | |

### THERAPEUTIC APPLICATIONS

A further aspect of the invention relates to a compound as described above for use in medicine.

Another aspect of the invention relates to a compound as described above for use in treating cancer or a neurodegenerative disorder.

Another aspect relates to the use of a compound as described above in the preparation of a medicament for treating or preventing a neurodegenerative disorder. Preferably, the neurodegenerative disorder is Parkinson's Disease.

Another aspect relates to the use of a compound as described above in the preparation of a medicament for treating or preventing a proliferative disorder, for example, cancer.

Preferably, the compound is administered in an amount sufficient to inhibit one or more kinases, preferably LRRK, even more preferably LRRK2.

Yet another aspect relates to the use of a compound of the invention in the preparation of a medicament for the prevention or treatment of a disorder caused by, associated with or accompanied by any abnormal activity against a biological target, wherein the target is a kinase, more preferably LRRK, even more preferably LRRK2.

Preferably, the disorder is Parkinson's Disease.

Preferably, the disease state is alleviated by the inhibition of the protein kinase LRRK, more preferably LRRK2.

Preferably, the mammal is a human.

The term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

The term "administering" as used herein refers to a method for bringing a compound of the present invention and a protein kinase together in such a manner that the compound can affect the enzyme activity of the protein kinase either directly; i.e., by interacting with the protein kinase itself or indirectly; i.e., by interacting with another molecule on which the catalytic activity of the protein kinase is dependent. As used herein, administration can be accomplished either *in vitro,* i.e. in a test tube, or *in vivo,* i.e., in cells or tissues of a living organism.

Herein, the term "treating" includes abrogating, substantially inhibiting, slowing or reversing the progression of a disease or disorder, substantially ameliorating clinical symptoms of a disease or disorder or substantially preventing the appearance of clinical symptoms of a disease or disorder.

Herein, the term "preventing" refers to a method for barring an organism from acquiring a disorder or disease in the first place.

The term "therapeutically effective amount" refers to that amount of the compound being administered which will relieve to some extent one or more of the symptoms of the disease or disorder being treated.

For any compound used in this invention, a therapeutically effective amount, also referred to herein as a therapeutically effective dose, can be estimated initially from cell culture assays. For example, a dose can be formulated in animal models to achieve a circulating concentration range that includes the IC₅₀ or the IC₁₀₀ as determined in cell culture. Such information can be used to more accurately determine useful doses in humans. Initial dosages can also be estimated from *in vivo* data. Using these initial guidelines one of ordinary skill in the art could determine an effective dosage in humans.

Moreover, toxicity and therapeutic efficacy of the compounds described herein can be determined by standard pharmaceutical procedures in cell cultures or experimental animals, e.g., by determining the LD₅₀ and the ED₅₀. The dose ratio between toxic and therapeutic effect is the therapeutic index and can be expressed as the ratio between LD₅₀ and ED₅₀. Compounds which exhibit high therapeutic indices are preferred. The data obtained from these cell cultures assays and animal studies can be used in formulating a dosage range that is not toxic for use in human. The dosage of such compounds lies preferably within a range of circulating concentrations that include the ED₅₀ with little or no toxicity. The dosage may vary within this range depending upon the dosage form employed and the route of administration utilized. The exact formulation, route of administration and dosage can be chosen by the individual physician in view of the patient's conditions. (see, e.g., Fingl et al, 1975, In: The Pharmacological Basis of Therapeutics, chapter 1, page 1).

Dosage amount and interval may be adjusted individually to provide plasma levels of the active compound which are sufficient to maintain therapeutic effect. Usual patient dosages for oral administration range from about 50-2000 mg/kg/day, commonly from about 100-1000 mg/kg/day, preferably from about 150-700 mg/kg/day and most preferably from about 250-500 mg/kg/day. Preferably, therapeutically effective serum levels will be achieved by administering multiple doses each day. In cases of local administration or selective uptake, the effective local concentration of the drug may not be related to plasma concentration. One skilled in the art will be able to optimize therapeutically effective local dosages without undue experimentation.

As used herein, "kinase related disease or disorder" refers to a disease or disorder characterized by inappropriate kinase activity or over-activity of a kinase as defined herein. Inappropriate activity refers to either; (i) kinase expression in cells which normally do not express said kinase; (ii) increased kinase expression leading to unwanted cell proliferation, differentiation and/or growth; or, (iii) decreased kinase expression leading to unwanted reductions in cell proliferation, differentiation and/or growth. Over-activity of kinase refers to either amplification of the gene encoding a particular kinase or production of a level of kinase activity, which can correlate with a cell proliferation, differentiation and/or growth disorder (that is, as the level of the kinase increases, the severity of one or more of the symptoms of the cellular disorder increases). Over activity can also be the result of ligand independent or constitutive activation as a result of mutations such as deletions of a fragment of a kinase responsible for ligand binding.

Preferred diseases or disorders that the compounds described herein may be useful in preventing, include cancer and neurodegenerative disorders such as Parkinson's Disease.

Thus, the present invention further provides use of compounds as defined herein for the manufacture of medicaments for the treatment of diseases where it is desirable to inhibit LRRK2. Such diseases include Parkinson's Disease.

### PHARMACEUTICAL COMPOSTIONS

For use according to the present invention, the compounds or physiologically acceptable salts thereof, described herein, may be presented as a pharmaceutical formulation, comprising the compounds or physiologically acceptable salt thereof, together with one or more pharmaceutically acceptable carriers therefore and optionally other therapeutic and/or prophylactic ingredients. The carrier(s) must be acceptable in the sense of being compatible with the other ingredients of the formulation and not deleterious to the recipient thereof. The pharmaceutical compositions may be for human or animal usage in human and veterinary medicine.

Examples of such suitable excipients for the various different forms of pharmaceutical compositions described herein may be found in the "Handbook of Pharmaceutical Excipients, 2nd Edition, (1994), Edited by A Wade and PJ Weller.

Acceptable carriers or diluents for therapeutic use are well known in the pharmaceutical art, and are described, for example, in Remington's Pharmaceutical Sciences, Mack Publishing Co. (A. R. Gennaro edit. 1985).

Examples of suitable carriers include lactose, starch, glucose, methyl cellulose, magnesium stearate, mannitol, sorbitol and the like. Examples of suitable diluents include ethanol, glycerol and water.

The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s), buffer(s), flavouring agent(s), surface active agent(s), thickener(s), preservative(s) (including anti-oxidants) and the like, and substances included for the purpose of rendering the formulation isotonic with the blood of the intended recipient.

Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol.

Examples of suitable lubricants include sodium oleate, sodium stearate, magnesium stearate, sodium benzoate, sodium acetate, sodium chloride and the like.

Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may be also used.

Pharmaceutical formulations include those suitable for oral, topical (including dermal, buccal and sublingual), rectal or parenteral (including subcutaneous, intradermal, intramuscular and intravenous), nasal and pulmonary administration e.g., by inhalation. The formulation may, where appropriate, be conveniently presented in discrete dosage units and may be prepared by any of the methods well known in the art of pharmacy. All methods include the step of bringing into association an active compound with liquid carriers or finely divided solid carriers or both and then, if necessary, shaping the product into the desired formulation.

Pharmaceutical formulations suitable for oral administration wherein the carrier is a solid are most preferably presented as unit dose formulations such as boluses, capsules or tablets each containing a predetermined amount of active compound. A tablet may be made by compression or moulding, optionally with one or more accessory ingredients. Compressed tablets may be prepared by compressing in a suitable machine an active compound in a free-flowing form such as a powder or granules optionally mixed with a binder, lubricant, inert diluent, lubricating agent, surface-active agent or dispersing agent. Moulded tablets may be made by moulding an active compound with an inert liquid diluent. Tablets may be optionally coated and, if uncoated, may optionally be scored. Capsules may be prepared by filling an active compound, either alone or in admixture with one or more accessory ingredients, into the capsule shells and then sealing them in the usual manner. Cachets are analogous to capsules wherein an active compound together with any accessory ingredient(s) is sealed in a rice paper envelope. An active compound may also be formulated as dispersible granules, which may for example be suspended in water before administration, or sprinkled on food. The granules may be packaged, e.g., in a sachet. Formulations suitable for oral administration wherein the carrier is a liquid may be presented as a solution or a suspension in an aqueous or non-aqueous liquid, or as an oil-in-water liquid emulsion.

Formulations for oral administration include controlled release dosage forms, e.g., tablets wherein an active compound is formulated in an appropriate release - controlling matrix, or is coated with a suitable release - controlling film. Such formulations may be particularly convenient for prophylactic use.

Pharmaceutical formulations suitable for rectal administration wherein the carrier is a solid are most preferably presented as unit dose suppositories. Suitable carriers include cocoa butter and other materials commonly used in the art. The suppositories may be conveniently formed by admixture of an active compound with the softened or melted carrier(s) followed by chilling and shaping in moulds. Pharmaceutical formulations suitable for parenteral administration include sterile solutions or suspensions of an active compound in aqueous or oleaginous vehicles.

Injectable preparations may be adapted for bolus injection or continuous infusion. Such preparations are conveniently presented in unit dose or multi-dose containers which are sealed after introduction of the formulation until required for use. Alternatively, an active compound may be in powder form which is constituted with a suitable vehicle, such as sterile, pyrogen-free water, before use.

An active compound may also be formulated as long-acting depot preparations, which may be administered by intramuscular injection or by implantation, e.g., subcutaneously or intramuscularly. Depot preparations may include, for example, suitable polymeric or hydrophobic materials, or ion-exchange resins. Such long-acting formulations are particularly convenient for prophylactic use.

Formulations suitable for pulmonary administration via the buccal cavity are presented such that particles containing an active compound and desirably having a diameter in the range of 0.5 to 7 microns are delivered in the bronchial tree of the recipient.

As one possibility such formulations are in the form of finely comminuted powders which may conveniently be presented either in a pierceable capsule, suitably of, for example, gelatin, for use in an inhalation device, or alternatively as a self-propelling formulation comprising an active compound, a suitable liquid or gaseous propellant and optionally other ingredients such as a surfactant and/or a solid diluent. Suitable liquid propellants include propane and the chlorofluorocarbons, and suitable gaseous propellants include carbon dioxide. Self-propelling formulations may also be employed wherein an active compound is dispensed in the form of droplets of solution or suspension.

Such self-propelling formulations are analogous to those known in the art and may be prepared by established procedures. Suitably they are presented in a container provided with either a manually-operable or automatically functioning valve having the desired spray characteristics; advantageously the valve is of a metered type delivering a fixed volume, for example, 25 to 100 microlitres, upon each operation thereof.

As a further possibility an active compound may be in the form of a solution or suspension for use in an atomizer or nebuliser whereby an accelerated airstream or ultrasonic agitation is employed to produce a fine droplet mist for inhalation.

Formulations suitable for nasal administration include preparations generally similar to those described above for pulmonary administration. When dispensed such formulations should desirably have a particle diameter in the range 10 to 200 microns to enable retention in the nasal cavity; this may be achieved by, as appropriate, use of a powder of a suitable particle size or choice of an appropriate valve. Other suitable formulations include coarse powders having a particle diameter in the range 20 to 500 microns, for administration by rapid inhalation through the nasal passage from a container held close up to the nose, and nasal drops comprising 0.2 to 5% w/v of an active compound in aqueous or oily solution or suspension.

Pharmaceutically acceptable carriers are well known to those skilled in the art and include, but are not limited to, 0.1 M and preferably 0.05 M phosphate buffer or 0.8% saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Preservatives and other additives may also be present, such as, for example, antimicrobials, antioxidants, chelating agents, inert gases and the like.

Formulations suitable for topical formulation may be provided for example as gels, creams or ointments. Such preparations may be applied e.g. to a wound or ulcer either directly spread upon the surface of the wound or ulcer or carried on a suitable support such as a bandage, gauze, mesh or the like which may be applied to and over the area to be treated.

Liquid or powder formulations may also be provided which can be sprayed or sprinkled directly onto the site to be treated, e.g. a wound or ulcer. Alternatively, a carrier such as a bandage, gauze, mesh or the like can be sprayed or sprinkle with the formulation and then applied to the site to be treated.

According to a further aspect of the invention, there is provided a process for the preparation of a pharmaceutical or veterinary composition as described above, the process comprising bringing the active compound(s) into association with the carrier, for example by admixture.

In general, the formulations are prepared by uniformly and intimately bringing into association the active agent with liquid carriers or finely divided solid carriers or both, and then if necessary shaping the product. The invention extends to methods for preparing a pharmaceutical composition comprising bringing a compound of general formula (I) in conjunction or association with a pharmaceutically or veterinarily acceptable carrier or vehicle.

### SALTS

The compounds of the invention can be present as salts, in particular pharmaceutically and veterinarily acceptable salts.

Pharmaceutically acceptable salts of the compounds of the invention include suitable acid addition or base salts thereof. A review of suitable pharmaceutical salts may be found in Berge et al, J Pharm Sci, 66, 1-19 (1977). Salts are formed, for example with strong inorganic acids such as mineral acids, e.g. hydrohalic acids such as hydrochloride, hydrobromide and hydroiodide, sulphuric acid, phosphoric acid sulphate, bisulphate, hemisulphate, thiocyanate, persulphate and sulphonic acids; with strong organic carboxylic acids, such as alkanecarboxylic acids of 1 to 4 carbon atoms which are unsubstituted or substituted (e.g., by halogen), such as acetic acid; with saturated or unsaturated dicarboxylic acids, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C₁-C₄)-alkyl- or aryl-sulfonic acids which are unsubstituted or substituted (for example, by a halogen) such as methane- or p-toluene sulfonic acid. Salts which are not pharmaceutically or veterinarily acceptable may still be valuable as intermediates.

Preferred salts include, for example, acetate, trifluoroacetate, lactate, gluconate, citrate, tartrate, maleate, malate, pantothenate, adipate, alginate, aspartate, benzoate, butyrate, digluconate, cyclopentanate, glucoheptanate, glycerophosphate, oxalate, heptanoate, hexanoate, fumarate, nicotinate, palmoate, pectinate, 3-phenylpropionate, picrate, pivalate, proprionate, tartrate, lactobionate, pivolate, camphorate, undecanoate and succinate, organic sulphonic acids such as methanesulphonate, ethanesulphonate, 2-hydroxyethane sulphonate, camphorsulphonate, 2-naphthalenesulphonate, benzenesulphonate, p-chlorobenzenesulphonate and p-toluenesulphonate; and inorganic acids such as hydrochloride, hydrobromide, hydroiodide, sulphate, bisulphate, hemisulphate, thiocyanate, persulphate, phosphoric and sulphonic acids.

### ENANTIOMERS/TAUTOMERS

In all aspects of the present invention previously discussed, the invention includes, where appropriate all enantiomers, diastereoisomers and tautomers of the compounds of the invention. The person skilled in the art will recognise compounds that possess optical properties (one or more chiral carbon atoms) or tautomeric characteristics. The corresponding enantiomers and/or tautomers may be isolated/prepared by methods known in the art.

Enantiomers are characterised by the absolute configuration of their chiral centres and described by the *R*- and *S*-sequencing rules of Cahn, Ingold and Prelog. Such conventions are well known in the art (e.g. see 'Advanced Organic Chemistry', 3rd edition, ed. March, J., John Wiley and Sons, New York, 1985).

Compounds of the invention containing a chiral centre may be used as a racemic mixture, an enantiomerically enriched mixture, or the racemic mixture may be separated using well-known techniques and an individual enantiomer may be used alone.

### STEREO AND GEOMETRIC ISOMERS

Some of the compounds of the invention may exist as stereoisomers and/or geometric isomers - e.g. they may possess one or more asymmetric and/or geometric centres and so may exist in two or more stereoisomeric and/or geometric forms. The present invention contemplates the use of all the individual stereoisomers and geometric isomers of those inhibitor agents, and mixtures thereof. The terms used in the claims encompass these forms, provided said forms retain the appropriate functional activity (though not necessarily to the same degree).

The present invention also includes all suitable isotopic variations of the agent or a pharmaceutically acceptable salt thereof. An isotopic variation of an agent of the present invention or a pharmaceutically acceptable salt thereof is defined as one in which at least one atom is replaced by an atom having the same atomic number but an atomic mass different from the atomic mass usually found in nature. Examples of isotopes that can be incorporated into the agent and pharmaceutically acceptable salts thereof include isotopes of hydrogen, carbon, nitrogen, oxygen, phosphorus, sulphur, fluorine and chlorine such as ²H, ³H, ¹³C, ¹⁴C, ¹⁵N, ¹⁷O, ¹⁸O, ³¹P, ³²P, ³⁵S, ¹⁸F and ³⁶Cl, respectively. Certain isotopic variations of the agent and pharmaceutically acceptable salts thereof, for example, those in which a radioactive isotope such as ³H or ¹⁴C is incorporated, are useful in drug and/or substrate tissue distribution studies. Tritiated, i.e., ³H, and carbon-14, i.e., ¹⁴C, isotopes are particularly preferred for their ease of preparation and detectability. Further, substitution with isotopes such as deuterium, i.e., ²H, may afford certain therapeutic advantages resulting from greater metabolic stability, for example, increased *in vivo* half-life or reduced dosage requirements and hence may be preferred in some circumstances. For example, the invention includes compounds of general formula (I) where any hydrogen atom has been replaced by a deuterium atom. Isotopic variations of the agent of the present invention and pharmaceutically acceptable salts thereof of this invention can generally be prepared by conventional procedures using appropriate isotopic variations of suitable reagents.

### SOLVATES

The present invention also includes solvate forms of the compounds of the present invention. The terms used in the claims encompass these forms.

### POLYMORPHS

The invention further relates to the compounds of the present invention in their various crystalline forms, polymorphic forms and (an)hydrous forms. It is well established within the pharmaceutical industry that chemical compounds may be isolated in any of such forms by slightly varying the method of purification and or isolation form the solvents used in the synthetic preparation of such compounds.

### ADMINISTRATION

The pharmaceutical compositions of the present invention may be adapted for rectal, nasal, intrabronchial, topical (including buccal and sublingual), vaginal or parenteral (including subcutaneous, intramuscular, intravenous, intraarterial and intradermal), intraperitoneal or intrathecal administration. Preferably the formulation is an orally administered formulation. The formulations may conveniently be presented in unit dosage form, i.e., in the form of discrete portions containing a unit dose, or a multiple or sub-unit of a unit dose. By way of example, the formulations may be in the form of tablets and sustained release capsules, and may be prepared by any method well known in the art of pharmacy.

Formulations for oral administration in the present invention may be presented as: discrete units such as capsules, gellules, drops, cachets, pills or tablets each containing a predetermined amount of the active agent; as a powder or granules; as a solution, emulsion or a suspension of the active agent in an aqueous liquid or a non-aqueous liquid; or as an oil-in-water liquid emulsion or a water-in-oil liquid emulsion; or as a bolus etc. Preferably, these compositions contain from 1 to 250 mg and more preferably from 10-100 mg, of active ingredient per dose.

For compositions for oral administration (e.g. tablets and capsules), the term "acceptable carrier" includes vehicles such as common excipients e.g. binding agents, for example syrup, acacia, gelatin, sorbitol, tragacanth, polyvinylpyrrolidone (Povidone), methylcellulose, ethylcellulose, sodium carboxymethylcellulose, hydroxypropyl-methylcellulose, sucrose and starch; fillers and carriers, for example corn starch, gelatin, lactose, sucrose, microcrystalline cellulose, kaolin, mannitol, dicalcium phosphate, sodium chloride and alginic acid; and lubricants such as magnesium stearate, sodium stearate and other metallic stearates, glycerol stearate stearic acid, silicone fluid, talc waxes, oils and colloidal silica. Flavouring agents such as peppermint, oil of wintergreen, cherry flavouring and the like can also be used. It may be desirable to add a colouring agent to make the dosage form readily identifiable. Tablets may also be coated by methods well known in the art.

A tablet maybe made by compression or moulding, optionally with one or more accessory ingredients. Compressed tablets may be prepared by compressing in a suitable machine the active agent in a free flowing form such as a powder or granules, optionally mixed with a binder, lubricant, inert diluent, preservative, surface-active or dispersing agent. Moulded tablets may be made by moulding in a suitable machine a mixture of the powdered compound moistened with an inert liquid diluent. The tablets may be optionally be coated or scored and may be formulated so as to provide slow or controlled release of the active agent.

Other formulations suitable for oral administration include lozenges comprising the active agent in a flavoured base, usually sucrose and acacia or tragacanth; pastilles comprising the active agent in an inert base such as gelatin and glycerin, or sucrose and acacia; and mouthwashes comprising the active agent in a suitable liquid carrier.

Other forms of administration comprise solutions or emulsions which may be injected intravenously, intraarterially, intrathecally, subcutaneously, intradermally, intraperitoneally or intramuscularly, and which are prepared from sterile or sterilisable solutions. Injectable forms typically contain between 10 - 1000 mg, preferably between 10 - 250 mg, of active ingredient per dose.

The pharmaceutical compositions of the present invention may also be in form of suppositories, pessaries, suspensions, emulsions, lotions, ointments, creams, gels, sprays, solutions or dusting powders.

An alternative means of transdermal administration is by use of a skin patch. For example, the active ingredient can be incorporated into a cream consisting of an aqueous emulsion of polyethylene glycols or liquid paraffin. The active ingredient can also be incorporated, at a concentration of between 1 and 10% by weight, into an ointment consisting of a white wax or white soft paraffin base together with such stabilisers and preservatives as may be required.

### DOSAGE

A person of ordinary skill in the art can easily determine an appropriate dose of one of the instant compositions to administer to a subject without undue experimentation. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. The dosages disclosed herein are exemplary of the average case. There can of course be individual instances where higher or lower dosage ranges are merited, and such are within the scope of this invention.

In accordance with this invention, an effective amount of a compound of general formula (I) may be administered to inhibit the kinase implicated with a particular condition or disease. Of course, this dosage amount will further be modified according to the type of administration of the compound. For example, to achieve an "effective amount" for acute therapy, parenteral administration of a compound of general formula (I) is preferred. An intravenous infusion of the compound in 5% dextrose in water or normal saline, or a similar formulation with suitable excipients, is most effective, although an intramuscular bolus injection is also useful. Typically, the parenteral dose will be about 0.01 to about 100 mg/kg; preferably between 0.1 and 20 mg/kg, in a manner to maintain the concentration of drug in the plasma at a concentration effective to inhibit a kinase. The compounds may be administered one to four times daily at a level to achieve a total daily dose of about 0.4 to about 400 mg/kg/day. The precise amount of an inventive compound which is therapeutically effective, and the route by which such compound is best administered, is readily determined by one of ordinary skill in the art by comparing the blood level of the agent to the concentration required to have a therapeutic effect.

The compounds of this invention may also be administered orally to the patient, in a manner such that the concentration of drug is sufficient to achieve one or more of the therapeutic indications disclosed herein. Typically, a pharmaceutical composition containing the compound is administered at an oral dose of between about 0.1 to about 50 mg/kg in a manner consistent with the condition of the patient. Preferably the oral dose would be about 0.5 to about 20 mg/kg.

No unacceptable toxicological effects are expected when compounds of the present invention are administered in accordance with the present invention. The compounds of this invention, which may have good bioavailability, may be tested in one of several biological assays to determine the concentration of a compound which is required to have a given pharmacological effect.

### COMBINATIONS

In a particularly preferred embodiment, the one or more compounds of the invention are administered in combination with one or more other active agents, for example, existing drugs available on the market. In such cases, the compounds of the invention may be administered consecutively, simultaneously or sequentially with the one or more other active agents.

Drugs in general are more effective when used in combination. In particular, combination therapy is desirable in order to avoid an overlap of major toxicities, mechanism of action and resistance mechanism(s). Furthermore, it is also desirable to administer most drugs at their maximum tolerated doses with minimum time intervals between such doses. The major advantages of combining chemotherapeutic drugs are that it may promote additive or possible synergistic effects through biochemical interactions and also may decrease the emergence of resistance.

Beneficial combinations may be suggested by studying the inhibitory activity of the test compounds with agents known or suspected of being valuable in the treatment of a particular disorder. This procedure can also be used to determine the order of administration of the agents, i.e. before, simultaneously, or after delivery. Such scheduling may be a feature of all the active agents identified herein.

### ASSAY

A further aspect of the invention relates to the use of a compound as described above in an assay for identifying further candidate compounds capable of inhibiting one or more kinases, more preferably LRRK, even more preferably, LRRK2.

Preferably, the assay is a competitive binding assay.

More preferably, the competitive binding assay comprises contacting a compound of the invention with a kinase, preferably LRRK, more preferably LRRK2, and a candidate compound and detecting any change in the interaction between the compound according to the invention and the kinase.

Preferably, the candidate compound is generated by conventional SAR modification of a compound of the invention.

As used herein, the term "conventional SAR modification" refers to standard methods known in the art for varying a given compound by way of chemical derivatisation.

Thus, in one aspect, the identified compound may act as a model (for example, a template) for the development of other compounds. The compounds employed in such a test may be free in solution, affixed to a solid support, borne on a cell surface, or located intracellularly. The abolition of activity or the formation of binding complexes between the compound and the agent being tested may be measured.

The assay of the present invention may be a screen, whereby a number of agents are tested. In one aspect, the assay method of the present invention is a high through-put screen.

This invention also contemplates the use of competitive drug screening assays in which neutralising antibodies capable of binding a compound specifically compete with a test compound for binding to a compound.

Another technique for screening provides for high throughput screening (HTS) of agents having suitable binding affinity to the substances and is based upon the method described in detail in WO 84/03564.

It is expected that the assay methods of the present invention will be suitable for both small and large-scale screening of test compounds as well as in quantitative assays.

Preferably, the competitive binding assay comprises contacting a compound of the invention with a kinase in the presence of a known substrate of said kinase and detecting any change in the interaction between said kinase and said known substrate.

A further aspect of the invention provides a method of detecting the binding of a ligand to a kinase, said method comprising the steps of:
(i) contacting a ligand with a kinase in the presence of a known substrate of said kinase;
(ii) detecting any change in the interaction between said kinase and said known substrate;
and wherein said ligand is a compound of the invention.

One aspect of the invention relates to a process comprising the steps of:
(a) performing an assay method described hereinabove;
(b) identifying one or more ligands capable of binding to a ligand binding domain; and
(c) preparing a quantity of said one or more ligands.

Another aspect of the invention provides a process comprising the steps of:
(a) performing an assay method described hereinabove;
(b) identifying one or more ligands capable of binding to a ligand binding domain; and
(c) preparing a pharmaceutical composition comprising said one or more ligands.

Another aspect of the invention provides a process comprising the steps of:
(a) performing an assay method described hereinabove;
(b) identifying one or more ligands capable of binding to a ligand binding domain;
(c) modifying said one or more ligands capable of binding to a ligand binding domain;
(d) performing the assay method described hereinabove;
(e) optionally preparing a pharmaceutical composition comprising said one or more ligands.

The invention also relates to a ligand identified by the method described hereinabove.

Yet another aspect of the invention relates to a pharmaceutical composition comprising a ligand identified by the method described hereinabove.

Another aspect of the invention relates to the use of a ligand identified by the method described hereinabove in the preparation of a pharmaceutical composition for use in the treatment of one or more disorders [insert list of disorders].

The above methods may be used to screen for a ligand useful as an inhibitor of one or more kinases.

Compounds of general formula (I) are useful both as laboratory tools and as therapeutic agents. In the laboratory certain compounds of the invention are useful in establishing whether a known or newly discovered kinase contributes a critical or at least significant biochemical function during the establishment or progression of a disease state, a process commonly referred to as 'target validation'.

### SYNTHESIS

Another aspect of the invention relates to a process for preparing compounds of formula I.

More specifically, the invention provides a process for preparing a compound of formula I as defined above, said process comprising converting a compound of formula II into a compound of formula I:

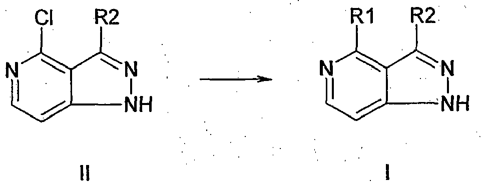

In one preferred embodiment of the invention, the process further comprises the step of preparing said compound of formula II by treating a compound of formula III with hydrazine monohydrate:

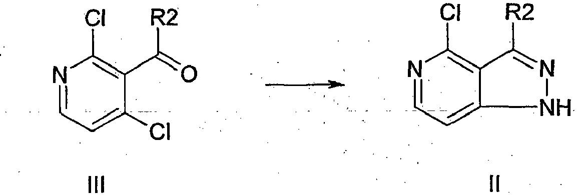

In one preferred embodiment of the invention, the process further comprises the step of preparing said compound of formula III by treating a compound of formula IV with an oxidizing agent:

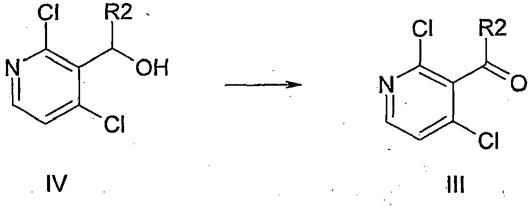

In one preferred embodiment of the invention, the process further comprises the step of preparing said compound of formula IV by treating a compound of formula V with R² -Mg-Cl:

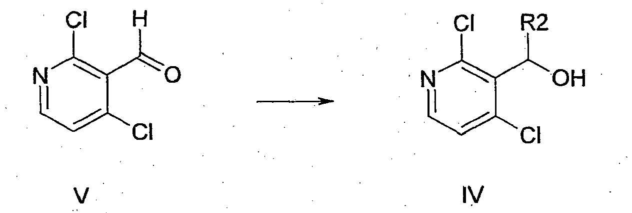

In one preferred embodiment of the invention, R¹ is -NHR³, and the process comprises reacting a compound of formula II with an amine of formula NH₂R³.

In another preferred embodiment of the invention, R¹ is an NH-containing C₄₋₇-heterocycloalkyl or an NH-containing fused aryl-C₄₋₇-heterocycloalkyl, and the process comprises reacting a compound of formula II with the NH-group of said C₄₋₇-heterocycloalkyl or fused aryl-C₄₋₇-heterocycloalkyl.

In another preferred embodiment of the invention, R¹ is selected from aryl, heteroaryl, C₄₋₇-heterocycloalkyl, fused aryl-C₄₋₇-heterocycloalkyl, -C₃₋₇ cycloalkyl and -C₁₋₆ alkyl, and said process comprises reacting a compound of formula II with X-R¹, where X is a 4,4,5,5-tetramethyl-1,3,2-dioxaborolan-2-yl group, in the presence of a coupling agent.

Preferably, the coupling agent is palladium diphenylphosphinoferrocene dichloride.

Another aspect of the invention relates to a process for preparing compounds of the invention, in accordance with the steps set forth below in Scheme 1.

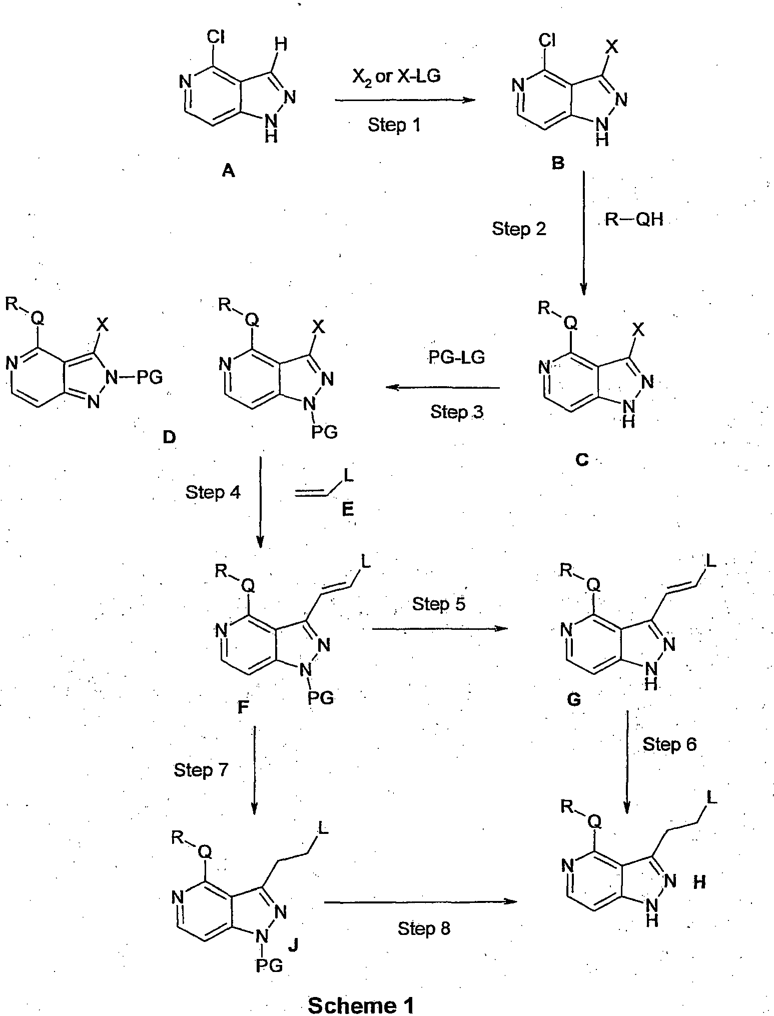

### Step 1

Step 1 describes the conversion of formula A into formula B, wherein X is a halogen, preferably bromine or iodine and LG is a leaving group such as succinimide.

The reaction is carried out in the presence of a suitable halogenating agent, such as iodine or N-bromosuccinimide, optionally in the presence of a base, such as potassium hydroxide in a suitable solvent.

Typical conditions (X=I), 1eq. of formula A, 2eq. of I₂, 3.7eq of KOH in dioxane at 75 °C for 4h; (X=Br), 1 eq. of formula A, 1eq. of N-bromosuccinimide in acetonitrile at reflux for 3h.

### Step 2

Step 2 describes the conversion of formula B into formula C, wherein X is a halogen, RQH can either be a primary or secondary amine, or an alcohol. The group R can optionally contain a functional group which can be manipulated at later stages in the synthetic process using standard conditions known to the skilled person.

The reaction involves nucleophlic displacement of the chloro group in formula B with a an amino group in a suitable solvent, optionally in the presence a Bronsted acid. This reaction generally requires heating, either thermally or with the use of microwave irradiation. Where RQH is an alcohol, the alcohol is deprotonated with a suitable base to the corresponding alkoxide followed by subsequent nucleophlic displacement of the chloro group in formula B.

Typical conditions (RQH = primary or secondary aliphatic amino group), 2.5eq. of amine, 1eq. of formula B in n-butanol, heated to 190 °C in the microwave for 20 min; (RQH = primary or secondary aromatic amino group), 2eq. of amine, 1eq of formula B, 3eq of conc. HCl(aq) in n-butanol, heated to 190 °C in the microwave for 45 min; (RQH = alcohol), 4eq. of alcohol is treated with 3.5 eq. of sodium hydride in dioxane at room temperature for 2h prior to addition of 1eq of formula B and subsequent heating in the microwave at 180 °C for 1.5h.

### Step 3

Step 3 describes the conversion of formula C into formula D, wherein PG is defined as a protecting group, including but not limited to *tert*-butoxycarbonyl-; benzyloxycarbonyl-; benzyl-; 4-methoxybenzyl-; 2,4-dimethoxybenzyl- or trityl-; LG is defined as a leaving group, such as a halogen or *tert*-butylcarbonate.

The reaction involves capping of the indazole NH with a protecting group. It will be appreciated by the skilled person, that that many protecting groups can be used for this purpose (see Greene, Theodora W. and Wuts, Peter G. M. Greene's Protective Groups in Organic Synthesis. 4th Ed. (2006)). The skilled person will also appreciate that it is possible to introduce the protecting group either at N1 or N2, and the ratio may change depending on the nature of PG or the precise reaction conditions deployed. The reaction conditions will depend on the nature of the protecting group.

Typical conditions (PG = 4-methoxybenzyl): 1eq of 4-methoxybenxyl chloride; 1eq of formula C, 2eq of potassium hydroxide is stirred in DMF at room temperature overnight.

### Step 4

Step 4 involves the conversion of formula D to formula F, wherein L is a group, such as but not limited to, aryl, substituted aryl, heteroaryl, substituted heteroaryl, an ester or an amide; X is a halogen, but preferably an iodine. The linker L can optionally contain a functional group which can be manipulated at later stages in the synthetic process using standard conditions known to the skilled person.

The reaction involves a cross coupling of a substituted vinyl derivative (formula E) with formula D in the presence of a suitable transition metal catalyst and a suitable base, preferably triethylamine and optionally additional additives, such as tetrabutyl ammonium iodide. This type of transformation is often known as a "Heck Reaction" to those skilled in the art.

Typical conditions: 1eq. of formula D, 10eq. of formula E, 2eq. of tetrabutylammonium iodide, 0.2eq. of Pd(dppf)Cl₂ in DMF;Water:triethylamine (6.25:1:1) is heated to 70 °C overnight.

### Step 5

Step 5 involves the conversion of formula F into formula G, wherein Q, PG, and L are as defined earlier.

The reaction involved removal of the protecting group from the indazole and the precise conditions will vary depending the nature of the protecting group (Greene, Theodora W. and Wuts, Peter G. M. Greene's Protective Groups in Organic Synthesis. 4th Ed. (2006). Typical conditions (QR is a substituted amino group and PG is 4-methoxybenzyl): Formula F is treated with trifluoroacetic acid at 70 °C overnight.

### Step 6

Step 6 involves the conversion of formula G into formula H, wherein PG, L, PG, RQ- are as defined earlier.

The reaction involves hydrogenation of the double bond to the corresponding saturated compound with a hydrogen source in the presence of a suitable transition metal catalyst in a suitable solvent. It may be necessary or desirable to add a Bronsted acid (such as HCl, or acetic acid) to facilitate this reaction. The person skilled in the art will appreciate that a number of different metal catalysts can be used for this type of reaction and that it may be necessary or desirable to carry out these reactions under pressure.

Typical conditions: formula G is treated with platinum oxide in glacial acetic acid under an atmosphere of hydrogen.

### Step 7

Step 7 involves the conversion of formula F into formula J, wherein PG, L, PG, RQ- are as defined earlier.

The reaction involves hydrogenation of the double bond to the corresponding saturated compound with a hydrogen source in the presence of a suitable transition metal catalyst, such as palladium on carbon or platinum oxide in a suitable solvent, such as ethanol, ethyl acetate or dioxane. It may be necessary or desirable to add a Bronsted acid (such as HCl, or acetic acid) to facilitate this reaction. The person skilled in the art will appreciate that a number of different metal catalysts can be used for this type of reaction and that it may be necessary or desirable to carry out these reactions under pressure.

Typical conditions: formula F is treated with 10% palladium on carbon in ethyl acetate under an atmosphere of hydrogen at room temperature overnight.

### Step 8

Step 8 involves the conversion of formula J to formula H, wherein PG, L, PG, RQ- are as defined earlier.

The reaction involves removal of the protecting group from the indazole, and the precise conditions will depend on the nature of the protecting group (Greene, Theodora W. and Wuts, Peter G. M. Greene's Protective Groups in Organic Synthesis. 4th Ed. (2006). Typical conditions (QR is a substituted amino group and PG is 4-methoxybenzyl): Formula F is treated with trifluoroacetic acid at 70 °C overnight.

### Step 9

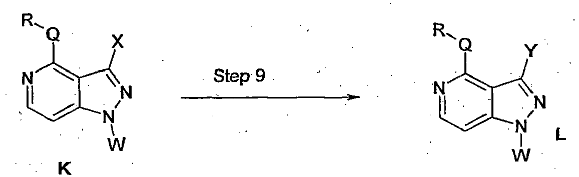

Step 9 describes the conversion of formula K into formula L wherein X and RQ are as defined previously, W can be either hydrogen or a protecting group, such as but not limited to 4-methoxybenzyl or trityl; Y can be aryl, substituted aryl, heteroaryl or substituted heteroaryl. The person skilled in the art will appreciate that where W is a protecting group, this can be removed at a later stage using standard conditions (Greene, Theodora W. and Wuts, Peter G. M. Greene's Protective Groups in Organic Synthesis. 4th Ed. (2006).

The reaction involves cross-coupling of the halide in formula K with a boronic acid or boronic ester in the presence of a transition metal catalyst in a suitable solvent. The reactions are typically carried out at elevated temperatures with either thermal or microwave heating. An inorganic base (such as sodium carbonate) is generally added to the reaction mixture. Transformations of this type are known as "Suzuki Couplings" to those skilled in the art.

Typical conditions: 1eq. of formula K, 0.09eq. of Pd(dppf)₂Cl₂, 1.5 eq. of the boronic acid (or boronic ester), 3.5eq. of 2M aqueous sodium carbonate in dioxane at 90 °C for 18h.

### Step 10-11

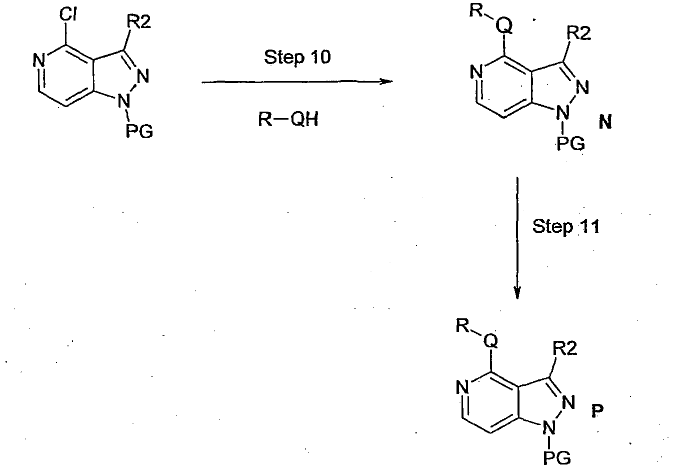

Step 10 describes the conversion of formula **M** into formula **N**, wherein R2 and RQH are as defined earlier and PG is a protecting group such as but not limited to 4-methoxybenzyl or trityl.

Where RQH is a primary or secondary amine, the reaction involves nucleophlic displacement of the chloro group in formula **M** with the amine. The reaction can be either carried out with or without solvent (such as but not limited to *n*-butanol or N-methylpyrrolidone), optionally in the presence of a Bronsted acid (such as but not limited to HCl) or an organic base (such as but not limited to *N,N*-diisopropylethylamine). This reaction generally requires heating, either thermally or with the use of microwave irradiation.

Alternatively, the reaction can be carried out by treatment of formula **M** with a primary or secondary amine in the presence or a transition metal catalyst, in the presence of a base in a suitable solvent.

Typical conditions: 1.4 equivalents of amine, 1 equivalent of formula **M**, 1 equivalent of cesium carbonate, 0.06 equivalents of palladium(II) acetate and 0.08 equivalents of 2,2'-bis(diphenylphosphino)-1,1'-binaphthyl (BINAP) is heated to 90 °C in 1,4-dioxane overnight.

Where RQH is an alcohol, the alcohol is deprotonated with a suitable base to the corresponding alkoxide followed by subsequent nucleophlic displacement of the chloro group in formula **M**. Alternatively, the reaction can be carried out by treatment of formula **M** with a primary or secondary alcohol in the presence or a transition metal catalyst, in the presence of a base in a suitable solvent.

Typical conditions (nucleophilic displacement): 2eq. of alcohol is treated with 1.5 eq. of sodium hydride in dioxane at room temperature for 3h prior to addition of 1eq of formula B and subsequent heating in the microwave at 180 °C for 1.5h.

Typical conditions (transition metal catalyzed): 2 equivalents of alcohol, 1 equivalent of formula **M**, 3 equivalents of sodium *tert*-butoxide, 0.06 equivalents of palladium(II) acetate and 0.08 equivalents of 2,2'-bis(diphenylphosphino)-1,1'-binaphthyl (BINAP) is heated to 100 °C in toluene overnight.

### Step 11

Step 8 involves the conversion of formula **N** to formula **P**, wherein R2, PG, RQ- are as defined earlier.

The reaction involves removal of the protecting group from the indazole, and the precise conditions will depend on the nature of the protecting group (Greene, Theodora W. and Wuts, Peter G. M. Greene's Protective Groups in Organic Synthesis. 4th Ed. (2006). Typical conditions (QR is a substituted amino group and PG is 4-methoxybenzyl): Formula **N** is treated with trifluoroacetic acid (neat) at 70 °C overnight.

Typical conditions (QR is an alkoxy group and PG is trityl): Formula **N** is treated with trifluoroacetic acid:DCM (1:10) for 18 h at room temperature.

The invention is further described by way of the following non-limiting examples, and with reference to the following figures, wherein:
Figure 1 shows the domain structure of LRRK1 and local mutations that have been linked to Parkinson's disease.

### EXAMPLES

### Materials and Methods

### Source and Purification of kinases

All LRRK2 protein kinases were of human origin and were sourced from Invitrogen Corporation (Carlsbad, CA 92008 USA) unless otherwise indicated. The active mutant used was recombinant human, catalytic domain (amino acids 970-2527) containing a G2019S mutation, GST-tagged, expressed in insect cells (Invitrogen Cat#PV4881). The wild type used was recombinant human, catalytic domain (amino acids 970-2527) GST - tagged, expressed in insect cells (Invitrogen Cat#PV4873). The kinase dead mutant used was recombinant human, catalytic domain (amino acids 970-2527) containing a D1994A mutation, GST-tagged, expressed in insect cells (Invitrogen Cat#PM4041AE). No special measures were taken to activate any of the kinases.

### Protein kinase assays

All assays were carried out at room temperature (∼21°C) and were linear with respect to time and enzyme concentration under the conditions used. Assays were performed for 180 min in a 96 well format. LRRK2 was present at a concentration of approximately 5nM. The enzyme was diluted and assayed in 50mM Tris-HCl pH7.5, 0.1 mM EGTA, 1mM DTT and 10mM MgCl₂. The concentration of magnesium chloride in the assay was 10mM. The [γ-33P] ATP (0.4µCi/well) was used at 134uM for G2019S mutant and at 57µM for the wild type kinase in order to be at Km. The peptide substrate in the assay was RLGWWRFYTLRRARQGNTKQR at 100µM.

The assays were initiated with Mg/ATP and stopped by the addition of 25µl/well 50% orthophosphoric acid. Reactions were harvested onto Whatman P81 Unifilter Plates (Fisher Scientific. Loughborough, LE115RG, UK. Cat# FDU-105-020U) using a Tomtec harvester. (Tomtec Hamden, Ct 06514. USA) Plates were counted using a Perkin Elmer Top Count NX7. (Perkin Elmer, Shelton CT 06484-4794 USA)

IC50 values of inhibitors were determined after carrying out assays at 10 different concentrations of each compound in duplicate.

### General procedures for synthesis of compounds

### Chromatography

Preparative high pressure liquid chromatography was carried out using apparatus made by Agilent. The apparatus is constructed such that the chromatography is monitored by a multi-wavelength UV detector (G1365B manufactured by Agilent) and an MM-ES+APCI mass spectrometer (G-1956A, manufactured by Agilent) connected in series, and if the appropriate criteria are met the sample is collected by an automated fraction collector (G1364B manufactured by Agilent). Collection can be triggered by any combination of UV or mass spectrometry or can be based on time. Typical conditions for the separation process are as follows: The gradient is run over a 10 minute period (gradient at start: 10% methanol and 90% water, gradient at finish: 100% methanol and 0% water; as buffer either 0.1% trifluoroacetic acid is added to the water (low pH buffer), or ammonium bicarbonate (10 mmol / l) and 35% ammonium hydroxide (1.6 ml / l) is added to the water (high pH buffer). It will be appreciated by those skilled in the art that it may be necessary or desirable to modify the conditions for each specific compound, for example by changing the solvent composition at the start or at the end, modifying the solvents or buffers, changing the run time, changing the flow rate and/or the chromatography column.

Flash chromatography refers to silica gel chromatography and carried out using an SP4 or an Isolara 4 MPLC system (manufactured by Biotage); pre-packed silica gel cartridges (supplied by Biotage); or using conventional glass column chromatography.

### Analytical Methods

¹H Nuclear magnetic resonance (NMR) spectroscopy was carried out using an ECX400 spectrometer (manufactured by JEOL) in the stated solvent at around room temperature unless otherwise stated. In all cases, NMR data were consistent with the proposed structures. Characteristic chemical shifts (δ) are given in parts-per-million using conventional abbreviations for designation of major peaks: e.g. s, singlet; d, doublet; t, triplet; q, quartet; dd, doublet of doublets; br, broad. Mass spectra were recorded using a MM-ES+APCI mass spectrometer (G-1956A, manufactured by Agilent). Where thin layer chromatography (TLC) has been used it refers to silica gel TLC using silica gel MK6F 60Å plates, R_{f} is the distance travelled by the compound divided by the distance travelled by the solvent on a TLC plate.

### Compound preparation

Where the preparation of starting materials is not described, these are commercially available, known in the literature, or readily obtainable by those skilled in the art using standard procedures. Where it is stated that compounds were prepared analogously to earlier examples or intermediates, it will be appreciated by the skilled person that the reaction time, number of equivalents of reagents and temperature can be modifed for each specific reaction and that it may be necessary or desirable to employ different work-up or purification techniques. Where reactions are carried out using microwave irradiation, the microwave used is an Initiator 60 supplied by Biotage. The actual power supplied varies during the course of the reaction in order to maintain a constant temperature.

### Abbreviations

- DCM: = Dichloromethane
- DMF: = N,N-Dimethylformamide
- THF: = Tetrahydrofuran
- MeOH: = Methanol
- TFA: = Trifluoroacetic acid
- Xantphos: = 4,5-Bis(diphenylphosphino)-9,9-dimethylxanthene
- HATU: =N,N,N',N'-Tetramethyl-O-(7-azabenzotriazol-1-yl)uronium-hexafluorophospate
- EDCl: = 1,3-Propanediamine, N3-(ethylcarbonimidoyl)-N1,N1-dimethyl-, hydrochloride
- DCC: = 1,3-Dicyclohexylcarbodiimide
- Pd₂(dba)₃: = tris(dibenzylideneacetone)dipalladium(0)
- TEA: = Triethylamine
- rm: = Reaction mixture
- rt: = Room temperature
- AcOH: = Acetic acid
- IPA: = Isopropanol
- DIPEA: = N,N-diisopropylethylamine
- TBSMSCI: = Tertiarybutyldimethylsilyl chloride
- MeCN: = Acetonitrile

- NH₃: = Ammonia
- EtOH: = Ethanol
- EtOAc: = Ethyl Acetate
- LCMS: = Mass spectrometry directed high pressure liquid chromatography
- UV: = Ultraviolet
- SCX: = Strong cation exchange
- TPAP: = Tetrapropylammonium perruthenate
- DMSO: = Dimethylsulphoxide
- BINAP: = 2,2'-bis(diphenylphosphino)-1,1'-binaphthyl

The structures of selected compounds of the invention are shown in the table below:

### Intermediate 1

### 1-(2,4-Dichloro-pyridin-3-yl)-ethanol

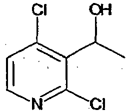

A solution of methylmagnesium chloride, 3M in THF (20.4 ml, 61.3 mmol) was added to 2,4-dichloro-pyridine-3-carbaldehyde (9.8 g, 55.7 mmol) in THF (200 ml) at -78°C. The reaction mixture was stirred at -78°C for 30 minutes and allowed to warm to rt. The mixture was quenched with saturated ammonium chloride solution (aq) and the product was extracted with EtOAc. The organic extract was washed with brine, dried and concentrated. The crude residue was purified by flash column chromatography over silica gel (300 g) eluting with 2:1 petroleum ether:EtOAc to provide a green coloured oil (7.8 g, 73%). ¹H NMR (400 MHz, CHLOROFORM-*d*) δ pom 1.67 (d, J=6.87 Hz, 3 H), 5.57 (q, *J*=6.87 Hz, 1 H), 7.29 (d, *J*=5.50 Hz, 1 H), 8.20 (d, *J*=5.50 Hz, 1 H).

### Intermediate 2

### 1-(2,4-Dichloro-pyridin-3-yl)-ethanone

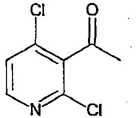

Freshly activated 4Å molecular sieves (9.0g) and NMO (7.1 g, 60.9 mmol) were added to a solution of Intermediate 1 (7.8 g, 40.6 mmol) in DCM (130 ml) and the mixture was stirred for 15 minutes. TPAP (403 mg, 1.15 mmol) was added and the reaction mixture was stirred for 2 hours at rt. The mixture was then filtered through Celite and the filtrate was concentrated. The crude residue was purified by flash column chromatography over silica gel (270 g) eluting with 4:1 petroleum ether:EtOAc to give a pale yellow coloured oil (6.2 g, 80%). ¹H NMR (400 MHz, CHLOROFORM-*d*) δ ppm 2.62 (s, 3 H) 7.34 (d, *J*=5.50 Hz, 1 H) 8.34 (d, *J*=5.50 Hz, 1 H).

### Intermediate 3

### 4-Chloro-3-methyl-1H-pyrazolo[4,3-c]pyridine

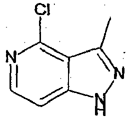

1-(2,4-Dichloro-pyridin-3-yl)-ethanone (6.2 g, 32.6 mmol) in 65% hydrazine monohydrate (45 ml) was stirred at rt overnight. The mixture was diluted with EtOAc and water. The organic extract was washed with brine, dried and concentrated to give a white solid. The crude product was purified by flash column chromatography over silica gel (200 g) eluting with 1:1 petroleum ether:EtOAc to give an off-white solid (3.5 g, 64%). ¹H NMR (400 MHz, DMSO-*d*₆) δ ppm 2.64 (s, 3 H) 7.47 (d, *J*=5.95 Hz, 1 H) 8.06 (d, *J*=5.95 Hz, 1 H). m/z (ES+APCl)⁺ : 168 / 170 [M+H]⁺.

### Intermediate 4

### 4-Chloro-1-(4-methoxy-benzyl)-3-methyl-1H-pyrazolo[4,3-c]pyridine

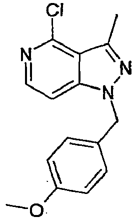

Intermediate 3 (1 g, 5.99 mmol), 4-methoxybenzylchloride (0.82 ml, 5.99 mmol) and potassium hydroxide (0.5 g, 8.98 mmol) were combined in DMF (20 ml) under nitrogen. The reaction was stirred at room temperature overnight. The reaction mixture was evaporated, the residue dissolved in EtOAc (20 ml) and partitioned with water (20 ml). The aqueous layer was extracted with EtOAc (20 ml) and then the combined organic layers were washed with brine, dried (MgSO₄) and evaporated. The crude product was purified by flash chromatography on the Biotage SP4, eluting with 0 to 60% EtOAc/petroleum ether to give a white solid (1.65 g, 96%). The product was isolated as a 4:1 mixture of N1 and N2 alkylated regioisomers:. Major regioisomer: ¹H NMR (400 MHz, DMSO-*d*₆) δ ppm 2.64 (s, 3 H), 3.70 (s, 3 H), 5.52 (s, 2 H), 6.87 (d, *J*=8.7 Hz, 2 H), 7.22 (d, *J*=8.7 Hz, 2 H), 7.77 (d, *J*=6.0 Hz, 1 H), 8.11 (d, *J*=6.0 Hz, 1 H). m/z (ES+APCl)⁺: 288 / 290. Minor regioisomer: ¹H NMR (400 MHz, DMSO-*d*₆) δ ppm 2.83 (s, 3 H), 3.71 (s, 3 H), 5.61 (s, 2 H), 6.90 (d, *J*=8.7 Hz, 2 H), 7.22 (d, *J*=8.7 Hz, 2 H), 7.49 (d, *J*=6.0 Hz, 1 H), 7.91 (d, *J*=6.4 Hz, 1 H); m/z (ES+APCI)⁺: 288 / 290.

### Intermediate 5

### 1-(4-Methoxy-benzyl)-3-methyl-1H-pyrazolo[4,3-c]pyridine-4-carbonitrile

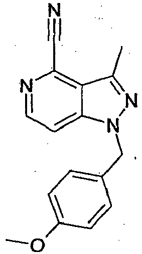

Intermediate 4 (200 mg, 0.70 mmol), zinc cyanide (81 mg, 0.70 mmol) and Pd(PPh₃)₄ (80 mg, 0.07 mmol) were combined in DMF (2.5 ml), degassed for 10 minutes and placed under an atmosphere of nitrogen. The reaction mixture was irradiated at 180 °C for 20 min in a Biotage I-60 microwave reactor. The reaction was diluted with EtOAc (5 ml) and partitioned with saturated NaHCO₃ aqueous solution (10 ml). The aqueous layer was then extracted with EtOAc (2x 20 ml). The combined organic layers were washed with brine, dried (MgSO₄) and evaporated. The crude product was purified by flash chromatography on the Biotage SP4, eluting with 0 to 60% EtOAc/petroleum ether to give a a white solid (148 mg, 76%). NMR data indicate a mixture of N-1 and N-2 regioisomers:
*Major product:* ¹H NMR (400 MHz, DMSO-*d*₆) δ ppm 2.70 (s, 3 H), 3.70 (s, 3 H), 5.59 (s; 2 H), 6.85 - 6.89 (m, 2 H), 7.22 - 7.26 (m, 2 H), 8.11 (d, *J*=6.0 Hz, 1 H), 8.52 (d, *J*=6.0 Hz, 1 H); m/z (ES+APCI)⁺: 279 [M+H]⁺.
*Minor product:* ¹H NMR (400 MHz, DMSO-*d*₆) δ ppm 2.88 (s, 3 H), 3.72 (s, 3 H), 5.68 (s, 2 H), 6.89 - 6.93 (m, 2 H), 7.24 - 7.27 (m, 2 H), 7.87 (d, *J*=6.0 Hz, 1 H), 8.32 (d, *J*=5.9 Hz, 1 H); m/z (ES+APCI)⁺: 279 [M+H]⁺.

### Intermediate 6

### 1-(4-Methoxy-benzyl)-3-methyl-1H-pyrazolo[4,3-c]pyridine-4-carboxylic acid

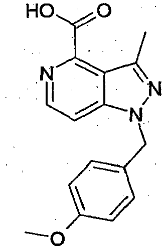

Potassium hydroxide (1.45 g, 25.9 mmol) was added to a solution of Intermediate 5 (720 mg, 2.51 mmol) in EtOH (20 ml) and H₂O (3 ml), and the mixture was refluxed for 18 h. The reaction was allowed to cool, then diluted with H₂O (100 ml) and adjusted to pH3 with concentrated HCl(aq). During extraction with EtOAc, a white solid crashed out of the aqueous phase, which was filtered and dried to give a white solid (217 mg, 28%). ¹H NMR (400 MHz, DMSO-*d*₆) δ ppm 2.61 (s, 3 H), 3.70 (s, 3 H), 5.57 (s, 2 H), 6.83 - 6.91 (m, 2 H), 7.18 - 7.25 (m, 2 H), 7.93 (d, *J*=6.0 Hz, 1 H), 8.34 (d, *J*=6.0 Hz, 1 H). m/z (ES+APCI)⁺: 298 [M+H]⁺.

### Intermediate 7

### 4-[1-(4-Methoxy-benzy/)-3-methy/-1H-pyrazolo[4,3-c]pyridin-4-ylamino]-piperidine-1-carboxylic acid tert-butyl ester

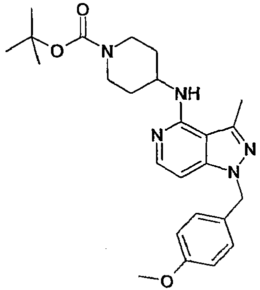

Intermediate 4 (1 g, 3.48 mmol), 4-amino-1-boc-piperidine (0.98 g, 4.88 mmol), Pd(OAc)₂ (47 mg, 0.21 mmol), BINAP (174 mg, 0.28 mmol) and cesium carbonate (3.39 g, 10.5 mmol) were combined in dioxane (20 ml). The mixture was degassed and placed under an atmosphere of nitrogen, then stirred at 90°C for 18h. The mixture was diluted with DCM (50 ml), partitioned with H₂O (50 ml) and the aqueous layer extracted with DCM (2x 50 ml). The combined organic layers were washed with brine, dried (MgSO₄) and evaporated. The crude product was purified by flash chromatography on the Biotage SP4, eluting with 0 to 100% EtOAc/petroleum ether to give a yellow solid (950 mg, 60%). H NMR (400 MHz, DMSO-*d*₆) δ ppm 1.40 (s, 9 H), 1.44 - 1.57 (m, 2 H), 1.85 - 1.93 (m, 2 H), 2.56 (s, 3 H), 2.74 - 2.93 (m, 2 H), 3.69 (s, 3 H), 3.88 - 4.00 (m, 2 H), 4.18 - 4.28 (m, 1 H), 5.32 (s, 2 H), 5.68 - 5.73 (m, 1 H), 6.79 (d, *J*=6.0 Hz, 1 H), 6.81 - 6.88 (m, 2 H), 7.12 - 7.17 (m, 2 H), 7.69 (d, *J*=6.0 Hz, 1 H). m/z (ES+APCI)⁺: 452 [M+H]⁺

### Intermediate 8

### [1-(4-Methoxy-benzyl)-3-methyl-1H-pyrazolo[4,3-c]pyridin-4-yl]-piperidin-4-yl-amine hydrochloride salt.

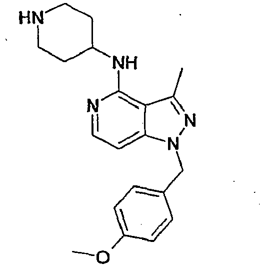

Intermediate 7 (0.92 g, 2.04 mmol) and 4M hydrochloric acid (20 ml) were combined and stirred at room temperature for 3h. The reaction mixture was evaporated to give a white solid (0.85 g, 100%). ¹H NMR (400 MHz, DMSO-*d*₆) δ ppm 1.93 - 2.05 (m, 2 H), 2.06 - 2.14 (m, 2 H), 2.66 (s, 3 H), 2.94 - 3.08 (m, 2 H), 3.31 - 3.45 (m, 2 H), 3.71 (s, 3 H), 4.21 - 4.31 (m, 1 H), 5.50 (s, 2 H), 6.86 - 6.91 (m, 2 H), 7.19 - 7.25 (m, 2 H), 7.37 - 7.44 (m, 1 H), 7.67 - 7.73 (m, 1 H), 7.79 - 7.86 (m, 1 H), 8.95 (br. s., 1 H). m/z (ES+APCI)⁺: 352 [M+H]⁺.

### Intermediate 9

### 4-[1-(4-Methoxy-benzyl)-3-methyl-1H-pyrazolo[4,3-c]pyridin-4-ylamino]-benzoic acid

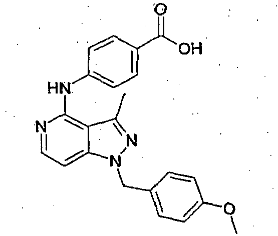

### Step 1

Intermediate 4 (1 g, 3.48 mmol), 4-amino-benzoic acid methyl ester (0.74 g, 4.88 mmol), Pd(OAc)₂ (47 mg, 0.21 mmol), BINAP (174 mg, 0.28 mmol) and cesium carbonate (3.4 g, 10.5 mmol) were combined in dioxane (20 ml). The mixture was degassed and placed under an atmosphere of nitrogen, then stirred at 90 °C for 18 h. The mixture was diluted with DCM (50 ml), partitioned with H₂O (50 ml) and the aqueous layer extracted with DCM (2x 50 ml). The combined organic layers were washed with brine, dried (MgSO₄) and evaporated. The crude product was purified by flash chromatography on the Biotage SP4, eluting with 0 to 60% EtOAc/petroleum ether to give a pale yellow solid (945 mg) which was used in the next step without further purification.

### Step 2

2M NaOH (aq) (3.5 ml, 7.05 mmol) was added to the crude product of Step 1 (945 mg) in EtOH (20 ml). The reaction mixture was stirred at 70°C for 4h. The reaction mixture was evaporated, dissolved in H₂O (20 ml) and adjusted to pH6 with 1M HCl (aq). The precipitate was filtered and washed with H₂O. The solid was azeotroped with toluene and then acetonitrile to give a yellow solid (832 mg, 91 %). NMR data indicate a mixture of N-1 and N-2 regioisomers:
*Major regioisomer:* ¹H NMR (400 MHz, DMSO-*d*₆) δ ppm 2.71 (s, 3 H), 3.60 (br. s., 1 H), 3.72 (s, 3 H), 5.53 (s, 2 H), 6.87 - 6.92 (m, 2 H), 7.23 - 7.27 (m, 2 H), 7.46 (d, *J*=6.0 Hz, 1 H), 7.67 (d, *J*=8.2 Hz, 2 H), 7.75 (d, *J*=6.9 Hz, 1 H), 8.00 (d, *J*=8.2 Hz, 2 H), 9.65 (br. s., 1 H); m/z (ES+APCl)⁺: 389 [M+H]⁺
*Minor regioisomer* ^{:1}H NMR (400 MHz, DMSO-*d*₆) δ ppm 2.91 (s, 3 H), 3.61 (br. s., 1 H), 3.73 (s, 3 H), 5.62 (s, 2 H), 6.92 - 6.96 (m, 2 H), 7.18 (d, *J*=7.3 Hz, 1 H), 7.25 - 7.29 (m, 2 H), 7.38 (d, *J*=6.9 Hz, 1 H), 7.62 (d, *J*=8.7 Hz, 2 H), 8.08 (d, *J*=8.7 Hz, 2 H), 9.65 (br. s., 1 H); m/z (ES+APCI)⁺: 389 [M+H]⁺

### Intermediate 10

### 2,4-Dichloro-Pyridine-3-carbaldehyde

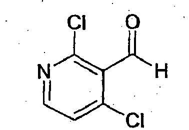

To a solution of n-butyllithium (1.6 M in hexane, 64 ml, 101 mmol) in THF (150 ml) at -78 °C was added diisopropylamine (14.3 ml, 101 mmol) dropwise. The reaction mixture was allowed to warm to 0 °C over 1 h, and then cooled down to -78 °C. 2,4-Dichloropyridine (11 ml, 101 mmol) was added dropwise and the solution was stirred at-78°C for 2.5 h. N-Formylpiperidine (11.2 ml, 101 mmol) was then added dropwise and the mixture stirred at -78 °C for a further 1.5 h. The solution was quenched at -78 °C with saturated NH₄Cl (aq) and then allowed to warm to room temperature. The reaction mixture was diluted with ethyl acetate and washed with 1M HCl (aq), the organic phase was separated, washed with saturated NaHCO₃ (aq), dried (MgSO₄) and evaporated to dryness. The crude residue was purified by flash chromatography, eluting with 0 to 20% ethyl acetate/petroleum ether gradient to give a yellow solid (9.7 g, 54%). ¹H NMR (400 MHz, DMSO-*d*₆) δ ppm 7.78 (d, *J*=5.04 Hz, 1 H), 8.56 (d, *J*=5.50 Hz, 1 H), 10.31 (s, 1 H). R_{f} (20% ethyl acetate in petroleum ether) = 0.70.

### Intermediate 11

### 4-Chloro-1H-pyrazolo[4,3-c]pyridine

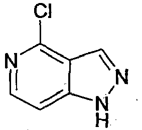

To a solution of Intermediate 11 (1.7 g, 9.7 mmol) in dimethoxyethane (12 ml) at room temperature was added hydrazine monohydrate (1.2 ml, 38.6 mmol) arid the resulting mixture was stirred at 75 °C overnight. The mixture was then concentrated to dryness and the crude residue was purified by flash chromatography, eluting with 20 to 100% ethyl acetate/petroleum ether gradient to give a white solid (0.82 g, 56%). ¹H NMR (400 MHz, DMSO-*d*₆) δ ppm 7.60 (d, *J*=6.9 Hz, 1 H), 8.14 (d, *J*=6.0 Hz, 1 H), 8.32 (s, 1 H); m/z (ES+APCl)⁺: 154 [M + H]⁺.

### Intermediate 12

### 4-Chloro-3-iodo-1H-pyrazolo[4,3-c]pyridine

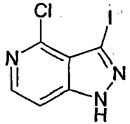

To a mixture of Intermediate 11 (5.8 g, 38 mmol) and KOH (8 g, 142 mmol) in dioxane (100 ml) at room temperature was added iodine (19g, 76 mmol). The reaction mixture was stirred at 75 °C for 4 h, and then allowed to cool to room temperature. The solution was diluted with saturated Na₂S₂O₃ (aq), and the resulting precipitate was filtered and dried to give a yellow solid (4.1 g). The filtrate was left standing for 3 days and filtration of the resulting precipitate yielded a further 2.35 g of the product. Combined yield (6.45 g, 61%). ¹H NMR (400 MHz, DMSO-*d*₆) δ ppm 7.64 (d, *J*=6.0 Hz, 1 H), 8.11 (d, *J=6.0* Hz, 1 H); m/z (ES+APCl)⁺: 280 [M + H]⁺.

### Intermediate 13

### 4-Chloro-3-iodo-1-(4-methoxy-benzyl)-1H-pyrazolo[4,3-c]pyridine

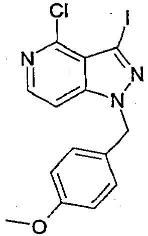

To a mixture of Intermediate 12 (1 g, 3.6 mmol) and KOH (0.3 mg, 5.4 mmol) in DMF (10 ml) at room temperature was added 4-methoxybenzyl chloride (0.5 ml, 3.6 mmol). The resulting mixture was stirred at room temperature for 2.5 h, and then evaporated to dryness. The crude residue was dissolved in EtOAc and washed with water. The organic phase was dried and purified by flash chromatography, eluting with 0 to 30% ethyl acetate/petroleum ether gradient to give a 9:1 mixture of N1:N2 regioisomers as a solid (1.3 g, 93%). *Major regioisomer.* ¹H NMR (400 MHz, DMSO-*d*₆) δ ppm 3.72 (s, 3 H), 5.62 (s, 2 H), 6.85 - 6.94 (m, 2 H), 7.20 - 7.27 (m, 2 H), 7.95 (d, *J*=6.0 Hz, 1 H), 8.20 (d, *J*=6.0 Hz, 1 H); m/z (ES+APCl)⁺: 400 [M + H]⁺.

### Intermediate 14

### Cyclohexyl-[3-iodo-1-(4-methoxy-benzyl)-1H-pyrazolo[4,3-c]pyridin-4-yl]-amine

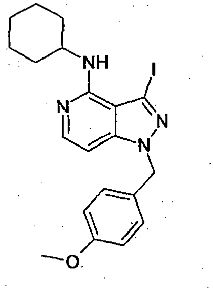

To a solution of Intermediate 13 (0.95 g, 2.4 mmol) in 1-butanol (5 ml) at room temperature was added cyclohexylamine (1.1 ml, 9.52 mmol). The resulting mixture was irradiated at 190 °C for 1 h in a Biotage I-60 microwave reactor. The reaction mixture was then evaporated to dryness and the crude residue was purified by flash chromatography eluting with 10 to 100% ethyl acetate/petroleum ether gradient to give a white solid (0.87 g, 80%) ¹H NMR (400 MHz, DMSO-*d*₆) δ ppm 1.24 - 1.50 (m, 5 H), 1.50 - 1.63 (m, 1 H), 1.63 - 1.80 (m, 2 H), 1.86 - 2.03 (m, 2 H), 3.72 (s, 3 H), 4.02 - 4.15 (m, 1 H), 5.43 (s, 2 H), 5.95 (d, *J*=7.3 Hz, 1 H), 6.85 - 6.90 (m, 2 H), 6.95 (d, *J*=6.0 Hz, 1 H), 7.15 - 7.24 (m, 2 H), 7.76 (d, *J*=6.0 Hz, 1 H); m/z (ES+APCl)⁺: 463 [M + H]⁺.

### Intermediate 15

### 3-Bromo-4-chloro-1H-pyrazolo[4,3-c]pyridine

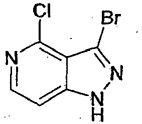

*N*-bromosuccinimide (1.87 g, 10.5 mmol) was added to a solution of Intermediate 11 (1.61 g, 10.5 mmol) in acetonitrile (50 ml), and the mixture was heated to reflux for 3 h. The solvents were evaporated and DCM (60 ml) was added to the crude solid and the mixture stirred at r.t. for 30 min. The beige solid was filtered off, washed with DCM, then dried under vacuum (1.98 g, 81%). ¹H NMR (400 MHz, DMSO-*d*₆) δ ppm 7.69 (d, *J*=6.0 Hz, 1 H), 8.22 (d, *J*=6.0 Hz, 1 H); m/z (ES+APCl)⁺: 232 / 234 / 236 [M + H]⁺.

### Intermediate 16

### Cyclopropyl-(2,4-dichloro-pyridin-3-yl)-methanol

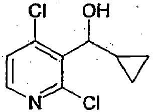

To a solution of Intermediate 10 (1.00 g, 5.68 mmol) in dry THF (100 ml), under nitrogen at -78 °C was added dropwise cyclopropyl magnesium bromide (0.5 M in THF, 12.5 ml, 6.25 mmol). After stiring at -78 °C for a further 3 h, the mixture was warmed up to -20 °C, and then quenched with saturated ammonium chloride. The aqueous phase was extracted twice with ethyl acetate and the combined organic extracts washed with brine, dried (MgSO₄) and concentrated. Purification by flash chromatography using a Biotage SP4 (ethyl acetate / petroleum ether gradient) gave the product (443 mg, 36%). ¹H NMR (400 MHz, DMSO-*d*₆) δ ppm 0.25 - 0.32 (m, 1 H), 0.38 - 0.46 (m, 1 H), 0.48 - 0.55 (m, 1 H), 0.62 - 0.70 (m, 1 H), 1.61 - 1.73 (m, 1 H), 4.47 (dd, *J*=9.2*,* 4.1 Hz, 1 H), 5.67 (d, *J*=4.1 Hz, 1 H), 7.62 (d, *J*=5.5 Hz, 1 H), 8.31 (d, *J*=5.5 Hz, 1 H); m/z (ES+APCl)⁺: 218 [M + H]⁺.

### Intermediate 17

### Cyclopropyl-(2,4-dichloro-pyridin-3-yl)-methanone

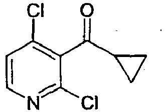

Freshly activated 4 A molecular sieves and *N*-methylmorpholine-*N*-oxide (336 mg, 2.87 mmol) were added to a solution of Intermediate 16 (417 mg, 1.91 mmol) in DCM (10 ml), under nitrogen and stirred for 15 min. After this time, TPAP (20 mg, 0.06 mmol) was added and stirring then continued for further 3.5 h at r.t. The reaction mixture was filtered through Celite and the filtrate concentrated. Purification by flash chromatography using a Biotage SP4 (ethyl acetate / petroleum ether gradient) gave the product (298 mg, 72%). ¹H NMR (400 MHz, DMSO-*d*₆) δ ppm 1.19 - 1.33 (m, 4 H), 2.43 - 2.52 (m, 1 H), 7.81 (d, *J*=5.5 Hz, 1 H), 8.53 (d, *J*=5.5 Hz, 1 H); Rf = 0.62 (1:1 petroleum ether/ethyl, acetate).

### Intermediate 18

### 4-Chloro-3-cyclopropyl-1H-pyrazolo[4,3-c]pyridine

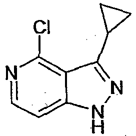

65% Hydrazine hydrate (1 ml) was added to Intermediate 17 (278 mg, 1.29 mmol) and the reaction stirred at r.t. for 19 h. The reaction mixture was partitioned between water and ethyl acetate and extracted twice with ethyl acetate. The combined organic extracts were washed with brine and dried (MgSO₄). Purification by flash chromatography using a Biotage SP4 (ethyl acetate / petroleum ether gradient) gave the product as a white solid (70 mg, 28%). ¹H NMR (400 MHz, DMSO-*d*₆) δ ppm 0.96 - 1.11 (m, 4 H), 2.56 - 2.64 (m, 1 H), 7.52 (d, *J*=6.0 Hz, 1 H), 8.10 (d, *J*=6.0 Hz, 1 H); m/z (ES+APCl)⁺: 194/196 [M + H]⁺.

### Intermediate 19

### 4-Cyclohexyloxy-3-iodo-1-(4-methoxy-benzyl)-1H-pyrazolo[4,3-c]pyridine

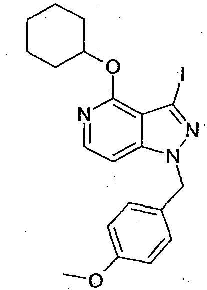

To a 2 - 5 ml microwave vial containing a solution of cyclohexanol (156 µl, 1.50 mmol) in dioxane (3 ml) was added NaH (60% dispersion, 45 mg, 1.13 mmol), the vessel was capped and flushed out with nitrogen and stirred for 3 h at r.t. A solution of Intermediate 13 (300 mg, 0.75 mmol) in dioxane (1 ml) was added and the vessel was irradiated at 180 °C for 1.5 h in the microwave. The solvents were evaporated, the crude mixture was partitioned between ethyl acetate and water, and the aqueous phase was extracted twice with ethyl acetate. The combined organic extracts washed with brine, dried (MgSO₄) concentrated. Purification by flash chromatography using a Biotage SP4 (ethyl acetate / petroleum ether gradient) gave a white solid (168 mg, 48%). ¹H NMR (400 MHz, DMSO-*d*₆) δ ppm 1.42 - 1.58 (m, 4 H), 1.65 - 1.78 (m, 2 H), 1.82 - 1.96 (m, 4 H), 3.75 (s, 3 H), 5.35 - 5.41 (m, 1 H), 5.55 (s, 2 H), 6.92 (d, *J*=9.2 Hz, 2 H), 7.25 (d, *J*=9.2 Hz, 2 H), 7.39 (d, *J*=6.0 Hz, 1 H), 7.90 (d, *J*=6.4 Hz, 1 H); m/z (ES+APCl)⁺: 464 [M + H]⁺.

### Intermediate 20

### (E)-3-[4-Cyclohexylamino-1-(4-methoxy benzyl)-1H-pyrazolo[4,3-c]pyridin-3-yl]acrylic acid methyl ester

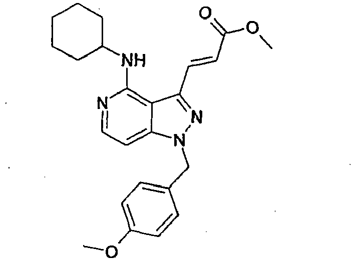

To a mixture of Intermediate 14 (3.1 g, 6.7 mmol) and tetrabutylammonium iodide (4.9 g, 13.4 mmol) in DMF/water/triethylamine (60 ml/9.2 ml/9.2 ml) at room temperature was added methyl acrylate (6 ml, 67 mmol) and Pd(dppf)Cl₂ (1.1 g, 1.34 mmol) respectively. The resulting mixture was heated at 70 °C overnight and then evaporated to dryness. The crude residue was dissolved in EtOAc and washed with water. The organic phase was dried, evaporated and purified by flash chromatography, eluting with 15 to 70% ethyl acetate/petroleum ether gradient to give a yellow solid (2 g, 71%) ¹H NMR (400 MHz, DMSO-*d*₆) δ ppm 1.16 - 1.26 (m, 1 H), 1.28 - 1.45 (m, 4 H), 1.62 (d, *J*=12.4 Hz, 1 H), 1.69 - 1.77 (m, 2 H), 1.92 - 1.99 (m, 2 H), 3.70 (s, 3 H), 3.76 (s, 3 H), 3.98 - 4.07 (m, 1 H), 5.49 (s, 2 H), 6.21 (d, *J*=7.8 Hz, 1 H), 6.67 (d, *J*=15.6 Hz, 1 H), 6.84 - 6.96 (m, 3 H), 7.21 - 7.25 (m, 2 H), 7.80 (d, 1 H), 8.05 (d, *J*=15.6 Hz, 1 H); m/z (ES+APCl)⁺: 421 [M + H]⁺.

### Intermediate 21

### 3-[4-Cyclohexylamino-1-(4-methoxy-benzyl)-1H-pyrazolo[4,3-c]pyridin-3-yl]-propionic acid methyl ester

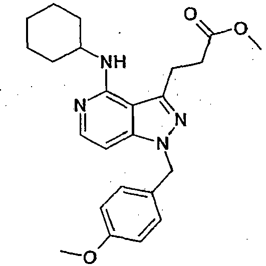

To a solution of Intermediate 20 (2 g, 4.7 mmol) in ethyl acetate (50 ml) at room temperature was added 10% Pd/C (0.4 g). The resulting mixture was stirred under hydrogen at room temperature overnight. The reaction mixture was then filtered through Celite^{™} and evaporated to give a gum (2 g, 100%). ¹H NMR (400 MHz, DMSO-*d*₆) δ ppm 1.13 -1.25 (m, 1 H), 1.27 - 1.43 (m, 4 H), 1.60 -1.72 (m, 1 H), 1.67 - 1.77 (m, 2 H), 1.89-1.99 (m, 2 H), 2.77 (t, 2 H), 3.27 (t, *J*=7.1 Hz, 2 H), 3.57 (s, 3 H), 3.69 (s, 3 H), 3.99 - 4.08 (m, 1 H), 5.32 (s, 2 H), 5.63 (d, *J*=7.8 Hz, 1 H), 6.74 (d, *J*=6.0 Hz, 1 H), 6.81 - 6.86 (m, 2 H), 7.10 - 7.15 (m, 2 H), 7.69 (d, *J*=6.0 Hz, 1 H); m/z (ES+APCl)⁺: 423 [M + H]⁺.

### Intermediate 22

### 3-(4-Cyclohexylamino-1H-pyrazolo[4,3-c]pyridin-3-yl)propionic acid methyl ester

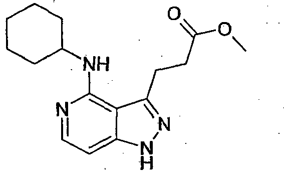

A solution of Intermediate **21** (1.37 g, 3.24 mmol) in TFA (12 ml) was stirred at 70 °C for 4 h, and then allowed to cool to room temperature overnight. 2M NaOH (aq) was added, followed by NH₃ (aq), and then the aqueous was extracted with EtOAc. The organic phase was dried (MgSO₄), evaporated and purified by flash chromatography, eluting with 50% ethyl acetate/petroleum ether to 10% methanol/ethyl acetate gradient to give a cream solid (1.0 g, 100%) ¹H NMR (400 MHz, DMSO-*d*₆) δ ppm 1.14 - 1.25 (m, 1 H), 1.29 - 1.44 (m, 4 H), 1.58 - 1.66 (m, 1 H), 1.69 - 1.79 (m, 2 H), 1.91 - 2.01 (m, 2 H), 2.79 (t, 2 H), 3.28 (t, *J*=7.1 Hz, 2 H), 3.60 (s, 3 H), 3.98 - 4.07 (m, 1 H), 5.68 (br. s., 1 H), 6.59 (d, *J*=6.0 Hz, 1 H), 7.67 (d, *J*=6.0 Hz, 1 H); m/z (ES+APCl)⁺: 303 [M + H]⁺.

### Intermediate 23

### 3-(4-Cyclohexylamino-1H-pyrazolo[4,3-c]pyridin-3-yl)-propionic acid

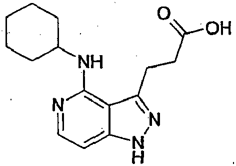

To a stirred solution of Intermediate 22 (0.98 g, 3.24 mmol) in methanol (10 ml) at room temperature was added 2M NaOH (4 ml, 8.11 mmol). The resulting mixture was stirred at room temperature overnight. Acetic acid (0.57 ml, 9.73 mmol) was then added, and the resulting precipitate was filtered and dried to give a white solid (0.7 g, 75%). ¹H NMR (400 MHz, DMSO-*d*₆) δ ppm 1.04 - 1.45 (m, 5 H), 1.60 -1.72 (m, 1 H), 1.66 - 1.78 (m, 2 H), 1.87 - 2.01 (m, 2 H), 2.68 (t, *J*=7.1 Hz, 2 H), 3.19 (t, 2 H), 3.97 - 4.07 (m, 1 H), 5.82 (br. s., 1 H), 6.57 (d, *J*=6.0 Hz, 1 H), 7.66 (d, *J*=6.0 Hz, 1 H); m/z (ES+APCl)⁺: 289 [M + H]⁺.

### Intermediate 24

### (E)-3-[4-Cyclohexylamino-1-(4-methoxy-benzyl)-1H-pyrazolo[4,3-c]pyridin-3-yl]-N,N-dimethyl-acrylamide

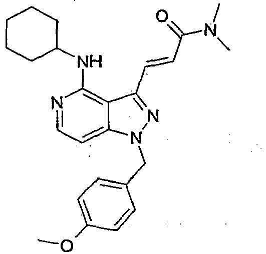

To a mixture of Intermediate 14 (0.25 g, 0.54 mmol) and tetrabutylammonium, iodide (0.4 g, 1.08 mmol) in DMF/water/triethylamine (5 ml/0.8 ml/0.8 ml) at room temperature was added *N,N*-dimethylacrylamide (0.56 ml, 5.4 mmol) and Pd(dppf)Cl₂ (88 mg, 0.11 mmol) respectively. The resulting mixture was heated at 65 °C overnight and then evaporated to dryness. The crude residue was dissolved in EtOAc and washed with water. The organic phase was dried (MgSO₄), evaporated and purified by flash chromatography, eluting with 50 to 100% ethyl acetate/petroleum ether gradient to give a brown gum (185 mg, 79%). ¹H NMR (400 MHz, DMSO-*d*₆) δ ppm 1.16 - 1.24 (m, 1 H), 1.28 - 1.42 (m, 4 H), 1.61 (m, 1 H), 1.68 - 1.75 (m, 2 H), 1.93 - 1.99 (m, 2 H), 2.95 (s, 3 H), 3.16 (s, 3 H), 3.70 (s, 3 H), 4.00 - 4.06 (m, 1 H), 5.49 (s, 2 H), 5.98 (d, *J*=7.8 Hz, 1 H), 6.85 - 6.90 (m, 3 H), 7.18 - 7.23 (m, 3 H), 7.77 - 7.80 (m, 2 H); m/z (ES+APCl)⁺: 434 [M + H]⁺.

### Intermediate 25

### 3-[4-Cyclohexylamino-1-(4-methoxy-benzyl)-1H-pyrazolo[4,3-c]pyridin-3-yl]-N,N-dimethyl propionamide

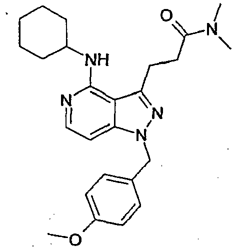

To Intermediate **24** (185 mg, 0.43 mmol) in ethyl acetate (3 ml) at room temperature was added 10% Pd/C (30 mg, 20% by wt). The resulting mixture was stirred under an atmosphere of hydrogen at room temperature overnight. The reaction mixture was then filtered through Celite^{™} and evaporated to give a gum (126 mg, 68%). ¹H NMR (400 MHz, DMSO-*d*₆) δ ppm 1.12 - 1.26 (m, 1 H), 1.26 - 1.46 (m, 4 H), 1.55 - 1.67 (m, 1 H), 1.67 - 1.80 (m, 2 H), 1.90 - 2.03 (m, 2 H), 2.75 (t, *J*=6.6 Hz, 2 H), 2.81 (s, 3 H), 2.94 (s, 3 H), 3.17 (t, *J*=6.6 Hz, 2 H), 3.69 (s, 3 H), 3.96 - 4.07 (m, 1 H), 5.34 (s, 2 H), 6.53 (d, *J*=7.3 Hz, 1 H), 6.74 (d, *J*=6.4 Hz, 1 H), 6.82 - 6.90 (m, 2 H), 7.11 - 7:19 (m, 2 H), 7.68 (d, *J*=6.4 Hz, 1 H); m/z (ES+APCl)⁺: 436 [M H]⁺.

### Intermediate 26

### Cyclohexyl-[1-(4-methoxy-benzyl)-3-((E)-styryl)-1H-pyrazolo[4,3-c]pyridin-4-yl]-amine

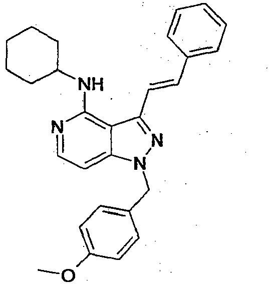

To a mixture of Intermediate 14 (0.25 g, 0.54 mmol) and tetrabutylammonium iodide (0.4 g, 1.08 mmol) in DMF/water/triethylamine (5 ml/0.8 ml/0.8 ml) at room temperature was added styrene (0.62 ml, 5.4 mmol) and Pd(dppf)Cl₂ (88 mg, 0.11 mmol) respectively. The resulting mixture was heated at 70 °C overnight and then evaporated to dryness. The crude residue was dissolved in DCM and washed with water. The organic phase was collected and dried using a phase separation cartridge and then evaporated. The crude residue was purified by flash chromatography, eluting with 20 to 40% ethyl acetate/petroleum ether gradient to give the desired product 50 mg, 63%) ¹H NMR (400 MHz, DMSO-*d*₆) δ ppm 1.15 - 1.26 (m, 1 H), 1.29 - 1.49 (m, 4 H), 1.59 - 1.66 (m, 1 H), 1.71 - 1.77 (m, 2 H), 1.95 - 2.02 (m, 2 H), 3.70 - 3.73 (m, 3 H), 4.01 - 4.12 (m, 1 H), 5.47 (s, 2 H), 6.04 (d, *J*=7.8 Hz, 1 H), 6.83 - 6.92 (m, 3 H), 7.19 - 7.25 (m, 2 H), 7.29 - 7.36 (m, 1 H), 7.39 - 7.45 (m, 3 H), 7.63 (d, *J*=16.0 Hz, 1 H), 7.68 - 7.74 (m, 2 H), 7.77 (d, 1 H); m/z (ES+APCl)⁺: 439 [M + H]⁺.

### Intermediate 27

### Cyclohexyl-[1-(4-methoxy-benzyl)-3-phenethyl-1H-pyrazolo[4,3-c]pyridin-4-yl]amine

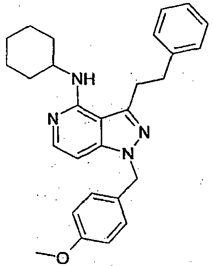

To Intermediate 26 (150 mg, 0.34 mmol) in ethyl acetate (5 ml) at room temperature was added 10% Pd/C (30 mg, 20% by wt). The resulting mixture was stirred under an atmosphere of hydrogen at room temperature over night. The reaction mixture was then filtered through Celite^{™} and evaporated to give a gum (140 mg, 93%). ¹H NMR (400 MHz, DMSO-*d*₆) δ ppm 1.12 - 1.26 (m, 1 H), 1.26 - 1.43 (m, 4 H), 1.57 - 1.66 (m, 1 H), 1.67 - 1.77 (m, 2 H), 1.90 - 2.01 (m, 2 H), 2.98 (m, 2 H), 3.29 (m, 2 H), 3.71 (s, 3 H), 3.97 - 4.06 (m, 1 H), 5.36 (s, 2 H), 5.67 (br. s., 1 H), 6.72 - 6.81 (m, 1 H), 6.75 - 6.90 (m, 2 H), 7.07 - 7.13 (m, 2 H), 7.15 - 7.21 (m, 1 H), 7.22 - 7.34 (m, 4 H), 7.69 (d, *J*=6.0 Hz, 1 H); m/z (ES+APCl)⁺: 441 [M + H]⁺.

### Intermediate 28

### Cyclohexyl-[1-(4-methoxy-benzyl)-3-((E)-2-pyridin-2-yl-vinyl)-1H-pyrazolo[4,3-c]pyridin-4-yl]-amine.

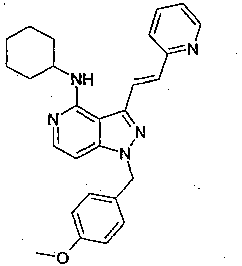

Prepared analogously to Intermediate 26 from Intermediate 14 and 2-vinylpyridine to give the desired product (200 mg, 84%). ¹H NMR (400 MHz, DMSO-*d*₆) δ ppm 1.15 - 1.27 (m, 1 H), 1.28 - 1.45 (m, 4 H), 1.57 - 1.64 (m, 1 H), 1.69 - 1.76 (m, 2 H), 1.93 - 2.03 (m, 2 H), 3.70 (s, 3 H), 4.00 - 4.09 (m, 1 H), 5.48 (s, 2 H), 5.95 (d, *J*=7.8 Hz, 1 H), 6.83 - 6.92 (m, 3 H), 7.20 - 7.25 (m, 2, H), 7.27 - 7.31 (m, 1 H), 7.42 (d, *J*=15.6 Hz, 1 H), 7.71 (d, *J*=8.2 Hz, 1 H), 7.78 (d, *J*=6.0 Hz, 1 H), 7.80 - 7.85 (m, 1 H), 7.95 (d, *J*=16.0 Hz, 1 H), 8.61 (d, *J*=3.7 Hz, 1 H); m/z (ES+APCl)⁺: 440 [M + H]⁺.

### Intermediate 29

### Cyclohexyl-[1-(4-methoxy-benzyl)-3-(2-pyridin-2-yl-ethyl)-1H-pyrazolo[4,3-c]pyridin-4-yl]-amine

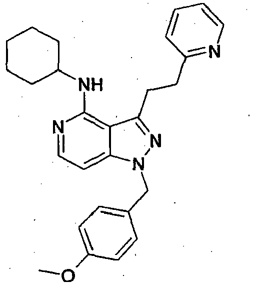

To a stirred solution of Intermediate 28 (150 mg, 0.34 mmol) in ethyl acetate (4.5 ml) at room temperature was added acetic acid (0.5 ml) and 10% Pd/C (30 mg). The resulting mixture was stirred under an atmosphere of hydrogen at room temperature for 72 h. The reaction mixture was then filtered through Celite^{™} and evaporated. The crude residue was dissolved in DCM and washed with 2M NaOH. The organic phase was dried and evaporated to give a brown gum (80 mg, 53%). ¹H NMR (400 MHz, DMSO-*d*₆) δ ppm 1.15 - 1.27 (m, 1 H), 1.27 - 1.45 (m, 4 H), 1.58 - 1.66 (m, 1 H), 1.70 - 1.79 (m, 2 H), 1.94 - 2.03 (m, 2 H), 3.11 - 3.18 (m, 2 H), 3.34 - 3.40 (m, 2 H), 3.72 (s, 3 H), 4.04 - 4.13 (m, 1 H), 5.34 (s, 2 H), 5.94 (d, *J*=7.8 Hz, 1 H), 6.74 (d, *J*=6.0 Hz, 1 H), 6.82 - 6.91 (m, 2 H), 7.09 - 7.14 (m, 2 H), 7.21 - 7.31 (m, 2 H), 7.68 - 7.73 (m, 2 H), 8.54 (d, *J*=4.1 Hz, 1 H); m/z (ES+APCl)⁺: 442 [M + H]⁺.

### Intermediate 30

### 3-[4-Cyclohexylamino-1-(4-methoxy-benzyl)-1H-pyrazolo[4,3-c]pyridin-3-yl]-propan-1-ol

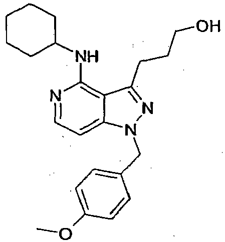

Lithium borohydride (132 mg, 6.0 mmol) was added to Intermediate **21** (0.85 g, 2.0 mmol) in THF (10 ml) at 0 °C. Methanol (0.25 ml, 6.0 mmol) was then added dropwise and the resulting mixture was stirred at 0 °C for 10 mins and then allowed to warm to room temperature overnight. The reaction mixture was diluted with methanol and evaporated. 6M HCl (aq) was then added and the resulting solution stirred at 50 °C for 35 mins and then evaporated. Saturated NaHCO₃ (aq) was added and then the aqueous phase was extracted with EtOAc (x 2). The combined organic phases were dried (MgSO₄) and evaporated to give the product as a gum (0.74 g, 93%) ¹H NMR (400 MHz, DMSO-*d*₆) δ ppm 1.13 - 1.27 (m, 1 H), 1.27 - 1.40 (m, 4 H), 1.57 - 1.65 (m, 1 H), 1.67 - 1.84 (m, 4 H), 1.90 - 2.01 (m, 2 H), 2.98 (t, *J*=7.6 Hz, 2 H), 3.44 - 3.50 (m, 2 H), 3.70 (s, 3 H), 3.99 - 4.09 (m, 1 H), 4.85 (t, *J*=4.6 Hz, 1 H), 5.34 (s, 2 H), 5.79 (d, 1 H), 6.74 (d, *J*=6.4 Hz, 1 H), 6.83 - 6.88 (m, 2 H), 7.12 - 7.17 (m, 2 H), 7.68 (d, *J*=6.0 Hz, 1 H); m/z (ES+APCl)⁺: 395 [M + H]⁺.

### Intermediate 31

### 3-[4-Cyclohexylamino-1-(4-methoxy-benzyl)-1H-pyrazolo[4,3-c]pyridin-3-yl]-propionic acid

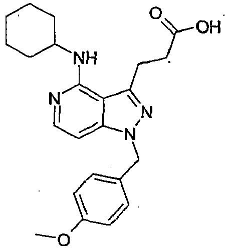

Prepared analogously to Intermediate 23 from Intermediate **21** to give the product (0.3 g, 89%). ¹H NMR (400 MHz, DMSO-*d*₆) δ ppm 1.14 - 1.43 (m, 5 H), 1.56 - 1.63 (m, 1 H), 1.67 - 1.75 (m, 2, H), 1.90 - 2.02 (m, 2 H), 2.44 (t, *J*=6.9 Hz, 2 H), 3.08 (t, *J*=6.6 Hz, 2 H), 3.69 (s, 3 H), 3.93 - 4.01 (m, 1 H), 5.31 (s, 2 H), 6.66 (d, *J*=6.4 Hz, 1 H), 6.82 - 6.86 (m, 2 H), 7.11 - 7.16 (m, 2 H), 7.65 (d, *J*=6.0 Hz, 1 H); R_{f} (100% ethyl acetate) = 0.10.

### Intermediate 32

### Benzoic acid N'-{3-[4-cyclohexylamino-1-(4-methoxy-benzyl)-1H-pyrazolo[4,3-c]pyridin-3-yl]propionyl}-hydrazide

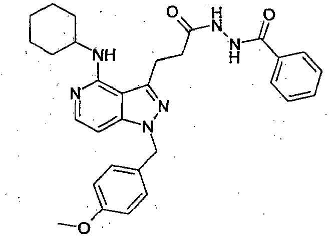

To a solution of Intermediate 31 (100 mg, 0.25 mmol) in DMF (2 ml) was added HATU (93 mg, 0.25 mmol) and *N,N*-diisopropylethylamine (250 µl, 1.5 mmol). Benzoic hydrazide (33 mg, 0.25 mmol) was then added and the resulting solution was left to stir at room temperature overnight. The volatiles were removed under reduced pressure and the crude product was dissolved in 10% MeOH/DCM and eluted though an Isolute-NH₂ cartridge and evaporated. The crude residue was re-dissolved in DCM and washed with water. The organic phase was separated, dried and evaporated to give the desired product (90 mg, 70%). ¹H NMR (400 MHz, DMSO-*d*₆) δ ppm 1.07 - 1.19 (m, 1 H), 1.22 - 1.45 (m, 4 H), 1.53 - 1.61 (m, 1 H), 1.65 - 1.76 (m, 2 H), 1.91 - 1.99 (m, 2 H), 2.65 - 2.77 (m, 2 H), 3.25 (t, *J*=6.9 Hz, 2 H), 3.69 (s, 3 H), 3.87 - 3.95 (m, 1 H), 5.43 (s, 2 H), 6.85 (d, 2 H), 6.89 - 7.01 (m, 1 H), 7.19 (d, *J*=8.7 Hz, 2 H), 7.41 - 7.52 (m, 2 H), 7.54 - 7.60 (m, 1 H), 7.65 - 7.69 (m, 1 H), 7.82 - 7.87 (m, 1 H), 7.95 (s, 1 H), 10.08 (s, 1 H), 10.33 (s, 1 H); m/z (ES+APCl)⁺: 527 [M + H]⁺.

### Intermediate 33

### Cyclohexyl-{1-(4-methoxy-benzyl)-3-[2-(5-pheny/-1,3,4-oxadiazol-2-yl)-ethyl]-1H-pyrazolo[4,3-c]pyridin-4-yl}-amine

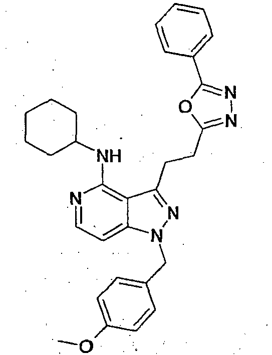

To Intermediate 32 (90 mg, 0.17 mmol) in THF (2 ml) at room temperature was added methyl-*N*-(triethylammoniumsulfonyl)carbamate inner salt (Burgess reagent) (51 mg, 0.21 mmol). The resulting mixture was irradiated at 100 °C for 30 mins in a Biotage I-60 microwave reactor, monitoring the reaction by LCMS. Irradiation was continued for a further 30 mins at 150 °C prior to concentration followed by flash chromatography, eluting with 20 to 100% ethyl acetate/petroleum ether gradient to give a the product as a gum (20 mg, 23%). ¹H NMR (400 MHz, CDCl₃) δ ppm 1.11 - 1.18 (m, 1 H), 1.19 - 1.62 (m, 4 H), 1.61 - 1.72 (m, 1 H), 1.72 - 1.85 (m, 2 H), 2.09 - 2.21 (m, 2 H), 3.46 - 3.61 (m, 4 H), 3.71 (s, 3 H), 4.09 - 4.26 (m, 1 H), 5.31 (s, 2 H), 6.50 (d, *J*=6.4 Hz, 1 H), 6.75 - 6.81 (m, 2 H), 7.08 - 7.16 (m, 2 H), 7.44 - 7.56 (m, 3 H), 7.82 (d, *J*=6.4 Hz, 1 H), 7.96 - 8.08 (m, 2 H); m/z (ES+APCl)⁺: 509 [M + H]⁺.

### Intermediate 34

### Cyclohexyl-[1-(4-methoxy-benzyl)-3-((E)-2-pyridin-4-yl-vinyl)-1H-pyrazolo[4,3-c]pyridin-4-yl]-amine

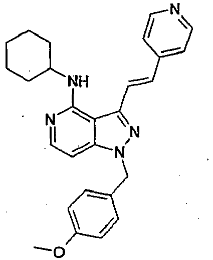

Prepared analogously to Intermediate 26 from Intermediate 14 and 4-vinylpyridine to give the product as a yellow solid (0.93 g, 65%) ¹H NMR (400 MHz, DMSO-*d*₆) δ ppm 1.13 - 1.26 (m, 1 H), 1.28 - 1.51 (m, 4 H), 1.59 - 1.68 (m, 1 H), 1.70 - 1.80 (m, 2 H), 1.95 - 2.04 (m, 2 H), 3.71 (s, 3 H), 4.02 - 4.13 (m, 1 H), 5.49 (s, 2 H), 6.17 (d, *J*=7.8 Hz, 1 H), 6.86 - 6.92 (m, 3 H), 7.16 - 7.25 (m, 2 H), 7.37 (d, *J*=16.0 Hz, 1 H), 7.68 (d, *J*=6.0 Hz, 2 H), 7.78 (d, *J*=6.0 Hz, 1 H), 7.89 (d, *J*=16.0 Hz, 1 H), 8.59 (d, *J*=6.0 Hz, 2 H); m/z (ES+APCl)⁺: 440 [M + H]⁺.

### Intermediate 35

### 3-(4-Cyclohexylamino-1H-pyrazolo[4,3-c]pyridin-3-yl)propan-1-ol

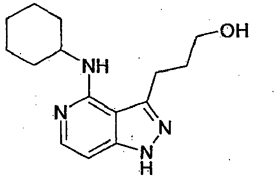

A solution of Intermediate 30 (0.74 g, 1.9 mmol) in TFA (5 ml) was stirred at 70 °C overnight. The reaction mixture was cooled to room temperature and then diluted with DCM, and saturated Na₂CO₃ (aq) was added. The organic phase was separated, filtered through a phase separation tube and evaporated. The crude residue was purified by flash chromatography, eluting with 20% ethyl acetate/petroleum ether to 10% methanol/ethyl acetate gradient to give a gum (0.47 g, 91%). ¹H NMR (400 MHz, DMSO-*d*₆) δ ppm 1.13 - 1.26 (m, 1 H), 1.32 - 1.58 (m, 4 H), 1.62 - 1.70 (m, 1 H), 1.75 - 1.88 (m, 4 H), 1.93 - 2.03 (m, 2 H), 3.04 - 3.14 (m, 2 H), 3.14 - 3.25 (m, 1 H), 3.48 (t, *J*=5.7 Hz, 2 H), 3.79 - 3.88 (m, 1 H), 5.20 (br. s., 1 H), 6.98 (d, 1 H), 7.52 - 7.68 (m, 1 H); m/z (ES+APCl)⁺: 275 [M + H]⁺.

### Intermediate 36

### Cyclohexyl-[3-((E)-2-pyridin-4-yl-vinyl)-1H-pyrazolo[4,3-c]pyridin-4-yl]-amine

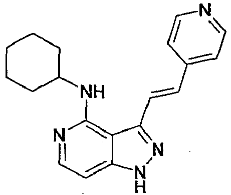

Prepared analogously to Intermediate 35 from Intermediate 34 to give the product as yellow solid (23 mg, 21%) ¹H NMR (400 MHz, MeOD) δ ppm 1.25 - 1.54 (m, 5 H), 1.60 - 1.74 (m, 1 H), 1.74 - 1.87 (m, 2 H), 1.99 - 2.22 (m, 2 H), 3.97 - 4.06 (m, 1 H), 6.74 (d, J=6.0 Hz, 1 H), 7.38 (d, *J*=16.0 Hz, 1 H), 7.65 (d, *J*=6.4 Hz, 2 H), 7.72 (d, *J*=6.0 Hz, 1 H), 7.84 (d, *J*=16.0 Hz, 1 H), 8.54 (d, *J*=6.4 Hz, 2 H); m/z (ES+APCl)⁺: 320 [M + H]⁺.

### Intermediate 37

### Cyclohexyl-[1-(4-methoxy-benzyl)-3-(2-pyridin-4-yl-ethyl)-1H-pyrazolo[4,3-c]pyridin-4-yl]-amine

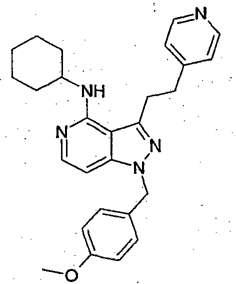

To a stirring solution of Intermediate 34 (0.53 g, 1.2 mmol) in ethanol (10 ml) at room temperature was added platinum oxide (53 mg) and 4M HCl in dioxane (0.3 ml). The resulting mixture was stirred under hydrogen at room temperature overnight. More platinum oxide (50 mg) was added and the mixture was stirred for a further 18 h at room temperature. The reaction mixture was then filtered through Celite^{™}, evaporated and partitioned between DCM and saturated Na₂CO₃ (aq). The organic phase was collected using a phase separation tube, evaporated and then purified by flash chromatography, eluting with ethyl acetate to 20% 2M NH₃ in methanol(aq) / ethyl acetate gradient to give the desired product (0.47 mg, 88%). ¹H NMR (400 MHz, CDCl₃) δ ppm 1.10 - 1.38 (m, 3 H), 1.38 - 1.60 (m, 2 H), 1.60 - 1.83 (m, 3 H), 2.08 - 2.37 (m, 2 H), 3.11 - 3.21 (m, 2 H), 3.21 - 3.32 (m, 2 H), 3.78 (s, 3 H), 4.11 - 4.26 (m, 1 H), 4.72 (d, *J*=7.3 Hz, 1 H), 5.32 (s, 2 H), 6.51 (d, *J*=6.4 Hz, 1 H), 6.79 - 6.88 (m, 2 H), 7.04 - 7.11 (m, 2 H), 7.15 (d, *J*=6.0 Hz, 2 H), 7.82 (d, *J*=6.4 Hz, 1 H), 8.51 (d, *J*=5.0 Hz, 2 H); m/z (ES+APCI)⁺: 442 [M + H]⁺.

### Intermediate 38

### Cyclohexyl-[1-(4-methoxy-benzyl)-3-(2-piperidin-4-yl-ethyl)-1H-pyrazolo[4,3-c]pyridin-4-yl]-amine

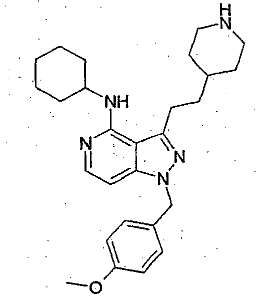

To a stirred solution of Intermediate 37 (0.47 g, 1.1 mmol) in ethanol (10 ml) at room temperature was added platinum oxide (50 mg) and 4M HCl in-dioxane (0.27 ml, 1.1). The resulting mixture was stirred under hydrogen at 50 °C overnight. The crude product was filtered through Celite^{™}, evaporated and then partitioned between DCM and saturated Na₂CO₃ (aq). The organic phase was collected, dried(MgSO₄) and evaporated to give the desired product (28 mg, 59%). ¹H NMR (400 MHz, DMSO-*d*₆) δ ppm 0.93 - 1.09 (m, 2 H), 1.14 - 1.41 (m, 6 H), 1.47 - 1.82 (m, 7 H), 1.84 - 2.04 (m, 2 H), 2.32 - 2.49 (m, 2 H), 2.86 - 2.94 (m, 2 H), 2.95 - 3.07 (m, 2 H), 3.71 (s, 3 H), 3.97 - 4.08 (m, 1 H), 5.34 (s, 2 H), 6.75 (d, *J*=6.0 Hz, 1 H), 6.82 - 6.89 (m, 2 H), 7.08 - 7.16 (m, 2 H), 7.68 (d, 1 H); m/z (ES+APCl)⁺: 448 [M + H]⁺.

### Intermediate 39

### Cyclohexyl-{1-(4-methoxy-benzyl)-3-[(E)-2-(3-nitro-phenyl)-vinyl]-1H-pyrazolo[4,3-c]pyridin-4-yl}-amine

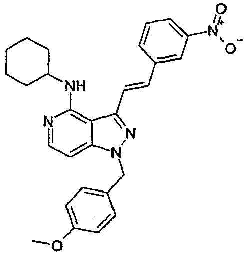

Prepared analogously to Intermediate 26 from Intermediate 14 and 3-nitrostyrene to give the product as a yellow solid (0.93 g, 59%). ¹H NMR (400 MHz, DMSO-*d*₆) δ ppm 1.17 - 1.25 (m, 1 H), 1.29 - 1.39 (m, 2 H), 1.39 - 1.50 (m, 2 H), 1.60 - 1.66 (m, 1 H), 1.71 - 1.78 (m, 2 H), 1.96 - 2.02 (m, 2 H), 3.70 (s, 3 H), 4.04 - 4.12 (m, 1 H), 5.48 (s, 2 H), 6.19 (d, *J*=7.8 Hz, 1 H), 6.86 - 6.91 (m, 3 H), 7.20 - 7.24 (m, 2 H), 7.52 (d, *J*=16.0 Hz, 1 H), 7.71 - 7.74 (m, 1 H), 7.77 (d, *J*=6.4 Hz, 1 H), 7.84 (d, *J*=15.6 Hz, 1 H), 8.13 - 8.16 (m, 1 H), 8.19 (d, *J*=7.8 Hz, 1 H), 8.55 (s, 1 H); m/z (ES+APCl)⁺: 484 [M + H]⁺.

### Intermediate 40

### Cyclohexyl-{3-[(E)-2-(3-nitro-phenyl)-vinyl]-1H-pyrazolo[4,3-c]pyridin-4-yl}-amine

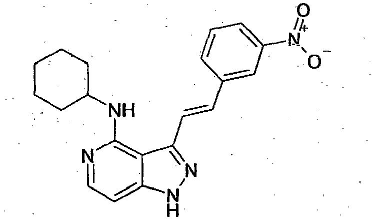

Prepared analogously to Intermediate 35 from Intermediate 39 to give the product (as a brown solid (0.48 g, 100%). ¹H NMR (400 MHz, DMSO-*d*₆) δ ppm 1.16 - 1.26 (m, 1 H), 1.29 - 1.50 (m, 4 H), 1.60 - 1.67 (m, 1 H), 1.72 - 1.79 (m, 2 H), 1.97 - 2.10 (m, 2 H), 4.04 - 4.13 (m, 1 H), 6.13 (d, *J*=7.8 Hz, 1 H), 6.67 (d, *J*=6.0 Hz, 1 H), 7.55 (d, *J*=16.0 Hz, 1 H), 7.70 - 7.76 (m, 2 H), 7.87 (d, *J*=16.0 Hz, 1 H), 8.13 - 8.17 (m, 1 H), 8.18 8.21 (m, 1 H), 8.55 (s, 1 H); m/z (ES+APCI)⁺: 364 [M + H]⁺.

### Intermediate 41

### 4-{(E)-2-[4-Cyclohexylamino-1-(4-methoxy-benzyl)-1H-pyrazolo[4,3-c]pyridin-3-yl]-vinyl}-benzoic acid methyl ester

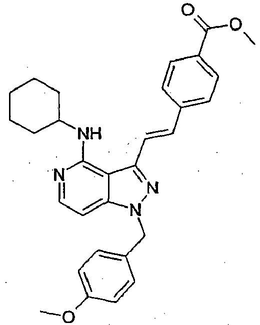

Prepared analogously to Intermediate 26 from Intermediate 14 and methyl-4-vinyl benzoate to give the desired product as a brown solid (190 mg, 70%). ¹H NMR (400 MHz, DMSO-*d*₆) δ ppm 1.15 - 1.24 (m, 1 H), 1.27 - 1.48 (m, 4 H), 1.59 - 1.65 (m, 1 H), 1.70 - 1.77 (m, 2 H), 1.94 - 2.01 (m, 2 H), 3.69 (s, 3 H), 3.86 (s, 3 H), 4.03 - 4.11 (m, 1 H), 5.47 (s, 2 H), 6.14 (d, *J*=7.8 Hz, 1 H), 6.85 = 6.90 (m, 3 H), 7.19 - 7.23 (m, 2 H), 7.45 (d, *J*=16.0 Hz, 1 H), 7.73 - 7.82 (m, 2 H), 7.85 (d, *J*=8.2 Hz, 2 H), 7.98 (d, *J*=8.7 Hz, 2 H);m/z (ES+APCl)⁺: 497 [M + H]⁺.

### Intermediate 42

### 4-{2-[4-Cyclohexylamino-1-(4-methoxy-benzyl)-1H-pyrazolo[4,3-c]pyridin-3-yl]-ethyl}-benzoic acid methyl ester

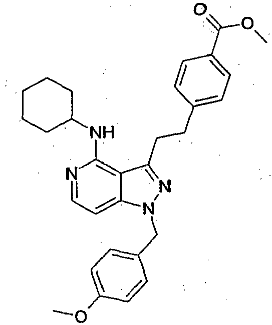

To Intermediate 41 (0.19 g, 0.38 mmol) in ethanol (5 ml) at room temperature was added 10% Pd/C (40 mg). The resulting mixture was stirred under an atmosphere of hydrogen at room temperature overnight. More 10% Pd/C (40 mg) was added and the mixture was stirred for a further 18 h at room temperature. The reaction mixture was then filtered through Celite^{™} and evaporated to give the product as a brown gum (185 mg, 97%). ¹H NMR (400 MHz, DMSO-*d*₆) δ ppm 1.12 - 1.27 (m, 1 H), 1.27 - 1.42 (m, 4 H), 1.57 - 1.65 (m, 1 H), 1.66 - 1.76 (m, 2 H), 1.92 - 1.99 (m, 2 H), 3.05 - 3.11 (m, 2 H), 3.34 - 3.41 (m, 2 H), 3.70 (s, 3 H), 3.83 (s, 3 H), 3.98 - 4.06 (m, 1 H), 5.32 (s, 2 H), 5.61 (d, *J*=6.4 Hz, 1 H), 6.74 (d, *J*=6.0 Hz, 1 H), 6.77 - 6.82 (m, 2 H), 7.01 - 7.06 (m, 2 H), 7.34 - 7.38 (m, 2 H), 7.68 (d, *J*=6.4 Hz, 1 H), 7.82 - 7.87 (m, 2 H); m/z (ES+APCl)⁺: 499 [M + H]⁺.

### Intermediate 43

### 4-[2-(4-Cyclohexylamino-1H-pyrazolo[4,3-c]pyridin-3-yl)-ethyl]benzoic acid methyl ester

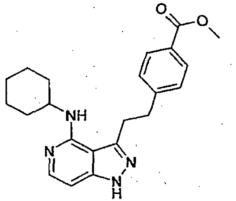

Prepared analogously to Intermediate 35 from Intermediate 42 to give the product as a brown solid (0.12 g, 86%). ¹H NMR (400 MHz, DMSO-*d*₆) δ ppm 1.13 - 1.24 (m, 1 H), 1.27 - 1.41 (m, 4 H), 1.58 - 1.66 (m, 1 H), 1.67 - 1.76 (m, 2 H), 1.92 - 2.01 (m, 2 H), 3.06 - 3.12 (m, 2 H), 3.34 - 3.42 (m, 2 H), 3.83 (s, 3 H), 3.99 - 4.07 (m, 1 H), 5.52 (d, *J*=7.8, Hz, 1 H), 6.58 (d, *J*=6.0 Hz, 1 H), 7.42 (d, *J*=8.2 Hz, 2 H), 7.67 (d, *J*=6.0 Hz, 1 H), 7.88 (d, *J*=8.7 Hz, 2 H); m/z (ES+APCl)⁺: 379 [M + H]⁺.

### Intermediate 44

### 4-[2-(4-Cyclohexylamino-1H-pyrazolo[4,3-c]pyridin-3-yl)-ethyl]-benzoic acid

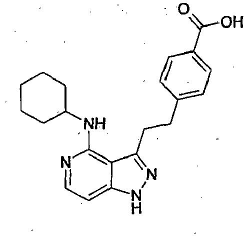

To a stirred solution of Intermediate 43 (0.1 g, 0.26 mmol) in methanol (2 ml) at room temperature was added 2M NaOH (0.33 ml, 0.66 mmol). The resulting mixture was stirred at 70 °C overnight. Acetic acid (40 µl, 0.66 mmol) was then added, and the resulting precipitate was filtered and dried to give a white solid (35 mg, 36%). ¹H NMR (400 MHz, DMSO-*d*₆) δ ppm 1.13 - 1.24 (m, 1 H), 1.27 - 1.43 (m, 4 H), 1.58 - 1.65 (m, 1 H), 1.68 - 1.78 (m, 2 H), 1.93 - 2.01 (m, 2 H), 3.05 - 3.12 (m, 2 H), 3.36 - 3.45 (m, 2 H), 3.96 - 4.05 (m, 1 H), 5.68 (br. s., 1 H), 6.62 (d, *J*=6.0 Hz, 1 H), 7.36 - 7.41 (m, 2 H), 7.66 (d, *J*=6.0 Hz, 1 H), 7.84 - 7.88 (m, 2 H); m/z (ES+APCl)⁺: 365 [M + H]⁺.

### Intermediate 45

### 4-Chloro-3-iodo-1-trityl-1H-pyrazolo[4,3-c]pyridine

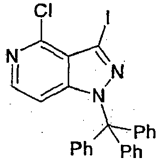

NaH (108 mg, 2.69 mmol, 60% dispersion) was added to a solution of Intermediate 12 (500 mg, 1.79 mmol) in DMF (2 ml) at 0 °C and the mixture was stirred at this temperature for 30 min. Trityl chloride (550 mg, 1.97 mmol) was then added and stirring continued at room tempearture for 19 h. Water was added, and the white precipitate was filtered and washed with water. The residue was then dissolved in DCM, washed with water, followed by brine, dried (MgSO₄) and solvents evaporated to give a white solid (900 mg, 96%). ¹H NMR (400 MHz, DMSO-*d*₆) δ ppm 6.33 (d, *J*=7.8 Hz, 1 H), 7.15 - 7.19 (m, 5 H), 7.31 - 7.46 (m, 10 H), 7.91 (d, *J*=6.4 Hz, 1 H); Rf = 0.52 (1:1, petroleum ether: ethyl acetate).

### Intermediate 46

### 4-Cyclohexyloxy-3-iodo-1-trityl-1H-pyrazolo[4,3-c]pyridine

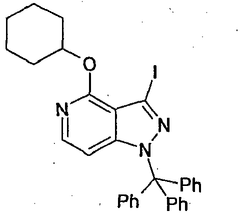

To a solution of cyclohexanol (223 µl, 2.15 mmol) in dioxane (1 ml), in a 2 - 5 ml microwave vial was added NaH (64 mg, 1.61 mmol, 60% dispersion). The vial was sealed and the mixture was stirred for 3 h at room temperature under nitrogen. After this time Intermediate 45 (559 mg, 1.07 mmol) in dioxane (2 ml) was added and the reaction irradiated in the microwave at 180 °C for 1.5 h. The solvents were evaporated and the crude product were partitioned between ethyl acetate and water, extracted twice with ethyl acetate, the organic extracts combined, washed with brine, dried (MgSO₄) and solvents removed. Purification by flash chromatography using a Biotage SP4 (ethyl acetate / petroleum ether gradient) gave a white solid (319 mg, 51%). ¹H NMR (400 MHz, DMSO-*d*₆) δ ppm 1.46 - 1.58 (m, 4 H), 1.66 - 1.77 (m, 2 H), 1.80 - 1.96 (m, 4 H), 5.27 - 5.39 (m, 1 H), 5.80 - 5.84 (m, 1 H), 7.13 - 7.20 (m, 5 H), 7.22 - 7.44 (m, 10 H), 7.57 (d, *J*=6.4 Hz, 1 H); m/z (ES+APCI)⁺: 586 [M + H]⁺.

### Intermediate 47

### (E)-3-[4-Chloro-1-(4-methoxy-benzyl)-1H-pyrazolo[4,3-c]pyridin-3-yl]-acrylic acid methyl ester

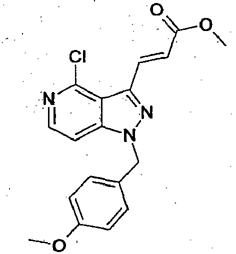

To a mixture of Intermediate 13 (2.0 g, 5.0 mmol) and tetrabutylammonium iodide (3.7 g, 10.0 mmol) in DMF/water/triethylamine (50 ml/8 ml/8 ml) at room temperature was added methyl acrylate (4.5 ml, 50 mmol) and Pd(dppf)Cl₂ (0.82 mg, 1.0 mmol) respectively. The resulting mixture was heated at 50 °C overnight and then evaporated to dryness. The crude residue was purified by flash chromatography, eluting with 20 to 100% ethyl acetate/petroleum ether gradient to give a brown solid (1.2 g, 67%) ¹H NMR (400 MHz, CDCl₃) δ ppm 3.80 (s, 3 H), 3.86 (s, 3 H), 5.52 (s, 2 H), 6.83 - 6.91 (m, 2 H), 6.97 (d, *J*=16.0 Hz, 1 H), 7.12 - 7.24 (m, 3 H), 8.13 (d, *J*=6.0 Hz, 1 H), 8.37 (d, *J*=16.0 Hz, 1 H); m/z (ES+APCI)⁺: 358 / 360 [M + H]⁺.

### Intermediate 48

### 3-[4-Chloro-1-(4-methoxy-benzyl)-1H-pyrazolo[4,3-c]pyridin-3-yl]-propionic acid methyl ester

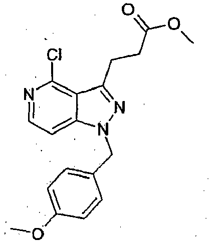

To a solution of Intermediate 47 (0.5 g, 1.4 mmol) in 1:1 mixture of 2-propanol/ethyl acetate (10 ml) at room temperature was added 5% Rh/Al₂O₃ (0.25 g). The resulting mixture was stirred under an atmosphere of hydrogen at room temperature overnight. A further 0.25 g of 5% Rh/Al₂O₃ was added and the mixture stirred for a further 18 h. The reaction mixture was then filtered through Celite^{™} and evaporated. The crude residue was purified by preparative LCMS (high pH buffer) to give the product as a white solid (0.15 mg, 30%). ¹H NMR (400 MHz, CDCl₃) δ ppm 2.89 - 2.95 (m, 2 H), 3.49 - 3.56 (m, 2 H), 3.70 (s, 3 H), 3.78 (s, 3 H), 5.43 (s, 2 H), 6.82 - 6.87 (m, 2 H), 7.08 - 7.17 (m, 3 H), 8.07 (d, *J*=6.0 Hz, 1 H); m/z (ES+APCI)⁺: 360 362 [M + H]⁺.

### Intermediate 49

### 3-[4-Chloro-1-(4-methoxy-benzyl)-1H-pyrazolo[4,3-c]pyridin-3-yl]-propionic acid

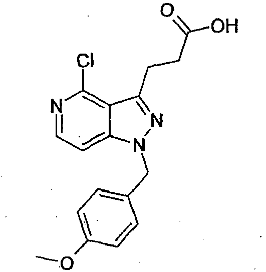

To a stirred solution of Intermediate 48 (0.15 g, 0.42 mmol) in methanol (1.5 ml) at room temperature was added 2M NaOH (0.52 ml, 1.0 mmol). The resulting mixture was stirred at room temperature overnight, then evaporated. The crude residue was dissolved in water, and then 2M HCl (0.52 ml, 1.0 mmol) was added. The resulting precipitate was filtered and dried to give a white solid (0.11 g, 77%). ¹H NMR (400 MHz, DMSO-*d*₆) δ ppm 2.77 (t, *J*=7.6 Hz, 2 H), 3.29, - 3.39 (m, 2 H), 3.71 (s, 3 H), 5.55 (s, 2 H), 6.84 - 6.90 (m, 2 H), 7.16 - 7.26 (m, 2 H), 7.76 (d, *J*=6.0 Hz, 1 H), 8.13 (d, *J*=6.0 Hz, 1 H); m/z (ES+APCI)⁺: 346 / 348 [M + H]⁺:

### Intermediate 50

### 3-[4-Chloro-1-(4-methoxy benzyl)-1H-pyrazolo[4,3-c]pyridin-3-yl]1-((R)-3-phenyl-piperidin-1-yl)-propan-1-one

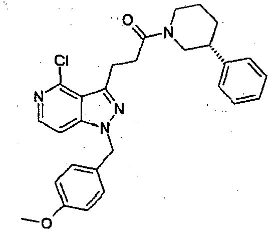

To a solution of Intermediate 49 (0.11 g, 0.32 mmol) in DMF (3 ml) was added HATU (0.13 g, 0.33 mmol) and *N*,*N*-diisopropylethylamine (332 µl, 1.9 mmol), followed by *(R)*-3-phenylpiperidine (52 mg, 0.32 mmol). The resulting solution was left to stir at room temperature overnight, and then evaporated. The crude product purified by flash chromatography eluting with 30-100% ethyl acetate/petroleum ether gradient to give a gum (70 mg, 45%). ¹H NMR (400 MHz, CDCl₃) δ ppm 1.53 - 1.78 (m, 2 H), 1.79 - 1.88 (m, 1 H), 2.00 - 2.26 (m, 1 H), 2.51 - 2.75 (m, 2 H), 2.84 - 3.11 (m, 3 H), 3.36 - 3.65 (m, 2 H), 3.77 (s, 3 H), 3.88 - 4.06 (m, 1 H), 4.67 - 4.88 (m, 1 H), 5.35 - 5.54 (m, 2 H), 6.72 - 6.95 (m, 2 H), 7.05 - 7.35 (m, 8 H), 7.99 - 8.14 (m, 1 H); m/z (ES+APCI)⁺: 489 / 491 [M + H]⁺.

### Intermediate 51

### (E)-3-[4-Methoxy-1-(4-methoxy-benzyl)-1H-pyrazolo[4,3-c]pyridin-3-yl]-acrylic acid

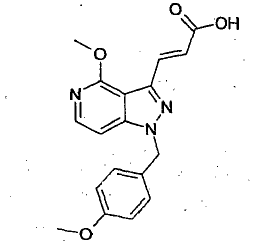

To a stirred solution of Intermediate 47 (0.5 g, 1.4 mmol) in methanol (10 ml) at room temperature was added 2M NaOH (1.75 ml, 3.5 mmol). The resulting mixture was stirred at room temperature overnight. The mixture was heated to 70 °C for a further 4 h, and evaporated. The crude residue was dissolved in water, and then 2M HCl (1.75 ml, 3.5 mmol) was added. The resulting precipitate was filtered and dried to give a brown solid (0.42 g, 87%). ¹H NMR (400 MHz, DMSO-*d*₆) δ ppm 3.70 (s, 3 H), 4.05 (s, 3 H), 5.59 (s, 2 H), 6.77 - 6.96 (m, 3 H), 7.15 - 7.36 (m, 2 H), 7.42 (d, *J*=6.0 Hz, 1 H), 7.84 (d, *J*=16.0 Hz, 1 H), 7.92 - 7.99 (m, 1 H); m/z (ES+APCI)⁺: 34.0 [M + H]⁺.

### Intermediate 52

### (E)-3-[4-Methoxy-1-(4-methoxy-benzyl)-1H-pyrazolo[4,3-c]pyridin-3-yl]-1-((R)-3-phenyl-piperidin-1-yl)-propenone

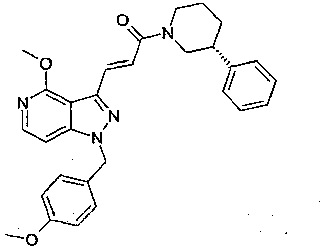

Prepared analogously to Intermediate 50 from Intermediate 51 and (R)-3-phenylpiperidine to yield a white solid (0.43 mg, 72%). ¹H NMR (400 MHz, DMSO-*d*₆) δ ppm 1.44 - 1.68 (m, 1 H), 1.71 - 1.92 (m, 2 H), 1.93 - 2.11 (m, 1 H), 2.62 - 2.83 (m, 4 H), 2.86 - 3.23 (m, 1 H), 3.74 (s, 3 H), 3.94 - 4.18 (m, 2 H), 4.42 (br. s, 1 H), 5.57 (s, 2 H), 6.89 (d, *J*=8.7 Hz, 2 H), 7.16 - 7.44 (m, 8 H), 7.53 - 7.66 (m, 1 H), 7.70 - 7.84 (m, 1 H), 7.93 (d, *J*=6.0 Hz, 1 H); m/z (ES+APCI)⁺: 483 [M + H]⁺.

### Intermediate 53

### 3-[4-Methoxy-1-(4-methoxy-benzyl)-1H-pyrazolo[4,3-c]pyridin-3-yl]-1-((R)-3-phenyl-piperidín-1-yl)-propan-1-one

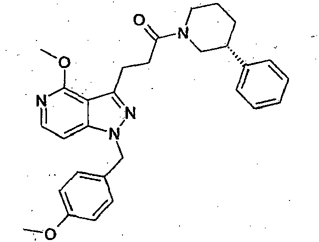

To a solution of Intermediate 52 (0.43 g, 0.89 mmol) in ethyl acetate (10 ml) at room temperature was added 10% Pd/C (90 mg). The resulting mixture was stirred under hydrogen at room temperature overnight. A further 100 mg of 10% Pd/C was added and the mixture stirred for a further 72 h. The reaction mixture was then filtered through Celite^{™} and evaporated to give a white foam (0.36 mg, 83%) ¹H NMR (400 MHz, DMSO-*d*₆) δ ppm 1.30 - 1.58 (m, 1 H), 1.64 - 1.77 (m, 2 H), 1.85 - 1.92 (m, 1 H), 2.52 - 2.65 (m, 2 H), 2.71 - 2.94 (m, 2 H), 3.02 - 3.23 (m, 3 H), 3.66 - 3.71 (m, 3 H), 3.79 - 4.15 (m, 4 H), 4.40 - 4.50 (m, 1 H), 5.45 (d, *J*=10.5 Hz, 2 H), 6.82 - 6.88 (m, 2 H), 7.13 - 7.34 (m, 8 H), 7.84 (dd, *J*=10.5, 6.0 Hz, 1 H); m/z (ES+APCI)⁺: 485 [M + H]⁺.

### Intermediate 54

### 4-Cyclohexyloxy-3-iodo-1H-pyrazolo[4,3-c]pyridine

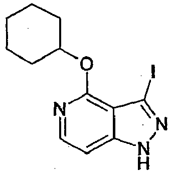

To a solution of cyclohexanol (1.5 ml, 14.3 mmol) in dioxane (28 ml) at room temperature was added sodium hydride (0.5 g, 12.5 mmol). The resulting mixture was stirred at room temperature for 1.5 h, then Intermediate 12 (1 g, 3.6 mmol) was added. The mixture was irradiated at 180 °C for 1.5 h in a Biotage I-60 microwave reactor and then evaporated to dryness. The crude residue was partitioned between DCM and H₂O, the aqueous phase was decanted and the organic phase was dry-loaded onto silica and purified by flash chromatography eluting with 20 to 100% ethyl acetate/petroleum ether gradient. The product was dissolved in 10% MeOH/EtOAc and eluted through an SCX cartridge, eluting first with 10% MeOH/EtOAc, followed by 2M/NH₃ in methanol to yield a white foam (0.84 g, 68%). ¹H NMR (400 MHz, DMSO-*d*₆) δ ppm 1.28 - 1.56 (m, 4 H), 1.56 - 1.80 (m, 2 H), 1.80 - 1.94 (m, 4 H), 5.20 - 5.40 (m, 1 H), 7.06 - 7.11 (m, 1 H), 7.79 - 7.84 (m, 1 H); m/z (ES+APCI)⁺: 344 [M + H]⁺.

### Intermediate 55

### 4-Cyclohexyloxy-3-iodo-pyrazolo[4,3-c]pyridine-1-carboxylic acid tert-butyl ester

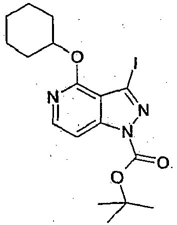

To a solution of Intermediate 54 (0.84 g, 2.45 mmol) and di-tert-butyl dicarbonate (1.38 g, 6.36 mmol) in THF (12 ml) at room temperature was added 4-(dimethylamino)pyridine (15 mg, 0.12 mmol). The resulting mixture was stirred at 70 °C for 2.5 h and then evaporated to dryness. The crude residue was partitioned between DCM and H₂O, and the organic phase collected and dried through a phase separating tube and evaporated to yield a yellow solid (1.1 g, 100%). ¹H NMR (400 MHz, DMSO-*d*₆) δ ppm 1.33 - 1.57 (m, 4 H), 1.57 - 1.78 (m, 11 H), 1.78 - 1.94 (m, 4 H), 5.33 - 5.40 (m, 1 H), 7.54 (d, *J*=6.0 Hz, 1 H), 8.12 (d, *J*=6.0 Hz, 1 H); m/z (ES+APCI)⁺: 444 [M + H]⁺.

### Intermediate 56

### 4-Cyclohexyloxy-3-((E)-2-methoxycarbonyl-vinyl)-pyrazolo[4,3-c]pyridine-1-carboxylic acid tert-butyl ester

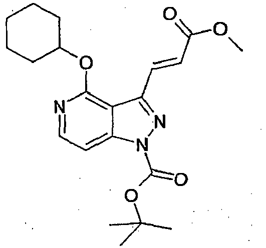

To a mixture of Intermediate 55 (0.6 g, 1.35 mmol) and tetrabutylammonium iodide (1 g, 2.7 mmol) in DMF/water/triethylamine (15 ml/2.4 ml/2.4 ml) at room temperature was added methyl acrylate (1.2 ml, 13.5 mmol) and Pd(dppf)Cl₂ (0.22 mg, 0.27 mmol) respectively. The resulting mixture was heated at 50 °C for 6 h and then evaporated to dryness. The crude residue was purified by flash chromatography, eluting with 5 to 20% ethyl acetate/petroleum ether gradient to give a white solid (80 mg, 15%) ¹H NMR (400 MHz, MeOD) δ ppm 1.40 - 1.67 (m, 4 H), 1.68 - 1.92 (m, 13 H), 1.99 - 2.13 (m, 2 H), 3.84 (s, 3 H), 5.21 - 5.49 (m, 1 H), 7.17 (d, *J*=16.0 Hz, 1 H), 7.48 - 7.74 (m, 1 H), 7.94 - 8.19 (m, 2 H); m/z (ES+APCI)⁺: 402 [M + H]⁺.

### Intermediate 57

### (E)-3-(4-Cyclohexyloxy-1H-pyrazolo[4,3-c]pyridin-3-yl)-acrylic acid

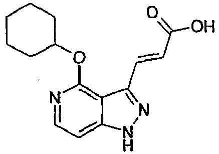

To a stirred suspension of Intermediate 56 (0.13 g, 0.32 mmol) in THF/methanol/water (2 ml/1 ml/ 1ml) at room temperature was added lithium hydroxide monohydrate (41 mg, 0.97 mmol). The resulting mixture was stirred at 65 °C overnight and then evaporated to dryness. The crude residue was dissolved in water and then 1M HCl (0.7 ml) was added. The resulting precipitate was filtered and dried to give a cream solid (60 mg, 65%). ¹H NMR (400 MHz, DMSO-*d*₆) δ ppm 1.31 - 1.71 (m, 6 H), 1.72 - 1.87 (m, 2 H), 1.91 - 2.16 (m, 2 H), 5.22 - 5.50 (m, 1 H), 6.93 (d, J=16.0 Hz, 1 H), 7.04 - 7.16 (m, 1 H), 7.85 - 7.98 (m, 2 H).

### Intermediate 58

### Cyclohexyl-{1-(4-methoxy-benzyl)-3-[(E)-2-(4-nitro-phenyl)-vinyl]-1H-pyrazolo[4,3-c]pyridin-4-yl}-amine

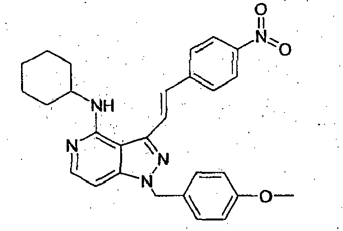

To a stirred solution of Intermediate 14 (3 g, 6.49 mmol) and tetrabutylammonium iodide (4.80 g, 13 mmol) in DMF/water/triethylamine (60 ml/9 ml/9 ml) was added 4-nitrostyrene (4.84 g, 32.4 mmol) and Pd(dppf)Cl₂ (1.06 g, 1.30 mmol). The reaction mixture was heated at 70 °C overnight under a nitrogen atmosphere. The mixture was allowed to cool to rt, concentrated and purified by column chromatography using a Biotage SP4 (petroleum ether/EtOAc gradient) gave the product as an orange solid (1.02 g). The column was further eluted with 100% EtOAc to 10-15% MeOH in EtOAc and the eluent was concentrated. The residue was diluted with EtOAc and H₂O. The organic layer was dried and concentrated. The residue was re-purified by column chromatography using a Biotage SP4 (DCM/MeOH gradient). The chromatographed product was then triturated in MeOH. The orange solid was collected by filtration, washed with MeOH and dried to yield a further 1.03 g of the product. Combined yield (2.05 g, 65% yield). ¹H NMR (400 MHz, CHLOROFORM-*d*) δ ppm 1.18 - 1.38 (m, 2 H) 1.38 - 1.58 (m, 2 H) 1.58 - 1.86 (m, 4 H) 2.14 (d, *J*=8.7 Hz, 2 H) 3.74 - 3.89 (m, 3 H) 4.10 - 4.25 (m, 1 H) 4.91 (d, *J*=7.8 Hz, 1 H) 5.43 (s, 2 H) 6.52 - 6.59 (m, 1 H) 6.80 - 6.93 (m, 2 H) 7.19 (d, *J*=7.8 Hz, 2 H) 7.33 - 7.59 (m, 2 H) 7.67 (d, *J*=7.8 Hz, 2 H) 7.89 (d, *J*=5.6 Hz, 1 H) 8.28 (d, *J*=7.8 Hz, 2 H); m/z (ES+APCI)⁺: 484 [M+H]⁺.

### Intermediate 59

### Cyclohexyl-{3-[(E)-2-(4-nitro-phenyl)-vinyl]-1H-pyrazolo[4,3-c]pyridin-4-yl}-amine

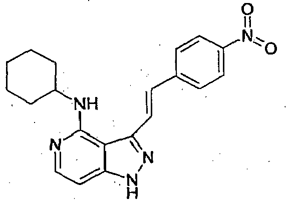

Intermediate 58 (2.05 g, 4.24 mmol) and TFA (20 ml) were combined and heated to 75 °C overnight under a nitrogen atmosphere. After cooling to rt, the solvent was evaporated, the residue was partitioned between DCM and saturated sodium carbonate (aq), and the organic layer was dried and concentrated. Purification by column chromatography using a Biotage SP4 (petroleum ether/EtOAc gradient) gave the desired product as a yellow solid (1.15 g, 75% yield). ¹H NMR (400 MHz, DMSO-*d*₆) δ ppm 1.12 - 1.25 (m, 1 H) 1.25 - 1.52 (m, 4 H) 1.63 (d, *J*=12.4 Hz, 1 H) 1.75 (d, *J*=12.8 Hz, 2 H) 1.94 - 2.07 (m, 2 H) 4.02 - 4.13 (m, 1 H) 6.13 (d, *J*=8.2 Hz, 1 H) 6.67 (d, *J*=6.0 Hz, 1 H) 7.54 (d, *J*=16.0 Hz, 1 H) 7.74 (d, *J*=6.0 Hz, 1 H) 7.86 - 8.05 (m, 3 H) 8.26 (d, *J*=8.7 Hz, 2 H); m/z (ES+APCI)⁺: 364 [M+H]⁺.

### Intermediate 60

### {3-[2-(4-Amino-phenyl)-ethyl]-1H-pyrazolo[4,3-c]pyridin-4-yl}-cyclohexyl-amine

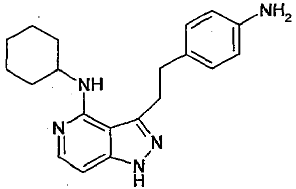

To a RB flask was added cyclohexyl Intermediate 59 (1.15 g) and 10% palladium on charcoal (115 mg) in ethanol (42 ml) and the mixture was stirred at rt under a hydrogen atmosphere for 18 hours. The reaction mixture was filtered through Celite and the filtrate was concentrated to afford the product as a pale yellow solid (1.07 g, 99% yield). ¹H NMR (400 MHz, DMSO-*d*₆) δ ppm 1.21 (d, *J*=8.7 Hz, 1 H) 1.25 - 1.48 (m, 4 H) 1.60 (d, *J*=11.9 Hz, 1 H) 1.66 - 1.81 (m, 2 H) 1.97 (br. s., 2 H) 2.78 (dd, *J*=9.4, 6.6 Hz, 2 H) 3.12 - 3.25 (m, 2 H) 4.01 (br. s., 1 H) 4.85 (s, 2 H) 5.36 (d, *J*=7.8 Hz, 1 H) 6.48 (m, *J*=8.2 Hz, 2 H) 6.57 (d, *J*=6.0 Hz, 1 H) 6.90 (m, *J*=8.2 Hz, 2 H) 7.66 (d, *J*=6.0 Hz, 1 H); m/z (ES+APCI)⁺: 336 [M+H]⁺.

### Intermediate 61

### 4-Chloro-3-methyl-1-trityl-1H-pyrazolo[4,3-c]pyridine

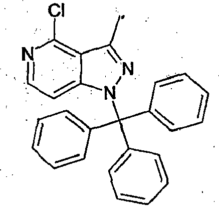

NaH (60% dispersion, 1.07 g, 27.0 mmol) was added to a solution of Intermediate 3 (3 g, 18.0 mmol) in DMF (50 ml). The suspension was stirred for 30 minutes at 0 °C. Triphenylmethyl chloride (5.51 g, 20.0 mmol) was added and the reaction stirred for 18 h at rt. The mixture was quenched with water (100 ml), extracted with EtOAc (x2), and the combined organic layers were washed water (x 3) followed by brine, (MgSO₄) and evaporated. Purification by flash chromatography using a Biotage SP4, eluting with 0 to 40% EtOAc/petroleum ether gave a pale yellow solid (5.2 g, 71%). The product was isolated as a 9:1 mixture of N1 (A) and N2 (B) alkylated regioisomers: m/z (ES+APCI)⁺: 410 [M+H]⁺

### Example 1

### (4-Chloro-benzyl)-(3-methyl-1H-pyrazolo[4,3-c]pyridin-4-yl)-amine

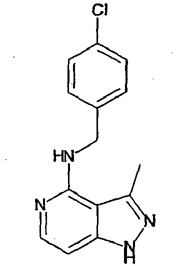

Intermediate 3 (60 mg, 0.357 mmol), 4-chlorobenzylamine (203 mg, 1.43 mmol) and 1-butanol (1 ml) were placed in a sealed microwave reactor vial. The vial was irradiated at 190 °C in a Biotage I-60 microwave reactor for 20 minutes. On cooling to rt the mixture was concentrated to dryness. The residue was dissolved in DMSO (1.2 ml) and purified by preparative LCMS to give a yellow solid (53 mg, 54%). ¹H NMR (400 MHz, DMSO-*d*₆) δ ppm 2.62 (m, 3 H) 4.67 (d, *J*=6.0 Hz, 2 H) 6.58 (d, *J*=5.95 Hz, 1 H) 6.82 (t, *J*=6.0 Hz, 1 H) 7.31 - 7.40 (m, 4 H) 7.60 (d, *J*=6.0 Hz, 1 H). m/z (ES+APCI)⁺: 273 /275 [M+H]⁺

### Example 2

### (3-Methyl-1H-pyrazofo[4,3-c]pyridin-4-yl)-(2-pyridin-2-yl-ethyl)-amine

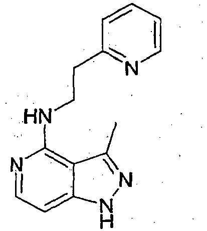

Example 2 was prepared analogously to Example 1 from Intermediate 3 and 2-Pyridin-2-yl-ethylamine to give the product (7 mg, 16%). ¹H NMR (400 MHz, DMSO-*d*₆) δ ppm 2.59 (s, 3 H), 3.12 (t, *J*=7.1 Hz, 2 H), 3.78 - 3.85 (m, 2 H), 6.52 (t, *J*=5.5 Hz, 1 H), 6.61 (d, *J*=6.0 Hz, 1 H), 7.26 - 7.30 (m, 1 H), 7.35 (d, *J*=7.8 Hz, 1 H), 7.72 (d, *J*=6.0 Hz, 1 H), 7.77 (m, 1 H), 8.56 (d, *J*=4.1 Hz, 1 H). m/z (ES+APCI)⁺: 254 [M + H]⁺.

### Example 3

### Cyclohexyl-(3-methyl-1H-pyrazolo[4,3-c]pyridin-4-yl)-amine

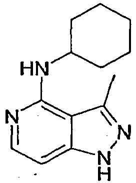

Example 3 was prepared analogously to Example 1 from Intermediate 3 and cyclohexylamine to give the product (2.5 mg, 5%). ¹H NMR (400 MHz, DMSO-*d*₆) δ ppm 1.17 - 1.47 (m, 6 H), 1.62 - 1.70 (m, 1 H), 1.71 - 1.83 (m, 2 H), 1.95 - 2.05 (m, 2 H), 2.60 (s, 3 H), 4.01 - 4.11 (m, 1 H), 5.53 (d, *J*=7.8 Hz, 1 H), 6.59 (d, *J*=6.0 Hz, 1 H), 7.69 (d, *J*=6.0 Hz, 1 H). m/z (ES+APCI)⁺: 231 [M + H]⁺.

### Example 4

### (3-Methyl-1H-pyrazolo[4,3-c]pyridin-4-yl)-(tetrahydro-pyran-4-yl)-amine

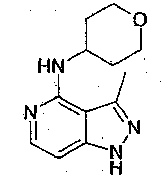

Example 4 was prepared analogously to Example 1 from Intermediate 3 and tetrahydropyran-4-ylamine to give the desired product as an off-white solid (27 mg, 64%). ¹H NMR (400 MHz, DMSO-*d*₆) δ ppm 1.57 - 1.71 (m, 2 H), 1.86 - 1.95 (m, 2 H), 2.59 (s, 3 H), 3.37 - 3.46 (m, 2 H), 3.84 - 3.93 (m, 2 H), 4.19 - 4.30 (m, 1 H), 5.62 - 5.69 (m, 1 H), 6.58 (d, *J*=6.0 Hz, 1 H), 7.66 (d, *J*=6.0 Hz, 1 H). m/z (ES+APCI)⁺: 233 [M+H]⁺

### Examples 5-46

Examples 5-46 in the table below were prepared analogously to Example 1 from Intermediate 3 and the corresponding amine:

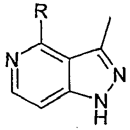

| **Example** | **R group** | **Name** | **m/z (ES+APCI)⁺** | **HPLC retention time (min)** |
|---|---|---|---|---|
| **5** | 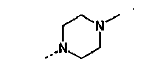 | *3-Methyl-4-(4-methyl-piperazin-1-yl)-1H-pyrazolo[4,3-c]pyridine* | 232 | 1.05 ^{c} |
| **6** | 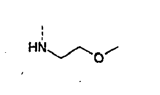 | *(2-Methoxy-ethyl)-(3-methyl-1H-pyrazolo[4,3-c]pyridin-4-yl)-amine* | 207 | 0.96 ^{c} |
| **7** | 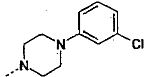 | *4-(4-(3-Chloro-phenyl)-piperazin-1-yl]-3-methyl-1H-pyrazolo[4,3-c]pyridine* | 328 | 1.91 ^{c} |
| **8** | 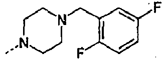 | *4-[4-(2,5-Difluoro-benzyl)-piperazin-1-yl]-3-methyl-1H-pyrazoio[4,3-c]pyridine* | 344 | 1.67 ^{c} |
| **9** | 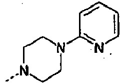 | *3-Methyl-4-(4-pyridin-2-yl-piperazin-1-y*/*)-1H-pyrazolo[4,3-c]pyridine* | 295 | 1.43 ^{c} |
| **10** | 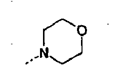 | *3-Methyl-4-morpholin-4-yl-1H-pyrazolo[4,3-c]pyridine* | 219 | 1.02 ^{c} |
| **11** | 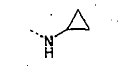 | *Cyclopropyl-(3-methyl-1H-pyrazolo[4,3-c]pyridin-4-yl)-amine* | 189 | 1.00 ^{c} |
| **12** | 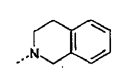 | *2-(3-Methyl-1H-pyrazolo[4,3-c]pyridin-4-yl)-1,2,3,4-tetrahydro-isoquinoline* | 265 | 1.71 ^{c} |
| **13** | 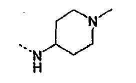 | *(1-Methyl-pipetidin-4-yl)-(3-methyl-1H-pyrazolo[4,3-c]pyridin-4-yl)-amine* | 246 | 2.34^{a} |
| **14** | 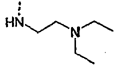 | *N,N-Diethyl-N'-(3-methyl-1H-pyrazolo[4,3-c]pyridin-4-yl)-ethane-1,2-diamine* | 248 | 2.05^{a} |
| **15** | 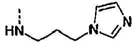 | *(3-Imidazol-1-yl-propyl)-(3-methyl-1H-pyrazolo[4,3-c]pyridin-4-yl)-amine* | 257 | 0.42^{b} |
| **16** | 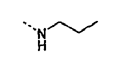 | *(3-Methyl-1H-pyrazolo[4,3-c]pyridin-4-yl)-propyl-amine* | 191 | 0.47 ^{b} |
| **17** | 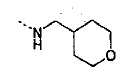 | *(3-Methyl-1H-pyrazolo[4,3-c]pyridin-4-yl)-(tetrahydro-pyran-4-ylmethyl)-amine* | 247 | 0.47 ^{b} |
| **18** | 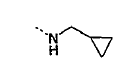 | *Cyclopropylmethyl-(3-methyl-1H-pyrazolo[4,3-c]pyridin-4-yl)-amine* | 203 | 2.77 ^{b} |
| **19** | 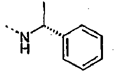 | *(3-Methyl-1H-pyrazolo[4,3-c]pyridin-4-yl)-((R)-1-phenyl-ethyl)-amine* | 253 | 1.72 ^{b} |
| **20** | 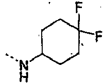 | *(4,4-Difluoro-cyclohexyl)-(3-methyl-1H-pyrazolo[4,3-c]pyridin-4-yl)-amine* | 267 | 1.87 ^{b} |
| **21** | 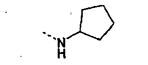 | *Cyclopentyl-(3-methyl-1H-pyrazolo[4,3-c]pyridin-4-yl)-amine* | 217 | 1.85 ^{b} |
| **22** | 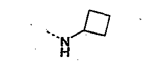 | *Cyclobutyl-(3-inethyl-1H-pyrazolo[4,3-c]pyridin-4-y*/*)-amine* | 203 | 1.67 ^{b} |
| **23** | 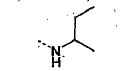 | *sec-Butyl-(3-methyl-1H-pyrazolo[4,3-c]pyridin-4-yl)-amine,* | 205 | 1.71 ^{b} |
| **24** | 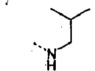 | *Isobutyl*-(*3*-*methyl*-*1H-pyrazolo[4,3-c]pyridin-4-yl)-amine* | 205 | 1.83 ^{b} |
| **25** | 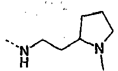 | *(3-Methyl-1H-pyrazolo[4,3-c]pyridin-4-yl)-[2-(1-methyl pyrrolidin-2-yl)-ethyl] amine* | 260 | 2.94^{a} |
| **26** | 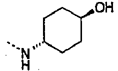 | *Trans-4-(3-Methyl-1H-pyrazolo[4,3-c]pyridin-4-ylamino)-cyclohexanol* | 247 | 1.49 ^{b} |
| **27** | 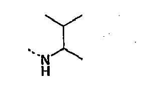 | *(1,2-Dimethyl-propyl)-(3-methyl-1H-pyrazolo[4,3-c]pyridin-4-yl)-amine* | 219 | 0.99^{d} |
| **28** | 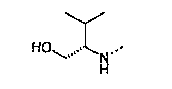 | *(S)-3-Methyl-2-(3-methyl-1H-pyrazolo[4,3-c]pyridin-4-ylamino)-butan-1-ol* | 235 | 1.75 ^{b} |
| **29** | 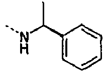 | *(3-Methyl-1H-pyrazolo[4,3-c]pyridin-4-yl)-((S)-1-phenylethyl)-amine* | 253 | 1.12^{d} |
| **30** | 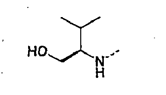 | *(R)-3-Methyl-2-(3-methyl-1H-pyrazolo[4,3-c]pyridin-4-ylamino)-butan-1-ol* | 235 | 1.65 ^{b} |
| **31** | 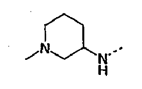 | *(1-Methyl-piperidin-3-yl)-(3-methyl-1H-pyrazolo[4,3-c]pyridin-4-yl)-amine* | 246 | 1.32^{c} |
| **32** | 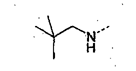 | *(2,2-Dimethyl-propyl)-(3-methyl-1H-pyrazolo[4,3-c]pyridin-4-yl)-amine* | 219 | 1.56^{c} |
| **33** | 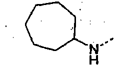 | *Cycloheptyl-(3-methyl-1H-pyrazolo[4,3-c]pyridin-4yl)-amine* | 245 | 1.76^{c} |
| **34** | 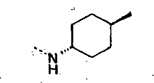 | *Trans-(4-Methylcyclohexyl)-(3-methyl-1H-pyrazolo[4,3-c]pyridin-4-yl)-amine* | 245 | 1.95^{c} |
| **35** | 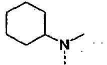 | *Cyclohexyl-methyl-(3-methyl-1H-pyrazolo[4,3-c]pyridin-4-yl)-amine* | 245 | 1.71 ^{c} |
| **36** | 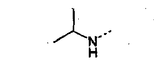 | *Isopropyl-(3-methyl-1H-pyrazolo[4,3-c]pyridin-4-yl)-amine* | 191 | 1.18^{c} |
| **37** | 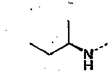 | *((R)-sec-Butyl)-(3-methyl-1H-pyrazolo[4,3-c]pyridin-4-yl)-amine* | 205 | 1.37^{c} |
| **38** | 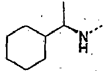 | *((R)-1-Cyclohexylethyl)-(3-methyl-1H-pyrazolo[4,3-c]pyridin-4-yl)-amine* | 259 | 1.85^{c} |
| **39** | 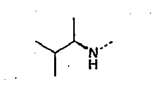 | *((R)-1,2-Dimethyl propyl)-(3-methyl-1H-pyrazolo[4,3-c]pyridin-4-yl)-amine* | 219 | 1.13^{d} |
| **40** | 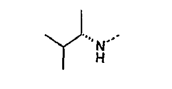 | *((S)-1,2-Dimethyl-propyl)-(3-methyl-1H-pyrazolo[4,3-c]pyridin-4-yl)-amine* | 219 | 1.51^{c} |
| **41** | 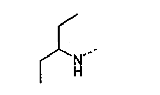 | *(1-Ethyl-propyl)-(3-methyl-1H-pyrazolo[4,3-c]pyridin-4-yl)-amine* | 219 | 1.12^{d} |
| **42** | 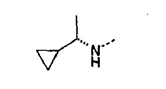 | *((S)-1-Cyclopropyl ethyl)-(3-methyl-1H-pyrazolo[4,3-c]pyridin-4-yl)-amine* | 217 | 1.42^{c} |
| **43** | 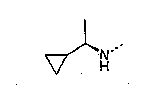 | *((R)-1-Cyclopropyl-ethyl)-(3-methyl-1H-pyrazolo[4,3-c]pyridin-4-yl)-amine* | 217 | 1.41^{c} |
| **44** | 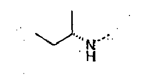 | *((S)-sec-Butyl)-(3-methyl-1H-pyrazolo[4,3-c]pyridin-4-yl)-amine* | 205 | 1.36^{c} |
| **45** | 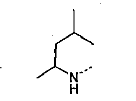 | *(1,3-Dimethyl-butyl)-(3-methyl-1H-pyrazolo[4,3-c]pyridin-4-yl)-amine* | 233 | 2.4^{c} |
| **46** | 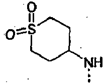 | *(1,1-Dioxo-hexahydro-1λ⁶-thiopyran-4-yl)-(3-methyl-1H-pyrazolo[4,3-c]pyridin-4-yl)-amine* | 281 | 1.02^{c} |

| | | | | |
|---|---|---|---|---|
| ^{a} HPLC column: 4.6x50mm (5µm) C-18 Xbridge; flow rate: 2ml/min; Run time: 4.6 min: Solvent A: 0.1% Ammonium Hydroxide in water, Solvent B: Methanol; Gradient - 10-100%B; Gradient time: 3.5min. ^{b} HPLC column: 4.6x50mm (5µm) C-18 Xbridge; flow rate: 2ml/min; Run time: 4.6 min: Solvent A: 0.1% Formic acid in water, Solvent B: Methanol; Gradient - 10-100%B; Gradient time: 3.5min. ^{c} HPLC column: 4.6x50mm (5µm) C-18 Xbridge; flow rate: 3ml/min; Run time: 3.2 min: Solvent A: 0.1% Ammonium Hydroxide in water, Solvent B: Acetonitrile; Gradient - 10-100%B; Gradient time: 2.35min. ^{d} HPLC column: 4.6x50mm (5µm) C-18 Xbridge; flow rate: 3ml/min; Run time: 3.2 min: Solvent A: 0.1% Formic acid in water, Solvent B: Acetonitrile; Gradient - 10-100%B; Gradient time: 2.35min. | | | | |

### Example 47

### 3-Methyl4-(1-methyl-1H-pyrazol-4-yl)-1H-pyrazolo[4,3-c]pyridine

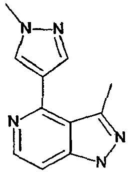

Intermediate 3 (50 mg, 0.298 mmol), 1-methyl-4-(4,4,5,5-tetramethyl-1,3,2-dioxaborolan-2-yl)-1H-pyrazole (93 mg, 0.446 mmol), palladium diphenylphosphinpferrocene dichloride (12.2 mg, 0.015 mmol), 2M aqueous sodium carbonate solution (521 □l, 1.04 mmol) and 1,4-dioxane (2 ml) were placed in a sealed microwave reactor vial. The vial was degassed and place under an atmosphere of nitrogen. The vial was irradiated at 160°C in a Biotage I-60 microwave reactor for 20 minutes. On cooling the reaction mixture was concentrated to dryness. The residue was dissolved in MeOH and filtered through a plug of Celite. The filtrate was concentrated and the residue dissolved in DMSO (1.2 ml) prior to purification. by mass-triggered preparative HPLC. A white solid was obtained (21 mg, 33%). ¹H NMR (400 MHz, DMSO-*d*₆) δ ppm 3.32 (s, 3 H) 3.94 (s, 3 H) 7.31 (d, *J*=5.5 Hz, 1 H) 7.84 (s, 1 H) 8.17 (s, 1 H) 8.23 (d, *J*=6.0 Hz, 1 H). m/z (ES+APCI)⁺ : 214 [M+H]⁺

### Example 48

### 4-Furan-2-yl-3-methyl-1H-pyrazolo[4,3-c]pyridine

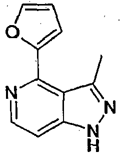

Example 48 was prepared analogously to Example 47 from Intermediate 3 and furan-2-boronic acid to give the product (34 mg, 58%). ¹H NMR (400 MHz, DMSO-*d*₆) δ ppm 2.37 (s, 3 H), 6.43 - 6.70 (m, 1 H), 7.04 (d, *J*=3.2 Hz, 1 H), 7.26 (d, *J*=6.0 Hz, 1 H), 7.84 (s, 1 H), 8.15 (d, *J*=6.0 Hz, 1 H). m/z (ES+APCI)⁺: 200 [M + H]⁺.

### Examples 49-58

Examples 49-58 in following table were prepared analogously to Example 47 from Intermediate 3 and the corresponding boronic acid or boronic ester:

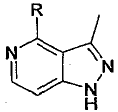

| **Example** | **R group** | **Name** | **m/z (ES+APCI)+** | **HPLC retention time (min)** |
|---|---|---|---|---|
| **49** | 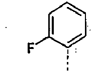 | *4-(2-Fluoro-phenyl)-3-methyl-1H-pyrazolo[4,3-c]pyridine* | 228 | 2.97^{a} |
| **50** | 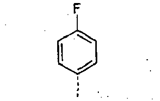 | *4-(4-Fluoro-phenyl)-3-methyl-1H-pyrazolo[4,3-c]pyridine* | 228 | 3.12^{a} |
| **51** | 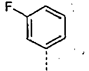 | *4-(3-Fluoro-phenyl)-3-methyl-1H-pyrazolo[4,3-c]pyridine* | 228 | 3.15^{a} |
| **52** | 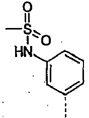 | *N-[3-(3-Methyl-1H-pyrazolo[4,3-c]pyridin-4-yl)-phenyl] methanesulfonamide* | 303 | 2.62 ^{a} |
| **53** | 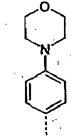 | *3-Methyl-4-(4-morpholin-4-yl-phenyl)-1H-pyrazolo[4,3-c]pyridine* | 295 | 2.97 ^{a} |
| **54** | 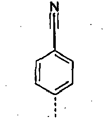 | *4-(3-Methyl-1H-pyrazolo[4,3-c]pyridin-4-yl)-benzonitrile* | 235 | 2.83 ^{a} |
| **55** | 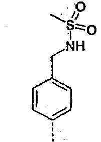 | *N-(4-(3-Methyl-1H-pyrazolo[4, 3-c]p yridin-4-yl)-benzyl]-methanesulfonamide* | 317 | 2.53 ^{a} |
| **56** | 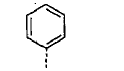 | *3-Methyl-4-phenyl-1H-pyrazolo[4,3-c]pyridine* | 210 | 3.07 ^{a} |
| **57** | 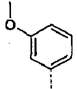 | *4-(3-Methoxy-phenyl)-3-methyl-1H-pyrazolo[4,3-c]pyridine* | 240 | 3.12 ^{a} |
| **58** | 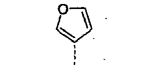 | *4-Furan-3-yl-3-methyl 1H-pyrazolo[4,3-c]pyridine* | 200 | 0.94^{c} |

| | | | | |
|---|---|---|---|---|
| ^{a} HPLC column: 4.6x50mm (5µm) C-18 Xbridge; flow rate: 2ml/min; Run time: 4.6 min: Solvent A: 0.1% Ammonium Hydroxide in water, Solvent B: Methanol; Gradient - 10-100%B; Gradient time: 3.5min. ^{c} HPLC column: 4.6x50mm (5µm) C-18 Xbridge; flow rate: 3ml/min; Run time: 3.2 min: Solvent A: 0.1% Ammonium Hydroxide in water, Solvent B: Acetonitrile; Gradient - 10-100%B; Gradient time: 2.35min. | | | | |

### Example 59

### (3-Chloro-phenyl)-(3-methyl-1H-pyrazolo[4,3-c]pyridin-4-yl)-amine

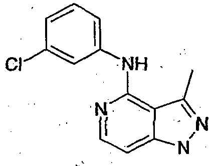

Intermediate 3 (40 mg, 0.24 mmol), 3-chloro-phenylamine (61 mg, 0.48 mmol) and concentrated hydrochloric acid (21 µl, 0.72 mmol), were dissolved in n-butanol (1.1 ml). The reaction mixture was irradiated at 190 °C for 45 minutes in a Biotage I-60 microwave reactor. The mixture was evaporated then purified by preparative LCMS (high pH buffer) to give the desired product as a white solid (49 mg, 79%). ¹H NMR (400 MHz, DMSO-*d*₆) δ ppm 2.72 (s, 3 H), 6.93 (d, *J*=6.0 Hz, 1 H), 6.96 - 7.00 (m, 1 H), 7.27 - 7.34 (m, 1 H), 7.61 - 7.68 (m, 1 H), 7.85 (d, *J*=6.0 Hz, 1 H), 7.92 - 7.96 (m, 1 H), 8.21 (br. s., 1 H). m/z (ES+APCI)⁺: 259 / 261 [M+H]⁺.

### Example 60

### 4-[5-(3-Methyl-1H-pyrazolo[4,3-c]pyridin-4-ylamino)-pyridin-3-yl]-benzonitrile

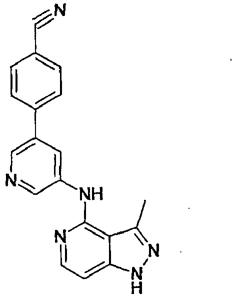

Example 60 was prepared analogously to Example 59 from Intermediate 3 and 4-(5-Amino-pyridin-3-yl)-benzonitrile to give the desired product as a white solid (4 mg, 4%). ¹H NMR (400 MHz, DMSO-*d*₆) δ ppm 2.81 (s, 3 H), 6.99 (d, *J*=6.0 Hz, 1 H), 7.88 (d, *J*=6.0 Hz, 1 H), 7.98 (m, 2 H), 8.04 (m, 2 H), 8.37 (s, 1 H), 8.53 (t, *J*=2.3 Hz, 1 H), 8.60 (d, *J*=2.3 Hz, 1 H), 9.07 (d, *J*=2.3 Hz, 1 H). m/z (ES+APCI)⁺: 327 [M + H]⁺.

### Example 61

### (3-Methyl-1H-pyrazolo[4,3-c]pyridin-4-yl)-(1-methyl-1H-pyrazol-3-yl)-amine

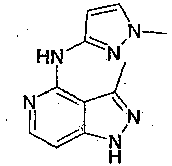

Example 61 was prepared analogously to Example 59 from Intermediate 3 and 1-methyl-1*H*-pyrazol-3-ylamine to give an off-white solid (16mg, 38%). ¹H NMR (400 MHz, METHANOL-*d₄*) δ ppm 2.74 (s, 3 H), 3.90 (s, 3 H), 6.76 (d, *J*=6.4 Hz, 1 H), 7.58 - 7.64 (m, 1 H), 7.74 (d, *J*=6.0 Hz, 1 H), 7.91 - 7.98 (m, 1 H). m/z (ES+APCI)⁺: 229 [M+H]⁺

### Example 62

### (2-Fluoro-phenyl)-(3-methyl-1H-pyrazolo[4,3-c]pyridin-4-yl)-amine

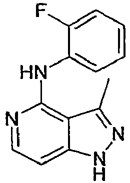

Example 62 was prepared analogously to Example 59 from Intermediate 3 and 2-fluoro-phenylamine to give a white solid (37mg, 64%). ¹H NMR (400 MHz, DMSO-*d*₆) δ ppm 2.70 (s, 3 H), 6.87 (d, *J*=6.0 Hz, 1 H), 7.05 - 7.12 (m, 1 H), 7.14 - 7.28 (m, 2 H), 7.76 (d, *J*=6.0 Hz, 1 H), 7.87 (br. s., 1 H), 8.04 - 8.10 (m, 1 H). m/z (ES+APCI)⁺: 243 [M+H]⁺

### Examples 63-91

Examples 63-91 in the following table were prepared analogously to Example 59 from Intermediate 3 and the corresponding amine:

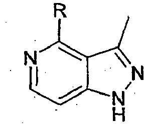

| **Example** | **R group** | **Name** | **m/z (ES+APCI)⁺** | **HPLC retention time (min)** |
|---|---|---|---|---|
| **63** | 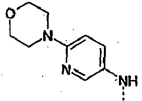 | *(3-Methyl-1H-pyrazolo[4,3-c]pyridin-4-yl)-(6-morpholin-4-yl pyridin-3-yl)-amine* | 311 | 1.5^{e} |
| **64** | 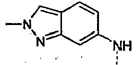 | *(2-Methyl-2H-indazol-6-yl)-(3-methyl-1H-pyrazolo[4,3-c]pyridin-4-yl)-amine* | 279 | 2.7^{a} |
| **65** | 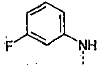 | *(3-Fluoro-phenyl)-(3-methyl-1H-pyrazolo[4,3-c]pyridin-4-yl)-amine* | 243 | 1.92^{e} |
| **66** | 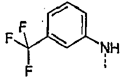 | *(3-Methyl-1H-pyrazolo[4,3-c]pyridin-4-yl*)-(*3*-*trifluoromethylphenyl)-amine* | 293 | 3.62^{a} |
| **67** | 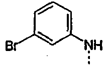 | *(3-Bromo-phenyl)-(3-methyl-1H-pyrazolo[4,3-c]pyridin-4-yl)-amine* | 303 / 305 | 3.60^{a} |
| **68** | 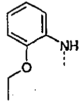 | *(2-Ethoxy-phenyl)-(3-methyl-1H-pyrazolo[4,3-c]pyridin-4-yl)-amine* | 269 | 3.73^{a} |
| **69** | 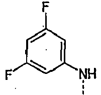 | *(3,5-Difluoro-phenyl)-(3-methyl-1H-pyrazolo[4,3-c]pyridin-4-yl)-amine* | 261 | 3.53^{a} |
| **70** | 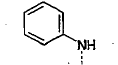 | *(3-Methyl-1H-pyrazolo[4,3-c]pyridin-4-yl)-phenyl-amine* | 225 | 3.13^{a} |
| **71** | 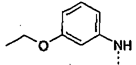 | *(3-Ethoxy-phenyl)-(3-methyl-1H-pyrazolo[4,3-c]pycidin-4-yl)-amine* | 269 | 1.08^{d} |
| **72** | 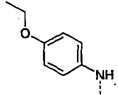 | *(4-Ethoxy-phenyl)-(3-methyl-1H-pyrazolo[4,3-c]pyridin-4-yl)-amine* | 269 | 1.07^{d} |
| **73** | 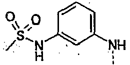 | *N-[3-(3-Methyl-1H-pyrazolo[4,3-c]pyridin-4-ylamino)-phenyl]-methanesulfonamide* | 318 | 0.88^{d} |
| **74** | 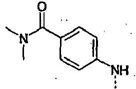 | *N,N-Dimethyl-4-(3-methyl-1H-pyrazolo[4,3-c]pyridin-4-ylamino)-benzamide* | 296 | 0.87^{d} |
| **75** | 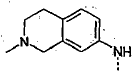 | *(3-Methyl-1H-pyrazolo[4,3-c]pyridin-4-yl)-(2-methyl-1,2,3,4-tetrahydro-isoquinolin-7-yl)-amine* | 294 | 0.44^{d} |
| **76** | 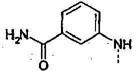 | *3-(3-Methyl-1H-pyrazolo[4,3-c]pyridin-4-ylamino)-benzamide* | 268 | 0.6^{d} |
| **77** | 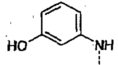 | *3-(3-Methyl-1H-pyrazolo[4,3-c]pyridin-4-ylamino)-phenol* | 241 | 0.98^{c} |
| **78** | 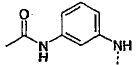 | *N-[3-(3-Methyl-1H-pyrazolo[4,3-c]pyridin-4-ylamino)-phenyl]-acetamide* | 282 | 1.24^{c} |
| **79** | 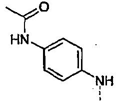 | *N-[4-(3-Methyl-1H-pyrazolo[4,3-c]pyridin-4-ylamino)-phenyl]-acetamide* | 282 | 0.79^{d} |
| **80** | 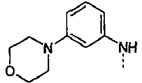 | *(3-Methyl-1H-pyrazolo[4,3-c]pyridin-4-yl)-(3-morpholin-4-yl phenyl)-amine* | 310 | 1.8^{b} |
| **81** | 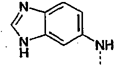 | *(3H-Benzimidazol-5-yl)-(3-methyl-1H-pyrazolo*[*4*,*3*-*c*]*pyridin*-*4-yl)-amine* | 265 | 1.12^{c} |
| **82** | 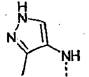 | *(3-Methyl-1H-pyrazolo[4,3-c]pyridin-4-yl)-(3-methyl-1H-pyrazol-4-yl)-amine* | 229 | 091^{c} |
| **83** | 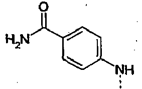 | *4-(3-Methyl-1H-pyrazolo[4,3-c]pyridin-4-ylamino)-benzamide* | 268 | 1.07^{c} |
| **84** | 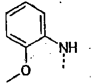 | *(2-Methoxy-phenyl)-(3-methyl-1H-pyrazolo[4,3-c]pyridin-4-yl)-amine* | 255 | 1.77^{c} |
| **85** | 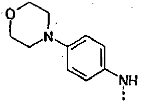 | *(3-Methyl-1H-pyrazolo[4,3-c]pyridin-4-yl)-(4-morpholin-4-yl-phenyl)-amine* | 310 | 1.34^{c} |
| **86** | 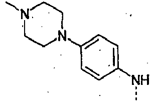 | *[4-(4-Methyl-piperazin-1-yl)-phenyl]-(3-methyl-1H-pyrazolo[4,3-c]pyridin-4-yl)-amine* | 323 | 1.27^{c} |
| **87** | 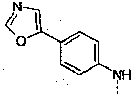 | *(3-Methyl-1H-pyrazolo[4,3-c]pyridin-4-yl)-(4-oxazol-5-yl-phenyl)-amine* | 292 | 1.43^{c} |
| **88** | 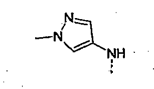 | *(3-Methyl-1H-pyrazolo[4,3-c]pyridin-4-yl)-(1-methyl-1H-pyrazol-4-yl)-amine* | 229 | 1.04^{c} |
| **89** | 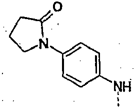 | *1-[4-(3-Methyl-1H-pyrazolo[4,3-c]pyridin-4-ylamino)-phenyl]-pyrrolidin-2-one* | 308 | 1.31^{c} |
| **90** | 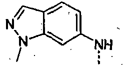 | *(1-Methy*/*-1H-indazol-6-yl)-(3-methyl-1H-pyrazolo[4,3-c]pyridin-4-yl)-amine* | 279 | 1.52^{c} |
| **91** | 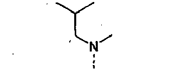 | *Isobutyl-methyl-(3-methyl-1H-pyrazolo[4,3-c]pyridin-4-yl)-amine* | 219 | 1.61^{c} |

| | | | | |
|---|---|---|---|---|
| ^{a} HPLC column: 4.6x50mm (5µm) C-18 Xbridge; flow rate: 2ml/min; Run time: 4.6 min: Solvent A: 0.1% Ammonium Hydroxide in water, Solvent B: Methanol; Gradient - 10-100%B; Gradient time: 3.5min. ^{b} HPLC column: 4.6x50mm (5µm) C-18 Xbridge; flow rate: 2ml/min; Run time: 4.6 min: Solvent A: 0.1% Formic acid in water, Solvent B: Methanol; Gradient 10-100%B; Gradient time: 3.5min. ^{c} HPLC column: 4.6x50mm (5µm) C-18 Xbridge; flow rate: 3ml/min; Run time: 3.2 min: Solvent A: 0.1% Ammonium Hydroxide in water, Solvent B: Acetonitrile; Gradient - 10-100%B; Gradient time: 2.35min. ^{d} HPLC column: 4.6x50mm (5µm) C-18 Xbridge; flow rate: 3ml/min; Run time: 3.2 min: Solvent A: 0.1% Formic acid in water, Solvent B: Acetonitrile; Gradient - 10-100%B; Gradient time: 2.35min. ^{e} HPLC column: 4.6x50mm (5µm) C-18 Xbridge; flow rate: 2ml/min; Run time: 4.6 min: Solvent A: 0.1 % Trifluoroacetic acid in water, Solvent B: Methanol; Gradient - 10-100%B; Gradient time: 3.5min. | | | | |

### Example 92

### N,N-Dimethyl-N'-(3-methyl-1H-pyrazolo[4,3-c]pyridin-4-yl)-benzene-1,3-diamine

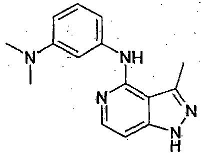

Intermediate 3 (40 mg, 0.24 mmol), N,N-dimethyl-benzene-1,3-diamine (65 mg, 0.48 mmol) and concentrated hydrochloric acid (21 µl, 0.72 mmol), were dissolved in n-butanol (1.1 ml). The reaction mixture was irradiated at 190°C for 45 minutes in a Biotage I-60 microwave reactor. The mixture was evaporated, purified by preparative LCMS (low pH buffer) then eluted through a 0.5gram lsolute-NH₂ cartridge with 9:1 DCM: methanol to give the free base as a white solid (15 mg, 23%). ¹H NMR (400 MHz, DMSO-*d*₆) δ ppm 2.71 (s, 3 H), 2.89 (s, 6 H), 6.35 - 6.40 (m, 1 H), 6.84 (d, *J*=6.0 Hz, 1 H), 7.05 - 7.13 (m, 3 H), 7.78 (d, *J*=6.0 Hz, 1 H), 7.83 (br. s., 1 H). m/z (ES+APCI)⁺: 268 [M+H]⁺

### Examples 93-95

Examples 93-95 in the following table were prepared analogously to Example 92 from Intermediate 3 and the corresponding amine.

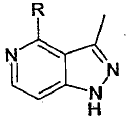

| **Example** | **R group** | **Name** | **m/z (ES+APCI)⁺** | **HPLC retention time (min)** |
|---|---|---|---|---|
| **93** | 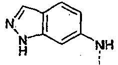 | *(1H-Indazol-6-yl)-(3-methyl-1H-pyrazolo[4,3-c]pyridin-4-yl)-amine* | 265 | 1.3^{c} |
| **94** | 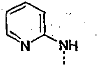 | *(3-Methyl-1H-pyrazolo[4,3-c]pyridin-4-yl)-pyridin-2-yl-amine* | 226 | 1.03^{d} |
| **95** | 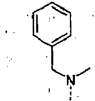 | *Benzyl-methyl-(3-methyl-1H-pyrazolo[4,3-c]pyridin-4*-*yl*)-*amine* | 253 | 1.42^{d} |

| | | | | |
|---|---|---|---|---|
| ^{c} HPLC column: 4.6x50mm (5µm) C-18 Xbridge; flow rate: 3ml/min; Run time: 3.2 min: Solvent A: 0.1% Ammonium Hydroxide in water, Solvent B: Acetonitrile; Gradient - 10-100%B; Gradient time: 2.35min. ^{d} HPLC column: 4.6x50mm (5µm) C-18 Xbridge; flow rate: 3ml/min; Run time: 3.2 min: Solvent A: 0.1% Formic acid in water, Solvent B: Acetonitrile; Gradient - 10-100%B; Gradient time: 2.35min. | | | | |

### Example 96

### (3-Methyl-1H-pyrazolo[4,3-c]pyridin-4-yl)-(4-1,2,4-triazol-1-yl-phenyl)-amine hydrochloride salt.

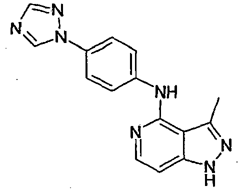

Intermediate 3 (50 mg, 0.3 mmol), 4-1,2,4-triazol-1-yl-phenylamine (95 mg, 0.60 mmol) and concentrated hydrochloric acid (27 µl, 0.39 mmol), were dissolved in n-butanol (1.1 ml). The reaction mixture was irradiated at 190°C for 45 minutes in a Biotage I-60 microwave reactor. The mixture was evaporated, dissolved in DMSO (1 ml), filtered, washed with water and dried to give the desired product as a pale green solid (46 mg, 47%). ¹H NMR (400 MHz, 60°C, DMSO-*d*₆) δ ppm 2.75 (s, 3 H), 7.14 (d, *J*=7.3 Hz, 1 H), 7.56 (d, *J*=6.9 Hz, 1 H), 7.73 (d, *J*=9.2 Hz, 2 H), 8.02 (d, *J*=9.2 Hz, 2 H), 8.25 (s, 1 H), 9.32 (s, 1 H), 9.99 (s, 1 H). m/z (ES+APCI)⁺: 292 [M+H]⁺

### Example 97

### (2,4-Dimethoxy-benzyl)-(3-methyl-1H-pyrazolo[4,3-c]pyridin-4-yl)-amine

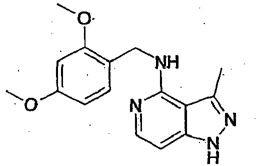

Intermediate 3 (200mg, 1.20 mmol), 2,4-dimethoxybenzylamine (0.72 ml, 4.79 mmol) and n-butanol (2.5 ml) were combined and irradiated at 190°C for 30 minutes in a Biotage I-60 microwave reactor. The reaction mixture was evaporated, the residue was partitioned between DCM (20 ml) and water (20 ml). The aqueous layer was extracted with DCM (20 ml) and then the combined organic layers were washed with brine (20 ml), dried (MgSO₄) and evaporated. The crude product was concentrated onto silica and purified by flash chromatography on the Biotage SP4, eluting with 0 to 100% EtOAc/petroleum ether to give the desired product as a pale yellow oil (303mg, 85%). ¹H NMR (400 MHz, DMSO-*d*₆) δ ppm 2.61 (s, 3 H), 3.71 (s, 3 H), 3.84 (s, 3 H), 4.57 (d, *J*=6.0 Hz, 2 H), 6.37 - 6.46 (m, 2 H), 6.52 - 6.59 (m, 2 H), 7.04 - 7.12 (m, 1 H), 7.60 (d, *J*=6.0 Hz, 1 H). m/z (ES+APCI)⁺: 299 [M+H]⁺

### Example 98

### (1-Benzyl-piperidin-4-yl)-(3-methyl-1H-pyrazolo[4,3-c]pyridin-4-yl)-amine

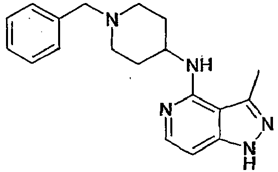

Intermediate 3 (200 mg, 1.20 mmol), 4-amino-1-benzylpiperidine (0.98 ml, 4.79 mmol) and n-butanol (2.5 ml) were combined and irradiated at 190 °C for 1.5 h in a Biotage I-60 microwave reactor. The reaction mixture was evaporated and the crude product was purified by flash chromatography on the Biotage SP4, eluting with 12 to 100% EtOAc/petroleum ether. Further purification by cation exchange chromatography using an Isolute SCX cartridge gave an off-white foam (153 mg, 40%). ¹H NMR (400 MHz, DMSO-*d*₆) δ ppm 1.58 - 1.71 (m, 2 H), 1.88 - 1:96 (m, 2 H), 2.03 - 2.12 (m, 2 H), 2.57 (s, 3 H), 2.76 - 2.84 (m, 2 H), 3.47 (s, 2 H), 3.98 - 4.10 (m, 1 H), 5.52 - 5.59 (m, 1 H), 6:56 (d, *J*=6.0 Hz, 1 H), 7.22 - 7.35 (m, 5 H), 7.64 (d, *J*=6.0 Hz, 1 H). m/z (ES+APCI)⁺: 322 [M+H]⁺

### Example 99

### (1-Methyl-1H-indazol-5-yl)-(3-methyl-1H-pyrazolo[4,3-c]pyridin-4-yl)-amine

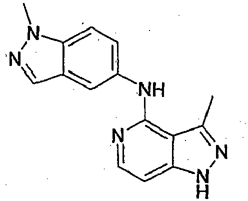

Intermediate 3 (40 mg, 0.24 mmol), 1-methyl-1H-indazol-5-amine (140mg, 0.95 mmol) and concentrated hydrochloric acid (21 µl, 0.72 mmol) were dissolved in n-butanol (1 ml). The reaction mixture was irradiated at 190°C for 1 h in a Biotage I-60 microwave reactor. The mixture was evaporated and then purified by preparative LCMS (high pH buffer). Further purification by cation exchange chromatography using an Isolute SCX cartridge eluting with MeOH then 2M NH₃ in MeOH, followed by anion exchange chromatography using an Isolute-NH₂ cartridge eluting with 9:1 DCM: MeOH gave an off-white solid (28 mg, 42%). ¹H NMR (400 MHz, DMSO-*d*₆) δ ppm 2.73 (s, 3 H), 4.03 (s, 3 H), 6.82 (d, *J*=6.0 Hz, 1 H), 7.54 - 7.62 (m, 2 H), 7.76 (d, *J*=6.0 Hz, 1 H), 7.96 - 7.98 (m, 2 H), 8.17 (dd, *J*=1.8, 0.9 Hz, 1 H). m/z (ES+APCI)⁺: 279 [M+H]⁺

### Example 100

### 3-Methyl-1H-pyrazolo[4,3-c]pyridine-4-carboxylic acid cyclohexylamide

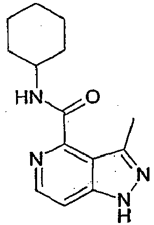

### Step 1

To a solution of cyclohexylamine (18 µl, 0.16 mmol) in DMF (2 ml) was added Intermediate 6 (70 mg, 0.24 mmol), HATU (96 mg, 0.25 mmol) and diisopropylethylamine (164 µl, 0.94 mmol), and the reaction was stirred at room temperature for 72 h. The mixture was evaporated, dissolved in a minimum amount of 10% MeOH in DCM and eluted through an Isolute-NH₂ cartridge. The filtrate was concentrated and used in the next step without purification.

### Step 2

The crude product from Step 1 (100 mg, 0.26 mmol) and trifluoroacetic acid (2 ml, 3.07 mmol) were combined and stirred at reflux for 18 h. The reaction mixture was evaporated and then purified by preparative LCMS (high pH buffer) to give the product as a white solid (28mg, 68%). ¹H NMR (400 MHz, DMSO-*d*₆) δ ppm 1.08 - 1.22 (m, 1 H), 1.26 - 1.42 (m, 4 H), 1.56 - 1.65 (m, 1 H), 1.68 - 1.78 (m, 2 H), 1.80 - 1.91 (m, 2 H), 2.63 (s, 3 H), 3.75 - 3.88 (m, 1 H), 7.57 (d, *J*=6.0 Hz, 1 H), 8.27 (d, *J*=6.0 Hz, 1 H), 8.46 - 8.53 (m, 1 H). m/z (ES+APCI)⁺: 259 [M+H]⁺

### Example 101

### 1-[4-(3-Methyl-1H-pyrazolo[4,3-c]pyridin-4-ylamino)-piperidin-1-yl]-ethanone

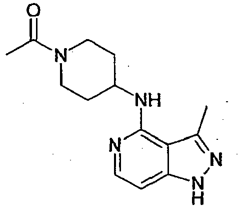

### Step 1

To a solution of Intermediate 8 (70 mg, 0.18 mmol) in DMF (2 ml) was added acetic acid (15 µl, 0.27 mmol), HATU (110 mg, 0.29 mmol) and diisopropylethylamine (188 µl, 1.08 mmol), and the reaction was stirred at room temperature for 18 h. The mixture was evaporated, dissolved in a minimum amount of 10% MeOH in DCM and eluted through an Isolute-NH₂ cartridge and the filtrate evaporated and used in the next step without further purification.

### Step 2

The crude product of Step 1 and trifluoroacetic acid (1 ml, 1.54 mmol) were combined and stirred at reflux for 18h. The reaction mixture was evaporated and then purified by preparative LCMS (high pH buffer) to give a white solid (24 mg, 48%). ¹H NMR (400 MHz, DMSO-*d*₆) δ ppm 1.40 - 1.51 (m, 1 H), 1.51 - 1.63 (m, 1 H), 1.89 - 2.03 (m, 5 H), 2.57 (s, 3 H), 2.66 - 2.75 (m, 1 H), 3.11 - 3.21 (m, 1 H), 3.79 - 3.86 (m, 1 H), 4.23 - 4.37 (m, 2 H), 5.62 - 5.68 (m, 1 H), 6.59 (d, *J*=6.0 Hz, 1 H), 7.66 (d, *J*=6.0 Hz, 1 H). m/z (ES+APCI)⁺: 274 [M+H]⁺

### Examples 102-103

### Examples 102-103 in the table below were prepared analogously to

### Example 101:

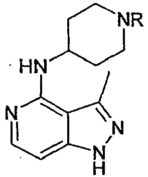

| **Example** | **R group** | **Name** | **m/z (ES+APCI)⁺** | **HPLC retention time (min)** |
|---|---|---|---|---|
| **102** | 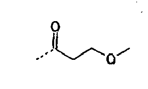 | *3-Methoxy-1-[4-(3-methyl-1H-pyrazolo[4,3-c]pyridin-4-ylamino)-piperidin-1-yl]-propan-1-one* | 318 | 1.04^{c} |
| **103** | 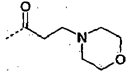 | *1-[4-(3-Methyl-1H-pyrazolo[4,3-c]pyridin-4-ylamino)-piperidin-1-yl]-3-morpholin-4-yl-propan-1-one* | 373 | 1.00^{c} |

| | | | | |
|---|---|---|---|---|
| ^{c} HPLC column: 4.6x50mm (5µm) C-18 Xbridge; flow rate: 3ml/min; Run time: 3.2 min: Solvent A: 0.1% Ammonium Hydroxide in water, Solvent B: Acetonitrile; Gradient - 10-100%B; Gradient time: 2.35min. | | | | |

### Example 104

### Cyclobutyl-[4-(3-methyl-1H-pyrazolo[4,3-c]pycidin-4-ylamino)-piperidin-1-yl]methanone

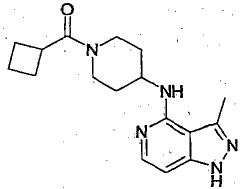

To a solution of Intermediate 8 (70 mg, 0.18 mmol) in DMF (2 ml) was added cyclobutyl acid (26 µl, 0.27 mmol), HATU (110 mg, 0.29 mmol) and diisopropylethylamine (188 µl, 1.08 mmol), and the mixture was stirred at room temperature for 18 h. The mixture was evaporated, dissolved in a minimum amount of 10% MeOH in DCM and eluted through an Isolute-NH₂ cartridge and the filtrate evaporated. The residue was combined with trifluoroacetic acid (1 ml) and stirred at reflux for 18h. The mixture was concentrated and the residue purified by preparative LCMS (low pH buffer). The product was then eluted through an Isolute-NH₂ cartridge with 10% MeOH in DCM to a white solid (20 mg, 35%). ¹H NMR (400 MHz, DMSO-*d*₆) δ ppm 1.37 - 1.56 (m, 2 H), 1.67 - 1.79 (m, 1 H), 1.83 - 1.99 (m, 3 H), 2.02 - 2.25 (m, 4 H), 2.57 (s, 3 H), 2.65 - 2.75 (m, 1 H), 3.01 - 3.10 (m, 1 H), 3.34 (s, 1 H), 3.67 - 3.75 (m, 1 H), 4.22 - 4.38 (m, 2 H), 5.61 - 5.68 (m, 1 H), 6.58 (d, *J*=6.4 Hz, 1 H), 7.66 (d, *J*=6.0 Hz, 1 H). m/z (ES+APCI)⁺: 314 [M+H]⁺.

### Example 105

### (4-Methoxy-phenyl)-[4-(3-methyl-1H-pyrazolo[4,3-c]pyridin-4-ylamino)-piperidin-1-yl] methanone

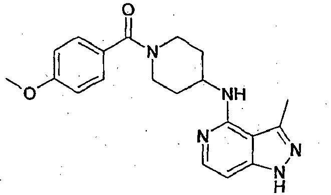

Example 105 was prepared analogously to Example 104 from Intermediate 8 and 4-methoxybenzoic acid to give the product (17mg, 26%). ¹H NMR (400 MHz, 60 °C, DMSO-*d*₆) δ ppm 1.56 - 1.68 (m, 2 H), 1.95 - 2.04 (m, 2 H), 2.60 (s, 3 H), 3.05 - 314 (m, 2 H), 3.81 (s, 3 H), 3.96 - 4.15 (m, 2 H), 4.31 - 4.42 (m, 1 H), 5.54 - 5.60 (m, 1 H), 6.59 (d, *J*=6.4 Hz, 1 H), 6.96 - 7.02 (m, 2 H), 7.34 - 7.40 (m, 2 H), 7.67 (d, *J*=6.0 Hz, 1 H). m/z (ES+APCI)⁺: 366 [M+H]⁺

### Example 106

### N-Cyclopentyl-4-(3-methyl-1H-pyrazolo[4,3-c]pyridin-4-ylamino)-benzamide

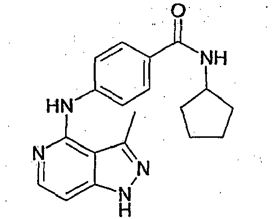

To a solution of cyclopentylamine (13 µl, 0.13 mmol) in DMF (2 ml) was added Intermediate 9 (78 mg, 0.20 mmol), HATU (81 mg, 0.21 mmol) and diisopropylethylamine (139 µl, 0.80 mmol) and the mixture was stirred at room temperature for 18 h. The mixture was evaporated, dissolved in a minimum amount of 10% MeOH in DCM and eluted through an lsolute-NH₂ cartridge and the filtrate concentrated. The residue was combined with trifluoroacetic acid (1 ml) and stirred at reflux for 18 h. The reaction mixture was evaporated and then purified by preparative LCMS (high pH buffer) to give a white solid (36 mg, 80%). ¹H NMR (400 MHz, DMSO-*d*₆) δ ppm 1.47 - 1.60 (m, 4 H), 1.64 - 1.76 (m, 2 H), 1.83 - 1.94 (m, 2 H), 2.72 (s, 3 H), 4.16 - 4:27 (m, 1 H), 6.94 (d, *J*=6.0 Hz, 1 H), 7.74 - 7.82 (m, 4 H), 7.85 (d, *J*=6.0 Hz, 1 H), 8.04 - 8.10 (m, 1 H), 8.28 (br. s., 1 H). m/z (ES+APCI)⁺: 336 [M+H]⁺

### Examples 107-113

Examples 107-113 in the table below were prepared analogously to Example 106 from Intermediate 44 and the appropriate amine:

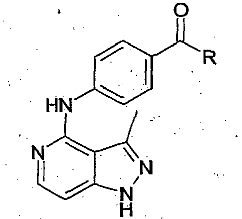

| **Example** | **R group** | **Name** | **m/z (ES+APCI)⁺** | **HPLC retention time (min)** |
|---|---|---|---|---|
| **107** | 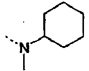 | *N-Cyclohexyl-N-methyl-4-(3-methyl-1H-pyrazolo[4,3-c]pyridin-4-ylamino)-benzamide* | 364 | 2.04^{b} |
| **108** | 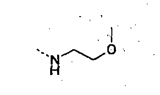 | *N-(2-Methoxy-ethyl)-4-(3-methyl-1H-pyrazolo[4,3-c]pyridin-4-ylamino)-benzamide* | 326 | 1.36^{b} |
| **109** | 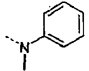 | *N-Methyl-4-(3-methyl-1H-pyrazo*/*o[4,3-c]pyridin-4-ylamino)-N-phenyl-benzamide* | 358 | 1.85^{b} |
| **110** | 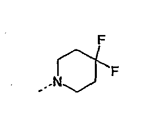 | *(4,4-Difluoro-piperidin-1-yl)-[4-(3-methyl-1H-pyrazolo[4,3-c]pyridin-4-ylamino)-phenyl]-methanone* | 372 | 1.49^{c} |
| **111** | 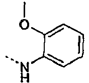 | *N-(2-Methoxy-phenyl)-4-(3-methyl-1H-pyrazolo[4,3-c]pyridin-4-ylamino)-benzamide* | 374 | 1.69^{c} |
| **112** | 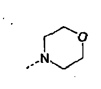 | *[4-(3-Methyl-1H-pyrazolo[4,3-c]pyridin-4-ylamino)-phenyl]-morpholin-4-yl-methanone* | 338 | 1.15^{c} |
| **113** | 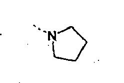 | *[4-(3-Methyl-1H-pyrazolo[4,3-c]pyridin-4-ylamino)-phenyl]-pyrrolidin-1-yl-methanone* | 322 | 1.30^{c} |

| | | | | |
|---|---|---|---|---|
| ^{b} HPLC column: 4.6x50mm (5µm) C-18 Xbridge; flow rate: 2ml/min; Run time: 4.6 min: Solvent A: 0.1% Formic acid in water, Solvent B: Methanol; Gradient - 10-100%B; Gradient time: 3.5min. ^{c} HPLC column: 4.6x50mm (5µm) C-18 Xbridge; flow rate: 3ml/min; Run time: 3.2 min: Solvent A: 0.1% Ammonium Hydroxide in water, Solvent B: Acetonitrile; Gradient - 10-100%B; Gradient time: 2.35min. | | | | |

### Example 114

### 4-Cyclohexyloxy-3-methyl-1H-pyrazolo[4,3-c]pyridine

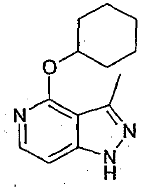

To a solution of cyclohexanol (249 µl, 2.40 mmol) in dioxane (2 ml) in a microwave vial was added sodium hydride (60% in mineral oil, 84 mg, 2.10 mmol). The mixture was allowed to stir at room temperature for 2h. A solution of Intermediate 3 (100 mg, 0.60 mmol) in dioxane (1 ml) was added, then the reaction mixture was irradiated at 180 °C for 1.5 h in a Biotage I-60 microwave reactor. The mixture was evaporated and water (20 ml) and ethyl acetate (20 ml) were added. The layers were separated and the aqueous extracted with further ethyl acetate (20 ml). The organic layers were combined and washed with brine (20 ml), dried and evaporated. The crude product was then purified by LCMS (high pH buffer) to give the desired product as a colourless oil (37 mg, 27%). ¹H NMR (400 MHz, DMSO-*d*₆) δ ppm 1.39 - 1.55 (m, 4 H), 1.57 - 1.68 (m, 2 H), 1.69 - 1.81 (m, 2 H), 1.87 - 2.00 (m, 2 H), 2.55 (s, 3 H), 5.17 - 5.38 (m, 1 H), 6.96 (d, *J*=6.4 Hz, 1 H), 7.76 (d, *J*=6.0 Hz, 1 H). m/z (ES+APCI)⁺: 232 [M+H]⁺.

### Examples 115-117

Examples 115-117 in the table below were prepared analogously to Example 114 from Intermediate 3 and the corresponding alcohol:

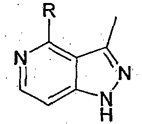

| **Example** | **R group** | **Name** | **m/z (ES+APCI)⁺** | **HPLC retention time (min)** |
|---|---|---|---|---|
| **115** | 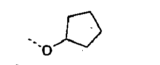 | *4-Cyclopentyloxy-3-methyl-1H-pyrazolo[4,3-c]pyridine* | 218 | 1.43^{d} |
| **116** | 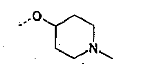 | *3-Methyl-4-(1-methyl-piperidin-4-yloxy)-1H-pyrazolo[4,3-c]pyridine* | 247 | 1.18^{c} |
| **117** | 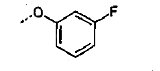 | *4-(3-Fluoro-phenoxy)-3-methyl-1H-pyrazolo[4,3-c]pyridine,* | 244 | 1.65^{c} |

| | | | | |
|---|---|---|---|---|
| ^{c} HPLC column: 4.6x50mm (5µm) C-18 Xbridge; flow rate: 3ml/min; Run time: 3.2 min: Solvent A: 0.1% Ammonium Hydroxide in water, Solvent B: Acetonitrile; Gradient - 10-100%B; Gradient time: 2.35min. ^{d} HPLC column: 4.6x50mm (5µm) C-18 Xbridge; flow rate: 3ml/min; Run time: 3.2 min: Solvent A: 0.1% Formic acid in water, Solvent B: Acetonitrile; Gradient - 10-100%B; Gradient time: 2.35min. | | | | |

### Example 118

### 3-Methyl-4-(tetrahydro-pyran-4-yloxy)-9H-pyrazolo[4,3-c]pyridine

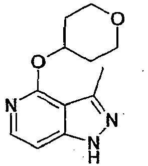

To a solution of tetrahydro-pyran-4-ol (137 µl, 1.48 mmol) in dioxane (2 ml) in a microwave vial was added sodium hydride (60% dispersion in mineral oil, 50 mg, 1.26 mmol) and the mixture was allowed to stir at room temperature for 2 h. A solution of Intermediate 3 (60 mg, 0.36 mmol) in dioxane (1 ml) was added and the reaction mixture was irradiated at 170 °C for 1.5 h in a Biotage I-60 microwave reactor. The mixture was evaporated, and partitioned between water and ethyl acetate. The layers were separated and the aqueous extracted with further ethyl acetate (20 ml). The organic layers were combined and washed with brine, dried (MgSO₄) and evaporated. The crude product was then purified by preparative LCMS (low pH buffer) then eluted through an Isolute-NH₂ cartridge with 9:1 DCM: methanol to give the desired product as a white solid (24 mg, 27%). ¹H NMR (400 MHz, DMSO-*d*₆) δ ppm 1.68 - 1.78 (m, 2 H), 1.99 - 2.08 (m, 2 H), 2.56 (s, 3 H), 3.53 - 3.63 (m, 2 H), 3.82 - 3.91 (m, 2 H), 5.39 - 5.48 (m, 1 H), 6.99 (d, *J*=6.0 Hz, 1 H), 7.77 (d, *J*=6.0 Hz, 1 H). m/z (ES+APCI)⁺: 234 [M+H]⁺

### Example 119

### 4-(1-Isopropyl-piperidin-4-yloxy)-3-methyl-1H-pyrazolo[4,3-c]pyridine

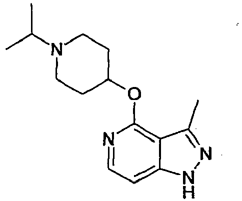

Example 119 was prepared analogously to Example 118 from Intermediate 3 and 1-Isopropyl-piperidin-4-ol to give a white solid (37mg, 64%). ¹H NMR (400 MHz, DMSO-d₆) d ppm 0.98 (d, *J*=6.9 Hz, 6 H), 1.70 - 1.80 (m, 2 H), 1.91 - 2.00 (m, 2 H), 2.37 - 2.45 (m, 2 H), 2.55 (s, 3 H), 2.65 - 2.75 (m, 3 H), 5.22 - 5.31 (m, 1 H), 6.96 (d, *J*=6.0 Hz, 1 H), 7.75 (d, *J*=6.0 Hz, 1 H). m/z (ES+APCI)⁺: 275 [M+H]⁺.

### Example 120

### 4-(4-Imidazol-1-yl-phenoxy)-3-methyl-1H-pyrazolo[4,3-c]pyridine

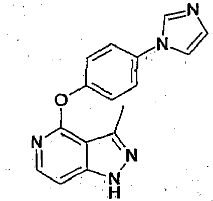

To a solution of 4-Imidazol-1-yl-phenylamine (230 mg, 1.48 mmol) in dioxane (2 ml) in a microwave vial was added sodium hydride (60% dispersion in mineral oil, 50 mg, 1.26 mmol) and the mixture was allowed to stir at room temperature for 2 h. A solution of Intermediate 3 (60 mg, 0.36 mmol) in dioxane (1 ml) was added and the reaction mixture was heated at 90 °C for 72 h. The mixture was evaporated, then water (20 ml) and ethyl acetate (20 ml) were added. The layers were separated and the aqueous extracted with further ethyl acetate (20 ml). The organic layers were combined and washed with brine, dried (MgSO₄) and evaporated. The crude product was then purified by preparative LCMS (low pH buffer) then eluted through an lsolute-NH₂ cartridge with 9:1 DCM: methanol to give the desired product as a white solid (2.3 mg, 2%). ¹H NMR (400 MHz, DMSO-*d*₆) δ ppm 2.66 (s, 3 H), 7.12 (s, 1 H), 7.16 (d, *J*=6.4 Hz, 1 H), 7.38 - 7.43 (m, 2 H), 7.68 - 7.75 (m, 3 H), 7.75 - 7.77 (m, 1 H), 8.25 - 8.26 (m, 1 H). m/z (ES+APCI)⁺: 292 [M+H]⁺

### Example 121

### 4-((S)-sec-Butoxy)-3-methyl-1H-pyrazolo[4,3-c]pyridine

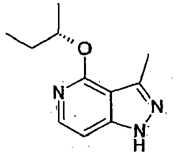

*(S)*-Butan-2-ol (91 µl, 0.98 mmol) was added to 1M potassium tert-butoxide in THF (1 ml, 0.98 mmol) and the mixture was stirred under a nitrogen atmosphere for 5 minutes before adding Intermediate 3 (0041 mg, 0.25 mmol). The reaction mixture was irradiated at 120 °C for 100 minutes in a Biotage I-60 microwave reactor. The mixture was quenched with 4M hydrogen chloride in dioxane (250 µl) and then diluted with H₂O (10 ml). The mixture was extracted twice with DCM (20 ml) and the combined organic layers were evaporated. The crude product was purified preparative LCMS (high pH buffer) to give the desired product as an off-white solid (19 mg, 38%). ¹H NMR (400 MHz, DMSO-d₆) δ ppm 0.95 (t,-*J*=7.4 Hz, 3 H), 1.32 (d, *J*=6.0 Hz, 3 H), 1.62 - 1.80 (m, 2 H), 2.53 (s, 3 H), 5.23 - 5.33 (m, 1 H), 6.96 (d, *J*=6.0 Hz, 1 H), 7.76 (d, *J*=6.0 Hz, 1 H). m/z (ES+APCI)⁺: 206 [M+H]⁺.

### Examples 122-123

Examples 122-123 in the following table were prepared analogously to Example 121 from Intermediate 3 and the corresponding alcohol:

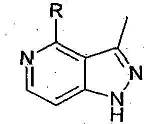

| **Example** | **R group** | **Name** | **m/z (ES+APCI)⁺** | **HPLC retention time (min)** |
|---|---|---|---|---|
| **122** | 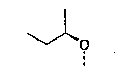 | 4-(*(R)*-sec-Butoxy)-3-methyl-1*H-*pyrazolo[4,3-c]pyridine | 206 | 1.68^{c} |
| **123** | 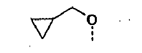 | 4-Cyclopropylmethoxy-3-methyl-1*H-*pyrazolo[4,3-c]pyridine | 204 | 1.55^{C} |

| | | | | |
|---|---|---|---|---|
| ^{c} HPLC column: 4.6x50mm (5µm) C-18 Xbridge; flow rate: 3ml/min; Run time: 3.2 min: Solvent A: 0.1% Ammonium Hydroxide in water, Solvent B: Acetonitrile; Gradient - 10-100%B; Gradient time: 2.35min. | | | | |

### Example 124

### 4-Ethoxy-3-methyl-1H-pyrazolo[4,3-c]pyridine

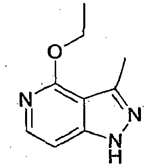

Concentrated KOH (aq) (0.5 ml) was added to a solution of Intermediate 3 (100 mg, 0.60 mmol) in ethanol (1 ml). The reaction mixture was irradiated at 120 °C for 1 h in a Biotage I-60 microwave reactor. The mixture was evaporated, diluted with H₂O (5 ml) and neutralised with dilute hydrochloric acid. The aqueous was then extracted with DCM (30 ml) twice, the combined organic layers were dried (MgSO₄), and evaporated. The crude product was purified by preparative LCMS.(high pH buffer) to give the desired product as a white solid (28 mg, 26%). ¹H NMR (400 MHz, DMSO-*d*₆) δ ppm 1.38 (t, *J*=7.1 Hz, 3 H), 2.54 (s, 3 H), 4.45 (q, *J*=7.2 Hz, 2 H), 6.99 (d, *J*=6.0 Hz, 1 H), 7.77 (d, *J*=6.0 Hz, 1 H). m/z (ES+APCI)⁺: 178 [M+H]⁺.

### Example 125

### 4-Benzyl-3-methyl-1H-pyrazolo[4,3-c]pyridine

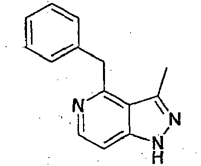

### Step 1

A 0.5M solution of benzyl zinc bromide in THF (2.1 ml, 1.05 mmol) was added to Intermediate 4 (150 mg, 0.52 mmol) and Pd(PPh₃)₄ (30 mg, 0.03 mmol) in THF (2 ml) under nitrogen at room temperature, and the resulting mixture was stirred at 60 °C overnight. The mixture was quenched with saturated NH₄Cl (aq) (10 ml) and extracted twice with EtOAc (20 ml). The combined organic layers were washed with brine (20 ml), dried (MgSO₄) and evaporated. The crude product was purified by flash chromatography on the Biotage SP4, eluting with 0 to 60% EtOAc/petroleum ether to give a yellow oil (78 mg) which was used in the next step without further purification.

### Step 2

The product of Step 1 (170 mg) and trifluoroacetic acid (3 ml, 4.61 mmol) were combined and stirred at reflux for 2.5 h. The reaction mixture was evaporated, the residue dissolved in EtOAc (20 ml) and partitioned with saturated NaHCO₃ (aq) (20 ml). The aqueous layer was extracted with EtOAc (10 ml), the combined organic layers were washed with brine, dried (MgSO₄) and evaporated. The crude product was purified by prep LCMS (high pH buffer) to give the desired product as a white solid (30 mg, 38%). ¹H NMR (400 MHz, DMSO-*d*₆) δ ppm 2.53 (s, 3 H), 4.46 (s, 2 H), 7.14 - 7.20 (m, 3 H), 7.23 - 7.29 (m, 2 H), 7.32 (d, *J*=6.0 Hz, 1 H), 8.19 (d, *J*=6.0 Hz, 1 H). m/z (ES+APCI)⁺: 224 [M+H]⁺.

### Examples 126-127

Examples 126-127 in the following table were prepared analogously to Example 125:

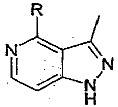

| **Example** | **R group** | **Name** | **m/z (ES+APCI)⁺** | **HPLC retention time (min)** |
|---|---|---|---|---|
| **126** | 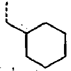 | *4-Cyclohexylmethyl-3-methyl-1H-pyrazolo[4,3-c]pyridine* | 230 | 1.66^{c} |
| **127** | 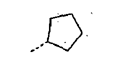 | *4-Cyclopentyl-3-methyl-1H-pyrazolo[4,3-c]pyridine* | 202 | 1.48^{c} |

| | | | | |
|---|---|---|---|---|
| ^{c}HPLC column: 4.6x50mm (5µm) C-18 Xbridge; flow rate: 3ml/min; Run time: 3.2 min: Solvent A: 0.1% Ammonium Hydroxide in water, Solvent B: Acetonitrile; Gradient - 10-100%B; Gradient time: 2.35min. | | | | |

### Example 128

### 3-Methyl-4-(3-methyl-butyl)-1H-pyrazolo[4,3-c]pyridine

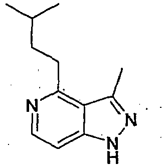

### Step 1

A 0.5M solution of 3-methylbutyl zinc bromide in THF (1.4 ml, 0.70 mmol) was added to Intermediate 4 (100 mg, 0.35 mmol) and Pd(PPh₃)₄ (20 mg, 0.02 mmol) in THF (2 ml) under nitrogen at room temperature. The reaction was stirred at 60 °C overnight. The mixture was quenched with saturated ammonium chloride aqueous solution (10 ml) and extracted twice with EtOAc (20 ml). The combined organic layers were washed with brine, dried (MgSO₄) and evaporated. The crude product was purified by flash chromatography on the Biotage SP4, eluting with 0 to 60% EtOAc/petroleum ether to give the desired product as a yellow oil which was used in Step 2 without further purification.

### Step 2

The product of step 1 (80 mg) and trifluoroacetic acid (2 ml, 3.07 mmol) were combined and stirred at reflux for 3 h. The crude product preparative LCMS (low pH buffer) then eluted through an Isolute-NH₂ cartridge with 9:1 DCM: methanol to give a white solid (8.4 mg, 12% over 2 steps). ¹H NMR (400 MHz, DMSO-*d*₆) δ ppm 0.96 (d, *J*=6.4 Hz, 6 H), 1.56 - 1.62 (m, 2 H), 1.63 - 1.73 (m, 1 H), 2.64 (s, 3 H), 3.04 - 3.10 (m, 2 H), 7.23 (d, *J*=6.0 Hz, 1 H), 8.12 (d, *J*=6.0 Hz, 1 H). m/z (ES+APCI)⁺: 204 [M+H]⁺

### Example 129

### 3-Methyl-4-oxazol-2-yl-1H-pyrazolo[4,3-c]pyridine

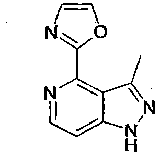

### Step 1

A solution of Intermediate 4 (80 mg, 0.28 mmol) in toluene (2 ml) was degassed for 10 minutes and placed under an atmosphere of nitrogen. 2-(Tri-*n*-butylstannyl) oxazole (70 µl, 0.33 mmol) and Pd(PPh₃)₄ (16 mg, 0.014 mmol) were added. The reaction vessel was evacuated backfilled with nitrogen,twice then stirred at reflux for 18 h. The mixture was evaporated, dry loaded onto silica and purified by flash chromatography on the Biotage SP4, eluting with 10 to 100% EtOAc/petroleum etherto give an orange oil (64 mg) which was used in Step 2 without further purification.

### Step 2

The product of Step 1 (62 mg, 0.19 mmol) and trifluoroacetic acid (0.5 ml, 0.77 mmol) were combined and stirred at reflux for 18 h. The reaction mixture was evaporated and then purified by preparative LCMS (high pH buffer) to give a white solid (19 mg, 34%). ¹H NMR (400 MHz, DMSO-*d*₆) 5 ppm 2.70 (s, 3 H), 7.56 (s, 1 H), 7.61 (d, *J*=6.0 Hz, 1 H), 8.38 (s, 1 H), 8.40 (d, *J*=6.0 Hz, 1 H). m/z (ES+APCI)⁺: 201 [M+H]⁺.

### Examples 130-131

Examples **130-131** in the following table were prepared analogously to Example 129:

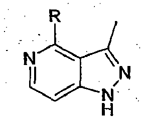

| **Example** | **R group** | **Name** | **m/z (ES+APCI)⁺** | **HPLC retention time (min)** |
|---|---|---|---|---|
| **130** | 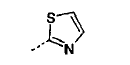 | *3-Methyl-4-thiazol-2-yl-1H-pyrazolo[4,3-c]pyridine* | 217 | 1.31^{c} |
| **131** | 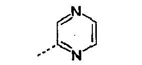 | *3-Methyl-4-pyrazin-2-yl-1H-pyrazolo[4,3-c]pyridine* | 212 | 0.92^{c} |

| | | | | |
|---|---|---|---|---|
| ^{c}HPLC column: 4.6x50mm (5µm) C-18 Xbridge; flow rate: 3ml/min; Run time: 3.2 min: Solvent A: 0.1% Ammonium Hydroxide in water, Solvent B: Acetonitrile; Gradient 10-100%B; Gradient time: 2.35min. | | | | |

### Example 132* (*outside scope of the invention)

### (3-Fluoro-phenyl)-(1H-pyrazolo[4,3-c]pyridin-4-yl)-amine

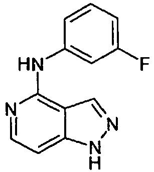

Intermediate 11 (30 mg, 0.20 mmol), 3-fluoroaniline (28 µl, 0.29 mmol) and conc. HCl (aq) (18 µl, 0.59 mmol) were combined in n-butanol (0.5 ml) and heated in the microwave at 190 °C for 1 h. The solvents were evaporated to dryness and the crude mixture purified by preparative LCMS to give a white solid (21 mg, 47%). ¹H NMR (400 MHz, DMSO-*d*₆) ppm 6.79 (td, *J*=8.4, 2.5 Hz, 1 H), 7.01 (d, *J*=7.3 Hz, 1 H), 7.33 - 7.41 (m, 1 H), 7.62 (d, *J*=7.3 Hz, 1 H), 7.95 (d, *J*=6.0 Hz, 1 H), 8.16 (dt, *J*=12.6, 2.4 Hz, 1 H), 8:50 (s, 1 H), 9.53 (s, 1 H); m/z (ES+APCI)⁺: 229 [M + H]⁺.

### Example 133* (*outside scope of the invention)

### Cyclohexyl-(1H-pyrazolo[4,3-c]pyridin-4-yl)-amine

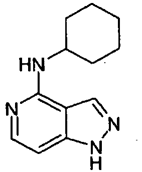

Intermediate 11 (30 mg, 0.20 mmol), cyclohexylamine (89 µl, 0.29 mmol) and conc. HCl (18 µl, 0.59 mmol) were combined in n-butanol (0.5 ml) and heated in the microwave at 190 °C for 1 h. The solvents were evaporated to dryness and the crude mixture purified by preparative LCMS to give a white solid (11 mg, 26%). ¹H NMR (400 MHz, DMSO-*d*₆) δ ppm 1.14 - 1.44 (m, 5 H), 1.63 - 1.71 (m, 1 H), 1.73 - 1.83 (m, 2 H), 1.96 - 2.06 (m, 2 H), 4.04 (m, 1 H), 6.61 (d, *J*=6.0 Hz, 1 H), 7.01 (d, *J*=7.8 Hz, 1 H), 7.70 (d, *J*=6.0 Hz, 1 H), 8.24 (s, 1 H); m/z (ES+APCI)⁺: 217 [M + H]⁺.

### Example 134

### (3-Bromo-1H-pyrazolo[4,3-c]pyridin-4 yl)-cyclohexyl-amine

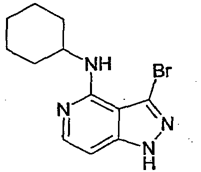

Intermediate 15 (1.9 g, 8.19 mmol) and cyclohexylamine (3.73 ml, 32.8 mmol) were combined in n-butanol (30 ml) and heated in the microwave for 1 h at 190 °C. The solvents were evaporated and the crude product purified by flash chromatography using a Biotage SP4 (ethyl acetate / petroleum ether gradient) to give a white solid (1.51 g, 63%). ¹H NMR (400 MHz, DMSO-*d*₆) δ ppm 1.24 - 1.48 (m, 5 H), 1.58 - 1.66 (m, 1 H), 1.68 - 1.79 (m, 2 H), 1.97 - 2.07 (m, 2 H), 4.05 - 4.14 (m, 1 H), 5.85 (d, *J*=7.3 Hz, 1 H), 6.72 (d, *J*=6.0 Hz, 1 H), 7.78 (d, 1 H); m/z (ES+APCI)⁺: 295 / 297 [M + H]⁺.

### Example 135

### Cyclohexyl-(3-pyridin-3-yl-1H-pyrazolo[4,3-c]pyridin-4-yl)-amine

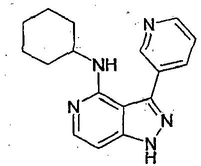

Example 134 (50 mg, 0.17 mmol), Pd(dppf)Cl₂ (14 mg, 0.02 mmol), 3-pyridineboronic acid (31 mg, 0.26 mmol) and 2 M sodium carbonate (aq) (298 µl, 0.60 mmol) were combined in dioxane (2 ml), the solvent degassed and the vial flushed out with nitrogen. The reaction mixture was then heated to 90 °C for 18 h. The solvents were evaporated and the crude residue re-dissolved in 9:1 DCM : methanol and filtered through a plug of silica, eluting with 9:1 DCM : methanol. The solvents were evaporated and the crude product purified by preparative LCMS to give a beige solid (0.8 mg, 22%). ¹H NMR (400 MHz, DMSO-*d*₆) δ ppm 1.13 - 1.27 (m, 3 H), 1.27 - 1.42 (m, 2 H), 1.51 - 1.65 (m, 3 H), 1.89 - 1.98 (m, 2 H), 3.99 - 4.09 (m, 1 H), 5.02 (d, *J*=7.8 Hz, 1 H), 6.79 (d, *J*=6.0 Hz, 1 H), 7.63 (dd, *J*=7.8, 5.5 Hz, 1 H), 7.84 (d, *J*=6.4 Hz, 1 H), 8.12 (dt, *J*=8.0,1.9 Hz, 1 H), 8.74 (dd, *J*=5.0, 1.8 Hz, 1 H), 8.91 (s, 1 H); m/z (ES+APCI)⁺: 294 [M + H]⁺.

### Example 136

### Cyclohexyl-(3-phenyl-1H-pyrazolo[4,3-c]pyridin-4-yl)-amine

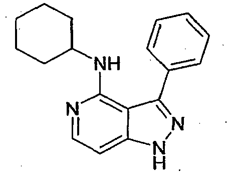

Example 134 (100 mg, 0.34 mmol), Pd(PPh₃)₄ (118 mg, 0.1 mmol), phenyl boronic acid (62 mg, 0.51 mmol) and 2 M sodium carbonate (aq) (340 µl, 0.68 mmol) were combined in a mixture of toluene (2.5 ml) and methanol (0.5 ml), the reaction content degassed and then heated to 65 °C, under nitrogen for 18 h. The solvents were evaporated and the crude residue re-dissolved in 9:1 DCM : methanol and filtered through a plug of silica, eluting with 9:1 DCM : methanol. The solvents were evaporated and the crude product purified by preparative LCMS to give a white solid (7 mg, 8%). ¹H NMR (400 MHz, DMSO-*d*₆) δ ppm 1.08 - 1.28 (m, 3 H), 1.29 - 1.41 (m, 2 H), 1.48 - 1.59 (m, 3 H), 1.86 - 1.94 (m, 2 H), 3.99 - 4.08 (m, 1 H), 4.96 (d, *J*=7.3 Hz, 1 H), 6.73 (d, *J*=6.0 Hz, 1 H), 7.54 - 7.64 (m, 3 H), 7.66 - 7.70 (m, 2 H), 7.81 (d, *J*=6.0 Hz, 1 H); m/z (ES+APCl)⁺: 293 [M + H]⁺.

### Example 137

### N-[3-(4-Cyclohexylamino-1H-pyrazolo[4,3-c]pyridin-3-yl)-phenyl]-acetamide

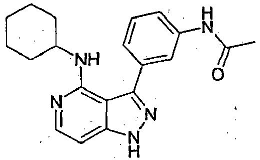

Example 134 (50 mg, 0.17 mmol), Pd(dppf)Cl₂ (14 mg, 0.02 mmol), 3-acetamidophenylboronic acid (46 mg, 0.26 mmol) and 2 M sodium carbonate (aq) (298 µl, 0.60 mmol) were combined in dioxane (2 ml), the solvent degassed and the vial flushed out with nitrogen. The reaction mixture was then heated to 90 °C for 18 h. The solvents were evaporated and the crude residue re-dissolved in 9:1 DCM : methanol and filtered through a plug of silica, eluting with 9:1 DCM : methanol. The solvents were evaporated and the crude product purified by preparative LCMS to give a brown solid (7 mg, 12%). ¹H NMR (400 MHz, DMSO-*d₆*) δ ppm 1.12 - 1.27 (m, 3 H), 1.27 - 1.40 (m, 2 H), 1.47 - 1.58 (m, 3 H), 1.84 - 1.93 (m, 2 H), 2.11 (s, 3 H), 3.98 - 4.07 (m, 1 H), 5.10 (d, *J*=7.8 Hz, 1 H), 6.74 (d, *J*=6.0 Hz, 1 H), 7.32 (d, *J*=7.8 Hz, 1 H), 7.52 (t, *J*=7.8 Hz, 1 H), 7.64 (d, *J*=9.2 Hz, 1 H), 7.80 (d, *J*=6.0 Hz, 1 H), 8.05 (s, 1 H), 10.19 (s, 1 H); m/z (ES+APCl)⁺: 350 [M + H]⁺.

### Example 138

### Cyclohexyl-(3-cyclopropyl-1H-pyrazolo[4,3-c]pyridin-4-yl)-amine

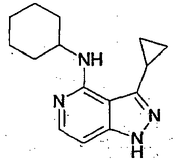

Intermediate **18** (30 mg, 0.16 mmol) and cyclohexylamine (71 µl, 0.62 mmol) were combined in n-butanol (0.5 ml) and heated in the microwave for 1 h at 190 °C. The solvents were evaporated and the crude product purified by preparative LCMS to give a white solid (7 mg, 18%). ¹H NMR (400 MHz, DMSO-*d*₆) δ ppm 0.84 - 0.92 (m, 2 H), 0.96 - 1.04 (m, 2 H), 1.21 - 1.34 (m, 1 H), 1.34 - 1.47 (m, 4 H), 1.61 - 1.68 (m, 1 H), 1.71 - 1.81 (m, 2 H), 1.97 - 2.06 (m, 2 H), 2.30 - 2.38 (m, 1 H), 4.05 - 4.15 (m, 1 H), 5.73 (d, *J*=8.2 Hz, 1 H), 6.58 (d, *J*=6.4 Hz, 1 H), 7.69 (d, *J*=6.0 Hz, 1 H); m/z (ES+APCI)⁺: 257 [M + H]⁺.

### Example 139

### 4-(4-Cyclohexylamino-1H-pyrazolo[4,3-c]pyridin-3-yl)-N-methyl-benzamide

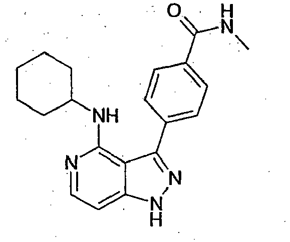

Intermediate 14 (50 mg, 0.11 mmol), Pd(dppf)Cl₂ (9 mg, 0.01 mmol), N-methyl-4-(4,4,5,5-tetramethyl-1,3,2-dioxaborolan-2-yl)benzamide (42 mg, 0.16 mmol) and 2 M aqueous sodium carbonate (189 µl, 0.38 mmol) were combined in dioxane (1 ml), the reaction mixture degassed and the vial flushed with nitrogen. The reaction mixture was then heated to 90 °C for 18 h. After heating to 90 °C for 18 h, the mixture was partitioned between DCM and water, the organic phase was collected using a phase separation cartridge and concentrated. The crude residue was treated with TFA (1 ml) at 60 °C for 19 h. The mixture was concentrated and the crude product purified by preparative LCMS (low pH buffer). The resulting salt was re-dissolved in methanol and eluted through an Isolute-NH₂ cartridge and solvents evaporated to give the final product (17 mg, 45%). ¹H NMR (400 MHz, DMSO-*d*₆) δ ppm 1.10 - 1.27 (m, 3 H), 1.30 - 1.42 (m, 2 H), 1.52 - 1.64 (m, 3 H), 1.91 - 1.98 (m, 2 H), 2.87 (d, *J*=4.6 Hz, 3 H), 4.01 - 4.09 (m, 1 H), 4.99 (d, *J*=7.8 Hz, 1 H), 6.77 (d, *J*=6.0 Hz, 1 H), 7.77 - 7.84 (m, 3 H), 8.06 (d, *J*=8.7 Hz, 2 H), 8.61 - 8.65 (m, 1 H); m/z (ES+APCI)⁺: 350 [M + H]⁺.

### Examples 140-154

Examples 140-154 in the following table were prepared analogously to Example 139 from Intermediate **14** and the appropriate boronic acid or boronic ester:

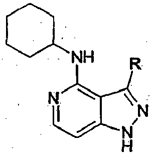

| **Example** | **R group** | **Name** | **m/z (ES+APGI)⁺** | **HPLC retention time (min)^{a,b}** |
|---|---|---|---|---|
| **140** | 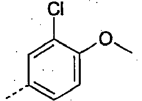 | *[3-(3-Chloro-4-methoxy-phenyl)-1H-pyrazolo[4,3-c]pyridin-4-yl]-cyclohexyl-amine* | 357 | 3.84^{a} |
| **141** | 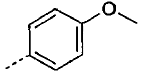 | *Cyclohexyl-[3-(4-methoxy-phenyl)-1H-pyrazolo[4,3-c]pyridin-4-yl]-amine* | 323 | 3.72^{a} |
| **142** | 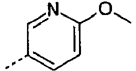 | *Cyclohexyl-[3-(6-methoxy-pyridin-3-yl)-1H-pyrazolo[4,3-c]pyridin-4-yl]-amine* | 324 | 3.64^{a} |
| **143** | 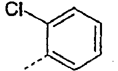 | *[3-(2-Chloro-phenyl)-1H-pyrazolo[4,3-c]pyridin-4-yl]-cyclohexyl-amine* | 327 | 3.66^{a} |
| **144** | 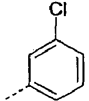 | *[3-(3-Chloro-phenyl)-1H-pyrazolo[4,3-c]pyridin-4-yl]-cyclohexyl-amine* | 327 | 2.16^{b} |
| **145** | 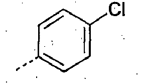 | *[3-(4-Chloro-phenyl)-1H-pyrazolo[4,3-c]pyridin-4-yl]-cyclohexyl-amine* | 327 | 3.94^{a} |
| **146** | 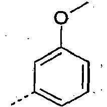 | *Cyclohexyl-[3-(3-methoxy-phenyl)-1H-pyrazolo[4,3-c]pyridin-4-yl] amine* | 323 | 3.74^{a} |
| **147** | 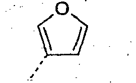 | *Cyclohexyl-(3-furan-2-yl-1H-pyrazolo[4,3-c]pyridin-4-yl)-amine* | 283 | 3.54^{a} |
| **148** | 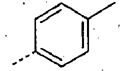 | *Cyclohexyl-(3-p-tolyl-1H-pyrazolo[4,3-c]pyridin-4-yl)-amine* | 307 | 3.86^{a} |
| **149** | 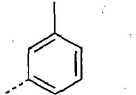 | *Cyclohexyl-(3-m-tolyl-1H-pyrazolo[4,3-c]pyridin-4-yl)-amine* | 307 | 3.87^{a} |
| **150** | 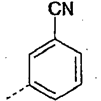 | *3-(4-Cyclohexylamino-1H-pyrazolo[4,3-c]pyridin-3-yl)-benzonitrile* | 318 | 3.52^{a} |
| **151** | 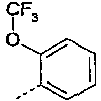 | *Cyclohexyl-[3-(2-trifluoromethoxy phenyl)-1H-pyrazolo[4,3-c]pyridin-4-yl]-amine* | 377 | 3.72^{a} |
| **152** | 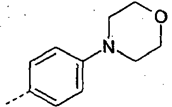 | *Cyclohexyl-[3-(4-morpholin-4-yl phenyl)-1H-pyrazolo[4,3-c]pyridin-4-yl]-amine* | 378 | 3.60^{a} |
| **153** | 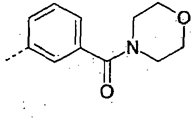 | *[3-(4-Cyclohexylamino-1H-pyrazolo[4,3-c]pyridin-3-yl)-phenyl]-morpholin-4-yl-methanone* | 406 | 3.29^{a} |
| **154** | 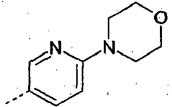 | *Cyclohexyl-[3-(6-morpholin-4-yl pyridin*-*3*-*yl*)-*1H-pyrazolo[4,3-c]pyridin-4-yl]-amine* | 379 | 3.47^{a} |

| | | | | |
|---|---|---|---|---|
| ^{a} HPLC column: 4.6x50mm (5µm) C-18 Xbridge; flow rate: 2ml/min; Run time: 4.6 min: Solvent A: 0.1% Ammonium Hydroxide in water, Solvent B: Methanol; Gradient - 10-100%B; Gradient time: 3.5min. ^{b} HPLC column: 4.6x50mm (5µm) C-18 Xbridge; flow rate: 3ml/min; Run time: 3.2 min: Solvent A: 0.1% Ammonium Hydroxide in water Solvent B: Acetonitrile; Gradient - 10-100%B; Gradient time: 2.35min. | | | | |

### Example 155

### Cyclohexyl-[1-(4-methoxy-benzyl)-3-(3-pyrazol-1-yl-phenyl)-1H-pyrazolo[4,3-c]pyridin-4-yl]-amine

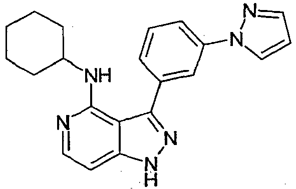

Intermediate 14 (50 mg, 0.11 mmol), Pd(dppf)Cl₂ (9 mg, 0.01 mmol), 1-[3-(4,4,5,5-tetramethyl-1,3,2-dioxaborolan-2-yl)phenyl]-1H-pyrazole (44 mg, 0.16 mmol) and 2 M aqueous sodium carbonate (189 µl, 0.38 mmol) were combined in dioxane (1 ml), the reaction mixture degassed and the vial flushed out with nitrogen. After heating to 90 °C for 18 h, the mixture was partitioned between DCM and water, the organic phase was collected using a phase separation cartridge and concentrated. The crude residue was treated with TFA (1 ml) at 60 °C for 19 h. Concentration under reduced pressure followed by purification by preparative LCMS (high pH buffer) gave the product (19 mg, 49%). ¹H NMR (400 MHz, DMSO-*d*₆) δ ppm 1.09 - 1.21 (m, 3 H), 1.25 - 1.36 (m, 2 H), 1.44 - 1.53 (m, 3 H), 1.83 - 1.91 (m, 2 H), 4.02 - 4.08 (m, 1 H), 5.10 (d, *J*=7.3 Hz, 1 H), 6.62 (d, *J*=1.8 Hz, 1 H), 6.78 (d, *J*=6.0 Hz, 1 H), 7.59 - 7.64 (m, 1 H), 7.74 (t, *J*=7.8 Hz, 1 H), 7.81 - 7.85 (m, 2 H), 8.04 (d, *J*=9.2 Hz, 1 H), 8.20 (s, 1 H), 8.68 (d, *J*=2.3 Hz, 1 H); m/z (ES+APCI)⁺: 359 [M + H]⁺.

### Examples 156-166

Examples 156-166 in the table below were prepared analogously to Example 155 from Intermediate **14** and the appropriate boronic acid or boronic ester.

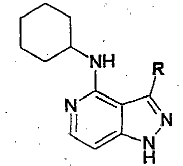

| **Example** | **R group** | **Name** | **m/z (ES+APCI)⁺** | **HPLC retention time (min)*** |
|---|---|---|---|---|
| **156** | 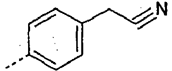 | *[4-(4-Cyclohexylamino-1H-pyrazolo[4,3-c]pridin-3-yl)-phenyl]-acetonitrile* | 332 | 1.77 |
| **157** | 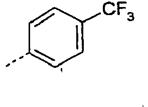 | *Cyclohexyl-[1-(4-methoxy-benzyl)-3-(4-trifluoromethyl phenyl)-1H-pyrazolo[4,3-c]pyridin-4-yl]-amine* | 361 | 2.20 |
| **158** | 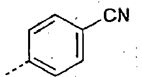 | *4-[4-Cyclohexylamino-1-(4-methoxy-benzyl)-1H-pyrazolo[4,3-c]pyridin-3-yl]-benzonitrile* | 318 | 1.87 |
| **159** | 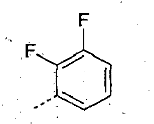 | *Cyclohexyl-[3-(2,3-difluorophenyl)-1-(4-methoxy-benzyl)-1H-pyrazolo[4,3-c]pyridin-4-yl]-amine* | 329 | 1.97 |
| **160** | 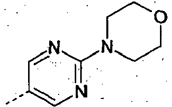 | *Cyclohexyl-[3-(2-morpholin-4-yl-pyrimidin-5-yl)-1H-pyrazolo[4,3-c]pyridin-4-yl]-amine* | 380 | 1.73 |
| **161** | 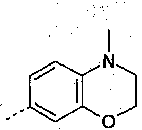 | *Cyclohexyl*-[*3*-(*4-methyl-3,4-dihydro-2H-1,4-benzoxazin-7-yl)-1H-pyrazolo[4,3-c]pyridin*-*4*-*yl*]-*amine* | 364 | 1.94 |
| **162** | 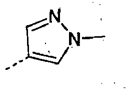 | *Cyclohexyl-[3-(1-methyl-1H-pyrazol-4-yl)-1H-pyrazolo[4,3-c]pyridin-4-yl]-amine* | 297 | 1.49 |
| **163** | 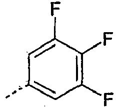 | *Cyclohexyl-[3-(3,4,5-trifluorophenyl)-1H-pyrazolo[4,3-c]pyridin-4-yl] amine* | 347 | 2.20 |
| **164** | 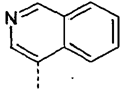 | *Cyclohexyl-(3-isoquinolin-4-yl-1H-pyrazolo[4,3-c)pyridin-4-yl)-amine* | 344 | 1.74 |
| **165** | 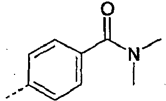 | *4-(4-Cyclohexylamino-1H-pyrazolo[4,3-c]pyridin-3-yl)-N,N-dimethyl-benzamide* | 364 | 1.60 |
| **166** | 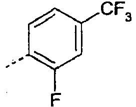 | *Cyclohexyl-[3-(2-fluoro-4-trifluoromethyl phenyl)-1H-pyrazolo[4,3-c]pyridin-4-yl]-amine* | 379 | 2.22 |

| | | | | |
|---|---|---|---|---|
| *HPLC column: 4.6x50mm (5µm) C-18 Xbridge; flow rate: 3ml/min; Run time: 3.2 min: Solvent A: 0.1% Ammonium Hydroxide in water Solvent B: Acetonitrile; Gradient - 10-100%B; Gradient time: 2.35min. | | | | |

### Example 167

### 3-Furan-3-yl-1H-pyrazolo[4,3-c]pyridin-4-ol

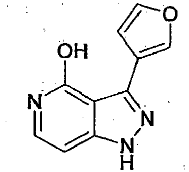

**Intermediate 19** (50 mg, 0.11 mmol), Pd(dppf)Cl₂ (9 mg, 0.01 mmol), furan-3-boronic acid (18 mg, 0.16 mmol) and 2 M aqueous sodium carbonate (189 µl, 0.38 mmol) were combined in dioxane (1 ml), the reaction mixture was degassed and heated to 90 °C, under nitrogen for 3 days. The mixture was partitioned between DCM and water, the organic phase was collected using a phase separation cartridge and concentrated. The crude residue was treated with TFA (1 ml) at 60 °C for 18 h. Concentration followed by purification by preparative LCMS yielded the title compound (3 mg, 14%). ¹H NMR (400 MHz, DMSO-*d*₆) δ ppm 6.45 (d, *J*=6.4 Hz, 1 H), 7.09 (br. s., 1 H), 7.13 - 7.22 (m, 1 H), 7.77 (s, 1 H), 8.87 (s, 1 H), 11.00 (br. s., 1 H); m/z (ES+APCI)⁺: 202 [M + H]⁺.

### Example 168

### 3-(4-Cyclohexylamino-1H-pyrazolo[4,3-c]pyridin-3-yl)-1-(4,4-difluoro-piperidin-1-yl)-propan-1-one

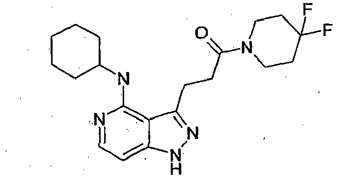

To a solution of Intermediate 23 (35 mg, 0.12 mmol) in DMF (1.5 ml) was added HATU (48 mg, 0.13 mmol) and *N,N*-diisopropylethylamine (126 µl, 0.73 mmol). 4,4-difluoro piperidine (19 µl, 0.18 mmol) was then added and the resulting solution was left to stir at room temperature overnight. The volatiles were removed under reduced pressure and the crude product was re-dissolved in 10% MeOH/DCM and eluted though an isolute-NH₂ cartridge. The crude product purified by flash chromatography eluting with 10% MeOH/DCM to give a yellow gum (28 mg, 61%). ¹H NMR (400 MHz, DMSO-*d*₆) δ ppm 1.14 - 1.26 (m, 1 H), 1.30 - 1.46 (m, 4 H), 1.60 - 1.72 (m, 1 H), 1.71 - 2.01 (m, 8 H), 2.87 (t, 2 H), 3.21 (t, *J*=6.4 Hz, 2 H), 3.49 - 3.60 (m, 4 H), 4.01 (br. s., 1 H), 6.67 (br. s., 1 H), 7.62 (d, *J*=6.0 Hz, 1 H); m/z (ES+APCI)⁺: 392 [M + H]⁺.

### Examples 169-171

Examples 169-171 were prepared analogously to Example 167, (the general structure is shown below followed by the tabulated examples).

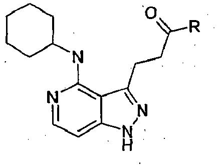

| **Example** | **R group** | **Name** | m/z (ES+APCl)⁺ | **HPLC retention time (min)*** |
|---|---|---|---|---|
| **169** | 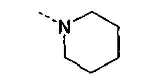 | *3-(4-Cyclohexylamino-1H-pyrazolo[4,3-c]pyridin-3-yl)-1-piperidin-1-yl-propan-1-one* | 356 | 1.76 |
| **170** | 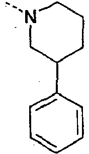 | *3-(4-Cyclohexylamino-1H-pyrazolo[4,3-c]pyridin-3-yl)-1-(3-phenyl-piperidin-1-yl)-propan-1-one* | 432 | 2.09 |
| **171** | 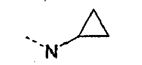 | *3-(4-Cyclohexylamino-1H-pyrazolo[4,3-c]pyridin-3-yl)-N-cyclopropyl-propionamide* | 328 | 1.48 |

| | | | | |
|---|---|---|---|---|
| *HPLC column: 4.6x50mm (5µm) C-18 Xbridge; flow rate: 3ml/min; Run time: 3.2 min: Solvent A: 0.1% Ammonium Hydroxide in water Solvent B: Acetonitrile; Gradient - 10-100%B; Gradient time: 2.35min. | | | | |

### Example 172

### 3-(4-Cyclohexylamino-1H-pyrazolo[4,3-c]pyridin-3-yl)-1-((R)-3-phenyl-piperidin-1-yl)-propan-1-one

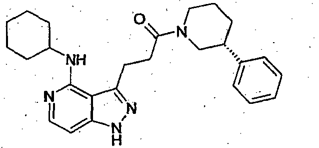

To a solution of Intermediate 23 (35 mg, 0.12 mmol) in DMF (1.5 ml) at room temperature was added HATU (48 mg, 0.13 mmol) and *N,N*-diisopropylethylamine (126 µl, 0.73 mmol). (*R*)-3-Phenylpiperidine (20 mg, 0.12 mmol) was then added, and the resulting solution was left to stir at room temperature overnight. The volatiles were removed under reduced pressure and the crude product was re-dissolved in 10% MeOH/DCM and eluted though an isolute-NH₂ cartridge. The solvents were removed and the crude product purified by preparative LCMS (high pH buffer) to give a white solid (4 mg, 8%). ¹H NMR (400 MHz, DMSO-*d*₆) δ ppm 1.13 - 1.43 (m, 6 H), 1.50 - 1.76 (m, 5 H), 1.76 - 1.87 (m, 1 H), 1.97 (br. s., 2 H), 2.32 - 2.48 (m, 1 H), 2.52 - 2.68 (m, 1 H), 2.68 - 2.94 (m, 2 H), 2.94 - 3.12 (m, 1 H), 3.12 - 3.31 (m, 2 H), 3.77 - 3.97 (m, 1 H), 3.97 - 4.10 (m, 1 H), 4.38 - 4.53 (m, 1 H), 6.13 - 6.40 (m, 1 H), 6.51 - 6.61 (m, 1 H), 7.08 - 7.35 (m, 5 H), 7.61 - 7.71 (m, 1 H); m/z (ES+APCI)⁺: 432 [M + H]⁺.

### Examples 173-209

Examples 173-209 in the table below were prepared analogously to Example 172 from Intermediate 23 and the appropriate amine:

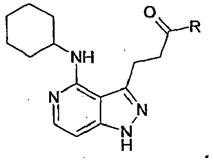

| **Example** | **R group** | **Name** | m/z (ES+APCl)⁺ | **HPLC retention time (min)*** |
|---|---|---|---|---|
| **173** | 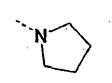 | *3-(4-Cyclohexylamino-1H-pyrazolo[4,3-c]pyridin-3-yl)-1-pyrrolidin-1-yl-propan-1-one* | 342 | 1.56 |
| **174** | 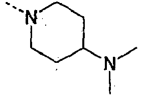 | *3-(4-Cyclohexylamino-1H-pyrazolo[4,3-c]pyridin-3-yl)-1-(4-dimethylamino-piperidin-1-yl)-propan-1-one* | 399 | 1.46 |
| **175** | 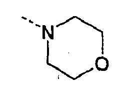 | *3-(4-Cyclohexylamino-1H-pyrazolo[4,3-c]pyridin-3-yl)-1-morpholin-4-yl-propan-1-one* | 358 | 1.46 |
| **176** | 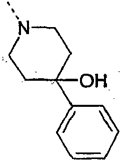 | *3-(4-Cyclohexylamino-1H-pyrazolo[4,3-c]pyridin-3-yl)-1-(4-hydroxy-4-phenyl-piperidin-1-yl)-propan-1-one* | 448 | 1.69 |
| **177** | 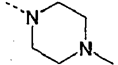 | *3-(4-Cyclohexylamino-1H-pyrazolo[4,3-c]pyridin-3-yl)-1-(4-methyl-piperazin-1-yl)-propan-1-one* | 371 | 1.40 |
| **178** | 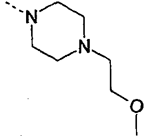 | *3-(4-Cyclohexylamino-1H-pyrazolo[4,3-c]pyridin-3-yl)-1-[4-(2-methoxy-ethyl)-piperazin-1-yl]-propan-1-one* | 415 | 1.44 |
| **179** | 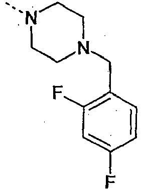 | *3-(4-Cyclohexylamino-1H-pyrazolo[4,3-c]pyridin-3-yl)-1-[4-(2,4-difluoro-benzyl)-piperazin-1-yl]-propan-1-one* | 483 | 1.94 |
| **180** | 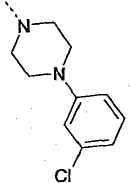 | *1-[4-(3-Chloro-phenyl)-piperazin-1-yl]-3-(4-cyclohexylamino-1H-pyrazolo[4,3-c]pyridin-3-yl)-propan-1-one* | 467 | 2.12 |
| **181** | 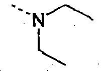 | *3-(4-Cyclohexylamino-1H-pyrazolo[4,3-c]pyridin-3-yl)-N,N-diethyl-propionamide* | 344 | 1.71 |
| **182** | 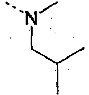 | *3-(4-Cyclohexylamino-1H-pyrazolo[4,3-c]pyridin-3-yl)-N-isobutyl-N-methyl-propionamide* | 358 | 1.87 |
| **183** | 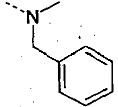 | *N-Benzyl-3-(4-cyclohexylamino-1H-pyrazolo[4,3-c]pyridin-3-yl)-N-methyl-propionamide* | 392 | 1.89 |
| **184** | 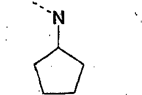 | *3-(4-Cyclohexylamino-1H-pyrazolo[4,3-c]pyridin-3-yl)-N-cyclopentyl-propionamide* | 356 | 1.73 |
| **185** | 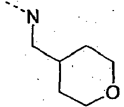 | *3-(4-Cyclohexylamino-1H-pyrazolo[4,3-c]pyridin-3-yl)-N-(tetrahydro-pyran-4-ylmethyl)-propionamide* | 386 | 1.43 |
| **186** | 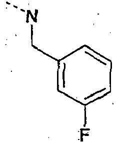 | *3-(4-Cyclohexylamino-1H-pyrazolo[4,3-c]pyridin-3-yl)-N-(3-fluoro-benzyl)-propionamide* | 396 | 1.79 |
| **187** | 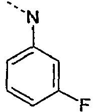 | *3-(4-Cyclohexylamino-1H-pyrazolo[4,3-c]pyridin-3-yl)-N-(3-fluoro-phenyl)-propionamide* | 382 | 1.93 |
| **188** | 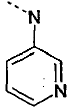 | *3-(4-Cyclohexylamino-1H-pyrazolo[4,3-c]pyridin-3-yl)-N-pyridin-3-yl-propionamide* | 365 | 1.52 |
| **189** | 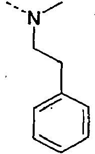 | *3-(4-Cyclohexylamino-1H-pyrazolo[4,3-c]pyridin-3-yl)-N-methyl-N-phenethyl-propionamide* | 406 | 1.96 |
| **190** | 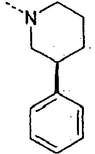 | *3-(4-Cyclohexylamino-1H-pyrazolo[4,3-c]pyridin-3-yl)-1-((S)-3-phenyl-piperidin-1-yl)-propan-1-one* | 432 | 2.09 |
| **191** | 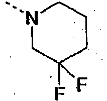 | *3-(4-Cyclohexylamino-1H-pyrazolo[4,3-6]pyridin-3-yl)-1-(3,3-difluoro-piperidin-1-yl)-propan-1-one* | 392 | 1.82 |
| **192** | 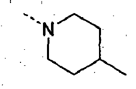 | *3-(4-Cyclohexylamino-1H-pyrazolo[4,3-c]pyridin-3-yl)-1-(4-methyl-piperidin-1-yl)-propan-1-one* | 370 | 1.93 |
| **193** | 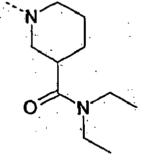 | *1-[3-(4-Cyclohexylamino-1H-pyrazolo[4,3]pyridin-3-yl)-propionyl]-piperidine-3-carboxylic acid diethylamide* | 455 | 1.64 |
| **194** | 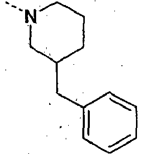 | *1-(3-Benzyl-piperidin-1-yl)-3-(4-cyclohexylamino-1H-pyrazolo[4,3-c]pyridin-3-yl)-propan-1-one* | 446 | 2.19 |
| **195** | 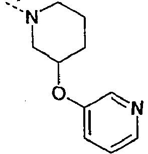 | *3-(4-Cyclohexylamino-1 H-pyrazolo*[*4*,*3*-*c*]*pyridin*-*3*-*yl*)-*1-[3-(pyridin-3-yloxy)-piperidin-1-yl]-propan-1-one* | 449 | 1.63 |
| **196** | 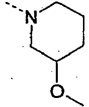 | *3-(4-Cyclohexylamino-1H-pyrazolo[4,3-c]pyridin-3-yl)-1-(3-methoxy-piperidin-1-yl)-propan-1-one* | 386 | 1.61 |
| **197** | 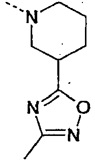 | *3-(4-Cyclohexylamino-1H-pyrazolo[4,3-c]pyridin-3-yl)-1-[3-(3-methyl-1,2,4-oxadiazol-5-yl)-piperidin-1-yl]-propan-1-one* | 438 | 1.67 |
| **198** | 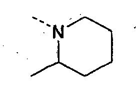 | *3-(4-Cyclohexylamino-1H-pyrazolo[4,3-c]pyridin-3-yl)-1-(2-methyl-piperidin-1-yl)-propan-1-one* | 370 | 1.86 |
| **199** | 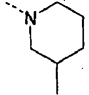 | *3-(4-Cyclohexylamino-1H-pyrazolo[4,3-c]pyridin-3-yl)-1-(3-methyl-piperidin-1-yl)-propan-1-one* | 370 | 1.88 |
| **200** | 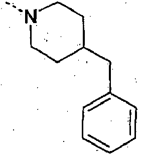 | *1-(3-Benzyl-piperidin-1-yl)-3-(4-cyclohexylamino-1H-pyrazolo[4,3-c]pyridin-3-yl)-propan-1-one* | 446 | 2.17 |
| **201** | 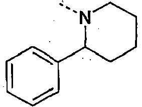 | *3-(4-Cyclohexylamino-1H-pyrazolo[4,3-c]pyridin-3-yl)-1-(2-phenyl-piperidin-1-yl)-propan-1-one* | 432 | 2.12 |
| **202** | 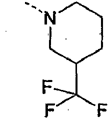 | *3-(4-Cyclohexylamino-1H-pyrazolo[4,3-c]pyridin-3-yl)-1-(3-trifluoromethyl-pipendin-1-yl)-propan-1-one* | 424 | 1.94 |
| **203** | 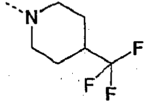 | *3-(4-Cyclohexylamino-1H-pyrazolo[4,3-c]pyridin-3-yl)-1-(4-trifluoromethyl piperidin-1-yl)-propan-1-one* | 424 | 1.88 |
| **204** | 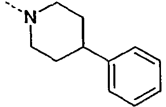 | *3-(4-Cyclohexylamino-1H-pyrazolo[4,3-c]pyridin-3-yl)-1-(4-phenyl-piperidin-1-yl)-propan-1-one* | 432 | 2.04 |
| **205** | 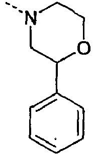 | *3-(4-Cyclohexylamino-1H-pyrazolo[4,3-c]pyridin-3-yl)-1-(2-phenyl-morpholin-4-y*/*)-propan-1-one* | 434 | 1.90 |
| **206** | 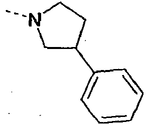 | *3-(4-Cyclohexylamino-1H-pyrazolo[4,3-c]pyridin-3-yl)-1-(3-phenyl-pyrrolidin-1-yl)-propan-1-one* | 418 | 1.94 |
| **207** | 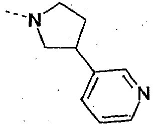 | *3-(4-Cyclohexylamino-1H-pyrazolo[4,3-c]pyridin-3-yl)-1-(3-pyridin-3-yl-pyrrolidin-1-yl)-propan-1-one* | 419 | 1.50 |
| **208** | 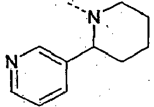 | *3-(4-Cyclohexylamino-1H-pyrazolo[4,3-c]pyridin-3-yl)-1-(3,4,5,6-tetrahydro-2H-[2,3']bipyridinyl-1-yl)-propan-1-one* | 433 | 1.67 |
| **209** | 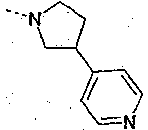 | *3-(4-Cyclohexylamino-1H-pyrazolo[4,3-c]pyridin-3-yl)-1-(3-pyridin-4-yl-pyrrolidin-1-yl)-propan-1-one* | 419 | 1.48 |

| | | | | |
|---|---|---|---|---|
| * HPLC column: 4.6x50mm (5µm) C-18 Xbridge; flow rate: 3ml/min; Run time: 3.2 min: Solvent A: 0.1% Ammonium Hydroxide in water Solvent B: Acetonitrile; Gradient - 10-100%B; Gradient time: 2.35min. | | | | |

### Example 210

### 3-(4-Cyclohexylamino-1H-pyrazolo[4,3-c]pyridin-3-yl)-N-methyl-propionamide

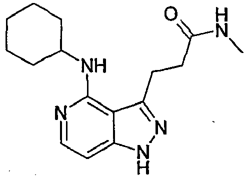

Intermediate 22 (80 mg, 0.26 mmol) was added to an excess of methylamine (33% in ethanol, 1 ml) at room temperature. The resulting mixture was irradiated at 150 °C for 1 h in a Biotage I-60 microwave reactor. The reaction mixture was then evaporated to dryness and the crude product purified by preparative LCMS (high pH buffer) to give the desired product (20 mg, 25%) ¹H NMR (400 MHz, CDCl₃) δ ppm 1.21 - 1.51 (m, 5 H), 1.58 - 1.71 (m, 1 H), 1.71 - 1.84 (m, 2 H), 2.03 - 2.32 (m, 2 H), 2.72 (t, *J*=6.9 Hz, 2 H), 2.79 (d, *J*=5.0 Hz, 3 H), 3.32 (t, *J*=7.1 Hz, 2 H), 4.08 - 4.19 (m, 1 H), 5.87 (br. s., 1 H), 5.95 - 6.08 (m, 1 H), 6.57 (d, *J*=6.0 Hz, 1 H), 7.76 - 7.81 (m, 1 H); m/z (ES+APCI)⁺: 302 [M + H]⁺.

### Example 211

### 3-(4-Cyclohexylamino-1H-pyrazolo[4,3-c]pyridin-3-yl)-N-(2-methoxy-ethyl)-propionamide

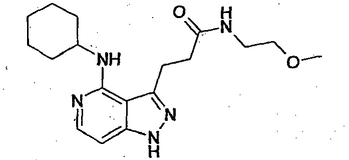

To a solution of Intermediate 22 (80 mg, 0.26 mmol) in ethanol (0.7 ml) at room temperature was added an excess of 2-methoxyethylamine (0.3 ml). The resulting mixture was irradiated at 150 °C for 1 h in a Biotage I-60 microwave reactor. Irradiation was continued at 190 °C for a further 30 mins, then the reaction mixture was evaporated to dryness and the crude product purified by mass triggered preparative LCMS (high pH buffer) to give the desired product (17mg, 19%) ¹H NMR (400 MHz, MeOD) δ ppm 1.12 - 1.38 (m, 1 H), 1.38 - 1.61 (m, 4 H), 1.61 - 1.76 (m, 1 H), 1.76 - 1.91 (m, 2 H), 2.03 - 2.15 (m, 2 H), 2.67 (t, *J*=7.1 Hz, 2 H), 3.21 (s, 3 H), 3.27 (t, *J*=7.1 Hz, 2 H), 3.29 - 3.35 (m, 4 H), 3.87 - 3.98 (m, 1 H), 6.66 (d, *J*=6.0 Hz, 1 H), 7.61 (d, 1 H); m/z (ES+APCI)⁺: 346 [M + H]⁺.

### Example 212

### 3-(4-Cyclohexylamino-1H-pyrazolo[4,3-c]pyridin~3-yl)-N,N-dimethyl-propionamide

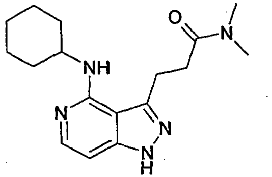

A solution of Intermediate 25 (115 mg, 0.27 mmol) in TFA (2 ml) was stirred at 70 °C for 4 h, and then allowed to cool to room temperature overnight. 2M NaOH (aq) was added and then the aqueous was extracted with EtOAc, dried (MgSO₄ and evaporated. The crude residue was purified by flash chromatography, eluting with 50% ethyl acetate/petroleum ether to 10% methanol/ethyl acetate gradient as a solid (84 mg, 100%). ¹H NMR (400 MHz, DMSO-*d*₆) δ ppm 1.15 - 1.28 (m, 1 H), 1.31 - 1.52 (m, 4 H), 1.60 - 1.68 (m, 1 H), 1.72 - 1.81 (m, 2 H), 1.95 - 2.05 (m, 2 H), 2.76 - 2.88 (m, 5 H), 2.96 (s, 3 H), 3.18 (t, *J*=6.2 Hz, 2 H), 3.87 - 3.97 (m, 1 H), 6.79 (d, *J*=6.4 Hz, 1 H), 7.60 (d, *J*=6.4 Hz, 1 H).

### Example 213

### Cyclohexyl-(3-phenethyl-1H-pyrazolo[4,3-c]pyridin-4-yl)-amine

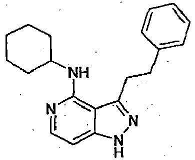

A solution of Intermediate 27 (140 mg, 0.32 mmol) in TFA (2 ml) was stirred at 70 °C for 2 h, and then allowed to cool to room temperature overnight. NH₃ (aq) was added slowly and then the aqueous was extracted with DCM, dried and evaporated. The crude residue was purified by mass triggered preparative LCMS (high Ph buffer) to give an off-white solid (36 mg, 36%) ¹H NMR (400 MHz, DMSO-*d*₆) δ ppm 1.15 - 1.24 (m, 1 H), 1.32 - 1.41 (m, 4 H), 1.58 - 1.65 (m, 1 H), 1.68 - 1.76 (m, 2 H), 1.94 - 2.02 (m, 2 H), 2.97 - 3.03 (m, 2 H), 3.27 - 3.30 (m, 2 H), 4.03 (br. s., 1 H), 5.47 (d, *J*=7.8 Hz, 1 H), 6.58 (d, *J*=6.0 Hz, 1 H), 7.17 - 7.22 (m, 1 H), 7.26 - 7.32 (m, 4 H), 7.68 (d, *J*=6.0 Hz, 1 H); m/z (ES+APCl)⁺: 321 [M + H]⁺.

### Example 214

### Cyclohexyl-[3-(2-pyridin-2-yl-ethyl)-1H-pyrazolo[4,3-c]pyridin-4-yl]-amine

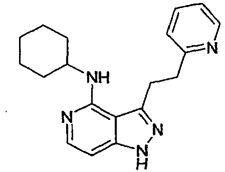

A solution of Intermediate 29 (80 mg, 0.32 mmol) in TFA (1.5 ml) was stirred at 70 °C for 24 h. The mixture was quenched by addition of ice, followed by 2M NaOH (aq) and NH₃ (aq). The aqueous was then extracted with DCM, dried and evaporated. The crude residue was purified by preparative LCMS (high pH buffer) to give the product as brown foam (6 mg, 10%). ¹H NMR (400 MHz, DMSO-*d*₆) δ ppm 1.17 - 1.26 (m, 1 H), 1.30 - 1.44 (m, 4 H), 1.60 - 1.66 (m, 1 H), 1.71 - 1.78 (m, 2 H), 1.98 - 2.04 (m, 2 H), 3.11 - 3.17 (m, 2 H), 3.33 - 3.40 (m, 2 H), 4.07 - 4.14 (m, 1 H), 5.90 (d, *J*=7.8 Hz, 1 H), 6.57 (d, *J*=6.0 Hz, 1 H), 7.23 - 7.27 (m, 1 H), 7.32 (d, *J*=7.8 Hz, 1 H), 7.66 - 7.74 (m, 2 H), 8.55 (d, *J*=4.1 Hz, 1 H). m/z (ES+APCl)⁺: 322 [M + H]⁺.

### Example 215

### Cyclohexyl-{3-[3-((S)-3-phenyl-piperidin-1-yl)-propyl]-1H-pyrazolo[4,3-c]pyridin-4-yl}-amine

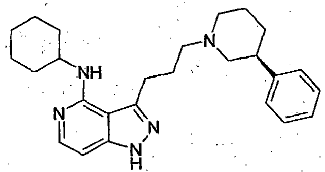

To Intermediate 35 (58 mg, 0.21 mmol) in acetonitrile (1 ml) at room temperature was added triphenylphosphine (83 mg, 0.32 mmol) and tetrabromomethane (105 mg, 0.32 mmol). The resulting mixture was stirred at room temperature for 2 hours. (*S*)-3-phenylpiperidine was added to this mixture and the resulting solution was irradiated at 100 °C for 30 mins in a Biotage I-60 microwave reactor. The reaction mixture was partitioned between water and DCM, and the organic phase was separated, dried and evaporated. The crude product purified by preparative LCMS (high pH buffer) to give the product as a gum (2 mg, 2%). ¹H NMR (400 MHz, DMSO-*d*₆) δ ppm 1.05 - 1.21 (m, 1 H), 1.28 - 1.47 (m, 2 H), 1.47 - 1.57 (m, 1 H), 1.58 - 1.78 (m, 4 H), 1.79 - 2.01 (m, 5 H), 2.06 - 2.27 (m, 2 H), 2.75 - 3.05 (m, 1 H), 3.05 - 3.26 (m, 6 H), 3.41 - 3.71 (m, 3 H), 3.82 - 4.11 (m, 1 H), 6.84 (br. s., 1 H), 7.19 - 7.49 (m, 5 H), 7.63 (d, *J*=6.0 Hz, 1 H); m/z (ES+APCl)⁺: 418 [M + H]⁺.

### Example 216

### Cyclohexyl-{3-[2-(5-phenyl-1,3,4-oxadiazol-2-yl)-ethyl]-1H-pyrazolo[4,3-c]pyridin-4-yl]-amine

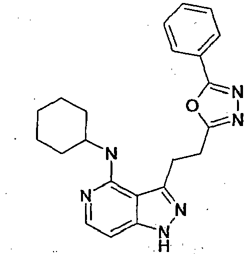

Prepared analogously to Intermediate 35 from Intermediate 33 to give the product as a white solid (4 mg, 26%). ¹H-NMR (400 MHz, DMSO-*d*₆) δ ppm 1.13 - 1.25 (m, 1 H), 1.28 - 1.45 (m, 4 H), 1.58 - 1.65 (m, 1 H), 1.69 - 1.77 (m, 2 H), 1.94 - 2.02 (m, 2 H), 3.40 (t, *J*=7.3 Hz, 2 H), 3.58 (t, *J*=7.1 Hz, 2 H), 4.01 - 4.10 (m, 1 H), 5.73 (d, *J*=7.8 Hz, 1 H), 6.59 (d, *J*=6.0 Hz, 1 H), 7.56 - 7.65 (m, 3 H), 7.69 (d, *J*=6.0 Hz, 1 H), 7.92 - 7.97 (m, 2 H); m/z (ES+APCl)⁺: 389 [M + H]⁺.

### Example 217

### Cyclohexyl-[3-(2-piperidin-4-yl-ethyl)-1H-pyrazolo[4,3-c]pyridin-4-yl]-amine

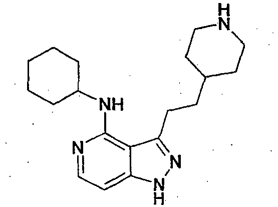

To a stirred solution of Intermediate 36 (23 mg, 0.07 mmol) in acetic acid (2 ml) at room temperature was added platinum oxide (10 mg). The resulting mixture was stirred under an atmosphere of hydrogen at room temperature overnight. The reaction mixture was then filtered through Celite^{™} and evaporated. The crude product was purified by preparative LCMS (high pH buffer) to give the desired product (4 mg, 17%). ¹H NMR (400 MHz, CDCl₃) δ ppm 1.10 - 1.36 (m, 3 H), 1.36 - 1.61 (m, 5 H), 1.62 - 1.91 (m, 7 H), 2.00 - 2.21 (m, 2 H), 2.66 - 2. 87 (m, 2 H), 2.87 - 3.14 (m, 2 H), 3.14 - 3.39 (m, 2 H), 4.10 - 4.24 (m, 1 H), 4.72 (d, *J*=7.8 Hz, 1 H), 6.60 (d, *J*=6.0 Hz, 1 H), 7.86 (d, *J*=6.0 Hz, 1 H); m/z (ES+APCl)⁺: 328 [M + H]⁺.

### Example 218

### Cyclohexyl-[3-(2-pyridin-4-yl-ethyl)-1H-pyrazolo[4,3-c]pyridin-4-yl]-amine

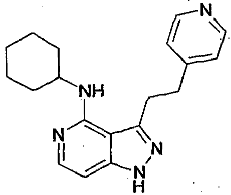

Prepared analogously to Intermediate 35 from Intermediate 37 to give the product as a white solid (4 mg, 26%). ¹H NMR (400 MHz, DMSO-*d*₆) δ ppm 1.10 - 1.24 (m, 1 H), 1.24 - 1.50 (m, 4 H), 1.50 - 1.66 (m, 1 H), 1.66 - 1.81 (m, 2 H), 1.89 - 2.04 (m, 2 H), 2.98 - 3.19 (m, 2 H), 3.19 - 3.59 (m, 2 H), 3.96 - 4.10 (m, 1 H), 5.54 (d, *J*=7.8 Hz, 1 H), 6.57 (d, *J*=6.0 Hz, 1 H), 7.29 (d, *J*=6.0 Hz, 2 H), 7.66 (d, *J*=6.0 Hz, 1 H), 8.42 - 8.48 (m, 2 H); m/z (ES+APCl)⁺: 322 [M + H]⁺.

### Example 219

### 1-{4-[2-(4-Cyclohexylamino-1H-pyrazolo[4,-3-c]pyridin-3-yl)-ethyl]-piperidin-1-yl}-ethanone

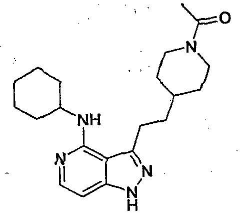

To a stirred solution of Intermediate 38 (45 mg, 0.1 mmol) and triethylamine (21 µl, 0.15 mmol) in DCM (2 ml) at 0 °C was added acetyl chloride (8 µl). The resulting mixture was allowed to warm to room temperature over 2 h. Water was added and the organic phase was collected using a phase separating cartridge and evaporated. TFA (1.5 ml) was added to the crude residue and the resulting solution was stirred at 70 °C overnight. The reaction mixture was evaporated and then partitioned between DCM, and saturated NaHCO₃ (aq). The organic phase was separated and dried using a phase separation tube and then evaporated. The crude residue was purified by preparative LCMS (high pH buffer) to give a white solid (25 mg, 67%) ¹H NMR (400 MHz, DMSO-*d*₆) δ ppm 0.92 - 1.31 (m, 3 H), 1.34 - 1.47 (m, 2 H), 1.50 - 1.89 (m, 10 H), 1.94 - 2.02 (m, 5 H), 2.93 - 3.04 (m, 1 H), 3.16 (t, *J*=7.3 Hz, 2 H), 3.29 (br. s., 1 H), 3.75 - 3.90 (m, 2 H), 4.31 - 4.39 (m, 1 H), 6.98 (d, *J*=7.3 Hz, 1 H), 7.57 (d, *J*=6.9 Hz, 1 H); m/z (ES+APCl)⁺: 370 [M + H]⁺.

### Example 220

### Cyclohexyl-{3-[2-(1-methanesulfonyl-piperidin-4-yl}-ethyl]-1H-pyrazolo[4,3-c]pyridin-4-yl}-amine

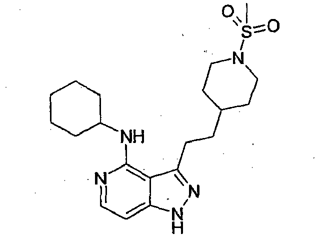

To a stirred solution of Intermediate 38 (45 mg, 0.1 mmol) and triethylamine (21 µl, 0.15 mmol) in DCM (2 ml) at 0 °C was added methanesulfonyl chloride (8.6 µl). The resulting mixture was allowed to warm to room temperature over 2 h. Water was added and the organic phase was collected using a phase separating tube and evaporated. TFA(1.5 ml) was added to the crude residue and the resulting solution was stirred at 70 °C overnight. The reaction mixture was evaporated and then partitioned between DCM, and saturated NaHCO₃ (aq). The organic phase was separated and dried using a phase separation cartridge and then evaporated. The crude residue was purified by preparative LCMS (high pH buffer) to give a white solid (11 mg, 26%) ¹H NMR (400 MHz, DMSO-*d*₆) δ ppm 1.15 - 1.27 (m, 3 H), 1.28 - 1.46 (m, 5 H), 1.58 - 1.69 (m, 3 H), 1.69 - 1.79 (m, 2 H), 1.80 - 1.88 (m, 2 H), 1.92 - 2.02 (m, 2 H), 2.60 - 2.69 (m, 2 H), 2.83 (s, 3 H), 2.99 - 3.07 (m, 2 H), 3.50 - 3.57 (m, 2 H), 3.97 - 4.06 (m, 1 H), 5.44 (br. s., 1 H), 6.59 (d, *J*=6.0 Hz, 1 H), 7.66 (d, *J*=6.0 Hz, 1 H); m/z (ES+APCl)⁺: 406 [M + H]⁺.

### Example 221

### {3-[2-(3-Amino-phenyl)-ethyl]-1H-pyrazolo[4,3-c]pyridin-4-yl}-cyclohexyl-amine

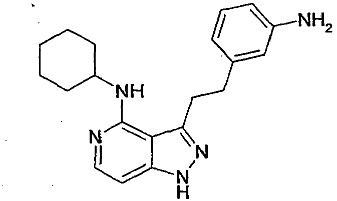

To Intermediate 40 (0.48 g, 1.3 mmol) in ethanol (10 ml) at room temperature was added 10% Pd/C (90 mg). The resulting mixture was stirred under an atmosphere of hydrogen at room temperature overnight. The reaction mixture was then filtered through Celite^{™} and evaporated. The crude residue was then purified by flash chromatography, eluting with ethyl acetate to 10% MeOH/ethyl acetate gradient to give a gum (0.21 g, 47%). ¹H NMR (400 MHz, DMSO-*d*₆) δ ppm 1.14 - 1.25 (m, 1 H), 1.25 - 1.47 (m, 4 H), 1.55 - 1.65 (m, 1 H), 1.65 - 1.77 (m, 2 H), 1.93 - 2.04 (m, 2 H), 2.78 - 2.85 (m, 2 H), 3.15 - 3.27 (m, 2 H), 3.97 - 4.10 (m, 1 H), 4.96 (s, 2 H), 5.38 (d, *J*=7.8 Hz, 1 H), 6.38 - 6.48 (m, 3 H), 6.58 (d, *J*=6.0 Hz, 1 H), 6.93 (t, *J*=7.8 Hz, 1 H), 7.67 (d, *J*=6.4 Hz, 1 H); m/z (ES+APCl)⁺: 336 [M + H]⁺.

### Example 222

### N-{3-[2-(4-Cyclohexylamino-1H-pyrazolo[4,3-c]pyridin-3-yl)-ethyl]-phenyl}-acetamide

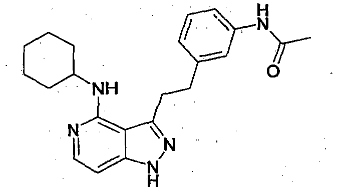

To a solution of acetic acid (7 µl, 0.12 mmol) in DMF (1 ml) at room temperature was added HATU (48 mg, 0.12 mmol) and *N,N*-diisopropylethylamine (125 µl, 0.72 mmol). Example 221 (40 mg, 0.12 mmol) was then added and the resulting solution was left to stir at room temperature overnight. The reaction mixture was diluted with DCM and washed with saturated NaHCO₃ (aq). The organic phase was separated and dried using a phase separation cartridge and the crude product purified by preparative LCMS (low pH buffer) to give the product as a white solid (4.5 mg, 10%). ¹H NMR (400 MHz, DMSO-*d*₆) δ ppm 1.13 - 1.26 (m, 1 H), 1.26 -1.42 (m, 4 H), 1.57 - 1.66 (m, 1 H), 1.66 - 1.77 (m, 2 H), 1.92 - 2.00 (m, 2 H), 2.02 (s, 3 H), 2.91 - 3.01 (m, 2 H), 3.22 - 3.31 (m, 2 H), 3.97 - 4.07 (m, 1 H), 5.42 (d, *J=7.8* Hz, 1 H), 6.58 (d, *J*=6.0 Hz, 1 H), 6.93 (d, *J*=7.8 Hz, 1 H), 7.20 (t, *J=7.8* Hz, 1 H), 7.41 (d, *J*=7.8 Hz, 1 H), 7.49 (s, 1 H), 7.67 (d, *J*=6.0 Hz, 1 H), 9.87 (s, 1 H);m/z (ES+APCl)⁺: 378 [M + H]⁺.

### Examples 223-228

Examples 223-228 in the table below were prepared analogously to Example 222:

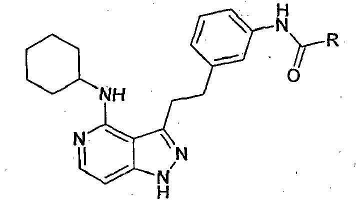

| **Example** | **R group** | **Name** | m/z (ES+APCl)⁺ | **HPLC retention time (min)*** |
|---|---|---|---|---|
| **223** | 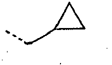 | *N-{3-[2-(4-Cyclohexylamino-1H-pyrazolo[4,3-c]pyridin-3-yl)-ethyl]-phenyl}-2-cyclopropyl-acetamide* | 418 | 1.88 |
| **224** | 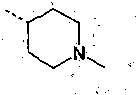 | *1-Methyl-piperidine-4-carboxylic acid {3-[2-(4-cyclohexylamino-1H-pyrazolo[4,3-c]pyridin-3-yl)-ethyl]*-*phenyl}*-*amide* | 461 | 1.70 |
| **225** | 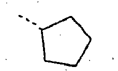 | *Cyclopentanecarboxylic acid* {*3*-*[2-(4-cyclohexylamino-1H-pyrazolo[4,3-c]pyridin-3-yl)-ethyl]-phenyl}-amide* | 432 | 2.02 |
| **226** | 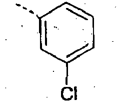 | *3-Chloro-N-{3-[2-(4-cyclohexylamino-1 H-pyrazolo[4,3-c]pyridin-3-yl)-ethyl]-phenyl}-benzamide* | 474 | 2.16 |
| **227** | 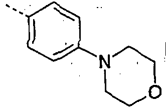 | *N-{3-[2-(4-Cyclohexylamino-1H-pyrazolo[4,3-c]pyridin-3-yl)-ethyl]-phenyl}-4-morpholin-4-yl-benzamide* | 525 | 1.92 |
| **228** | 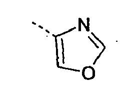 | *Oxazole-4-carboxylic acid {3-[2-(4-cyclohexylamino-1H-pyrazolo[4,3-c]pyridin-3-yl)-ethyl]-phenyl}-amide* | 431 | 1.78 |

| | | | | |
|---|---|---|---|---|
| * HPLC column: 4.6x50mm (5µm) C-18 Xbridge; flow rate: 3ml/min; Run time: 3.2 min: Solvent A: 0.1% Ammonium Hydroxide in water Solvent B: Acetonitrile; Gradient - 10-100%B; Gradient time: 2.35min. | | | | |

### Example 229

### {4-[2-(4-Cyclohexylamino-1H-pyrazolo[4,3-c]pyridin-3-yl)-ethyl]-phenyl}-(4-methyl-piperidin-1-yl)-methanone

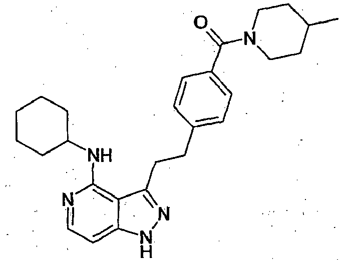

Prepared analogously to Example 172 from Intermediate 44 to give the desired product as a white solid (16 mg, 43%). ¹H NMR (400 MHz, DMSO-*d*₆) δ ppm 0.92 (d, *J*=6.0 Hz, 3 H), 0.98 - 1.25 (m, 3 H), 1.26 - 1.51 (m, 4 H), 1.51 - 1.79 (m, 6 H), 1.92 - 2.02 (m, 2 H), 2.72 (br. s., 1 H), 2.97 - 3.11 (m, 3 H), 3.27 - 3.31 (m, 2 H), 3.57 (br. s., 1 H), 3.98 - 4.09 (m, 1 H), 4.42 (br. s, 1 H), 5.53 (d, *J*=7.8 Hz, 1 H), 6.58 (d, *J*=6.0 Hz, 1 H), 7.25 - 7.35 (m, 4 H), 7.67 (d, *J*=6.0 Hz, 1 H); m/z (ES+APCl)⁺: 446 [M + H]⁺.

### Example 230

### 1-(4-Cyclohexylamino-1H-pyrazolo[4,3-c]pyridin-3-yl)-pyrrolidin-2-one

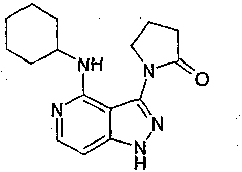

To a mixture of Intermediate 14 (100 mg, 0.2 mmol), copper iodide (10 mg, 0.054 mmol), N,N-dimethylethylenediamine (12 µl, 0.1 mmol) and potassium carbonate (45 mg, 0.32 mmol) in DMF (5 ml) at room temperature was added pyrrolidinone (25 µl, 0.32 mmol). The resulting mixture was irradiated at 150 °C for 30 mins in a Biotage I-60 microwave reactor. The reaction mixture was then partitioned between water and DCM, and the organic phase was collected, dried and evaporated. TFA (1 ml) was added to the crude residue and the resulting solution was stirred at 70 °C overnight. The reaction mixture was evaporated and then partitioned between DCM, and saturated Na₂CO₃ (aq). The organic phase was separated, dried and then evaporated. The crude residue was purified by preparative LCMS (high pH buffer) to give the product as a white solid (13 mg, 20%). ¹H NMR (400 MHz, DMSO-*d*₆) δ ppm 1.14 - 1.42 (m, 5 H), 1.52 - 1.63 (m, 1 H), 1.63 - 1.79 (m, 2 H), 1.89 - 2.01 (m, 2 H), 2.17 (quin, *J*=7.6 Hz, 2 H), 2.59 (t, *J*=8.0 Hz, 2 H), 3.89 - 4.03 (m, 3 H), 6.33 (d, *J*=7.3 Hz, 1 H), 6.60 (d, *J*=6.0 Hz, 1 H), 7.71 (d, *J*=6.0 Hz, 1 H); m/z (ES+APCl)⁺: 300 [M + H]⁺.

### Example 231

### 3-(4-Cyclohexylamino-1H-pyrazolo[4,3-c]pyridin-3-yl)-propan-1-ol

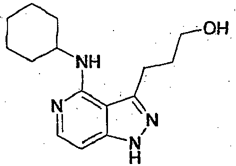

A solution of Intermediate 30 (0.74 g, 1.9 mmol) in TFA (5 ml) was stirred at 70 °C overnight. The reaction mixture was cooled to room temperature and then diluted with DCM, and saturated Na₂CO₃ (aq) was added. The organic phase was separated, filtered through a phase separation cartridge and evaporated. The crude residue was purified by flash chromatography, eluting with 20% ethyl acetate/petroleum ether to 10% methanol/ethyl acetate gradient to give a gum (0.47 g, 91%). ¹H NMR (400 MHz, DMSO-d₆) δ ppm 1.13 - 1.26 (m, 1 H), 1.32 - 1.58 (m, 4 H), 1.62 - 1.70 (m, 1 H), 1.75 - 1.88 (m, 4 H), 1.93 - 2.03 (m, 2 H), 3.04 - 3.14 (m, 2 H), 3.14 - 3.25 (m, 1 H), 3.48 (t, *J*=5.7 Hz, 2 H), 3.79 - 3.88 (m, 1 H), 5.20 (br. s., 1 H), 6.98 (d, 1 H), 7.52 - 7.68 (m, 1 H); m/z (ES+APCl)⁺: 275 [M + H]⁺.

### Example 232

### 4-[2-(4-Cyclohexylamino-1H-pyrazolo[4,3-c]pyridin-3-yl)-ethyl]-benzoic acid methyl ester

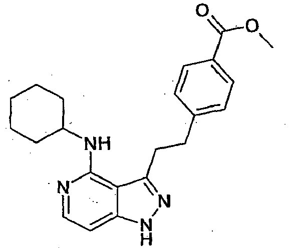

Prepared analogously to Intermediate 35 from Intermediate 42 to give the product (as a brown solid) (0.12 g, 86%). 1H NMR (400 MHz, DMSO-d6) δ ppm 1.13 - 1.24 (m, 1 H), 1.27 - 1.41 (m, 4 H), 1.58 - 1.66 (m, 1 H), 1.67 - 1.76 (m, 2 H), 1.92 - 2.01 (m, 2 H), 3.06 - 3.12 (m, 2 H), 3.34 - 3.42 (m, 2 H), 3.83 (s, 3 H), 3.99 - 4.07 (m, 1 H), 5.52 (d, *J*=7.8 Hz, 1 H), 6.58 (d, *J*=6.0 Hz, 1 H), 7.42 (d, *J*=8.2 Hz, 2 H), 7.67 (d, *J*=6.0 Hz, 1 H), 7.88 (d, *J*=8.7 Hz, 2 H); m/z (ES+APCl)⁺: 379 [M + H]⁺.

### Example 233

### Cyclohexyl-{3-[1-(2-morpholin-4-yl-ethyl)-1H-pyrazol-4-yl]-1H-pyrazolo[4,3-c]pyridin-4-yl}-amine

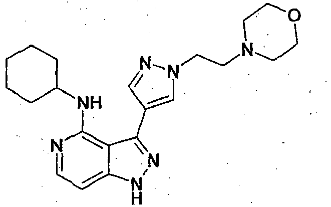

Example **233** was prepared analogously to Example 155 from Intermediate 14 and 1-(2-morpholinoethyl)-1H-pyrazole-4-boronic acid pinacol ester to give the product as a brown solid (20 mg, 39%). ¹H NMR (400 MHz, DMSO-*d*₆) 5 ppm 1.16 - 1.29 (m, 3 H), 1.32 - 1.44 (m, 2 H), 1.54 - 1.67 (m, 3 H), 1.94 - 2.02 (m, 2 H), 2.42 - 2.52 (m, 4 H), 2.82 (t, *J*=6.6 Hz, 2 H), 3.57 - 3.63 (m, 4 H), 3.98 - 4.06 (m, 1 H), 4.38 (t, *J*=6.4 Hz, 2 H), 5.19 (d, *J*=6.9 Hz, 1 H), 6.71 (d, *J*=6.0 Hz, 1 H), 7.74 - 7.79 (m, 2 H), 8.17 (s, 1 H); m/z (ES+APCl)⁺: 396 [M + H]⁺.

### Examples 234-235

Examples 234-235 in the following table were prepared analogously to Example 139 from Intermediate 14 and the appropriate boronic acid or boronic ester (the general structure is shown below followed by the tabulated examples).

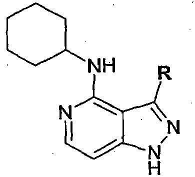

| **Example** | **R group** | **Name** | **m/z (ES+APCl)⁺** | **HPLC retention time (min)*** |
|---|---|---|---|---|
| **234** | 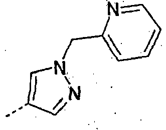 | *Cyclohexyl-[3-(1-pyridin-2-ylmethyl-1H-pyrazol-4-yl)-1H-pyrazolo*[*4*,*3-c]pyridin-4-yl]-amine* | 374 | 1.53 |
| **235** | 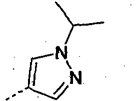 | *Cyclohexyl-[3-(1-isopropyl-1H-pyrazol-4-yl)-1H-pyrazolo[4,3-c]pyridin-4-yl]-amine* | 325 | 1.69 |

| | | | | |
|---|---|---|---|---|
| HPLC column: 4.6x50mm (5µm) C-18 Xbridge; flow rate: 3ml/min; Run time: 3.2 min: Solvent A: 0.1 % Ammonium Hydroxide in water Solvent B: Acetonitrile; Gradient - 10-100%B; Gradient time: 2.35min. | | | | |

### Example 236

### 4-Cyclohexyloxy-3-furan-3-yl-1H-pyrazolo[4,3-c]pyridine

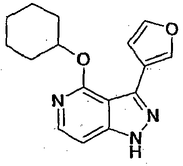

### Step 1

### 4-Cyclohexyloxy-3-furan-3-yl-1-trityl-1H-pyrazolo[4,3-c]pyridine

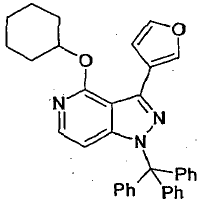

Intermediate 46 (96 mg, 0.16 mmol), Pd(dppf)Cl₂ (13 mg, 0.02 mmol), furan-3-boronic acid 28 mg, 0.25 mmol) and 2 M sodium carbonate (287 µl, 0.58 mmol) were combined in dioxane (1 ml), the solution degassed with nitrogen and then heated to 90 °C for 18 h. After evaporating the solvents, the crude material was re-dissolved in 1:1 DCM:MeOH, then dry loaded onto silica and purified by flash chromatography using a Biotage SP4 (ethyl acetate / petroleum ether gradient) to give a white solid (69 mg, 80%). ¹H NMR (400 MHz, DMSO-*d*₆) δ ppm 1.23 - 1.49 (m, 3 H), 1.55 - 1.66 (m, 3 H), 1.80 (m, *J*=8.7, 4.1 Hz, 2 H), 2.11 - 2.19 (m, 2 H), 5.22 - 5.29 (m, 1 H), 5.78 (d, *J*=6.0 Hz, 1 H), 6.79 (d, *J*=1.8 Hz, 1 H), 7.14 - 7.26 (m, 5 H), 7.27 - 7.44 (m, 10 H), 7.59 (d, *J*=6.4 Hz, 1 H), 7.76 - 7.78 (m, 1 H), 8.27 - 8.38 (m, 1 H); m/z (ES+APCl)⁺: 526 [M + H]⁺.

### Step 2

The product of step 1 (42 mg, 0.08 mmol) was dissolved in 2:8 TFA:DCM mixture and stirred at room temperature for 4 h. The solvents were evaporated and the crude material purified by preparative LCMS (high pH buffer) to give a white solid (2.8 mg, 12%). ¹H NMR (400 MHz, DMSO-*d*₆) δ ppm 1.41 (t, *J*=1.0 Hz, 3 H), 1.54 -1.68 (m, 3 H), 1.76 - 1.85 (m, 2 H), 2.12 - 2.21 (m, 2 H), 5.20 - 5.41 (m, H), 7.08 (d, *J*=0.9 Hz, 1 H), 7.11 (d, *J*=6.0 Hz, 1 H), 7.81 - 7.82 (m, 1 H), 7.89 (d, *J*=6.4 Hz, 1 H), 8.38 - 8.39 (m, 1 H); m/z (ES+APCl)⁺: 284 [M + H]⁺.

### Example 237

### 4-Cyclohexyloxy-3-pyrrolidin-1-yl-1H-pyrazolo[4,3-c]pyridine

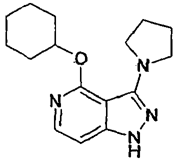

### Step 1

### 4-Cyclohexyloxy-3-pyrrolidin-1-yl-1-trityl-1H-pyrazolo[4,3-c]pyridine

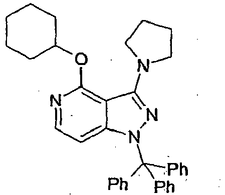

Intermediate 46 (203 mg, 0.35 mmol), pyrrolidine (327 µl, 3.99 mmol), Pd₂(dba)₃ (32 mg, 0.03 mmol), xantphos (12 mg, 0.02 mmol) and sodium *t*-butoxide (50 mg, 0.52 mmol) were combined in dioxane (3 ml). The solvent was degassed, the vial flushed out with nitrogen and the solution heated to 90 °C for 18 h. The solvents were evaporated, the crude material re-dissolved in 1:9 MeOH:DCM, directly dry loaded onto silica and purified by flash chromatography using a Biotage SP4 (ethyl acetate/ petroleum ether gradient) to give a yellow solid (142 mg, 78%). ¹H NMR (400 MHz, DMSO-*d*₆) δ ppm 1.26 - 1.46 (m, 4 H), 1.47 - 1.63 (m, 3 H), 1.72 - 1.81 (m, 2 H), 1.84 - 1.95 (m, 3 H), 2.02 - 2.10 (m, 2 H), 3.39 - 3.44 (m, 4 H), 5.17 - 5.24 (m, 1 H), 5.63 (d, *J*=6.0 Hz, 1 H), 7.17 - 7.26 (m, 5 H), 7.29 - 7.38 (m, 10 H), 7.45 (d, *J*=6.4 Hz, 1 H); m/z (ES+APCl)⁺: 529 [M + H]⁺.

### Step 2

The product of step 1 (80 mg, 0.15 mmol) was dissolved in 1:9 TFA:DCM mixture and stirred at room temperature for 1.5 h. The solvents were evaporated and the crude material purified by flash chromatography using a Biotage Isolera 4 (ethyl acetate/ petroleum ether gradient). The material was then further purified by preparative LCMS (high pH buffer) to give the product (1.5 mg, 3%). ¹H NMR (400 MHz, DMSO-*d*₆) δ ppm 1.24 - 1.67 (m, 8 H), 1.71 - 1.83 (m, 2 H), 1.86 - 1.98 (m, 4 H), 2.02 - 2.13 (m, 2 H), 3.35 - 3.41 (m, 2 H), 5.13 - 5.33 (m, 1 H), 6.86 (d, *J*=6.0 Hz, 1 H), 7.73 (d, *J*=6.0 Hz, 1 H), 12.18 (s, 1 H); m/z (ES+APCl)⁺: 287 [M + H]⁺.

### Examples 238-246

Examples 238-246 in the table below were prepared analogously to Example 168 from Intermediate 23 and the corresponding amine.

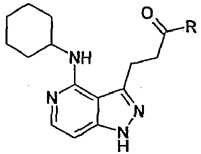

| **Example** | **R** | **Name** | m/z (ES+APCl)⁺ | **HPLC retention time (min)*** |
|---|---|---|---|---|
| 238 | 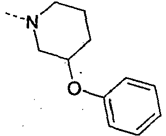 | *3-(4-Cyclohexylamino-1H-pyrazolo[4,3-c]pyridin-3-yl)-1-(3-phenoxy-piperidin-1-y*/*)-propan-1-one* | 448 | 2.04 |
| 239 | 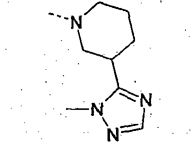 | *3-(4-Cyclohexylamino-1H-pyrazolo[4,3-c]pyridin-3-y*/*)-1-[3-(2-methyl-2H-1,2,4-triazol-3-yl)-piperidin-1-yl]-propan-1-one* | 437 | 1.33 |
| 240 | 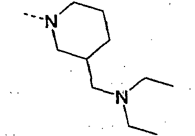 | *3-(4-Cyclohexylamino-1H-pyrazolo[4,3-c]pyridin-3-yl)-1-(3-diethylaminomethyl-piperidin-1-yl)-propan-1-one* | 441 | 2.04 |
| 241 | 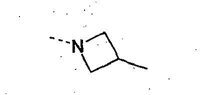 | *3-(4-Cyclohexylamino-1H-pyrazolo[4,3-c]pyridin-3-yl)-1-(3-methyl-azetidin-1-yl)-propan-1-one* | 342 | 1.54 |
| 242 | 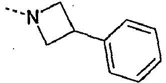 | *3-(4-Cyclohexylamino-1H-pyrazolo[4,3-c]pyridin-3-yl)-1-(3-phenyl-azetidin-1-yl)-propan-1-one* | 404 | 1.80 |
| 243 | 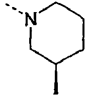 | *3-(4-Cyclohexylamino*-*1H-pyrazolo[4,3-c]pyridin-3-yl)-1-((R)-3-methyl-piperidin-1-yl)-propan-1-one* | 370 | 1.88 |
| 244 | 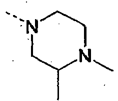 | *3-(4-Cyclohexylamino-1H-pyrazolo[4,3-c]pyridin-3-yl)-1-(3,4-dimethyl-piperazin-1-yl)-propan-1-one* | 385 | 1.44 |
| 245 | 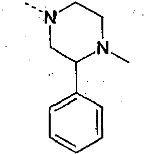 | *3-(4-Cyclohexylamino-1H-pyrazolo[4,3-c]pyridin-3-yl)-1-(4-methyl-3-phenyl-piperazin-1-yl)-propan-1-one* | 447 | 1.82 |
| 246 | 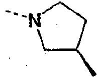 | *3-(4-Cyclohexylamino-1H-pyrazolo[4,3-c]pyridin-3-yl)-1-((R)-3-methyl-pyrrolidin-1-yl)-propan-1-one* | 356 | 1.69 |

| | | | | |
|---|---|---|---|---|
| * HPLC column: 4.6x50mm (5µm) C-18 Xbridge; flow rate: 3ml/min; Run time: 3.2 min: Solvent A: 0.1% Ammonium Hydroxide in water Solvent B: Acetonitrile; Gradient - 10-100%B; Gradient time: 2.35min. | | | | |

### Examples 247-256

Examples 247-256 in the table below were prepared analogously to Example 229, from Intermediate 44 and the appropriate amine.

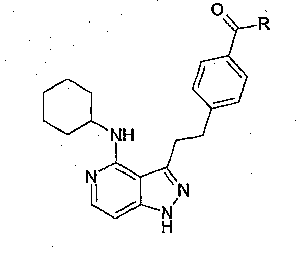

| **Example** | **R group** | **Name** | m/z (ES+APCl)⁺ | **HPLC retention time (min)*** |
|---|---|---|---|---|
| **247** | 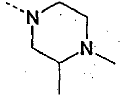 | *{4-[2-(4-Cyclohexylamino-1H-pyrazolo[4,3-c]pyridin-3-yl)-ethyl]-phenyl}-(3,4-dimethyl-piperazin-1-yl)-methanone* | 461 | 1.60 |
| **248** | 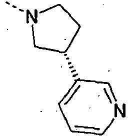 | *{4-[2-(4-Cyclohexylamino-1H-pyrazolo[4,3-c]pyrodin-3-yl)-ethyl]-phenyl}-((R)-3-pyridin-3-yl-pyrrolidin-1-yl)-methanone* | 495 | 1.66 |
| **249** | 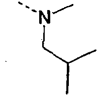 | *4-[2-(4-Cyclohexylamino-1H-pyrazolo[4,3-c]pyridin-3-yl)-ethyl]*-*N-isobutyl*-*N-methyl-benzamide* | 434 | 2.00 |
| **250** | 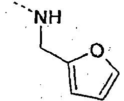 | *4-[2-(4-Cyclohexylamino-1H-pyrazolo[4,3-c]pyridin-3-yl)-ethyl]-N-furan-2-ylmethyl-benzamide* | 444 | 1.83 |
| **251** | 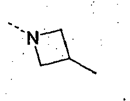 | *{4-[2-(4-Cyclohexylamino-1H-pyrazolo[4,3-c]pyridin-3-yl)-ethyl]-phenyl}-(3-methyl-azetidin-1-yl)-methanone* | 418 | 1.79 |
| **252** | 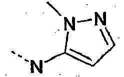 | *4-[2-(4-Cyclohexylamino-1H-pyrazolo[4,3-c]pyridin-3-yl)-ethyl]-N-(2 methyl-2H-pyrazol-3-yl)-benzamide* | 444 | 1.63 |
| **253** | 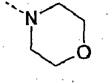 | *{4-{2-(4-Cyclohexylamino- 1H-pyrazolo[4,3-c]pyridin-3-yl)-ethyl]-phenyl}-morpholin-4-yl-methanone* | 434 | 1.64 |
| **254** | 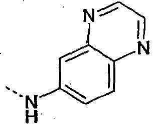 | *4-[2-(4-Cyclohexylamino-1H-pyrazolo[4,3-c]pyridin-3-yl)-ethyl]-N-quinoxalin-6-yl-benzamide* | 492 | 1.78 |
| **255** | 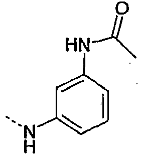 | *N*-(3-Acetylamino-phenyl)-4-[2-(4-cyclohexylamino-1*H-*pyrazolo[4,3-*c*]pyridin-3-yl)-ethyl]-benzamide | 497 | 1.68 |
| **256** | 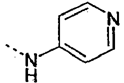 | *4-[2-(4-Cyclohexylamino-1H-pyrazolo[4,3-c]pyridin-3-yl)-ethyl]-N-pyridin-4-yl-benzamide* | 441 | 1.72 |

| | | | | |
|---|---|---|---|---|
| * HPLC column: 4.6x50mm (5µm) C-18 Xbridge; flow rate: 3ml/min; Run time: 3.2 min: Solvent A: 0.1 % Ammonium Hydroxide in water Solvent B: Acetonitrile; Gradient - 10-100%B; Gradient time: 2.35min. | | | | |

### Example 257

### 3-(4-Hydroxy-1H-pyrazolo[4,3-c]pyridin-3-yl)-1-((R)-3-phenyl-piperidin-1yl)-propan-9-one

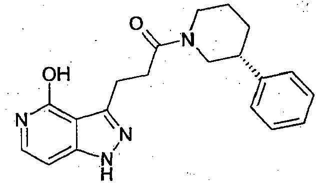

A solution of Intermediate 50 (70 mg, 0.14 mmol) in TFA (1 ml) was stirred at 50 °C for 4 h, and then allowed to cool to room temperature overnight. A further 1 ml of TFA was added, and mixture heated at 60 °C overnight, then evaporated. The crude residue was partitioned between saturated Na₂CO₃ (aq) and DCM. The organic phase was collected and dried (MgSO₄) using a phase separation tube and then evaporated. The crude residue was purified by preparative LCMS (high pH buffer) to give the product as a white solid (9 mg, 17%). ¹H NMR (400 MHz, CDCl₃) δ ppm 1.47 - 1.89 (m, 3 H), 1.96 - 2.08 (m, 1 H), 2.41 - 2.70 (m, 2 H), 2.85 - 3.14 (m, 3 H), 3.24 - 3.53 (m, 2 H), 3.99 (t, *J*=15.3 Hz, 1 H), 4.06 - 4.42 (m, 1 H), 4.73 (t, *J*=13.7 Hz, 1 H), 6.48 (t, *J*=6.9 Hz, 1 H), 6.82 - 7.11 (m, 1 H), 7.11 - 7.36 (m, 5 H), 10.20 (br. s., 1 H); m/z (ES+APCl)⁺: 351 [M + H]⁺.

### Example 258

### (E)-3-(4-Methoxy-1H-pyrazolo[4,3-c]pyridin-3-yl)-1-((R)-3-phenyl-piperidin-1-yl)-propenone

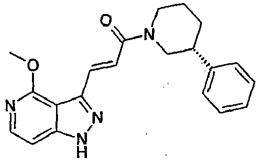

A solution of Intermediate 52 (0.1 g, 0.4 mmol) in TFA (1.5 ml) was stirred at 70 °C overnight. The reaction mixture was cooled to room temperature and then evaporated. The crude residue was re-dissolved in DCM and eluted though an Isolute-NH₂ cartridge. The solvents were removed and the crude product purified by mass triggered preparative LCMS (high pH buffer to yield a white solid (30 mg, 20%). ¹H NMR (400 MHz, DMSO-*d*₆) δ ppm 1.46 - 1.61 (m, 1 H), 1.72 - 1.90 (m, 2 H), 1.90 - 1.98 (m, 1 H), 2.61 - 2.83 (m, 2 H), 3.17 - 3.29 (m, 1 H), 3.82 - 4.25 (m, 4 H), 4.50 - 4.63 (m, 1 H), 7.15 (m, 1 H), 7.21 - 7.37 (m, 5 H), 7.63 (m, 1 H), 7.79 (m, 1 H), 7.90 (m, 1 H); m/z (ES+APCl)⁺: 363 [M + H]⁺.

### Example 259

### (E)-3-(4-Methoxy-1H-pyrazolo[4,3-c]pyridin-3-yl)-1-((R)-3-methyl-piperidin-1-yl)-propenone

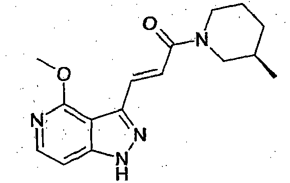

To a solution of Intermediate 51 (0.16 g, 0.47 mmol) in DMF (1.5 ml) was added HATU (0.19 g, 0.49 mmol) and *N,N*-diisopropylethylamine (492 µl, 2.83 mmol), followed by *(R)-*3-methylpiperidine (56 mg, 0.56 mmol). The resulting solution was left to stir at room temperature overnight, and then evaporated. The crude residue was re-dissolved in DCM and eluted though an Isolute-NH₂ cartridge. The solvents were removed and the crude product purified by flash chromatography eluting with 50-70% ethyl acetate/petroleum ether gradient to give a gum. TFA (1.5 ml) was then added and the resulting mixture stirred at 65 °C overnight and then evaporated. The crude residue was re-dissolved in DCM and eluted though an Isolute-NH₂ cartridge. The solvents were removed and the crude product purified by mass triggered preparative LCMS (high pH buffer to yield a white solid (30 mg, 21%). ¹H NMR (400 MHz, MeOD) δ ppm 0.77 - 1.08 (m, 3 H), 1.20 - 1.38 (m, 1 H), 1.40 - 2.09 (m, 4 H), 2.38 - 3.27 (m, 2 H), 4.00 - 4.29 (m, 4 H), 4.29 - 4.54 (m, 1 H), 7.08 (d, *J*=6.4 Hz, 1 H), 7.67 (dd, *J*=15.6, 2.7 Hz, 1 H), 7.80 - 7.98 (m, 2 H); m/z (ES+APCl)⁺: 301 [M + H]⁺.

### Example 260

### 3-(4-Methoxy-1H-pyrazolo[4,3-c]pyridin-3-yl)-1-((R)-3-phenyl-piperidin-1-yl)-propan-9-one

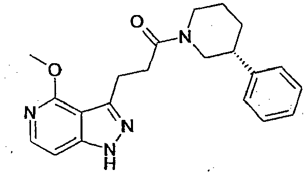

A solution of Intermediate 53 (0.15 g, 0.31 mmol) in TFA (1.5 ml) was stirred at 60 °C overnight. The reaction mixture was cooled to room temperature, evaporated, and the crude residue was re-dissolved in DCM and eluted through an SCX cartridge, efuting first with DCM, followed by 2M/NH₃ in methanol to yield a white solid (80 mg, 71%). ¹H NMR (400 MHz, DMSO-*d*₆) δ ppm 1.36 - 1.56 (m, 1 H), 1.65 - 1.79 (m, 2 H), 1.86 - 1.93 (m, 1 H), 2.51 - 2.60 (m, 1 H), 2.61 - 2.85 (m, 3 H), 3.02 - 3.20 (m, 3 H), 3.70 - 4.10 (m, 4 H), 4.40 - 4.51 (m, 1 H), 7.03 (dd, *J*=8.7, 6.0 Hz, 1 H), 7.19 - 7.34 (m, 5 H), 7.80 (dd, *J*=10.5, 6.0 Hz, 1 H); m/z (ES+APCl)⁺: 365 [M + H]⁺.

### Example 261

### (E)-3-(4-Cyclohexyloxy-1H-pyrazolo[4,3-c]pyridin-3-yl)-1-((R)-3-phenyl-piperidin-1-yl)-propenone

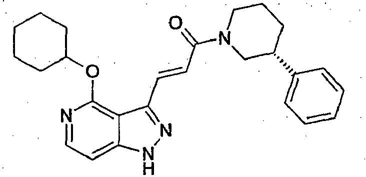

Prepared analogously to Example 172 from Intermediate 57 and *(R)*-3-phenylpiperidine to give the desired product as a white solid (2.2 mg, 2%) ¹H NMR (400 MHz, MeOD) δ ppm 1.38 - 1.60 (m, 4 H), 1.61 - 1.77 (m, 3 H), 1.84 - 2.14 (m, 6 H), 2.71 - 2.90 (m, 2 H), 3.25 - 3.43 (m, 2 H), 4.25 - 4.37 (m, 1 H), 4.71 (t, J=13.3 Hz, 1 H), 5.21 - 5.37 (m, 1 H), 7.01 - 7.13 (m, 1 H), 7.18 - 7.27 (m, 1 H), 7.29 - 7.37 (m, 4 H), 7.58 - 7.76 (m, 1 H), 7.85 (t, J=6.9 Hz, 1 H), 8.06 (dd, *J*=15.6, 10.5 Hz, 1 H); m/z (ES+APCI)⁺: 431 [M + H]⁺.

### Example 262

### N-{4-[2-(4-Cyclohexylamino-1H-pyrazolo[4,3-c]pyridin-3-yl)-ethyl]phenyl}-acetamide

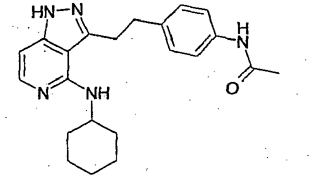

To a chromacol tube was added acetic acid (7 mg, 0.12 mmol) and HATU (48 mg, 0.125 mmol) in DMF (0.5 ml). The reaction mixture was stirred for 5 minutes followed by the addition of Intermediate 60 (40 mg, 0.12 mmol) and DIPEA (125 µl, 0.72 mmol) in DMF (0.5 ml), and the resulting mixture was allowed to stir at rt overnight. The mixture was diluted with DCM and saturated sodium bicarbonate (aq), and the organic layer was dried and concentrated. The residue was purified by mass triggered preparative HPLC (high pH buffer) to give the desired product as a white solid (26 mg, 58%). ¹H NMR (400 MHz, DMSO-d₆) δ ppm 1.13 - 1.24 (m, 1 H) 1.24 - 1.41 (m, 4 H) 1.60 (d, J=11.9 Hz, 1 H) 1.66 - 1.77 (m, 2 H) 1.91 - 2.08 (m, 5 H) 2.88 - 2.97 (m, 2 H) 3.22 - 3.31 (m, 2 H) 4.01 (br. s., 1 H) 5.43 (d, *J*=7.8 Hz, 1 H) 6.58 (d, *J*=6.0 Hz, 1 H) 7.17 (m, *J*=8.2 Hz, 2 H) 7.47 (m, *J*=8.7 Hz, 2 H) 7.66 (d, J=6.0 Hz, 1 H) 9.9 (s, 1 H); m/z (ES+APCI)⁺: 378 [M+H]⁺.

### Examples 263-276

Examples 263-276 in the table below were prepared analogously to Example 262 from Intermediate 60 and the appropriate carboxylic acid.

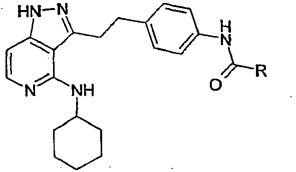

| **Example** | **R group** | **Name** | **m/z (ES+APCI)⁺** | **HPLC retention time (min)*** |
|---|---|---|---|---|
| **263** | 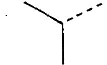 | *N-{4-[2-(4-Cyclohexylamino-1H-pyrazolo[4,3-c]pyridin-3-yl)-ethyl]phenyl}-isobutyramide* | 1.87 | 406 |
| **264** | 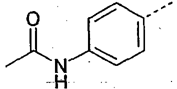 | *4-Acetylamino-N-{4-[2-(4-cyclohexylamino-1H-pyrazolo[4,3-c]pyridin-3-yl)-ethyl]-phenyl}-benzamide* | 1.70 | 497 |
| **265** | 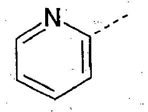 | *Pyridine-2-carboxylic acid {4-[2-(4-cyclohexylamino-1H-pyrazolo[4,3-c]pyridin-3-yl)-ethyl]phenyl}-amide* | 2.01 | 441 |
| **266** | 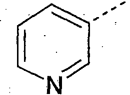 | *N-{4-[2-(4-Cyclohexylamino-1H-pyrazo*/*o[4,3-c]pyridin-3-yl)-ethyl]-phenyl}-nicotinamide* | 1.69 | 441 |
| **267** | 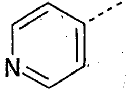 | *N-{4-[2-(4-Cyclohexylamino-1H-pyrazolo[4,3-c]pyridin-3-yl)-ethyl]-phenyl}-isonicotinamide* | 1.70 | 441 |
| **268** | 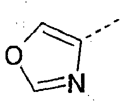 | *OXazole-4-carboxylic acid {4-[2-(4-cyclohexylamino-1H-pyrazolo[4,3-c]pyridin-3₋yl)-ethyl]-phenyl}-amide* | 1.78 | 431 |
| **269** | 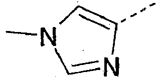 | *1-Methyl-1H-imidazole-4-carboxylic acid {4-[2-(4-cyclohexylamino-1H-pyrazolo[4,3-c]pyridin-3-yl)-ethyl]-phenyl}-amide* | 1.65 | 444 |
| **270** | 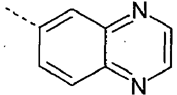 | *Quinoxaline-6-carboxylic acid {4-[2-(4-cyclohexylamino-1H-pyrazolo[4,3-c]pyridin-3-yl)-ethyl]phenyl}-amide* | 1.80 | 492 |
| **271** | 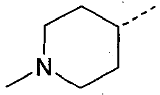 | *1-Methyl-piperidine-4-carboxylic acid {4-[2-(4-cyclohexylamino-1H-pyrazolo[4,3-c]pyridin-3-yl)-ethyl]-phenyl}-amide* | 1.68 | 461 |
| **272** | 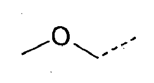 | *N-{4-[2-(4-Cyclohexylamino-1H-pyrazolo[4,3-c]pyridin-3-yl)-ethyl]-phenyl}-2-methoxy-acetamide* | 1.73 | 408 |
| **273** | 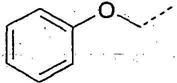 | *N-{4-[2-(4-Cyc*/*ohexylamino-1H-pyrazolo[4,3-c]pyridin-3-yl)-ethyl]phenyl}-2-phenoxy-acetamide* | 2.05 | 470 |
| **274** | 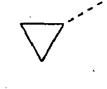 | *Cyclopropanecarboxyli c acid {4-[2-(4-cyclohexylamino-1H-pyrazolo[4,3-c]pyridin-3-yl)-ethyl]-phenyl}-amide* | 1.79 | 404 |
| **275** | 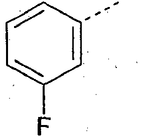 | *N-{4-[2-(4-Cyclohexylamino-1H-pyrazolo[4,3-c]pyridin-3-yl)-ethyl]-phenyl}-3-fluoro-benzamide* | 2.05 | 458 |
| **276** | 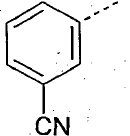 | *3-Cyano-N-{4-[2-(4-cyclohexylamino-1H-pyrazolo[4,3-c]pyridin-3-yl)-ethyl]phenyl}-benzamide* | 1.96 | 465 |

| | | | | |
|---|---|---|---|---|
| *HPLC column: 4.6x50mm (5µm) C-18 Xbridge; flow rate: 3ml/min; Run time: 3.2 min: Solvent A: 0.1% Ammonium Hydroxide in water Solvent B: Acetonitrile; Gradient - 10-100%B; Gradient time: 2.35min. | | | | |

### Examples 277-293

Examples 277-293 in the following table were prepared analogously to Example 114 from Intermediate 3 and the corresponding alcohol.

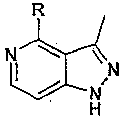

| **Example** | **R group** | **Name** | **m/z (ES+APCI)⁺** | **HPLC retention time (min)** |
|---|---|---|---|---|
| **277** | 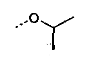 | *4-Isopropoxy-3-methyl-1H-pyrazolo[4,3-c]pyridine* | 192 | 1.59^{c} |
| **278** | 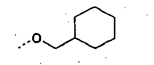 | *4-Cyclohexylmethoxy-3-methyl-1H-pycazolo[4,3-c]pyridine* | 246 | 2.07^{c} |
| **279** | 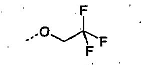 | *3-Methyl-4-*(2,2,2-*trifluoro-ethoxy)-1H-pyrazolo[4,3-c]pyridine* | 232 | 1.64^{c} |
| **280** | 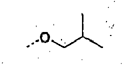 | *4-Isobutoxy-3-methyl-1H-pyrazolo[4,3-c]pyridine* | 206 | 1.69^{c} |
| **281** | 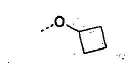 | *4-Cyclobutoxy-3-methyl-1H-pyrazolo[4,3-c]pyridine* | 204 | 1.60^{c} |
| **282** | 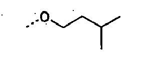 | *3-Methyl-4-(3-methyl-*butoxy)-1H-*pyrazolo[4,3-c]pyridine* | 220 | 1.86^{c} |
| **283** | 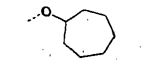 | *4-Cycloheptyloxy-3-methyl-1H-pyrazolo[4,3-c]pyridine* | 246 | 2.08^{c} |
| **284** | 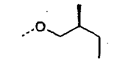 | *3-Methyl-4-((S)-2-methyl-butoxy)-1H-pyrazolo[4,3-c]pyridine* | 220 | 1.85^{c} |
| **285** | 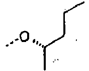 | *3-Methyl-4-((S)-1-methyl-butoxy)-1H-pyrazolo[4,3-c]pyridine* | 220 | 1.87^{c} |
| **286** | 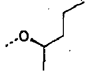 | *3-Methyl-4-((R)-1-methyl-butoxy)-1H-pyrazolo[4,3-c]pyridine* | 220 | 1.87^{c} |
| **287** | 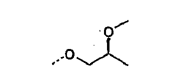 | *4-((S)-2-Methoxy propoxy)-3-methyl-1H-pyrazolo[4,3-c]pyridine* | 222 | 1.32^{c} |
| **288** | 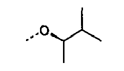 | *4-((R)-1,2-Dimethyl-propoxy)-3-methyl-1H-pyrazolo[4,3-c]pyiidine* | 220 | 1.83^{c} |
| **289** | 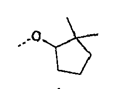 | *4-(2,2-Dimethyl-cyclopentyloxy)-3-methyl-1H-pyrazolo[4,3-c]pyridine* | 246 | 2.01^{c} |
| **290** | 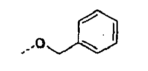 | *4-Benzyloxy-3-methyl-1H-pyrazolo[4,3-c]pyridine* | 240 | 1.73^{c} |
| **291** | 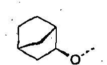 | *4-exo-Bicyclo[2.2.1]hept-2-yloxy)-3-methyl-1H-pyrazolo[4,3-c]pyridine (Racemic)* | 244 | 1.94^{c} |
| **292** | 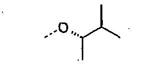 | *4-((S)-1, 2-Dimethyl-propoxy)-3-methyl-1H-pyrazolo[4,3-c]pyridine* | 220 | 1.83^{c} |
| **293** | 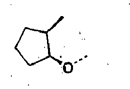 | *Cis-3-Methyl-4-(2-methyl-cyclopentyloxy)-1H-pyrazolo[4,3-c]pyridine (Racemic)* | 232 | 1.63^{c} |

| | | | | |
|---|---|---|---|---|
| ^{c} HPLC column: 4.6x50mm (5µm) C-18 Xbridge; flow rate: 3ml/min; Run time: 3.2 min: Solvent A: 0.1% Ammonium Hydroxide in water, Solvent B: Acetonitrile; Gradient - 10-100%B; Gradient time: 2.35min. ^{d} HPLC column: 4.6x50mm (5µm) C-18 Xbridge; flow rate: 3ml/min; Run time: 3.2 min: Solvent A: 0.1% Formic acid in water, Solvent B: Acetonitrile; Gradient - 10-100%B; Gradient time: 2.35min. | | | | |

### Example 294

### Trans-3-Methyl-4-(4-methyl-cyclohexyloxy)-1H-pyrazolo[4,3-c]pyridine

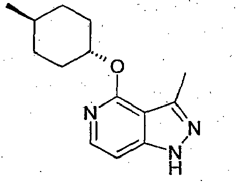

Intermediate 61 (100 mg, 0.24 mmol), *trans*-4-methylcyclohexanol (61 µl, 0.49 mmol), Pd(OAc)₂ (3.3 mg, 0.015 mmol), BINAP (12 mg, 0.02 mmol) and sodium tert-butoxide (70 mg, 0.73 mmol) were combined in toluene (3 ml). The mixture was degassed and placed under an atmosphere of nitrogen, then stirred at 100 °C for 18 h. The mixture was diluted with DCM, washed with H₂O, the organic layer was recovered using a phase separation cartridge, dried (MgSO₄) and evaporated. The crude product was dissolved in trifluoroacetic acid (0.2 ml, 2.70 mmol) in DCM (2 ml) and stirred at rt for 18 h. The reaction mixture was evaporated and then purified by cation exchange chromatography using an Isolute SCX cartridge. The crude product was then purified by preparative LCMS (high pH buffer) to give the product as a white solid (17 mg, 28%). ¹H NMR (400 MHz, DMSO-d₆) δ ppm 0.91 (d, *J*=6.9 Hz, 3 H), 1.04 - 1.16 (m, 2 H), 1.40 - 1.52 (m, 3 H), 1.70 - 1.78 (m, 2 H), 2.09 - 2.16 (m, 2 H), 2.51 (s, 3 H), 5.01 - 5.19 (m, 1 H), 6.95 (d, *J*=6.0 Hz, 1 H), 7.75 (d, *J*=6.0 Hz, 1 H); m/z (ES+APCI)⁺: 246 [M+H]⁺.

### Examples 295-300

Examples 295-300 in the table below were prepared analogously to Example 294 from Intermediate 4 and the corresponding alcohol.

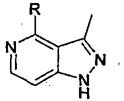

| **Example** | **R group** | **Name** | **m/z (ES+APCI)⁺** | **HPLC retention time (min)** |
|---|---|---|---|---|
| **295** | 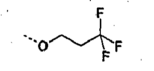 | *3-Methyl-4-(3,3,3-trifluoro-propoxy)-1H-pyrazolo[4,3-c]pyridine* | 246 | 1.57^{c} |
| **296** | 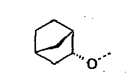 | 4-endo-*Bicyclo[2.2.1]hept-2-yloxy)-3-methyl-1H-pyrazolo[4,3-c]pyridine (Racemic)* | 244 | 1.93^{c} |
| **297** | 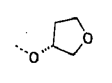 | *3-Methyl-4-[(R)-(tetrahydro-furan-3-yl)oxy]-1H-pyrazolo[4,3-c]pyridine* | 220 | 1.14^{c} |
| **298** | 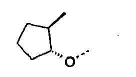 | *Trans-3-Methyl-4-(2-methyl-cyclopentyloxy)-1H-pyrazolo[4,3-c]pyridine (Racemic)* | 232 | 1.92^{c} |
| **299** | 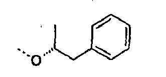 | *3-Methyl-4-((R)-1-methyl-2-phenyl-ethoxy)-1H-pyrazolo[4,3-c]pyridine* | 268 | 1.89^{c} |
| **300** | 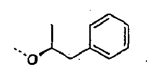 | *3-Methyl-4-((S)-1-methyl-2-phenyl-ethoxy)-1H-pyrazolo[4,3-c]pyridine* | 268 | 1.93^{c} |

| | | | | |
|---|---|---|---|---|
| ^{c} HPLC column: 4.6x50mm (5µm) C-18 Xbridge; flow rate: 3ml/min; Run time: 3.2 min: Solvent A: 0.1% Ammonium Hydroxide in water, Solvent B: Acetonitrile; Gradient - 10-100%B; Gradient time: 2.35min. | | | | |

### Examples 301-392

Examples 301-392 in the table below were prepared analogously to procedures described earlier, either by nucleophilic displacement of the 4-chloro group of Intermediate 3 with the appropriate amine (c.f. Example 1 or Example 59);
OR
Nucleophilic displacement of the 4-chloro group of Intermediate 4 with the corresponding amine (c.f. Intermediate 14), followed by removal of the protecting group (c.f. Intermediate 35);
OR
Palladium catalyzed amination of Intermediate 4 with the corresponding amine (c.f. Intermediate 7 and Intermediate 9, Step 1) followed by removal of the protecting group.

The person skilled in the art will appreciate that it may be necessary or desirable to modify the conditions for each specific compound, such as changing the number or equivalents of reagents, changing the solvent, changing the temperature, changing the reaction time. In the case of palladium catalysed reactions, using a different palladium salt, ligand or base. It may also be necessary or desirable to employ different work-up or purification techniques.

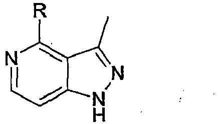

| **Example** | **R group** | **Name** | **m/z (ES+APCI)⁺** | **HPLC retention time (min)*** |
|---|---|---|---|---|
| **301** | 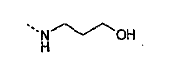 | *3-(3-Methyl-1H-pyrazolo[4,3-c]pyridin-4-ylamino)-propan-1-ol* | 207 | 0.79 |
| **302** | 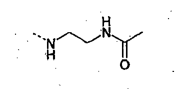 | *N-[2-(3-Methyl-1H-pyrazolo[4,3-c]pyridin-4-ylamino)-ethyl]-acetamide* | 234 | 0.76 |
| **303** | 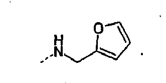 | *Furan-2-ylmethyl-(3-methyl-1H-pyrazolo[4,3-c]pyridin-4-yl)-amine* | 229 | 1.27 |
| **304** | 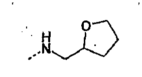 | *(3-Methyl-1H-pyrazolo[4,3-c]pyridin-4-yl)-(tetrahydro-furan-2-ylmethyl)-amine* | 233 | 1.10 |
| **305** | 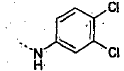 | *(3,4-Dichloro-phenyl)-(3-methyl-1H-pyrazolo[4,3-c]pyridin-4-yl)-amine* | 293 | 1.94 |
| **306** | 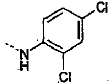 | *(2,4-Dichloro-phenyl)-(3-methyl-1H-pyrazolo[4,3-c]pyridin-4-yl)-amine* | 293 | 2.12 |
| **307** | 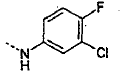 | *(3-Chloro-4-fluoro-phenyl)-(3-methyl-1H-pyrazolo[4,3-c]pyridin-4-yl)-amine* | 277 | 1.76 |
| **308** | 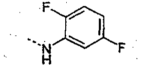 | *(2,5-Difluoro-phenyl)-(3-methyl-1H-pyrazolo[4,3-c]pyridin-4-yl)-amine* | 261 | 1.75 |
| **309** | 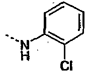 | *(2-Chloro-phenyl)-(3-methyl-1H-pyrazolo[4,3-c]pyridin-4-yl)-amine* | 259 | 1.82 |
| **310** | 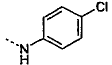 | *(4-Chloro-phenyl)-(3-methyl-1H-pyrazolo[4,3-c]pyridin-4-yl)-amine* | 259 | 1.73 |
| **311** | 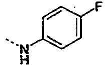 | *(4-Fluoro-phenyl)-(3-methyl-1H-pyrazolo[4,3-c]pyridin-4-yl)-amine* | 243 | 1.51 |
| **312** | 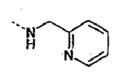 | *(3-Methyl-1H-pyrazolo[4,3-c]pyridin-4-yl)-pyridin-*2-*ylmethyl-amine* | 240 | 1.11 |
| **313** | 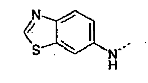 | *Benzothiazol-6-yl-*(3-*methyl-1H-pyrazolo[4,3-c]pyridin-4-yl)-amine* | 282 | 1.33 |
| **314** | 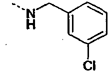 | *(3-Chloro-benzyl)-(3-methyl-1H-pyrazolo[4,3-c]pyridin-4-yl)-amine* | 273 | 1.63 |
| **315** | 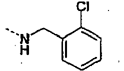 | *(2-Chloro-benzyl)-(3-methyl-1H-pyrazolo[4,3-c]pyridin-4-yl)-amine* | 273 | 1.65 |
| **316** | 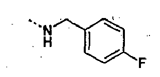 | *(4-Fluoro-benzyl)-(3-methyl-1H-pyrazolo[4,3-c]pyridin-4-yl)-amine* | 257 | 1.52 |
| **317** | 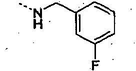 | *(3-Fluoro-benzyl)-(3-methyl-1H-pyrazolo[4,3-c]pyridin-4-yl)-amine* | 257 | 1.51 |
| **318** | 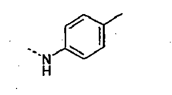 | *(4-Methyl-phenyl)-(3-methyl-1H-pyrazolo[4,3-c]pyridin-4-yl)-amine* | 239 | 1.61 |
| **319** | 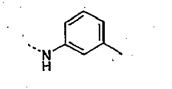 | *(3-Methyl-phenyl)-(3-methyl-1H-pyrazolo[4,3-c]pyridin-4-yl)-amine* | 239 | 1.63 |
| **320** | 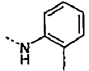 | *(2-Methyl-phenyl)-(3-methyl-1H-pyrazolo[4,3-c]pyridin-4-yl)-amine* | 239 | 1.50 |
| **321** | 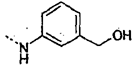 | *[3-(3-Methyl-1H-pyrazolo[4,3-c]pyridin-4-ylamino)-phenyl]-methanol* | 255 | 1.14 |
| **322** | 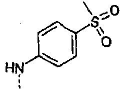 | *(4-Methanesulfonyl-phenyl)-(3-methyl-1H-pyrazolo[4,3-c]pyridin-4-yl)-amine* | 303 | 1.23 |
| **323** | 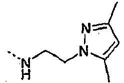 | *[2-(3,5-Dimethyl-pyrazol-1-yl)-ethyl]-(3-methyl-1H-*pyrazolo*[4,3-c]*pyridin-*4-yl)-amine* | 271 | 1.25 |
| **324** | 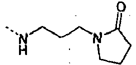 | *1-[3-(3-Methyl-1H-pyrazolo[4,3-c]pyridin-4-ylamino)-propyl]-pyrrolidin-2-one* | 274 | 1.01 |
| **325** | 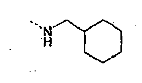 | *Cyclohexylmethyl-(3-methyl-1H-pyrazolo[4,3-c]pyridin-4-yl)-amine* | 245 | 1.69 |
| **326** | 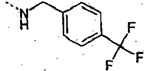 | *(3-Methyl-1H-pyrazolo[4,3-c]pyridin-4-yl)-(4-trifluoromethyl-benzyl)-amine* | 307 | 1.74 |
| **327** | 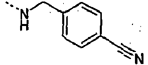 | *4-(3-Methyl-1H-pyrazolo[4,3-c]pyridin-4-ylamino)-methyl]-benzonitrile* | 264 | 1.37 |
| **328** | 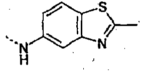 | *(2-Methyl-benzothiazol-5-yl)-(3-methyl-1H-pyrazolo[4,3-c]pyridin-4-yl)-amine* | 296 | 1.45 |
| **329** | 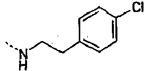 | *[2-(4-Chloro-phenyl)-ethyl]-(3-methyl-1H-pyrazolo[4,3-c]pyridin-4-yl)-amine* | 287 | 1.70 |
| **330** | 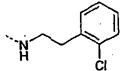 | *[2-(2-Chloro-phenyl)-ethyl]-(3-methyl-1H-pyrazolo[4,3-c]pyridin-4-yl)-amine* | 287 | 1.68 |
| 331 | 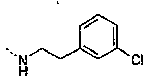 | *[2-(3-Chloro-phenyl)-ethyl]-(3-methyl-1H-pyrazolo[4,3-c]pyridin-4-yl)-amine* | 287 | 1.69 |
| **332** | 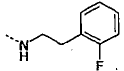 | *[2-(2-Fluoro-phenyl)-ethyl]-(3-methyl-1H-pyrazolo[4,3-c]pyridin-4-yl)-amine* | 271 | 1.56 |
| **333** | 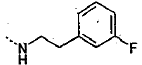 | *[2-(3-Fluoro-phenyl)-ethyl]-(3-methyl-1H-pyrazolo[4,3-c]pyridin-4-yl)-amine* | 271 | 1.59 |
| **334** | 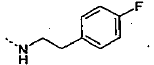 | *[2-(4-Fluoro-phenyl)-ethyl]-(3-methyl-1H-pyrazolo[4,3-c]pyridin-4-yl)-amine* | 271 | 1.57 |
| **335** | 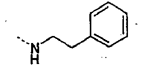 | *(3-Methyl-1H-pyrazolo[4,3-c]pyridin-4-yl)-phenethyl-amine* | 253 | 1.54 |
| **336** | 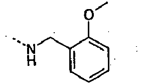 | *(2-Methoxy-benzyl)-(3-methyl-1H-pyrazolo[4,3-c]pyridin-4-yl)-amine* | 269 | 1.51 |
| **337** | 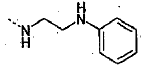 | *N-(3-Methyl-1H-pyrazolo[4,3-c]pyridin-4-yl)-N'-phenyl-ethane-1,2-diamine* | 268 | 1.47 |
| **338** | 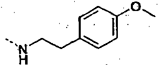 | *[2-(4-Methoxy-phenyl)-ethyl]-(3-methyl-1H-pyrazolo[4,3-c]pyridin-4-yl)-amine* | 283 | 1.15 |
| **339** | 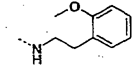 | *[2-(2-Methoxy-phenyl)-ethyl]-(3-methyl-1H-pyrazolo[4,3-c]pyridin-4-yl)-amine* | 283 | 0.97 |
| **340** | 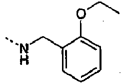 | *(2-Ethoxy-benzyl)-(3-methyl-1H-pyrazolo[4,3-c]pyridin-4-yl)-amine* | 283 | 1.28 |
| **341** | 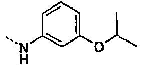 | *(3-Isopropoxy-phenyl)-(3-methyl-1H-pyrazolo[4,3-c]pyridin-4-yl)-amine* | 283 | 1.49 |
| **342** | 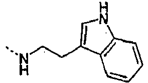 | *[2-(1H-Indol-3-yl)-ethyl]-(3-methyl-1H-pyrazolo[4,3-c]pyridin-4-yl)-amine* | 292 | 1.47 |
| **343** | 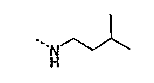 | *(3-Methyl-butyl)-(3-methyl-1H-pyrazolo[4,3-c]pyridin-4-yl)-amine* | 219 | 1.52 |
| **344** | 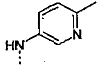 | *(3-Methyl-1H-pyrazolo[4,3-c]pyridin-4-yl)-(6-methyl-pyridin-3-yl)-amine* | 240 | 1.15 |
| **345** | 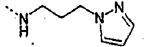 | *(3-Methyl-1H-pyrazolo[4,3-c]pyridin-4-yl)-(3-pyrazol-1-yl-propyl)-amine* | 257 | 1.11 |
| **346** | 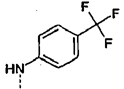 | *(3-Methyl-1H-pyrazolo[4,3-c]pyridin-4-y*/*)-(4-trifluoromethyl-phenyl)-amine* | 293 | 1.85 |
| **347** | 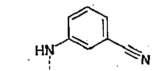 | *3-(3-Methyl-1H-pyrazolo[4,3-c]pyridin-4-ylamino)-benzonitrile* | 250 | 1.47 |
| **348** | 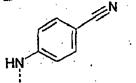 | *4-(3-Methyl-1H-pyrazolo[4,3-c]pyridin-4-ylamino)-benzonitrile* | 250 | 1.45 |
| **349** | 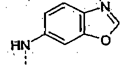 | *Benzoxazol-6-yl-(3-methyl-1H-pyrazolo[4,3-c]pyridin-4-yl)-amine* | 266 | 1.23 |
| **350** | 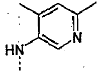 | *(4,6-Dimethyl-pyridin-3-yl)-(3-methyl-1H-pyrazolo[4,3-c]pyridin-4-yl)-amine* | 254 | 1.16 |
| **351** | 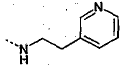 | *(3-Methyl-1H-pyrazolo[4,3-c]pyridin-4-yl)-(2-pyridin-3-yl-ethyl)-amine* | 254 | 1.07 |
| **352** | 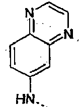 | *(3-Methyl-1H-pyrazolo[4,3-c]pyridin-4-yl)-quinoxalin-6-yl amine* | 277 | 1.18 |
| **353** | 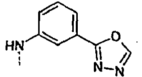 | *(3-Methyl-1H-pyrazolo[4,3-c]pyridin-4-yl)-(3-1,3,4-oxadiazol-2-yl-phenyl)-amine* | 293 | 1.27 |
| **354** | 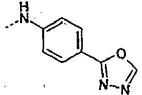 | *(3-Methyl-1H-pyrazolo[4,3-c]pyridin-4-yl)-(4-1,3,4-oxadiazol-2-yl-phenyl)-amine* | 293 | 1.23 |
| **355** | 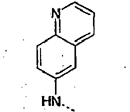 | *(3-Methyl-1H-pyrazolo[4,3-c]pyridin-4-yl)-quinolin-6-yl-amine* | 276 | 1.29 |
| **356** | 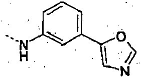 | *(3-Methyl-1H-pyrazolo[4,3-c]pyridin-4-yl)-(3-oxazol-5-yl-phenyl)-amine* | 292 | 1.41 |
| **357** | 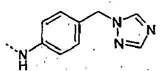 | *(3-Methyl-1H-pyrazolo[4,3-c]pyridin-4-yl)-(4-1,2,4-triazol-1-ylmethyl-phenyl)-amine* | 306 | 1.13 |
| **358** | 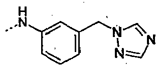 | *(3-Methyl-1H-pyrazolo[4,3-c]pyridin-4-yl)-(3-1,2,4-triazol-1-ylmethyl-phenyl)-amine* | 306 | 1.17 |
| **359** | 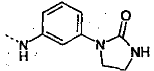 | *1-[3-(3-Methyl-1H-pyrazolo[4,3-c]pyridin-4-ylamino)-phenyl]-imidazolidin-2-one* | 309 | 1.17 |
| **360** | 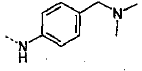 | (4-*Dimethylaminomethyl phenyl)-(3-methyl-1H-pyrazolo[4,3-c]pyridin-4-yl)-amine* | 282 | 1.38 |
| **361** | 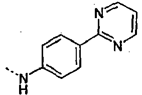 | *(3-Methyl-1H-pyrazolo[4,3-c]pyridin-4-yl)-(4-pyrimidin-2-yl-phenyl)-amine* | 303 | 1.40 |
| **362** | 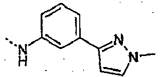 | *(3-Methyl-1H-pyrazolo[4,3-c]pyridin-4-yl)-[3-(1-methyl-1H-pyrazol-3-yl)-phenyl]-amine* | 305 | 1.43 |
| **363** | 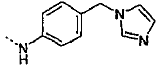 | *(4-Imidazol-1-ylmethyl-phenyl)-(3-methyl-1H-pyrazolo[4,3-c]pyridin-4-yl)-amine* | 305 | 1.41 |
| **364** | 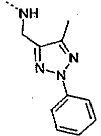 | *(5-Methyl-2-phenyl-2H-1,2,3-triazol-4-ylmethyl)-(3-methyl-1H-pyrazolo[4,3-c]pyridin-4-yl)-amine* | 320 | 1.70 |
| **365** | 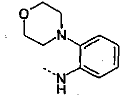 | *(3-Methyl-1H-pyrazolo[4,3-c]pyridin-4-yl)-(2-morpholin-4-yl-phenyl)-amine* | 310 | 1.68 |
| **366** | 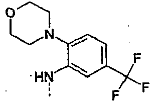 | *(5-Trifluoromethyl-2-morpholin-4-yl-phenyl)-(3-methyl-1H-pyrazolo[4,3-c]pyridin-4-yl)-amine* | 378 | 1.97 |
| **367** | 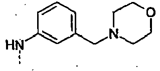 | *(3-Methyl-1H-pyrazolo[4,3-c]pyridin-4-yl)-(3-morpholin-4-ylmethyl-phenyl)-amine* | 324 | 1.34 |
| **368** | 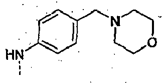 | *(3-Methyl-1H-pyrazolo[4,3-c]pyridin-4-yl)-(4-morpholin-4-ylmethyl-phenyl)-amine* | 324 | 1.29 |
| **369** | 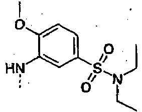 | *N,N-Diethyl-4-methoxy-3-(3-methyl 1H-pyrazolo[4,3-c]pyridin-4-ylamino)-benzenesulfonamide* | 390 | 1.82 |
| **370** | 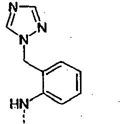 | *(3-Methyl-1H-pyrazolo[4,3-c]pyridin-4-yl)-(2-1,2,4-triazol-1-ylmethyl-phenyl)-amine* | 306 | 1.12 |
| **371** | 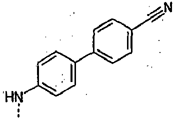 | *4'-(3-Methyl-1H-pyrazolo[4,3-c]pyridin-4-ylamino)-biphenyl-4-carbonitrile* | 326 | 1.82 |
| **372** | 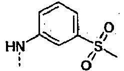 | *(3-Methanesulfonyl-phenyl)-(3-methyl-1H-pyrazolo[4,3-c]pyridin-4-yl)-amine* | 303 | 1.23 |
| **373** | 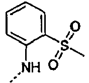 | *(2-Methanesulfonyl-phenyl)-(3-methyl-1H-pyrazolo[4,3-c]pyridin-4-yl)-amine* | 303 | 1.39 |
| **374** | 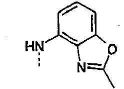 | *(2-Methyl-benzoxazol-4-yl)-(3-methyl-1H-pyrazolo[4,3-c]yridin-4-yl)-amine* | 280 | 1.64 |
| **375** | 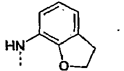 | *(2,3-Dihydro-benzofuran-7-yl)-(3-methyl-1H-pyrazolo[4,3-c]pyridin-4-yl)-amine* | 267 | 1.58 |
| **376** | 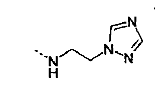 | *(3-Methyl-1H-pyrazolo[4,3-c]pyridin-4-yl)-(2-1,2,4-triazol-1-yl-ethyl)-amine* | 244 | 0.81 |
| **377** | 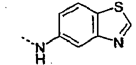 | *Benzothiazol-5-yl-(3-methyl-1H-pyrazolo[4,3-c]pyridin-4-yl)-amine* | 282 | 1.34 |
| **378** | 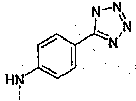 | *(3-Methyl-1H-pyrazolo[4,3-c]pyridin-4-yl)-[4-(1H-tetrazol-5-yl)-phenyl]-amine* | 293 | 0.50 |
| **379** | 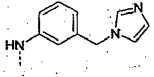 | *(3-Imidazol-1-ylmethyl-pheny*/*)-(3-methy*/*-1H-pyrazolo[4,3-c]pyridin-4-yl)-amine* | 306 | 1.08 |
| **380** | 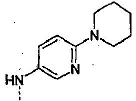 | *(3-Methyl-1H-pyrazolo[4,3-c]pyridin-4-yl)-(3,4,5,6-tetrahydro-2H-[1,2']bipyridinyl-5'-yl)-amine* | 309 | 1.07 |
| **381** | 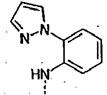 | *(3-Methyl-1H-pyrazolo[4,3-c]pyridin-4-yl)-(2-pyrazol-1-yl-phenyl)-amine* | 291 | 1.27 |
| **382** | 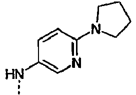 | *(3-Methyl-1H-pyrazolo[4,3-c]pyridin-4-yl)-(6-pyrrolidin-1-yl-pyridin-3-yl)-amine* | 295 | 1.15 |
| **383** | 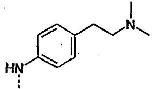 | *[4-(2-Dimethylamino-ethyl)-phenyl]-(3-methyl-1H-pyrazolo[4,3-c]pyridin-4-yl)-amine* | 296 | 1.57 |
| **384** | 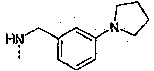 | *(3-Methyl-1H-pyrazolo[4,3-c]pyridin-4-yl)-(3-pyrrolidin-1-yl benzyl)-amine* | 308 | 1.78 |
| **385** | 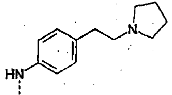 | *(3-Methyl-1H-pyrazolo[4,3-c]pyridin-4-yl)-[4-(2-pyrrolidin-1-yl-ethyl)-phenyl]-amine* | 322 | 1.36 |
| **386** | 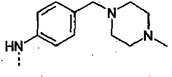 | *4-(4-Methyl-piperazin-1-ylmethyl)-phenyl]-(3-methyl-1H-pyrazolo[4,3-c]pyridin-4-yl)-amine* | 337 | 1.20 |
| **387** | 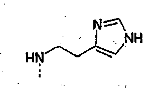 | *[2-(1H-Imidazol-4-yl)-ethyl]-(3-methyl-1H-pyrazolo[4,3-c]pylidin-4-yl)-amine* | 243 | 0.85 |
| **388** | 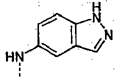 | *(1H-Indazol-5-yl)-(3-methyl-1H-pyrazolo[4,3-c]pyridin-4-yl)-amine* | 265 | 1.09 |
| **389** | 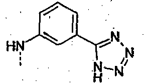 | *(3-Methyl-1H-pyrazolo[4,3-6]pyridin-4-yl)-[3-(1H-tetrazol-5-yl)-phenyl]-amine* | 293 | 0.33 |
| **390** | 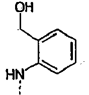 | *[2-(3-Methyl-1H-pyrazolo[4,3-c]pyiidin-4-ylamino)-phenyl]-methanol* | 255 | 1.23 |
| **391** | 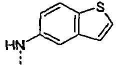 | *Benzo*[*b*]*thiophen*-*5*-*yl-(3-methyl-1H-pyrazolo[4,3-c]pyridin-4-yl)-amine* | 281 | 1.72 |
| **392** | 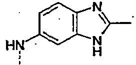 | *(2-Methyl-3H-benzoimidazol-5-yl)-(3-methyl*-*1H-pyrazolo[4,3-c]pyridin-4-yl)-amine* | 279 | 1.01 |

| | | | | |
|---|---|---|---|---|
| *HPLC column: 4.6x50mm (5µm) C-18 Xbridge; flow rate: 3ml/min; Run time: 3.2 min: Solvent A: 0.1% Ammonium Hydroxide in water, Solvent B: Acetonitrile; Gradient - 10-100%B; Gradient time: 235min. | | | | |

### Results

### LRRK2 Potency

Potency scores for selected compounds of the invention against LRRK2 are shown in Table 1.

### Kinase selectivity data

Kinase selectivity data of representative compounds is shown in Table 2. Values are expressed as percentage inhibition of the each specific kinase at 1µM inhibitor concentration.

Various modifications and variations of the described aspects of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes of carrying out the invention which are obvious to those skilled in the relevant fields are intended to be within the scope of the following claims.

### REFERENCES

1 Paisan-Ruiz, C., Jain, S., Evans, E. W., Gilks, W. P., Simon, J., van der Brug, M., Lopez de Munain, A., Aparicio, S., Gil, A. M., Khan, N., Johnson, J., Martinez, J. R., Nicholl, D., Carrera, I. M., Pena, A. S., de Silva, R., Lees, A., Marti-Masso, J. F., Perez-Tur, J., Wood, N. W. and Singleton, A. B. (2004) Cloning of the gene containing mutations that cause PARK8-linked Parkinson's disease. Neuron. 44, 595-600
2 Mata, I. F., Wedemeyer, W. J., Farrer, M. J., Taylor, J. P. and Gallo, K. A. (2006) LRRK2 in Parkinson's disease: protein domains and functional insights. Trends Neurosci. 29, 286-293
3 Taylor, J. P., Mata, I. F. and Farrer, M. J. (2006) LRRK2: a common pathway for parkinsonism, pathogenesis and prevention? Trends Mol Med. 12, 76-82
4 Farrer, M., Stone, J., Mata, I. F., Lincoln, S., Kachergus, J., Hulihan, M., Strain, K. J. and Maraganore, D. M. (2005) LRRK2 mutations in Parkinson disease. Neurology. 65, 738-740
5 Zabetian, C. P., Samii, A., Mosley, A. D., Roberts, J. W., Leis, B. C., Yearout, D., Raskind, W. H. and Griffith, A. (2005) A clinic-based study of the LRRK2 gene in Parkinson disease yields new mutations. Neurology. 65, 741-744
6 Bosgraaf, L. and Van Haastert, P. J. (2003) Roc, a Ras/GTPase domain in complex proteins. Biochim Biophys Acta. 1643, 5-10
7 Marin, I. (2006) The Parkinson disease gene LRRK2: evolutionary and structural insights. Mol Biol Evol. 23, 2423-2433
8 Manning, G., Whyte, D. B., Martinez, R., Hunter, T. and Sudarsaham, S. (2002) The protein kinase complement of the human genome. Science. 298, 1912-1934
9 West, A. B., Moore, D. J., Biskup, S., Bugayenko, A., Smith, W. W., Ross, C. A., Dawson, V. L. and Dawson, T. M. (2005) Parkinson's disease-associated mutations in leucine-rich repeat kinase 2 augment kinase activity. Proc Natl Acad Sci U S A. 102, 16842-16847
10 Greggio, E., Jain, S., Kingsbury, A., Bandopadhyay, R., Lewis, P., Kaganovich, A., van der Brug, M. P., Beilina, A., Blackinton, J., Thomas, K. J., Ahmad, R., Miller, D. W., Kesavapany, S., Singleton, A., Lees, A., Harvey, R. J., Harvey, K. and Cookson, M. R. (2006) Kinase activity is required for the toxic effects of mutant LRRK2/dardarin. Neurobiol Dis. 23, 329-341
11 Jaleel, M., Nichols, R. J., Deak, M., Campbell, D. G., Gillardon, F., Knebel, A. and Alessi, D. R. (2007) LRRK2 phosphorylates moesin at threonine-558: characterization of how Parkinson's disease mutants affect kinase activity. Biochem J. 405, 307-317
12 Goldberg, J. M., Bosgraaf, L., Van Haastert, P. J. and Smith, J. L. (2002) Identification of four candidate cGMP targets in Dictyostelium. Proc Natl Acad Sci U S A. 99, 6749-6754
13 Bosgraaf, L., Russcher, H., Smith, J. L., Wessels, D., Soll, D. R. and Van Haastert, P. J. (2002) A novel cGMP signalling pathway mediating myosin phosphorylation and chemotaxis in Dictyostelium. Embo J. 21, 4560-4570
14 Cohen, P. and Knebel, A. (2006) KESTREL: a powerful method for identifying the physiological substrates of protein kinases. Biochem J. 393, 1-6
15 Bretscher, A., Edwards, K. and Fehon, R. G. (2002) ERM proteins and merlin: integrators at the cell cortex Nat Rev Mol Cell Biol. 3, 586-599
16 Polesello, C. and Payre, F. (2004) Small is beautiful: what flies tell us about ERM protein function in development. Trends Cell Biol. 14, 294-302
17 Nichols, R. J., Dzamko, N., Hutti, J. E., Cantley, L. C., Deak, M., Moran, J., Bamborough, P., Reith, A. D. and Alessi, D. R. (2009) Substrate specificity and inhibitors of LRRK2, a protein kinase mutated in Parkinson's disease. Biochem J. 424, 47-60

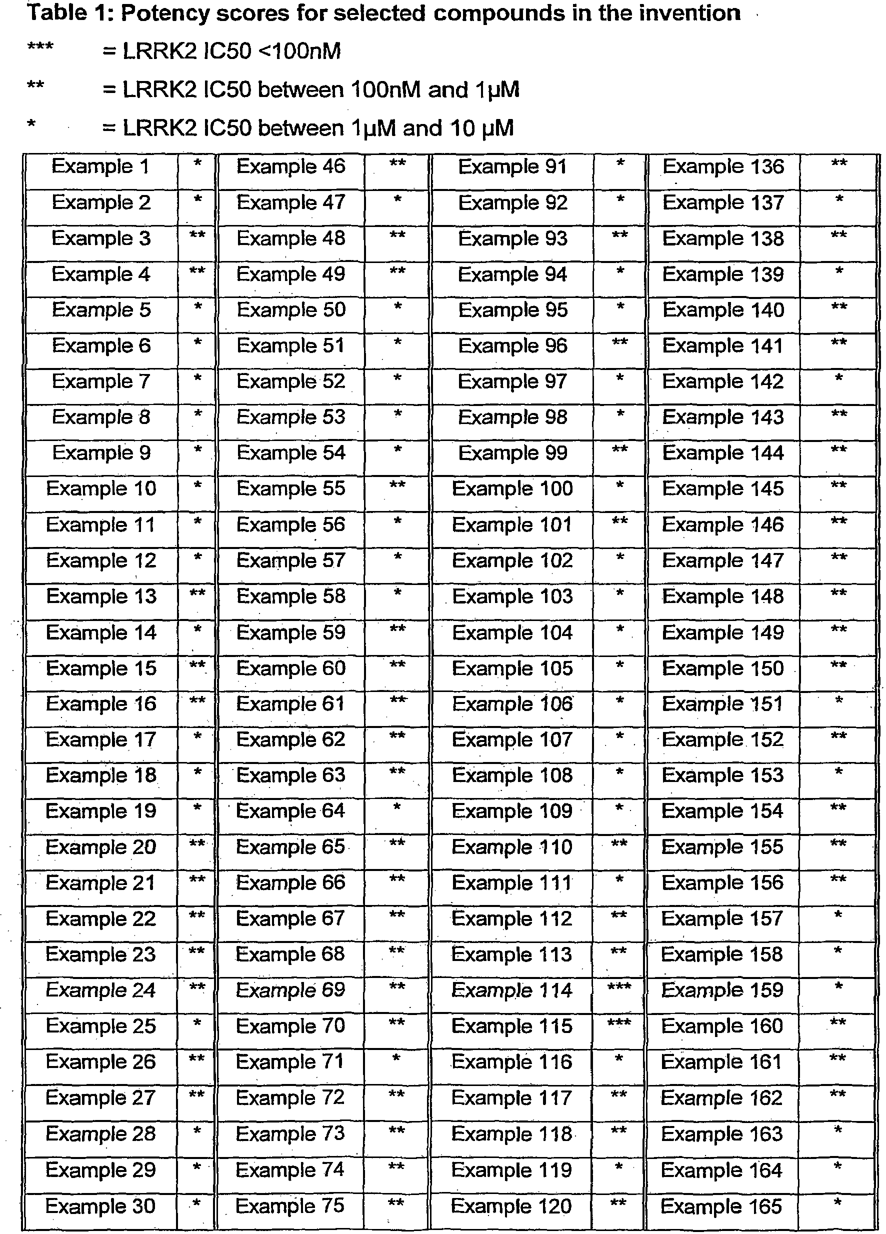

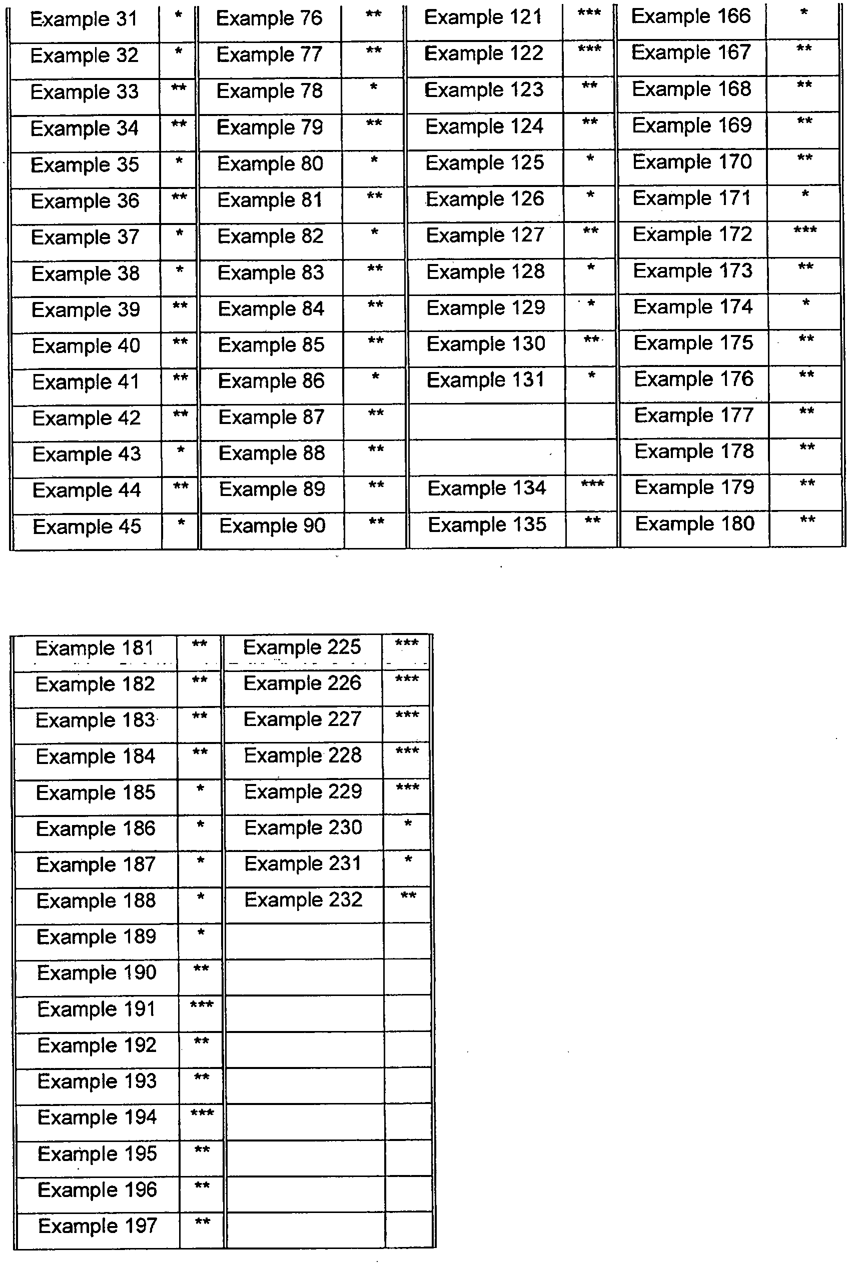

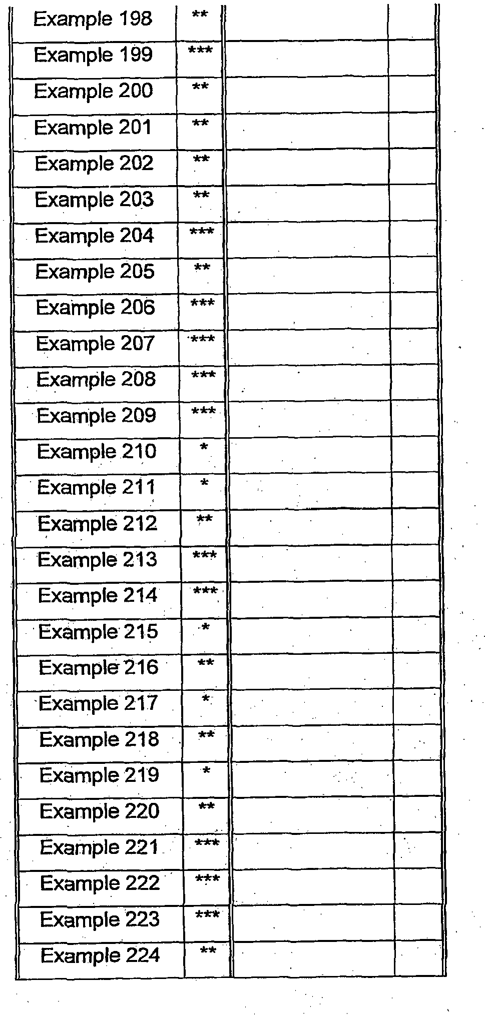

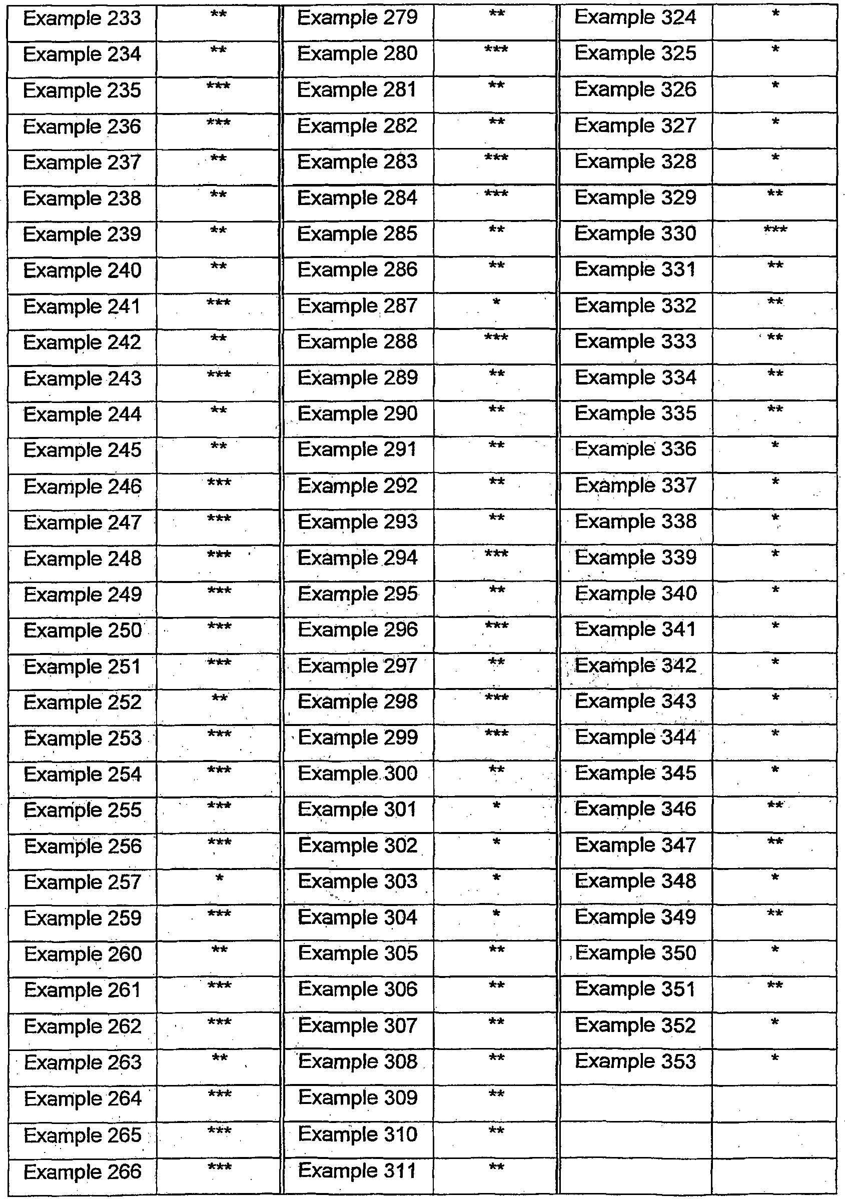

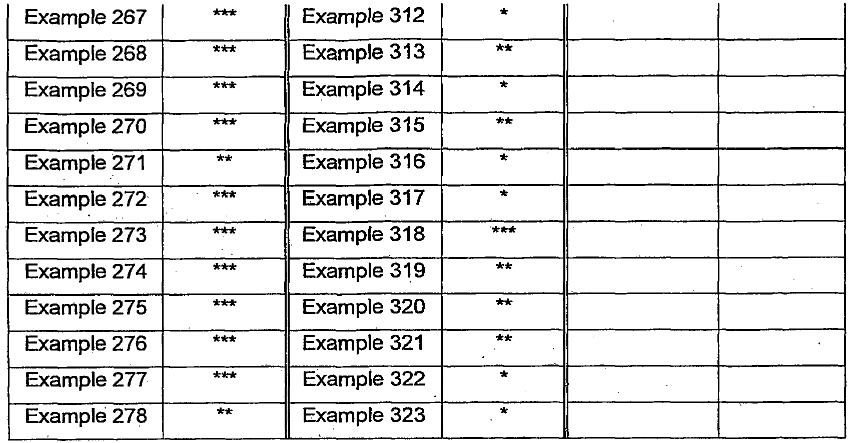

**Table 2: Kinase selectivity data of representative compounds**

| Data are expressed as percentage inhibition of each specific kinase at 1 µM inhibitor concentration | | | | | |
|---|---|---|---|---|---|
| Kinase | Example 121 | Example 172 | Kinase | Example 121 | Example 172 |
| AMPK | 29 | 0 | PIM1 | 15 | 0 |
| BRSK2 | 13 | 0 | PKA | 26 | 5 |
| BTK | 0 | 12 | PKBa | 19 | 0 |
| CAMK1 | 5 | 14 | PKBb | 3 | 0 |
| CAMKKb | 12 | 21 | PKCa | 2 | 0 |
| CDK2-Cyclin A | 61 | 0 | PKD1 | 29 | 1 |
| CHK1 | 10 | 0 | PLK1 | 19 | 3 |
| CHK2 | 13 | 40 | PRAK | 0 | 0 |
| CK1 | 31 | 6 | PRK2 | 39 | 16 |
| CK2 | 0 | 3 | ROCK 2 | 57 | 7 |
| CSK | 6 | 11 | S6K1 | 0 | 14 |
| DYRK1A | 27 | 27 | SGK1 | 23 | 1 |
| EPH-B3 | 6 | 3 | SmMLCK | 22 | 18 |
| ERK1 | 1 | 0 | Src | 0 | 6 |
| ERK2 | 0 | 9 | SRPK1 | 21 | 17 |
| FGF-R1 | 36 | 0 | SYK | 0 | 4 |
| GSK3b | 25 | 5 | TBK1 | 6 | 14 |
| HIPK2 | 0 | 0 | Aurora B | 0 | 15 |
| IKKb | 15 | 22 | EF2K | 0 | 0 |
| IKKe | 19 | 17 | IGF-1R | 46 | 18 |
| IRR | 22 | 15 | PKCz | 0 | 10 |
| JNK1 | 7 | 17 | Aurora A | 46 | 0 |
| JNK2 | 0 | 22 | EPH-A2 | 8 | 0 |
| Lck | 0 | 25 | GCK | 68 | 19 |
| MARK3 | 12 | 18 | HER4 | 0 | 10 |
| MELK | 61 | 5 | IR | 8 | 4 |
| MKK1 | 47 | 17 | IRAK4 | 12 | 0 |
| MNK1 | 1 | 0 | JAK2 | 47 | 12 |
| MNK2 | 7 | 0 | LKB1 | 42 | 8 |
| MSK1 | 48 | 1 | MEKK1 | 0 | 7 |
| MST2 | 55 | 10 | MINK1 | 17 | 18 |
| MST4 | 23 | 10 | MLK1 | 19 | 13 |
| NEK2a | 0 | 5 | MLK3 | 23 | 29 |
| NEK6 | 6 | 1 | NUAK1 | 59 | 14 |
| p38a MAPK | 0 | 6 | RIPK2 | 1 | 0 |
| PAK4 | 26 | 24 | TAK1 | 72 | 9 |
| PDK1 | 13 | 0 | TrkA | 26 | 5 |
| PHK | 7 | 26 | TTK | 30 | 14 |

## Claims

1. A compound of formula I, or a pharmaceutically acceptable salt thereof, 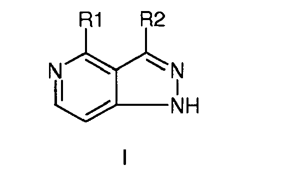 wherein:
R¹ is selected from:
aryl;
heteroaryl;
-NHR³;
fused aryl-C₄₋₇-heterocycloalkyl;
-CONR⁴R⁵;
-NHCOR⁶;
-C₃₋₇-cycloalkyl;
-NR³R⁶;
OR³;
OH;
NR⁴R⁵; and
-C₁₋₆ alkyl optionally substituted with a substituent selected from R¹¹ and a group A;
wherein said aryl, heteroaryl, fused aryl-C₄₋₇-heterocycloalkyl and C₄₋₇- heterocycloalkyl are each optionally substituted with one or more substituents selected from C₁₋₆-alkyl, C₃₋₇-cycloalkyl, heteroaryl, C₄₋₇-heterocycloalkyl, aryl and a group A, and said C₁₋₆-alkyl, C₃₋₇-cycloalkyl, heteroaryl, C₄₋₇-heterocycloalkyl, and aryl substituents are in turn each optionally substituted with one or more groups selected from R¹¹ and a group A;
R² is selected from aryl, C₁₋₆-alkyl, C₂₋₆-alkenyl, C₃₋₇-cycloalkyl, heteroaryl, C₄₋₇ heterocycloalkyl, fused aryl-C₄₋₇-heterocycloalkyl and halogen, wherein said C₁₋₆-alkyl, C₂₋₆-alkenyl, aryl, heteroaryl, fused aryl-C₄₋₇-heterocycloalkyl and C₄₋₇-heterocycloalkyl are each optionally substituted with one or more substituents selected from R¹¹ and A;
each R³ is selected from aryl, heteroaryl, C₄₋₇-heterocycloalkyl, C₃₋₇-cycloalkyl, fused aryl-C₄₋₇-heterocycloalkyl and C₁₋₆-alkyl, each of which is optionally substituted with one or more substituents selected from R¹¹ and A;
R⁴ and R⁵ are each independently selected from hydrogen, C₃₋₇-cycloalkyl, C₁₋₆-alkyl-C₃₋₇-cycloalkyl, aryl, heteroaryl, C₁₋₆-alkyl and a C₃₋₆-heterocycloalkyl ring optionally further containing one or more groups selected from oxygen, sulfur, nitrogen and CO, and optionally substituted by one or more R¹⁰ groups, wherein each C₁₋₆-alkyl, heteroaryl and aryl is optionally substituted by one or more substituents selected from C₁₋₆-alkyl, halogen, cyano, hydroxyl, aryl, halo-substituted aryl, heteroaryl, -NR⁸R⁹, -NR⁶R⁷, NR⁷(CO)R⁶, -NR⁷COOR⁶, -NR⁷(SO₂)R⁶, -COOR⁶, -CONR⁸R⁹, OR⁶, -SO₂R⁶ and a C₃₋₆-heterocycloalkyl ring optionally further containing one or more groups selected from oxygen, sulfur, nitrogen and CO and optionally substituted by one or more or R¹⁰ groups; or
R⁴ and R⁵ together with the N to which they are attached form a C₃₋₆-heterocycloalkyl ring optionally further containing one or more groups selected from oxygen, sulfur, nitrogen and CO, wherein said C₃₋₆-heterocycloalkyl ring is saturated or unsaturated and is optionally substituted with one or more groups selected from A, NR⁸R⁹ and R¹⁰;
each R⁶ is independently selected from C₁₋₆-alkyl, C₃₋₇ cycloalkyl, C₄₋₇-heterocycloalkyl, aryl and heteroaryl, each of which is optionally substituted by one or more substituents selected from R¹⁰, R¹¹ and A;
each R⁷ is selected from hydrogen, C₁₋₆-alkyl and C₃₋₇-cycloalkyl, wherein said C₁₋₆-alkyl is optionally substituted by one or more halogens;
each of R⁸ and R⁹ is independently selected from hydrogen and C₁₋₆-alkyl, wherein said C₁₋₆-alkyl group is optionally substituted by one or more halogens; or
R⁸ and R⁹ together with the N to which they are attached form a C₄₋₆-heterocycloalkyl ring optionally further containing one or more heteroatoms selected from oxygen and sulfur, wherein said C₄₋₆-heterocycloalkyl ring is optionally substituted by one or more R¹⁰ groups; and
each R¹⁰ is selected from C₃₋₇-cycloalkyl, aryl, heteroaryl, O-heteroaryl, aralkyl and C₁₋₆-alkyl, each of which is optionally substituted by one or more A groups, wherein where R¹⁰ is C₁₋₆-alkyl and two or more R¹⁰ groups are attached to the same carbon atom, the R¹⁰ groups may be linked to form a spiroalkyl group; and
each R¹¹ is independently selected from C₁₋₆-alkyl, C₃₋₇-cycloalkyl, C₁₋₆-alkyl-C₃₋₇-cycloalkyl, C₁₋₆-alkyl-heteroaryl, C₄₋₇-heterocycloalkyl, aryl and heteroaryl, each of which is optionally substituted with one or more substituents selected from A; and
A is selected from halogen, -NR⁴SO₂R⁵, -CN, -OR⁶, -NR⁴R⁵, -NR⁷R¹¹, hydroxyl, -CF₃, - CONR⁴R⁵, -NR⁴COR⁵, -NR⁷(CO)NR⁴R⁵, -NO₂, -CO₂H, -CO₂R⁶, -SO₂R⁶, -SO₂NR⁴R⁵, - NR⁴COR⁵,-NR⁴COOR⁵, C₁₋₆-alkyl, aryl and -COR⁶.

2. A compound according to claim 1 wherein R² is selected from:
halogen, more preferably bromine;
aryl optionally substituted by one or more substituents selected from R¹¹ and A;
C₁₋₆-alkyl optionally substituted by one or more substituents selected from R¹¹ and A;
C₂₋₆-alkenyl optionally substituted by one or more A substituents;
C₃₋₇-cycloalkyl;
heteroaryl optionally substituted by one or more substituents selected from R¹¹ and A; C₄₋₇-heterocycloalkyl; and
fused aryl-C₄₋₇-heterocycloalkyl.

3. A compound according to claim 1 or claim 2 wherein R² is selected from:
aryl optionally substituted by one or more substituents selected from -NR⁴R⁵, -NR⁴COR⁵, - CONR⁴R⁵, OR⁶, halogen, optionally substituted C₁₋₆-alkyl, CN, C₄₋₇-heterocycloalkyl and
heteroaryl;
C₁₋₆-alkyl optionally substituted by one or more substituents selected from -NR⁴COR⁵, - CONR⁴R⁵, -NR⁴R⁵, OR⁶, optionally substituted aryl, optionally substituted heteroaryl and C₄₋₇-heterocycloalkyl;
C₂₋₆-alkenyl optionally substituted by one or more -CONR⁴R⁵ substituents;
C₃₋₇-cycloalkyl, more preferably cyclopropyl;
heteroaryl optionally substituted by one or more substituents selected from -NR⁴R⁵, C₄₋₇-heterocycloalkyl, C₁₋₆-alkyl, C₃₋₇-cycloalkyl, C₁₋₆-alkyl-C₃₋₇-cycloalkyl and OR⁶;
C₄₋₇-heterocycloalkyl; and
fused aryl-C₄₋₇-heterocycloalkyl.

4. A compound according to any preceding claim wherein R² is selected from:
a phenyl group optionally substituted by one or more substituents selected from -NHCO-C₁₋₆-alkyl, -CONHC₁₋₆-alkyl, CO-(N-morpholinyl), Cl, F, -OC₁₋₆-alkyl, -CONMe₂, OCF₃, CN, CF₃, C₁₋₆-alkyl-(A), N-morpholinyl and pyrazolyl;
a heteroaryl group selected from pyridinyl, quinolinyl, pyrazoyl, furanyl and pyrimidinyl, each of which may be optionally substituted by one or more substituents selected from C₁₋₆-alkyl, aralkyl, OC₁₋₆-alkyl, N-morpholinyl;
a C₁₋₆-alkyl group optionally substituted by one or more substituents selected from -CONR⁴R⁵, phenyl, pyridinyl and oxadiazolyl and piperidinyl, wherein said phenyl, pyridinyl and oxadiazolyl and piperidinyl groups are each optionally further substituted by one or more - NR⁴COR⁵, -CONR⁴R⁵, COR⁶, SO₂R⁶ or aryl groups.

5. A compound according to claim 4 wherein each -CONR⁴R⁵ group is independently selected from:
-CO(N-morpholinyl), -CO(N-piperidinyl), -CO(N-pyrrolidinyl), -CO-(N-piperazinyl), each of which may be optionally further substituted by one or more substituents selected from aryl, heteroaryl, -OR⁶, CF₃, aralkyl, -NR⁴COR⁵-CONR⁴R⁵, -NR⁴R⁵, halogen, C₁₋₆-alkyl; and
-CON(C₁₋₆-alkyl)₂, CONH(C₁₋₆-alkyl), CON(C₁₋₆-alkyl)(aralkyl), CONH(C₃₋₇-cycloalkyl), - CONH(aryl), -CONH(heteroaryl), wherein said C₁₋₆-alkyl, aralkyl, aryl and heteroaryl groups are each optionally further substituted by one or more R¹¹ or A groups.

6. A compound according to any preceding claim wherein R² is a C₁₋₆-alkyl group optionally substituted by one or more substituents selected from -NR⁴COR⁵, -CONR⁴R⁵, - NR⁴R⁵, OR⁶, C₄₋₇-heterocycloalkyl, heteroaryl and aryl, wherein said aryl group is optionally substituted by one or more substituents selected from -NR⁴COR⁵ and -CONR⁴R⁵.

7. A compound according to any one of claims 1 to 3 wherein R² is selected from - CH₂CH₂CO-NR⁴R⁵, C₁₋₆-alkyl, C₃₋₇ cycloalkyl and a heteroaryl selected from furanyl and pyrazolyl, wherein said furanyl and pyrazolyl groups may be optionally substituted by one or more substituents selected from C₁₋₆-alkyl, C₃₋₇-cycloalkyl and C₁₋₆-alkyl-C₃₋₇-cycloalkyl.

8. A compound according to claim 7 wherein R² is selected from Me, 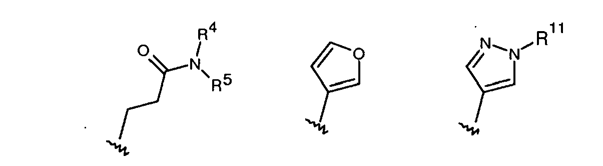 wherein R⁴ and R⁵ together with the N to which they are attached form a C₃₋₆-heterocycloalkyl ring optionally further containing one or more groups selected from oxygen, sulfur, nitrogen and CO, wherein said C₃₋₆-heterocycloalkyl ring is saturated or unsaturated and is optionally substituted with one or more groups selected from A, NR⁸R⁹ and R¹⁰.

9. A compound according to any one of claims 1 to 5 wherein R² is an unsubstituted C₁₋₆-alkyl group, more preferably methyl.

10. A compound according to any preceding claim wherein R¹ is selected from:
-NHR³;
aryl;
heteroaryl;
C₄₋₇-heterocycloalkyl;
fused aryl-C₄₋₇-heterocycloalkyl;
-C₃₋₇-cycloalkyl;
-NR³R⁶;
OR³;
NR⁴R⁵; and
-C₁₋₆ alkyl optionally substituted with a substituent selected from R¹¹ and a group A;
wherein said aryl, heteroaryl, fused aryl-C₄₋₇-heterocycloalkyl and C₄₋₇- heterocycloalkyl are each optionally substituted with one or more substituents selected from C₁₋₆-alkyl, C₃₋₇-cycloalkyl, heteroaryl, C₄₋₇-heterocycloalkyl, aryl and a group A, and said C₁₋₆-alkyl, C₃₋₇-cycloalkyl, heteroaryl, C₄₋₇-heterocycloalkyl, and aryl substituents are in turn each optionally substituted with one or more groups selected from R¹¹ and a group A.

11. A compound according to any preceding claim wherein R¹ is -NHR³, wherein R³ is selected from C₁₋₆-alkyl, C₃₋₇-cycloalkyl, C₄₋₇-heterocycloalkyl and aryl, each of which may be optionally substituted by one or more with one or more substituents selected from R¹¹ and A.

12. A compound according to claim 11 wherein R¹ is -NHR³ and R³ is selected from:
C₁₋₆-alkyl, optionally substituted by one or more -OR⁶, NR⁴COR⁵, heteroaryl, aryl, C₄₋₇-heterocycloalkyl, and C₃₋₇-cycloalkyl groups, wherein said aryl and heteroaryl groups are each independently optionally further substituted by one or more groups selected from CF₃, halogen, C₁₋₆-alkyl, -OR⁶ and -NR⁴R⁵;
a phenyl group optionally substituted by one or more substituents selected from -OR⁶, NR⁴COR⁵, -CONR⁴R⁵, aryl, -NR⁴R⁵, C₁₋₆-alkyl-heteroaryl, heteroaryl, halogen, -SO₂R⁶, CN, CF₃, C₁₋₆-alkyl, -SO₂NR⁴R⁵, -NR⁴SO₂R⁵, wherein said C₁₋₆-alkyl, heteroaryl and aryl groups are each independently optionally further substituted by one or more groups selected from CN, CF₃, halogen, C₁₋₆-alkyl, -OR⁶ and -NR⁴R⁵;
a heteroaryl group optionally substituted by one or more substituents selected from aryl, C₁₋₆-alkyl, and -NR⁴R⁵, wherein said aryl group is optionally further substituted by one or more A groups;
a C₄₋₇-heterocycloalkyl optionally substituted by one or more -COR⁶ groups;
a C₃₋₇-cycloalkyl group optionally substituted by one or more halogen or C₁₋₆-alkyl groups.

13. A compound according to any one of claims 1 to 10 wherein R¹ is -OR³, wherein R³ is selected from C₁₋₆-alkyl, C₃₋₇-cycloalkyl, C₄₋₇-heterocycloalkyl and aryl, each of which may be optionally substituted by one or more with one or more substituents selected from R¹¹ and A.

14. A compound according to any one of claims 1 to 10 wherein R¹ is aryl or heteroaryl, each of which may be optionally substituted by one or more with one or more substituents selected from R¹¹ and A.

15. A compound according to any one of claims 1 to 10 wherein R¹ is -NH-C₃₋₇-cycloalkyl or NH-C₄₋₇-heterocycloalkyl, each of which may be optionally substituted by one or more A substituents.

16. A compound according to any preceding claim wherein R³ is cyclohexyl or tetrahydropyranyl, each of which may be optionally substituted by one or more A substituents.

17. A compound according to any preceding claim wherein R¹ is selected from the following: 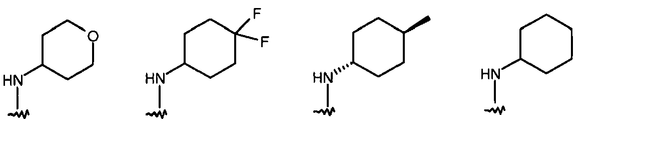 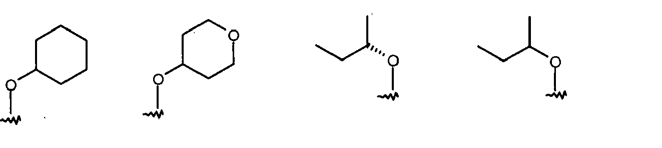 and wherein R¹ is preferably -NH-cyclohexyl.

18. A compound according to claim 1 wherein R¹ is -NHR³ and R² is an unsubstituted C₁₋₆-alkyl group, more preferably methyl.

19. A compound according to claim 1 wherein R¹ is -NHR³ and R² is a C₁₋₆-alkyl group substituted by one or more -CONR⁴R⁵ groups.

20. A compound according to claim 1 wherein R¹ is -NHR³ and R² is an aryl or heteroaryl group, each of which may be optionally substituted by one or more substituents selected from C₄₋₇-heterocycloalkyl, C₁₋₆-alkyl, C₃₋₇-cycloalkyl, C₁₋₆-alkyl-C₃₋₇-cycloalkyl and OR⁶.

21. A compound according to claim 1 wherein R¹ is selected from: 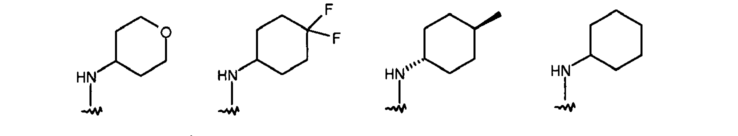 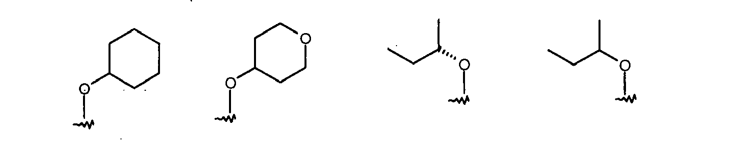 and R² is selected from Me 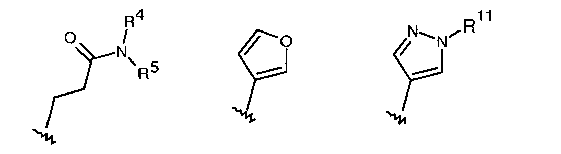 wherein R⁴ and R⁵ together with the N to which they are attached form a C₃₋₆-heterocycloalkyl ring optionally further containing one or more groups selected from oxygen, sulfur, nitrogen and CO, wherein said C₃₋₆-heterocycloalkyl ring is saturated or unsaturated and is optionally substituted with one or more groups selected from A, NR⁸R⁹ and R¹⁰.

22. A compound which is selected from the following:
| | | | |
|---|---|---|---|
| 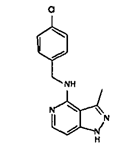 | Example 1 | 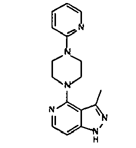 | Example 9 |
| 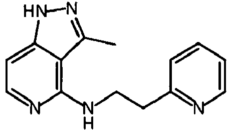 | Example 2 | 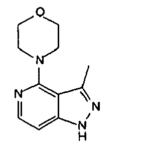 | Example 10 |
| 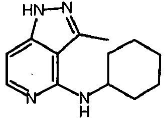 | Example 3 | 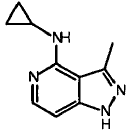 | Example 11 |
| 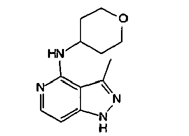 | Example 4 | 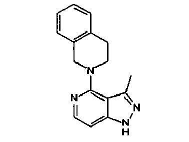 | Example 12 |
| 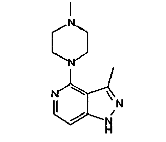 | Example 5 | 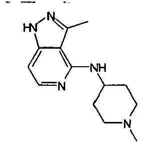 | Example 13 |
| 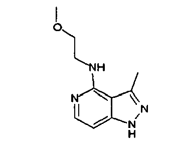 | Example 6 | 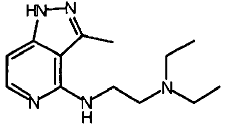 | Example 14 |
| 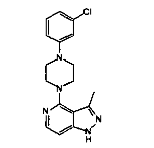 | Example 7 | 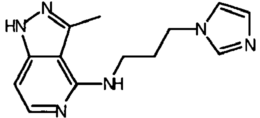 | Example 15 |
| 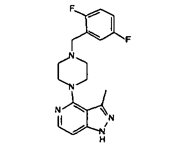 | Example 8 | 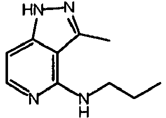 | Example 16 |
| 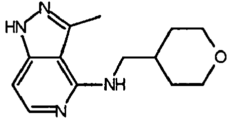 | Example 17 | 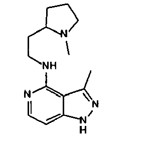 | Example 25 |
| 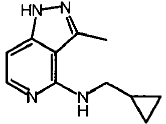 | Example 18 | 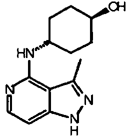 | Example 26 |
| 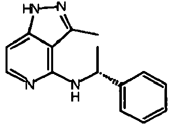 | Example 19 | 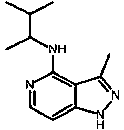 | Example 27 |
| 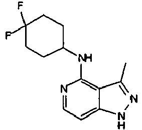 | Example 20 | 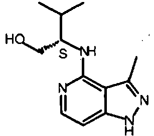 | Example 28 |
| 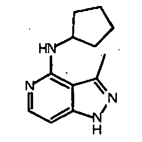 | Example 21 | 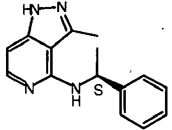 | Example 29 |
| 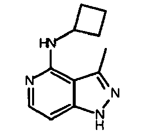 | Example 22 | 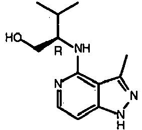 | Example 30 |
| 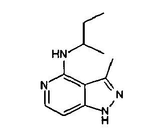 | Example 23 | 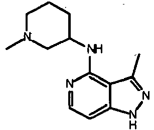 | Example 31 |
| 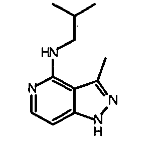 | Example 24 | 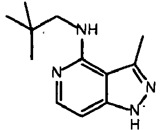 | Example 32 |
| 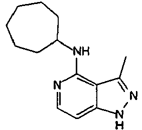 | Example 33 | 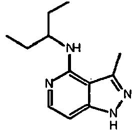 | Example 41 |
| 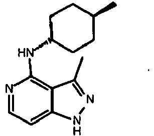 | Example 34 | 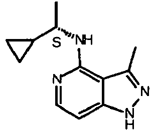 | Example 42 |
| 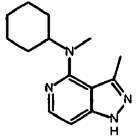 | Example 35 | 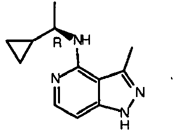 | Example 43 |
| 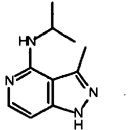 | Example 36 | 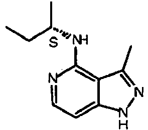 | Example 44 |
| 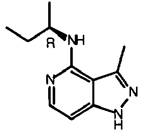 | Example 37 | 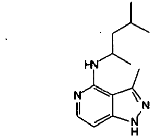 | Example 45 |
| 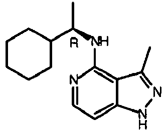 | Example 38 | 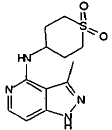 | Example 46 |
| 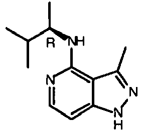 | Example 39 | 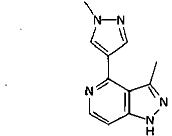 | Example 47 |
| 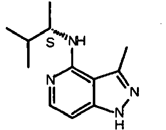 | Example 40 | 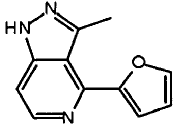 | Example 48 |
| 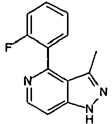 | Example 49 | 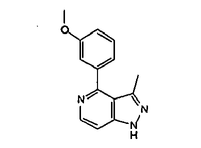 | Example 57 |
| 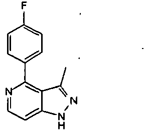 | Example 50 | 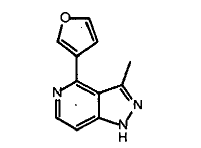 | Example 58 |
| 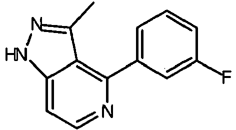 | Example 51 | 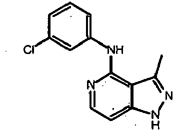 | Example 59 |
| 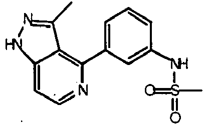 | Example 52 | 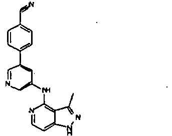 | Example 60 |
| 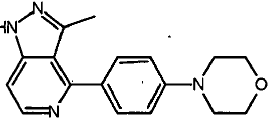 | Example 53 | 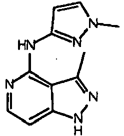 | Example 61 |
| 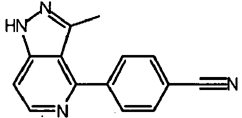 | Example 54 | 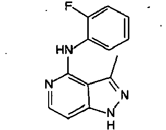 | Example 62 |
| 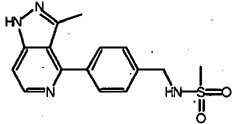 | Example 55 | 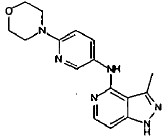 | Example 63 |
| 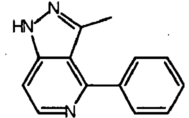 | Example 56 | 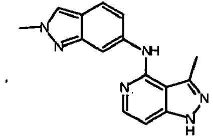 | Example 64 |
| 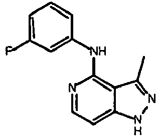 | Example 65 | 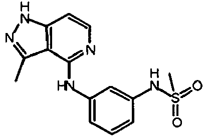 | Example 73 |
| 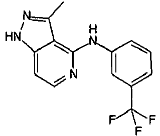 | Example 66 | 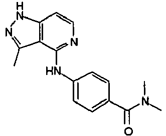 | Example 74 |
| 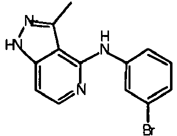 | Example 67 | 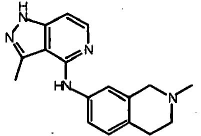 | Example 75 |
| 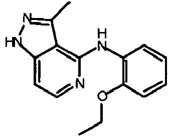 | Example 68 | 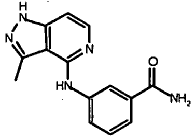 | Example 76 |
| 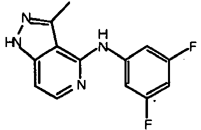 | Example 69 | 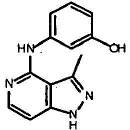 | Example 77 |
| 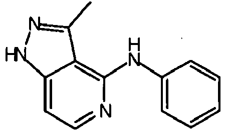 | Example 70 | 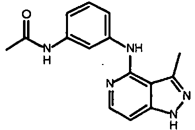 | Example 78 |
| 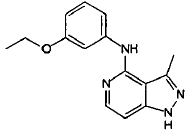 | Example 71 | 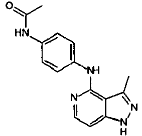 | Example 79 |
| 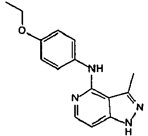 | Example 72 | 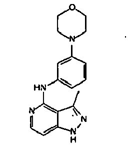 | Example 80 |
| 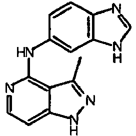 | Example 81 | 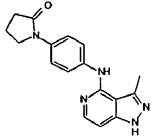 | Example 89 |
| 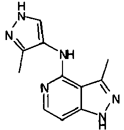 | Example 82 | 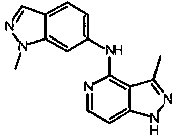 | Example 90 |
| 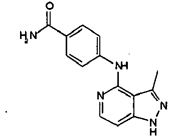 | Example 83 | 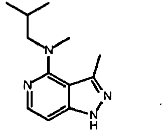 | Example 91 |
| 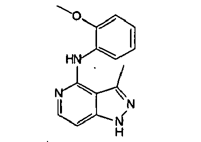 | Example 84 | 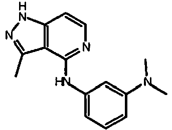 | Example 92 |
| 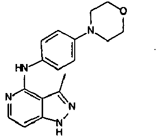 | Example 85 | 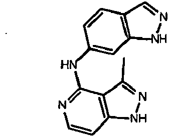 | Example 93 |
| 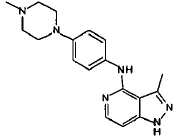 | Example 86 | 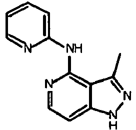 | Example 94 |
| 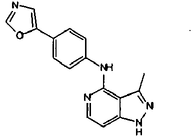 | Example 87 | 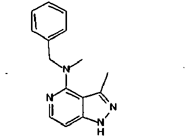 | Example 95 |
| 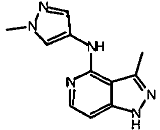 | Example 88 | 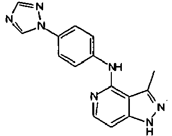 | Example 96 |
| 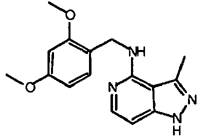 | Example 97 | 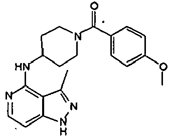 | Example 105 |
| 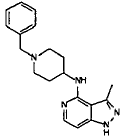 | Example 98 | 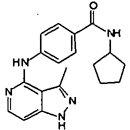 | Example 106 |
| 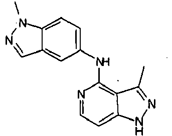 | Example 99 | 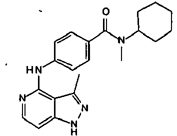 | Example 107 |
| 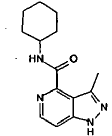 | Example 100 | 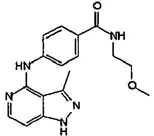 | Example 108 |
| 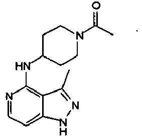 | Example 101 | 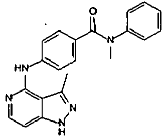 | Example 109 |
| 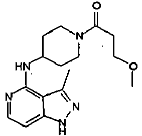 | Example 102 | 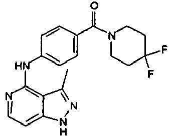 | Example 110 |
| 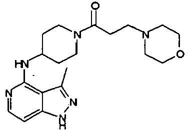 | Example 103 | 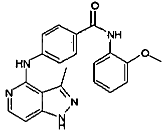 | Example 111 |
| 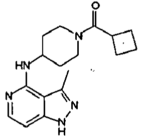 | Example 104 | 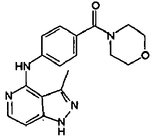 | Example 112 |
| 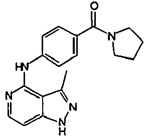 | Example 113 | 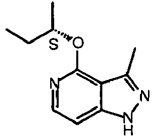 | Example 121 |
| 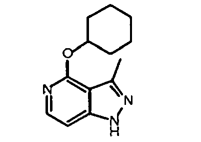 | Example 114 | 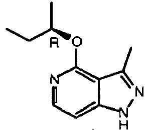 | Example 122 |
| 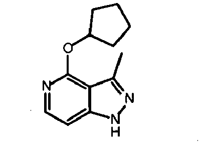 | Example 115 | 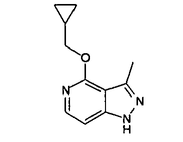 | Example 123 |
| 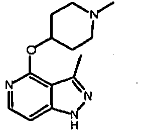 | Example 116 | 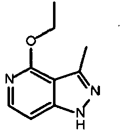 | Example 124 |
| 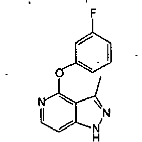 | Example 117 | 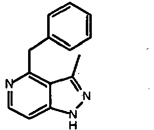 | Example 125 |
| 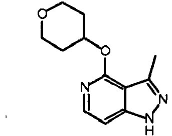 | Example 118 | 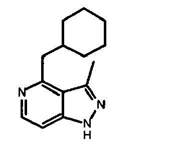 | Example 126 |
| 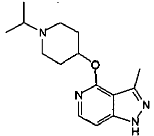 | Example 119 | 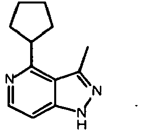 | Example 127 |
| 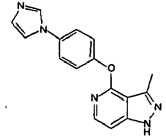 | Example 120 | 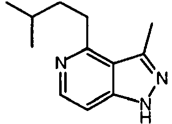 | Example 128 |
| 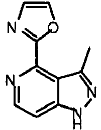 | Example 129 | 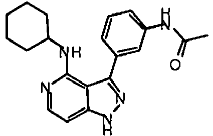 | Example 137 |
| 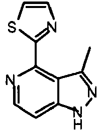 | Example 130 | 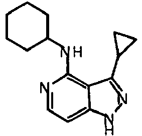 | Example 138 |
| 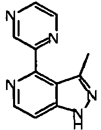 | Example 131 | 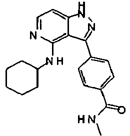 | Example 139 |
| | | 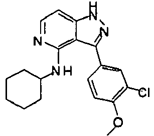 | Example 140 |
| | | 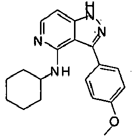 | Example 141 |
| 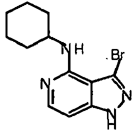 | Example 134 | 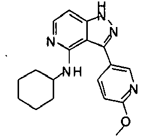 | Example 142 |
| 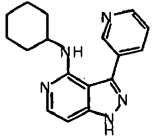 | Example 135 | 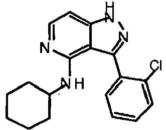 | Example 143 |
| 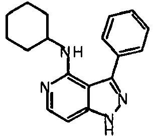 | Example 136 | 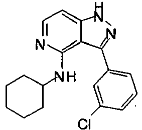 | Example 144 |
| 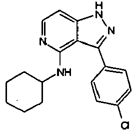 | Example 145 | 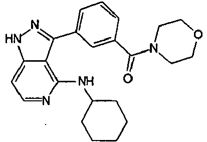 | Example 153 |
| 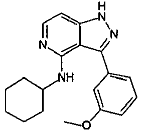 | Example 146 | 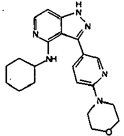 | Example 154 |
| 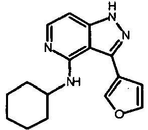 | Example 147 | 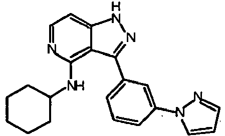 | Example 155 |
| 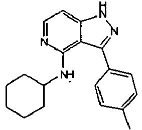 | Example 148 | 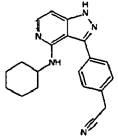 | Example 156 |
| 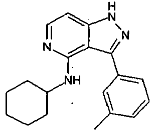 | Example 149 | 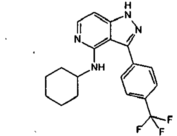 | Example 157 |
| 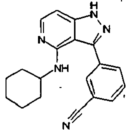 | Example 150 | 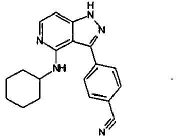 | Example 158 |
| 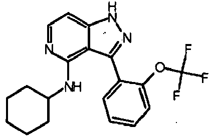 | Example 151 | 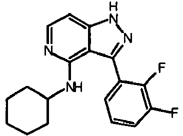 | Example 159 |
| 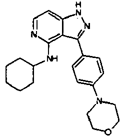 | Example 152 | 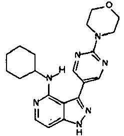 | Example 160 |
| 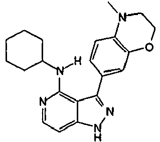 | Example 161 | 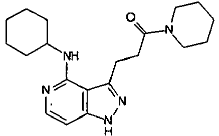 | Example 169 |
| 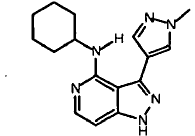 | Example 162 | 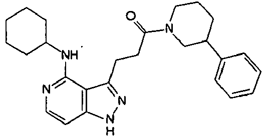 | Example 170 |
| 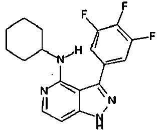 | Example 163 | 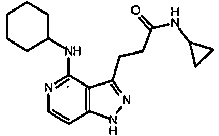 | Example 171 |
| 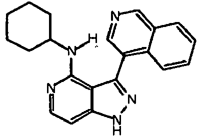 | Example 164 | 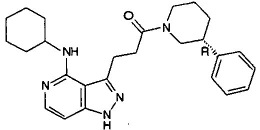 | Example 172 |
| 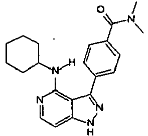 | Example 165 | 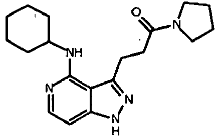 | Example 173 |
| 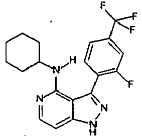 | Example 166 | 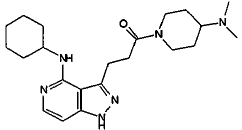 | Example 174 |
| 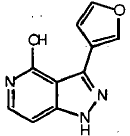 | Example 167 | 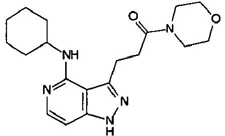 | Example 175 |
| 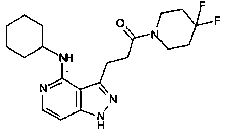 | Example 168 | 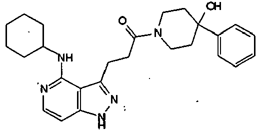 | Example 176 |
| 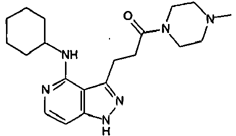 | Example 177 | 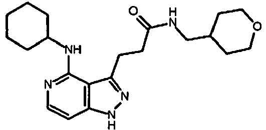 | Example 185 |
| 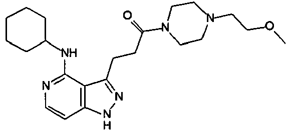 | Example 178 | 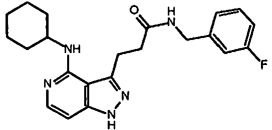 | Example 186 |
| 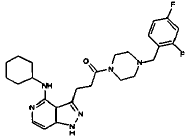 | Example 179 | 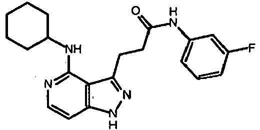 | Example 187 |
| 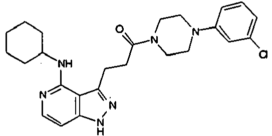 | Example 180 | 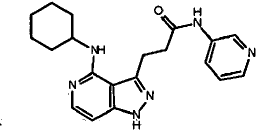 | Example 188 |
| 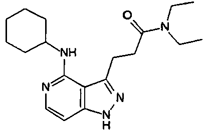 | Example 181 | 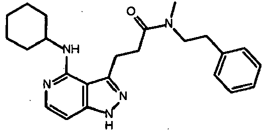 | Example 189 |
| 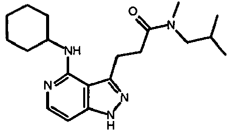 | Example 182 | 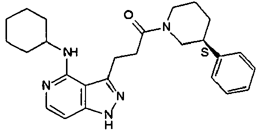 | Example 190 |
| 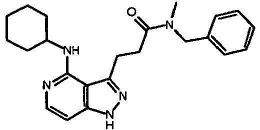 | Example 183 | 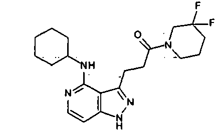 | Example 191 |
| 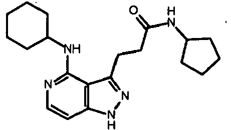 | Example 184 | 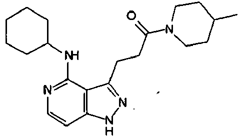 | Example 192 |
| 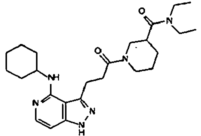 | Example 193 | 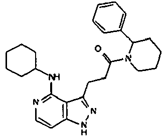 | Example 201 |
| 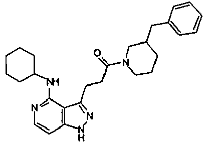 | Example 194 | 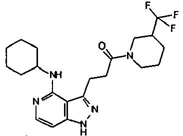 | Example 202 |
| 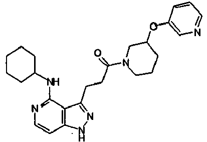 | Example 195 | 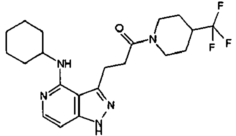 | Example 203 |
| 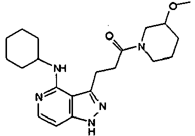 | Example 196 | 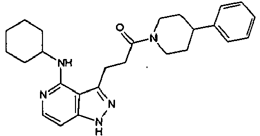 | Example 204 |
| 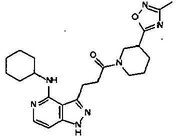 | Example 197 | 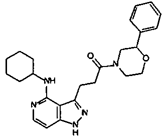 | Example 205 |
| 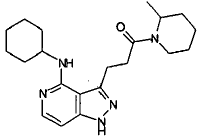 | Example 198 | 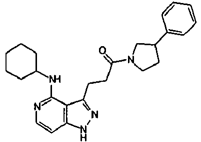 | Example 206 |
| 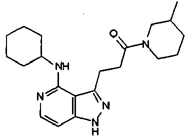 | Example 199 | 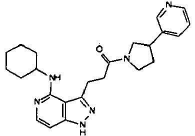 | Example 207 |
| 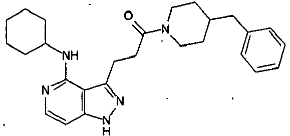 | Example 200 | 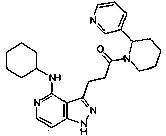 | Example 208 |
| 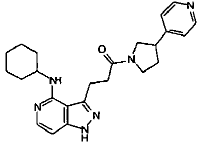 | Example 209 | 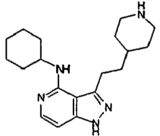 | Example 217 |
| 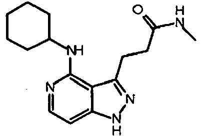 | Example 210 | 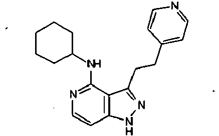 | Example 218 |
| 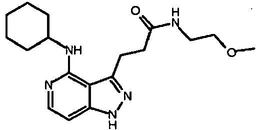 | Example 211 | 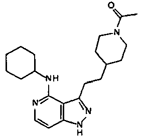 | Example 219 |
| 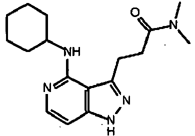 | Example 212 | 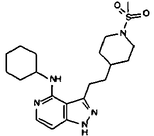 | Example 220 |
| 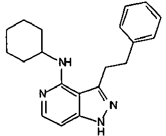 | Example 213 | 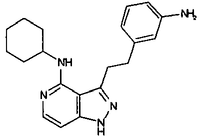 | Example 221 |
| 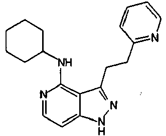 | Example 214 | 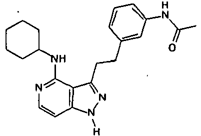 | Example 222 |
| 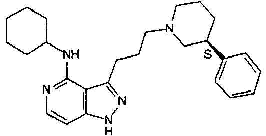 | Example 215 | 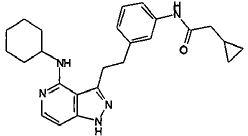 | Example 223 |
| 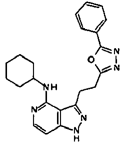 | Example 216 | 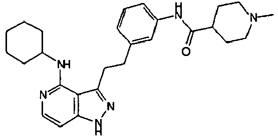 | Example 224 |
| 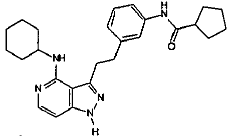 | Example 225 | | |
| 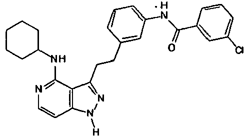 | Example 226 | | |
| 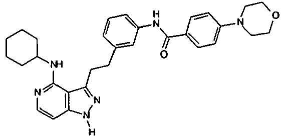 | Example 227 | | |
| 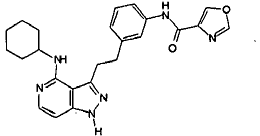 | Example 228 | | |
| 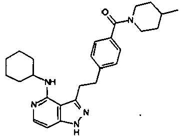 | Example 229 | | |
| 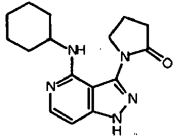 | Example 230 | | |
| 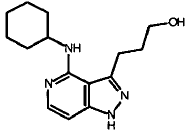 | Example 231 | | |
| 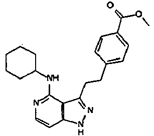 | Example 232 | | |
| 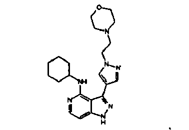 | Example 233 | 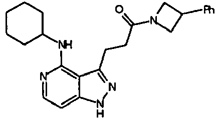 | Example 242 |
| 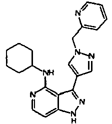 | Example 234 | 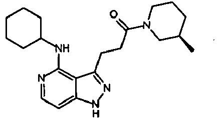 | Example 243 |
| 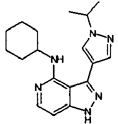 | Example 235 | 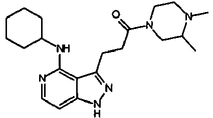 | Example 244 |
| 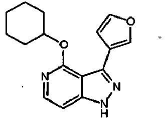 | Example 236 | 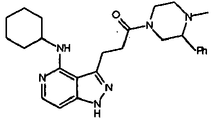 | Example 245 |
| 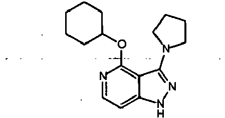 | Example 237 | 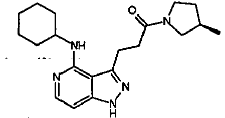 | Example 246 |
| 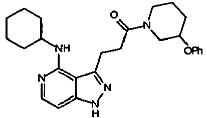 | Example 238 | 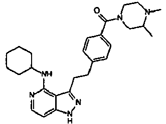 | Example 247 |
| 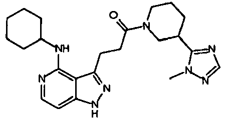 | Example 239 | 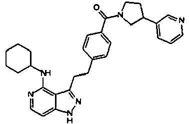 | Example 248 |
| 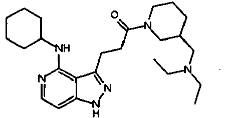 | Example 240 | 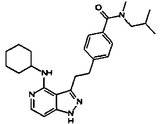 | Example 249 |
| 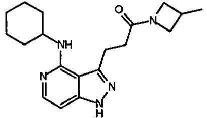 | Example 241 | 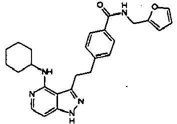 | Example 250 |
| 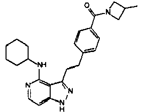 | Example 251 | 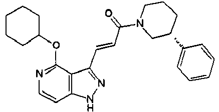 | Example 261 |
| 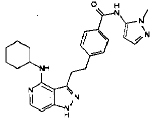 | Example 252 | 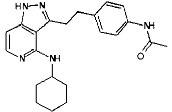 | Example 262 |
| 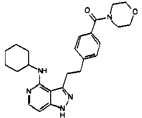 | Example 253 | 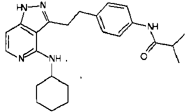 | Example 263 |
| 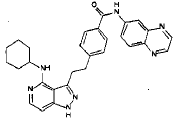 | Example 254 | 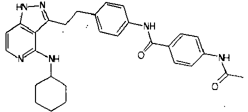 | Example 264 |
| 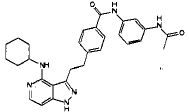 | Example 255 | 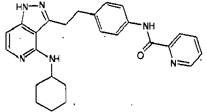 | Example 265 |
| 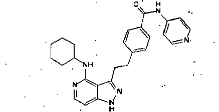 | Example 256 | 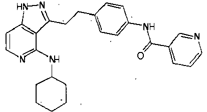 | Example 266 |
| 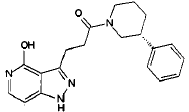 | Example 257 | 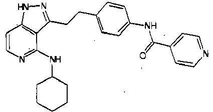 | Example 267 |
| 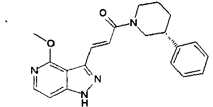 | Example 258 | 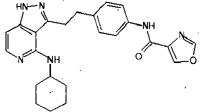 | Example 268 |
| 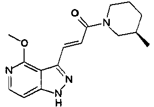 | Example 259 | 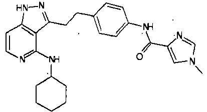 | Example 269 |
| 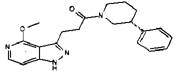 | Example 260 | | |
| 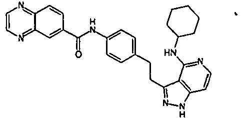 | Example 270 | 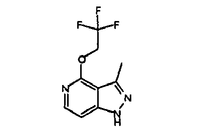 | Example 279 |
| 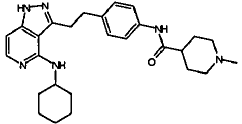 | Example 271 | 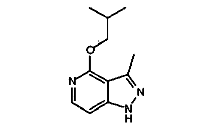 | Example 280 |
| 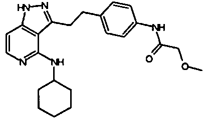 | Example 272 | 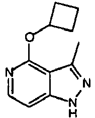 | Example 281 |
| 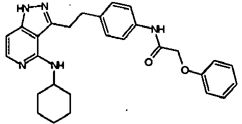 | Example 273 | 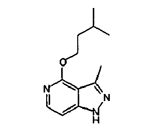 | Example 282 |
| 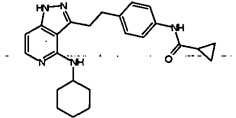 | Example 274 | 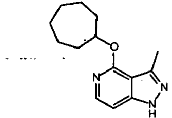 | Example 283 |
| 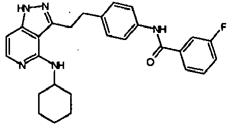 | Example 275 | 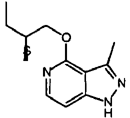 | Example 284 |
| 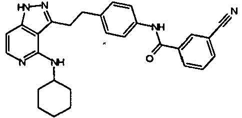 | Example 276 | 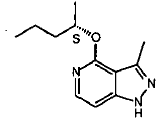 | Example 285 |
| 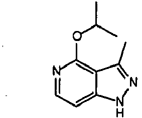 | Example 277 | 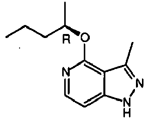 | Example 286 |
| 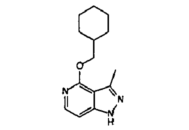 | Example 278 | 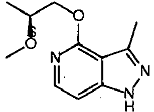 | Example 287 |
| 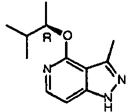 | Example 288 | 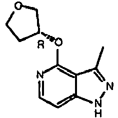 | Example 297 |
| 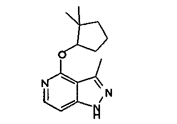 | Example 289 | 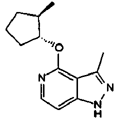 | Example 298 |
| 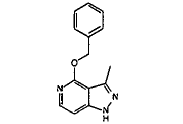 | Example 290 | 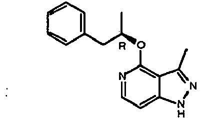 | Example 299 |
| 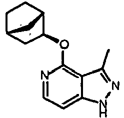 | Example 291 | 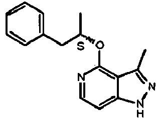 | Example 300 |
| 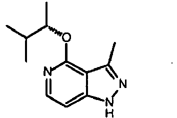 | Example 292 | 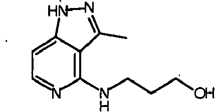 | Example 301 |
| 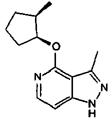 | Example 293 | 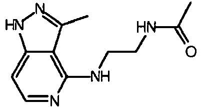 | Example 302 |
| 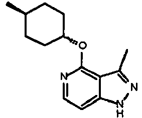 | Example 294 | 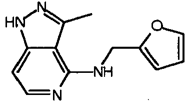 | Example 303 |
| 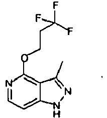 | Example 295 | 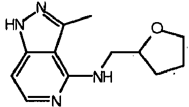 | Example 304 |
| 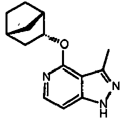 | Example 296 | 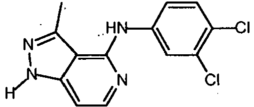 | Example 305 |
| 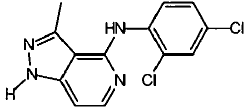 | Example 306 | 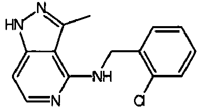 | Example 315 |
| 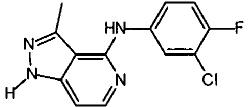 | Example 307 | 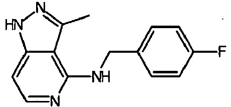 | Example 316 |
| 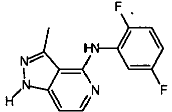 | Example 308 | 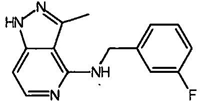 | Example 317 |
| 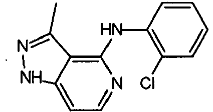 | Example 309 | 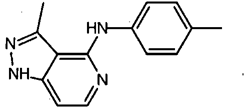 | Example 318 |
| 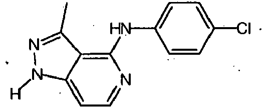 | Example 310 | 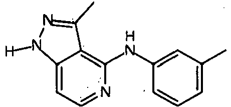 | Example 319 |
| 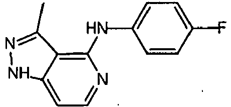 | Example 311 | 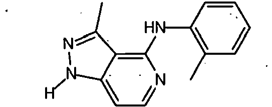 | Example 320 |
| 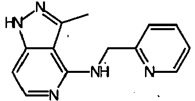 | Example 312 | 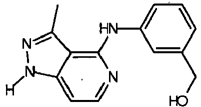 | Example 321 |
| 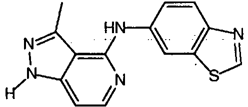 | Example 313 | 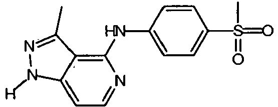 | Example 322 |
| 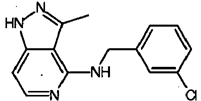 | Example 314 | 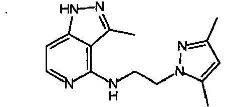 | Example 323 |
| 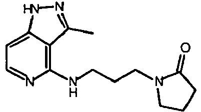 | Example 324 | 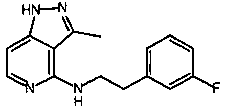 | Example 333 |
| 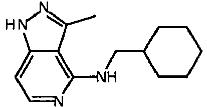 | Example 325 | 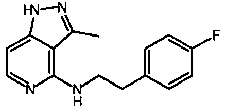 | Example 334 |
| 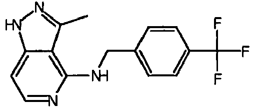 | Example 326 | 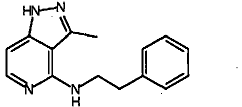 | Example 335 |
| 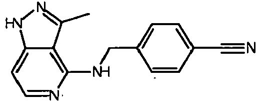 | Example 327 | 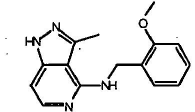 | Example 336 |
| 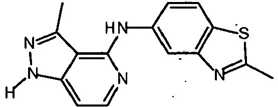 | Example 328 | 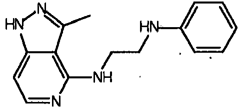 | Example 337 |
| 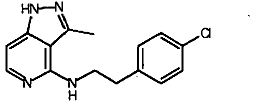 | Example 329 | 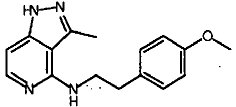 | Example 338 |
| 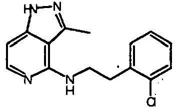 | Example 330 | 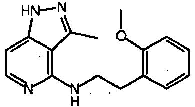 | Example 339 |
| 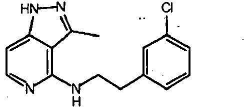 | Example 331 | 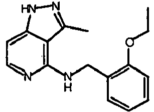 | Example 340 |
| 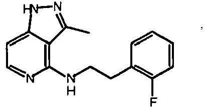 | Example 332 | 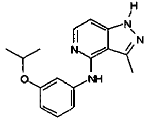 | Example 341 |
| 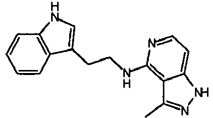 | Example 342 | 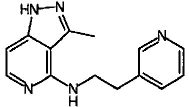 | Example 351 |
| 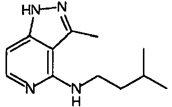 | Example 343 | 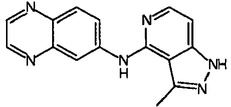 | Example 352 |
| 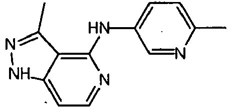 | Example 344 | 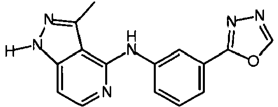 | Example 353 |
| 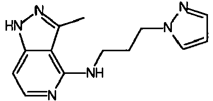 | Example 345 | 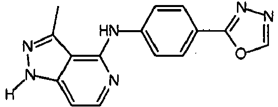 | Example 354 |
| 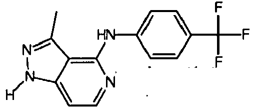 | Example 346 | 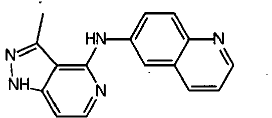 | Example 355 |
| 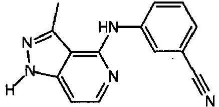 | Example 347 | 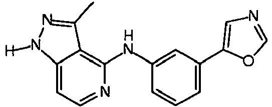 | Example 356 |
| 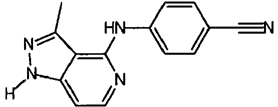 | Example 348 | 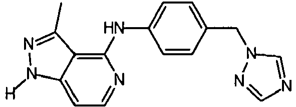 | Example 357 |
| 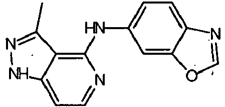 | Example 349 | 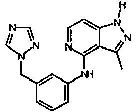 | Example 358 |
| 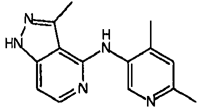 | Example 350 | 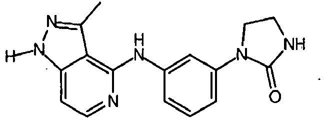 | Example 359 |
| 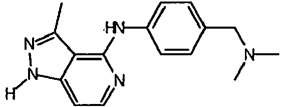 | Example 360 | 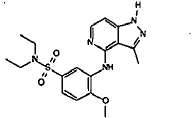 | Example 369 |
| 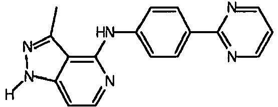 | Example 361 | 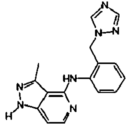 | Example 370 |
| 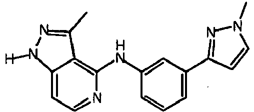 | Example 362 | 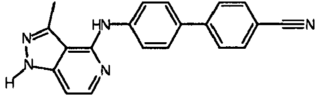 | Example 371 |
| 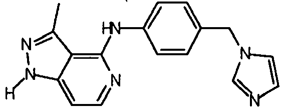 | Example 363 | 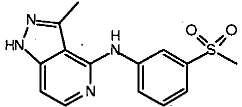 | Example 372 |
| 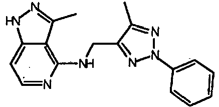 | Example 364 | 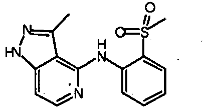 | Example 373 |
| 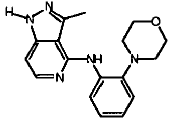 | Example 365 | 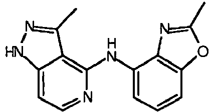 | Example 374 |
| 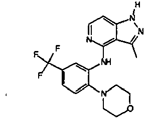 | Example 366 | 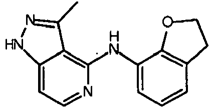 | Example 375 |
| 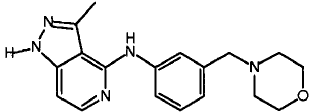 | Example 367 | 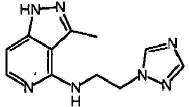 | Example 376 |
| 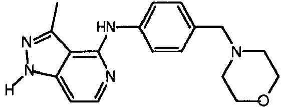 | Example 368 | 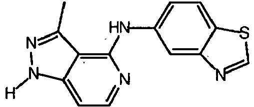 | Example 377 |
| 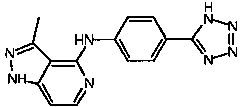 | Example 378 | 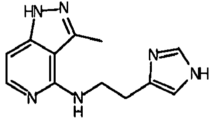 | Example 387 |
| 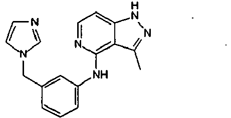 | Example 379 | 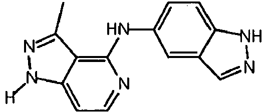 | Example 388 |
| 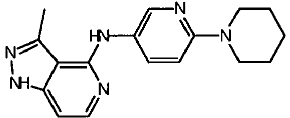 | Example 380 | 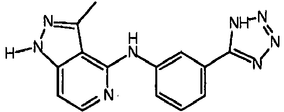 | Example 389 |
| 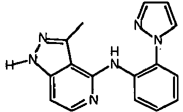 | Example 361 | 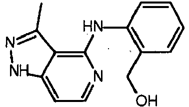 | Example 390 |
| 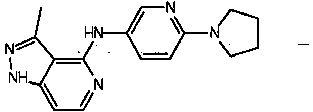 | Example 382 | 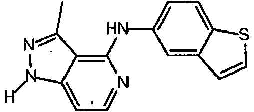 | Example 391 |
| 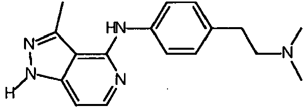 | Example 383 | 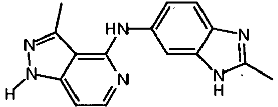 | Example 392 |
| 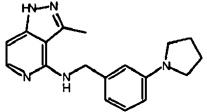 | Example 384 | | |
| 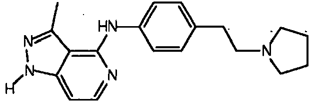 | Example 385 | | |
| 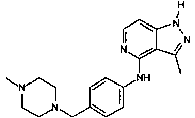 | Example 386 | | |

23. A pharmaceutical composition comprising a compound according to any one of claims 1 to 22 and a pharmaceutically acceptable carrier, diluent or excipient, wherein said pharmaceutical composition optionally further comprises a second therapeutic agent.

24. A compound according to any one of claims 1 to 22 for use in medicine.

25. A compound according to any one of claims 1 to 22 for use in treating a disorder selected from cancer and neurodegenerative diseases.

26. Use of a compound according to any one of claims 1 to 22 in the preparation of a medicament for treating or preventing a disorder selected from cancer and neurodegenerative diseases, or for the prevention or treatment of a disorder caused by, associated with or accompanied by abnormal kinase activity, preferably abnormal LRRK2 activity.

27. Use of a compound according to any one of claims 1 to 22 in an assay for identifying further candidate compounds capable of inhibiting LRRK, more preferably LRRK2.

28. A process for preparing a compound of formula I as defined in claim 1, said process comprising converting a compound of formula II into a compound of formula I: 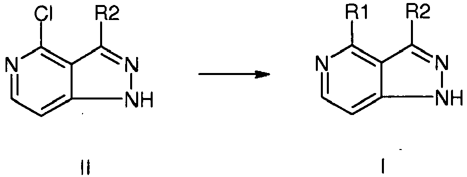 wherein said process optionally further comprises the step of preparing said compound of formula II by treating a compound of formula III with hydrazine monohydrate: 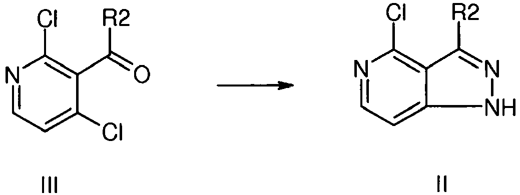 wherein said process optionally further comprises the step of preparing said compound of formula III by treating a compound of formula IV with an oxidizing agent: 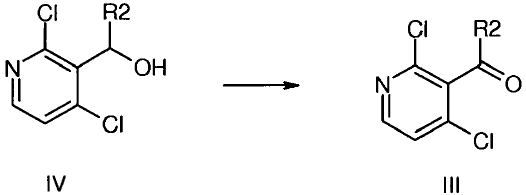 wherein said process optionally further comprises the step of preparing said compound of formula IV by treating a compound of formula V with R²-Mg-Cl:

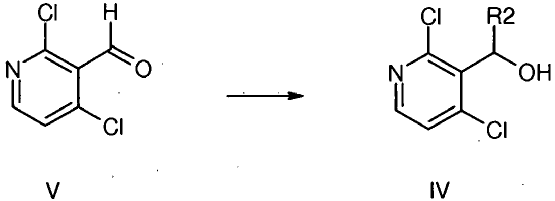

29. A process according to claim 28 where R¹ is -NHR³, and said process comprises reacting a compound of formula II with an amine of formula NH₂R³.

30. A process according to claim 28 where R¹ is an NH-containing C₄₋₇-heterocycloalkyl or an NH-containing fused aryl-C₄₋₇-heterocycloalkyl, and said process comprises reacting a compound of formula II with the NH-group of said C₄₋₇-heterocycloalkyl or fused aryl-C₄₋₇-heterocycloalkyl.

31. A process according to claim 28 wherein R¹ is selected from aryl, heteroaryl, C₄₋₇-heterocycloalkyl, fused aryl-C₄₋₇-heterocyoloalkyl, -C₃₋₇ cycloalkyl and -C₁₋₆ alkyl, and said process comprises reacting a compound of formula II with X-R¹, where X is a 4,4,5,5-tetramethyl-1,3,2-dioxaborolan-2-yl group, in the presence of a coupling agent.

32. A combination comprising a compound according to any one of claims 1 to 22 and a further therapeutic agent.

## Patentansprüche

1. Verbindung der Formel I oder ein pharmazeutisch unbedenkliches Salz davon, 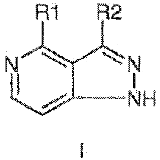 wobei:
R¹ aus:
Aryl;
Heteroaryl;
-NHR³ ;
anelliertem Aryl-C₄₋₇-heterocycloalkyl;
-CONR⁴R⁵;
-NHCOR⁶;
-C₃₋₇-Cycloalkyl;
-NR³R⁶;
OR³;
OH;
NR⁴R⁵ und
-C₁₋₆-Alkyl, das gegebenenfalls durch einen aus R¹¹ und einer Gruppe A ausgewählten Substituenten substituiert ist;
ausgewählt ist;
wobei das Aryl, Heteroaryl, annellierte Aryl-C₄₋₇-heterocycloalkyl und C₄₋₇-Heterocycloalkyl jeweils gegebenenfalls durch einen oder mehrere Substituenten, die aus C₁₋₆-Alkyl, C₃₋₇-Cycloalkyl, Heteroaryl, C₄₋₇-Heterocycloalkyl, Aryl und einer Gruppe A ausgewählt sind, substituiert sind und die C₁₋₆-Alkyl-, C₃₋₇-Cycloalkyl-, Heteroaryl-, C₄₋₇-Heterocycloalkyl- und Arylsubstituenten ihrerseits jeweils gegebenenfalls durch eine oder mehrere Gruppen, die aus R¹¹ und einer Gruppe A ausgewählt sind, substituiert sind;
R² aus Aryl, C₁₋₆-Alkyl, C₂₋₆-Alkenyl, C₃₋₇-Cycloalkyl, Heteroaryl, C₄₋₇-Heterocycloalkyl, anelliertem Aryl-C₄₋₇-heterocycloalkyl und Halogen ausgewählt ist, wobei das C₁₋₆-Alkyl, C₂₋₆-Alkenyl, Aryl, Heteroaryl, anellierte Aryl-C₄₋₇-heterocycloalkyl und C₄₋₇-Heterocycloalkyl gegebenenfalls durch einen oder mehrere Substituenten, die aus R¹¹ und A ausgewählt sind, substituiert sind;
R³ jeweils aus Aryl, Heteroaryl, C₄₋₇-Hydrocycloalkyl, C₃₋₇-Cycloalkyl, anelliertem Aryl-C₄₋₇-heterocycloalkyl und C₁₋₆-Alkyl ausgewählt ist, wobei jede dieser Gruppen gegebenenfalls durch einen oder mehrere Substituenten, die aus R¹¹ und A ausgewählt sind, substituiert ist;
R⁴ und R⁵ jeweils unabhängig voneinander aus Wasserstoff, C₃₋₇-Cycloalkyl, C₁₋₆-Alkyl-C₃₋₇-cycloalkyl, Aryl, Heteroaryl, C₁₋₆-Alkyl und einem C₃₋₆-Heterocycloalkylring, der gegebenenfalls ferner eine oder mehrere Gruppen, die aus Sauerstoff, Schwefel, Stickstoff und CO ausgewählt sind, enthält und gegebenenfalls durch eine oder mehrere R¹⁰-Gruppen substituiert ist, ausgewählt sind, wobei C₁₋₆-Alkyl, Heteroaryl und Aryl jeweils gegebenenfalls durch einen oder mehrere Substituenten, die aus C₁₋₆-Alkyl, Halogen, Cyano, Hydroxyl, Aryl, halogensubstituiertem Aryl, Heteroaryl, -NR⁸R⁹, -NR⁶R⁷, NR⁷(CO)R⁶, -NR⁷COOR⁶, -NR⁷(SO₂)R⁶, -COOR⁶, -CONR⁸R⁹, OR⁶, -SO₂R⁶ und einem C₃₋₆-Heterocycloalkylring, der gegebenenfalls ferner eine oder mehrere Gruppen, die aus Sauerstoff, Schwefel, Stickstoff und CO ausgewählt sind, enthält und gegebenenfalls durch eine oder mehrere R¹⁰-Gruppen substituiert ist, ausgewählt sind, substituiert ist; oder
R⁴ und R⁵ zusammen mit dem N, an das sie gebunden sind, einen C₃₋₆-Heterocycloalkylring bilden, der gegebenenfalls ferner eine oder mehrere Gruppen, die aus Sauerstoff, Schwefel, Stickstoff und CO ausgewählt sind, enthält, wobei der C₃₋₆-Heterocycloalkylring gesättigt oder ungesättigt ist und gegebenenfalls durch eine oder mehrere Gruppen, die aus A, NR⁸R⁹ und R¹⁰ ausgewählt sind, substituiert ist;
R⁶ jeweils unabhängig voneinander aus C₁₋₆-Alkyl, C₃₋₇-Cycloalkyl, C₄₋₇-Heterocycloalkyl, Aryl und Heteroaryl ausgewählt ist, wobei jede dieser Gruppen gegebenenfalls durch einen oder mehrere Substituenten, die aus R¹⁰, R¹¹ und A ausgewählt sind, substituiert ist;
R⁷ jeweils aus Wasserstoff, C₁₋₆-Alkyl und C₃₋₇-Cycloalkyl ausgewählt ist, wobei das C₁₋₆-Alkyl gegebenenfalls durch ein oder mehrere Halogene substituiert ist;
R⁸ und R⁹ jeweils unabhängig voneinander aus Wasserstoff und C₁₋₆-Alkyl ausgewählt sind, wobei die C₁₋₆-Alkylgruppe gegebenenfalls durch einen oder mehrere Halogene substituiert ist; oder
R⁸ und R⁹ zusammen mit dem N, an das sie gebunden sind, einen C₄₋₆-Heterocycloalkylring bilden, der gegebenenfalls ferner ein oder mehrere Heteroatome, die aus Sauerstoff und Schwefel ausgewählt sind, enthält, wobei der C₄₋₆-Heterocycloalkylring gegebenenfalls durch eine oder mehrere R¹⁰-Gruppen substituiert ist; und
R¹⁰ jeweils aus C₃₋₇-Cycloalkyl, Aryl, Heteroaryl, 0-Heteroaryl, Aralkyl und C₁₋₆-Alkyl ausgewählt ist, wobei jede dieser Gruppen gegebenenfalls durch eine oder mehrere A-Gruppen substituiert ist, wobei dann, wenn R¹⁰ für C₁₋₆-Alkyl steht und zwei oder mehr R¹⁰-Gruppen an dasselbe Kohlenstoffatom gebunden sind, die R¹⁰-Gruppen unter Bildung einer Spiroalkylgruppe verknüpft sein können; und
R¹¹ jeweils unabhängig voneinander aus C₁₋₆-Alkyl, C₃₋₇-Cycloalkyl, C₁₋₆-Alkyl-C₃₋₇-cycloalkyl, C₁₋₆-Alkyl-heteroaryl, C₄₋₇-Heterocycloalkyl, Aryl und Heteroaryl ausgewählt sind, wobei jede dieser Gruppen gegebenenfalls durch einen oder mehrere Substituenten, die aus A ausgewählt sind, substituiert ist; und
A aus Halogen, -NR⁴SO₂R⁵, -CN, -OR⁶, -NR⁴R⁵, -NR⁷R¹¹, Hydroxyl, -CF₃, -CONR⁴R⁵, -NR⁴COR⁵, -NR⁷(CO)NR⁴R⁵, -NO₂, -CO₂H, -CO₂R⁶, -SO₂R⁶, -SO₂NR⁴R⁵, -NR⁴COR⁵, -NR⁴COOR⁵, C₁₋₆-Alkyl, Aryl und -COR⁶ ausgewählt ist.

2. Verbindung nach Anspruch 1, wobei R² aus:
Halogen, weiter bevorzugt Brom;
Aryl, das gegebenenfalls durch einen oder mehrere Substituenten, die aus R¹¹ und A ausgewählt sind, substituiert ist;
C₁₋₆-Alkyl, das gegebenenfalls durch einen oder mehrere Substituenten, die aus R¹¹ und A ausgewählt sind, substituiert ist;
C₂₋₆-Alkenyl, das gegebenenfalls durch einen oder mehrere A-Substituenten substituiert ist;
C₃₋₇-Cycloalkyl;
Heteroaryl, das gegebenenfalls durch einen oder mehrere Substituenten, die aus R¹¹ und A ausgewählt sind, substituiert ist;
C₄₋₇-Heterocycloalkyl und
anelliertem Aryl-C₄₋₇-heterocycloalkyl
ausgewählt ist.

3. Verbindung nach Anspruch 1 oder Anspruch 2, wobei R² aus :
Aryl, das gegebenenfalls durch einen oder mehrere Substituenten, die aus -NR⁴R⁵, -NR⁴COR⁵, -CONR⁴R⁵, OR⁶, Halogen, gegebenenfalls substituiertem C₁₋₆-Alkyl, CN, C₄₋₇-Heterocycloalkyl und Heteroaryl ausgewählt sind, substituiert ist;
C₁₋₆-Alkyl, das gegebenenfalls durch einen oder mehrere Substituenten, die aus -NR⁴COR⁵, -CONR⁴R⁵, -NR⁴R⁵, OR⁶, gegebenenfalls substituiertem Aryl, gegebenenfalls substituiertem Heteroaryl und C₄₋₇-Heterocycloalkyl ausgewählt sind, substituiert ist;
C₂₋₆-Alkenyl, das gegebenenfalls durch einen oder mehrere -CONR⁴R⁵-Substituenten substituiert ist;
C₃₋₇-Cycloalkyl, weiter bevorzugt Cyclopropyl;
Heteroaryl, das gegebenenfalls durch einen oder mehrere Substituenten, die aus -NR⁴R⁵, C₄₋₇-Heterocycloalkyl, C₁₋₆-Alkyl, C₃₋₇-Cycloalkyl, C₁₋₆-Alkyl-C₃₋₇-cycloalkyl und OR⁶ ausgewählt sind, substituiert ist;
C₄₋₇-Heterocycloalkyl und
anelliertem Aryl-C₄₋₇-heterocycloalkyl
ausgewählt ist.

4. Verbindung nach einem der vorhergehenden Ansprüche, wobei R² aus:
einer Phenylgruppe, die gegebenenfalls durch einen oder mehrere Substituenten, die aus -NHCO-C₁₋₆-Alkyl, -CONH-C₁₋₆-Alkyl, CO-(N-Morpholinyl), Cl, F, -O-C₁₋₆-Alkyl, -CONMe₂, OCF₃, CN, CF₃, C₁₋₆-Alkyl-(A), N-Morpholinyl und Pyrazolyl ausgewählt sind, substituiert ist;
einer Heteroarylgruppe, die aus Pyridinyl, Chinolinyl, Pyrazolyl, Furanyl und Pyrimidinyl ausgewählt ist, wobei jede dieser Gruppen gegebenenfalls durch einen oder mehrere Substituenten, die aus C₁₋₆-Alkyl, Aralkyl, O-C₁₋₆-Alkyl und N-Morpholinyl ausgewählt sind, substituiert sein kann;
einer C₁₋₆-Alkylgruppe, die gegebenenfalls durch einen oder mehrere Substituenten, die aus -CONR⁴R⁵, Phenyl, Pyridinyl, Oxadiazolyl und Piperidinyl ausgewählt sind, substituiert ist, wobei die Phenyl-, Pyridinyl- und Oxadiazolyl- und Piperidinylgruppen jeweils gegebenenfalls ferner durch eine oder mehrere -NR⁴COR⁵-, -CONR⁴R⁵-, COR⁶-, SO₂R⁶- oder Arylgruppen substituiert sind;
ausgewählt ist.

5. Verbindung nach Anspruch 4, wobei jede -CONR⁴R⁵-Gruppe unabhängig voneinander aus:
-CO(N-Morpholinyl), -CO(N-Piperidinyl), -CO(N-Pyrrolidinyl), -CO-(N-Piperazinyl), wobei jede dieser Gruppen gegebenenfalls ferner durch einen oder mehrere Substituenten, die aus Aryl, Heteroaryl, -OR⁶, CF₃, Aralkyl, -NR⁴COR⁵-CONR⁴R⁵, -NR⁴R⁵, Halogen und C₁₋₆-Alkyl ausgewählt sind, substituiert sein kann und
-CON(C₁₋₆-Alkyl)₂, CONH(C₁₋₆-Alkyl), CON(C₁₋₆-Alkyl)(aralkyl), CONH(C₃₋₇-Cycloalkyl), -CONH(Aryl), -CONH(Heteroaryl), wobei die C₁₋₆-Alkyl-, Aralkyl-, Aryl- und Heteroarylgruppen jeweils gegebenenfalls ferner durch eine oder mehrere R¹¹- oder A-Gruppen substituiert sein können;
ausgewählt ist.

6. Verbindung nach einem der vorhergehenden Ansprüche, wobei R² für eine C₁₋₆-Alkylgruppe, die gegebenenfalls durch einen oder mehrere Substituenten, die aus -NR⁴COR⁵, -CONR⁴R⁵, -NR⁴R⁵, OR⁶, C₄₋₇-Heterocycloalkyl, Heteroaryl und Aryl ausgewählt sind, substituiert ist, steht, wobei die Arylgruppe gegebenenfalls durch einen oder mehrere Substituenten, die aus -NR⁴COR⁵ und -CONR⁴R⁵ ausgewählt sind, substituiert ist.

7. Verbindung nach einem der Ansprüche 1 bis 3, wobei R² aus -CH₂CH₂CO-NR⁴R⁵, C₁₋₆-Alkyl, C₃₋₇-Cycloalkyl und einem Heteroaryl, das aus Furanyl und Pyrazolyl ausgewählt ist, ausgewählt ist, wobei die Furanyl- und Pyrazolylgruppen gegebenenfalls durch einen oder mehrere Substituenten, die aus C₁₋₆-Alkyl, C₃₋₇-Cycloalkyl und C₁₋₆-Alkyl-C₃₋₇-cycloalkyl ausgewählt sind, substituiert sein können.

8. Verbindung nach Anspruch 7, wobei R² aus Me, 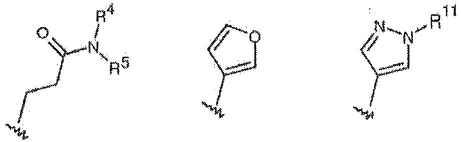 ausgewählt ist, wobei R⁴ und R⁵ zusammen mit dem N, an das sie gebunden sind, einen C₃₋₆-Heterocycloalkylring bilden, der gegebenenfalls ferner eine oder mehrere Gruppen, die aus Sauerstoff, Schwefel, Stickstoff und CO ausgewählt sind, enthält, wobei der C₃₋₆-Heterocycloalkylring gesättigt oder ungesättigt ist und gegebenenfalls durch eine oder mehrere Gruppen, die aus A, NR⁸R⁹ und R¹⁰ ausgewählt sind, substituiert ist.

9. Verbindung nach einem der Ansprüche 1 bis 5, wobei R² für eine unsubstituierte C₁₋₆-Alkylgruppe, weiter bevorzugt Methyl, steht.

10. Verbindung nach einem der vorhergehenden Ansprüche, wobei R¹ aus:
-NHR³;
Aryl;
Heteroaryl;
C₄₋₇-Heterocycloalkyl;
anelliertem Aryl-C₄₋₇-heterocycloalkyl;
-C₃₋₇-Cycloalkyl;
-NR³R⁶;
OR³;
NR⁴R⁵; und
C₁₋₆-Alkyl, das gegebenenfalls durch einen Substituenten, der aus R¹¹ und einer Gruppe A ausgewählt ist, substituiert ist;
ausgewählt ist; wobei das Aryl, Heteroaryl, anellierte Aryl-C₄₋₇-heterocycloalkyl und C₄₋₇-Heterocycloalkyl jeweils gegebenenfalls durch einen oder mehrere Substituenten, die aus C₁₋₆-Alkyl, C₃₋₇-Cycloalkyl, Heteroaryl, C₄₋₇-Heterocycloalkyl, Aryl und einer Gruppe A ausgewählt sind, substituiert sind und die C₁₋₆-Alkyl-, C₃₋₇-Cycloalkyl-, Heteroaryl-, C₄₋₇-Heterocycloalkyl- und Arylsubstituenten wiederum jeweils gegebenenfalls durch eine oder mehrere Gruppen, die aus R¹¹ und einer Gruppe A ausgewählt sind, substituiert sind.

11. Verbindung nach einem der vorhergehenden Ansprüche, wobei R¹ für -NHR³ steht, wobei R³ aus C₁₋₆-Alkyl, C₃₋₇-Cycloalkyl, C₄₋₇-Heterocycloalkyl und Aryl ausgewählt ist, wobei jede dieser Gruppen gegebenenfalls durch einen oder mehrere Substituenten, die aus R¹¹ und A ausgewählt sind, substituiert sein kann.

12. Verbindung nach Anspruch 11, wobei R¹ für -NHR³ steht und R³ aus:
C₁₋₆-Alkyl, das gegebenenfalls durch eine oder mehrere -OR⁶-, NR⁴COR⁵, Heteroaryl-, Aryl-, C₄₋₇-Heterocycloalkyl- und C₃₋₇-Cycloalkylgruppen substituiert ist, wobei die Aryl- und Heteroarylgruppen jeweils unabhängig voneinander gegebenenfalls ferner durch eine oder mehrere Gruppen, die aus CF₃, Halogen, C₁₋₆-Alkyl, -OR⁶ und -NR⁴R⁵ ausgewählt sind, substituiert sein können;
einer Phenylgruppe, die gegebenenfalls durch einen oder mehrere Substituenten, die aus -OR⁶, NR⁴COR⁵, -CONR⁴R⁵, Aryl, -NR⁴R⁵, C₁₋₆-Alkyl-heteroaryl, Heteroaryl, Halogen, -SO₂R⁶, CN, CF₃, C₁₋₆-Alkyl, -SO₂NR⁴R⁵, -NR⁴SO₂R⁵ ausgewählt sind, substituiert ist, wobei die C₁₋₆-Alkyl-, Heteroaryl- und Arylgruppen jeweils unabhängig voneinander gegebenenfalls ferner durch eine oder mehrere Gruppen, die aus CN, CF₃, Halogen, C₁₋₆-Alkyl, -OR⁶ und -NR⁴R⁵ ausgewählt sind, substituiert sind;
einer Heteroarylgruppe, die gegebenenfalls durch einen oder mehrere Substituenten, die aus Aryl, C₁₋₆-Alkyl und -NR⁴R⁵ ausgewählt sind, substituiert ist, wobei die Arylgruppe gegebenenfalls ferner durch eine oder mehrere A-Gruppen substituiert ist;
einem C₄₋₇-Heterocycloalkyl, das gegebenenfalls durch eine oder mehrere -COR⁶-Gruppen substituiert ist;
einer C₃₋₇-Cycloalkylgruppe, die gegebenenfalls durch einen oder mehrere Halogen- oder C₁₋₆-Alkylgruppen substituiert ist;
ausgewählt ist.

13. Verbindung nach einem der Ansprüche 1 bis 10, wobei R¹ für -OR³ steht, wobei R³ aus C₁₋₆-Alkyl, C₃₋₇-Cycloalkyl, C₄₋₇-Heterocycloalkyl und Aryl ausgewählt ist, wobei jede dieser Gruppen gegebenenfalls durch einen oder mehrere Substituenten, die aus R¹¹ und A ausgewählt sind, substituiert sein kann.

14. Verbindung nach einem der Ansprüche 1 bis 10, wobei R¹ für Aryl oder Heteroaryl steht, wobei jede dieser Gruppen gegebenenfalls durch einen oder mehrere Substituenten, die aus R¹¹ und A ausgewählt sind, substituiert sein kann.

15. Verbindung nach einem der Ansprüche 1 bis 10, wobei R¹ für -NH-C₃₋₇-Cycloalkyl oder NH-C₄₋₇-Heterocycloalkyl steht, wobei jede dieser Gruppen gegebenenfalls durch einen oder mehrere A-Substituenten substituiert sein kann.

16. Verbindung nach einem der vorhergehenden Ansprüche, wobei R³ für Cyclohexyl oder Tetrahydropyranyl steht, wobei jede dieser Gruppen gegebenenfalls durch einen oder mehrere A-Substituenten substituiert sein kann.

17. Verbindung nach einem der vorhergehenden Ansprüche, wobei R¹ aus den folgenden ausgewählt ist: 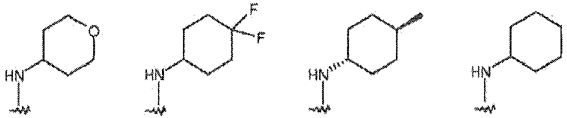 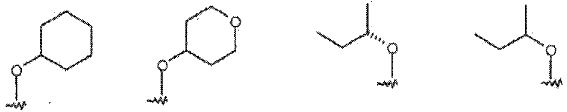 und wobei R¹ vorzugsweise für -NH-Cyclohexyl steht.

18. Verbindung nach Anspruch 1, wobei R¹ für -NHR³ steht und R² für eine unsubstituierte C₁₋₆-Alkylgruppe, weiter bevorzugt Methyl, steht.

19. Verbindung nach Anspruch 1, wobei R¹ für -NHR³ steht und R² für eine C₁₋₆-Alkylgruppe, die durch eine oder mehrere -CONR⁴R⁵-Gruppen substituiert ist, steht.

20. Verbindung nach Anspruch 1, wobei R¹ für -NHR³ steht und R² für eine Aryl- oder Heteroarylgruppe steht, wobei jede dieser Gruppen gegebenenfalls durch einen oder mehrere Substituenten, die aus C₄₋₇-Heterocycloalkyl, C₁₋₆-Alkyl, C₃₋₇-Cycloalkyl, C₁₋₆-Alkyl-C₃₋₇-cycloalkyl und OR⁶ ausgewählt sind, substituiert sein kann.

21. Verbindung nach Anspruch 1, wobei R¹ aus: 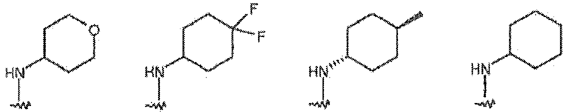 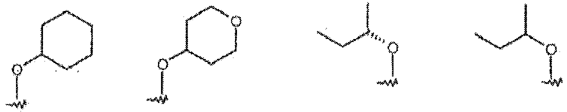 ausgewählt ist und R² aus Me, 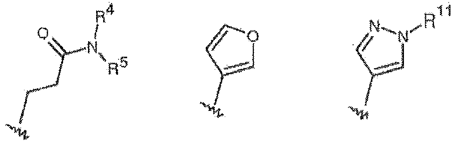 ausgewählt ist, wobei R⁴ und R⁵ zusammen mit dem N, an das sie gebunden sind, einen C₃₋₆-Heterocycloalkylring bilden, der gegebenenfalls ferner eine oder mehrere Gruppen, die aus Sauerstoff, Schwefel, Stickstoff und CO ausgewählt sind, enthält, wobei der C₃₋₆-Heterocycloalkylring gesättigt oder ungesättigt ist und gegebenenfalls durch eine oder mehrere Gruppen, die aus A, NR⁸R⁹ und R¹⁰ ausgewählt sind, substituiert ist.

22. Verbindung, die aus den folgenden ausgewählt ist:
| | |
|---|---|
| 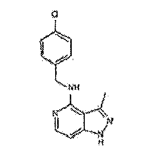 | Beispiel 1 |
| 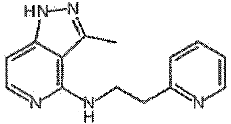 | Beispiel 2 |
| 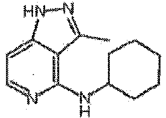 | Beispiel 3 |
| 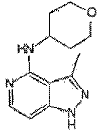 | Beispiel 4 |
| 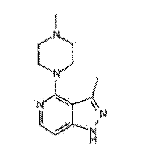 | Beispiel 5 |
| 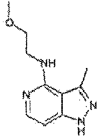 | Beispiel 6 |
| 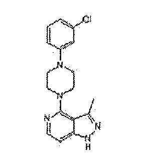 | Beispiel 7 |
| 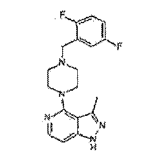 | Beispiel 8 |
| 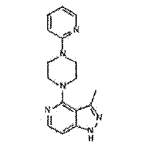 | Beispiel 9 |
| 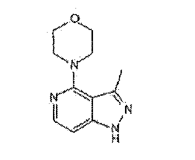 | Beispiel 10 |
| 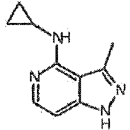 | Beispiel 11 |
| 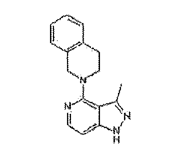 | Beispiel 12 |
| 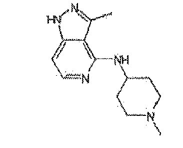 | Beispiel 13 |
| 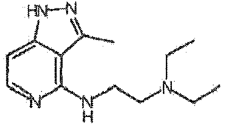 | Beispiel 14 |
| 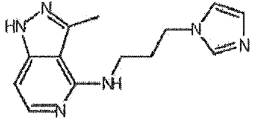 | Beispiel 15 |
| 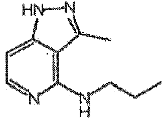 | Beispiel 16 |
| 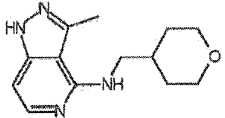 | Beispiel 17 |
| 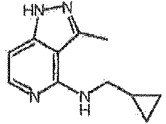 | Beispiel 18 |
| 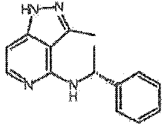 | Beispiel 19 |
| 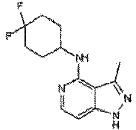 | Beispiel 20 |
| 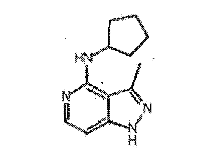 | Beispiel 21 |
| 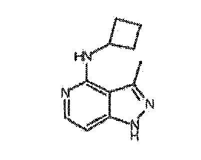 | Beispiel 22 |
| 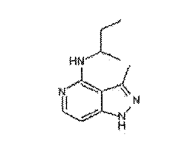 | Beispiel 23 |
| 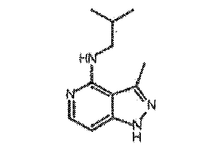 | Beispiel 24 |
| 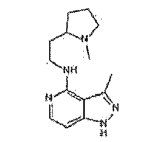 | Beispiel 25 |
| 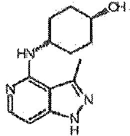 | Beispiel 26 |
| 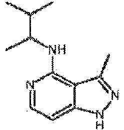 | Beispiel 27 |
| 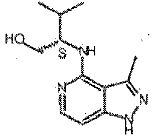 | Beispiel 28 |
| 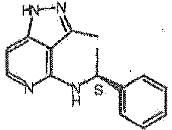 | Beispiel 29 |
| 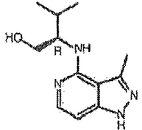 | Beispiel 30 |
| 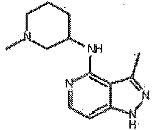 | Beispiel 31 |
| 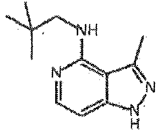 | Beispiel 32 |
| 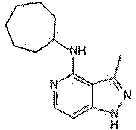 | Beispiel 33 |
| 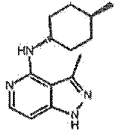 | Beispiel 34 |
| 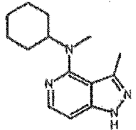 | Beispiel 35 |
| 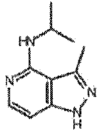 | Beispiel 36 |
| 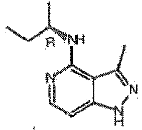 | Beispiel 37 |
| 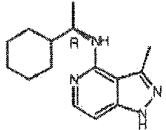 | Beispiel 38 |
| 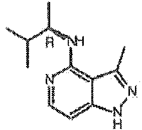 | Beispiel 39 |
| 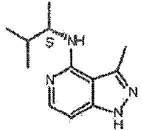 | Beispiel 40 |
| 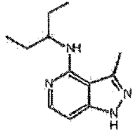 | Beispiel 41 |
| 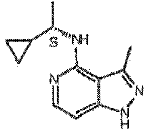 | Beispiel 42 |
| 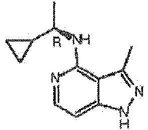 | Beispiel 43 |
| 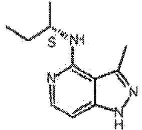 | Beispiel 44 |
| 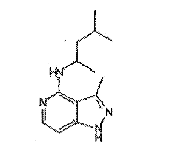 | Beispiel 45 |
| 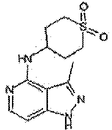 | Beispiel 46 |
| 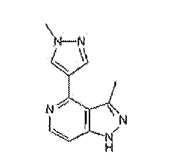 | Beispiel 47 |
| 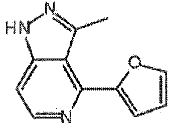 | Beispiel 48 |
| 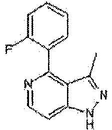 | Beispiel 49 |
| 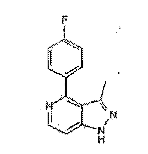 | Beispiel 50 |
| 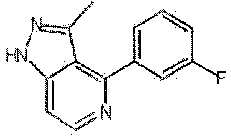 | Beispiel 51 |
| 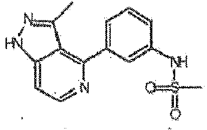 | Beispiel 52 |
| 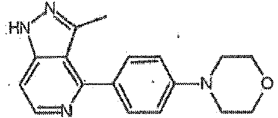 | Beispiel 53 |
| 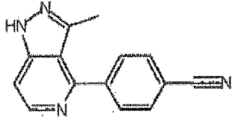 | Beispiel 54 |
| 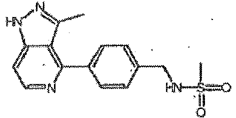 | Beispiel 55 |
| 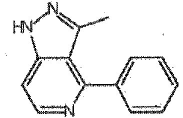 | Beispiel 56 |
| 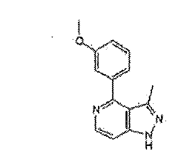 | Beispiel 57 |
| 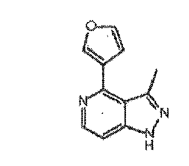 | Beispiel 58 |
| 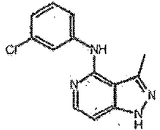 | Beispiel 59 |
| 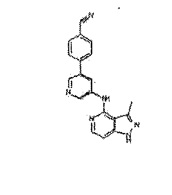 | Beispiel 60 |
| 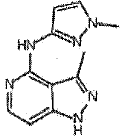 | Beispiel 61 |
| 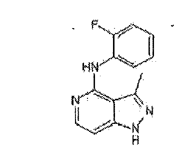 | Beispiel 62 |
| 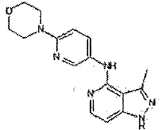 | Beispiel 63 |
| 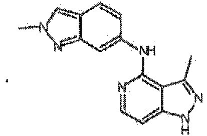 | Beispiel 64 |
| 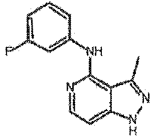 | Beispiel 65 |
| 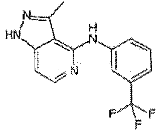 | Beispiel 66 |
| 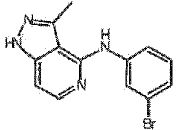 | Beispiel 67 |
| 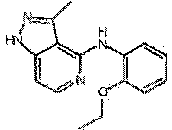 | Beispiel 68 |
| 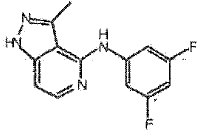 | Beispiel 69 |
| 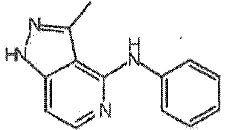 | Beispiel 70 |
| 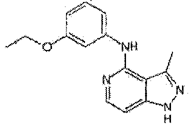 | Beispiel 71 |
| 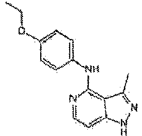 | Beispiel 72 |
| 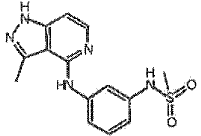 | Beispiel 73 |
| 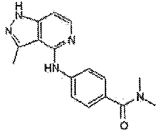 | Beispiel 74 |
| 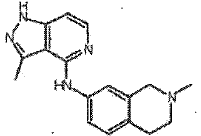 | Beispiel 75 |
| 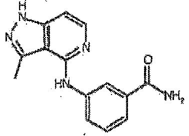 | Beispiel 76 |
| 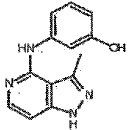 | Beispiel 77 |
| 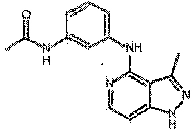 | Beispiel 78 |
| 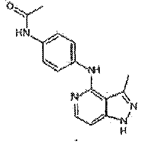 | Beispiel 79 |
| 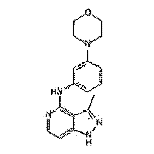 | Beispiel 80 |
| 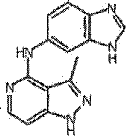 | Beispiel 81 |
| 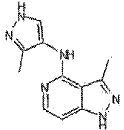 | Beispiel 82 |
| 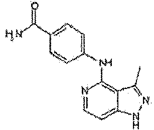 | Beispiel 83 |
| 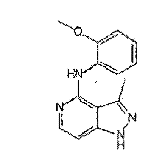 | Beispiel 84 |
| 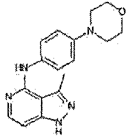 | Beispiel 85 |
| 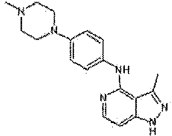 | Beispiel 86 |
| 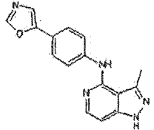 | Beispiel 87 |
| 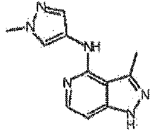 | Beispiel 88 |
| 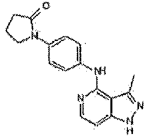 | Beispiel 89 |
| 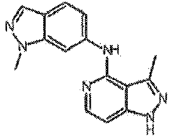 | Beispiel 90 |
| 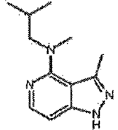 | Beispiel 91 |
| 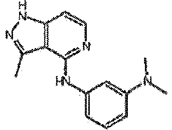 | Beispiel 92 |
| 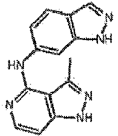 | Beispiel 93 |
| 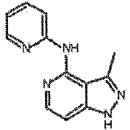 | Beispiel 94 |
| 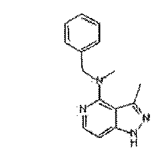 | Beispiel 95 |
| 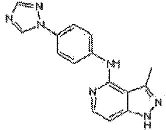 | Beispiel 96 |
| 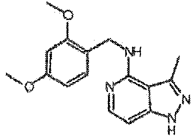 | Beispiel 97 |
| 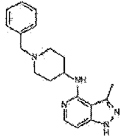 | Beispiel 98 |
| 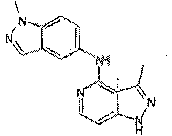 | Beispiel 99 |
| 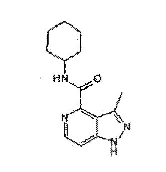 | Beispiel 100 |
| 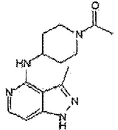 | Beispiel 101 |
| 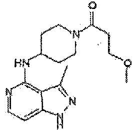 | Beispiel 102 |
| 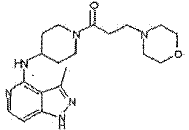 | Beispiel 103 |
| 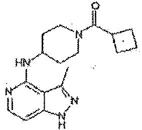 | Beispiel 104 |
| 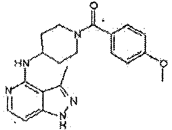 | Beispiel 105 |
| 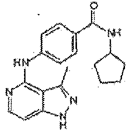 | Beispiel 106 |
| 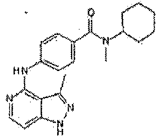 | Beispiel 107 |
| 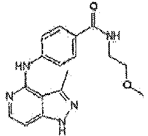 | Beispiel 108 |
| 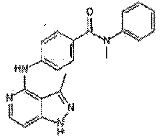 | Beispiel 109 |
| 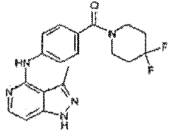 | Beispiel 110 |
| 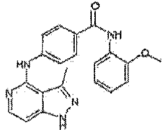 | Beispiel 111 |
| 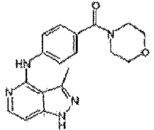 | Beispiel 112 |
| 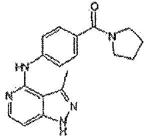 | Beispiel 113 |
| 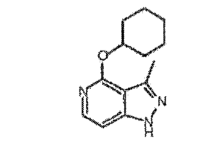 | Beispiel 114 |
| 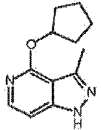 | Beispiel 115 |
| 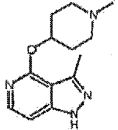 | Beispiel 116 |
| 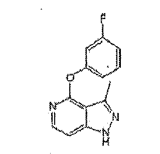 | Beispiel 117 |
| 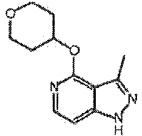 | Beispiel 118 |
| 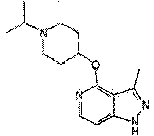 | Beispiel 119 |
| 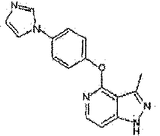 | Beispiel 120 |
| 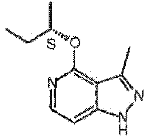 | Beispiel 121 |
| 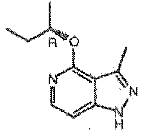 | Beispiel 122 |
| 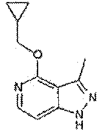 | Beispiel 123 |
| 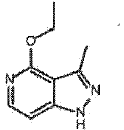 | Beispiel 124 |
| 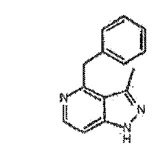 | Beispiel 125 |
| 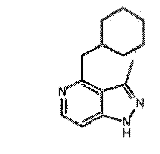 | Beispiel 126 |
| 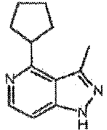 | Beispiel 127 |
| 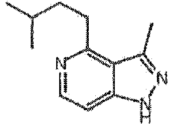 | Beispiel 128 |
| 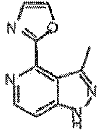 | Beispiel 129 |
| 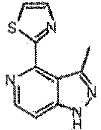 | Beispiel 130 |
| 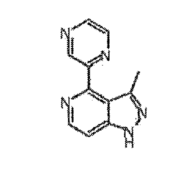 | Beispiel 131 |
| 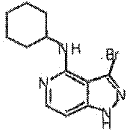 | Beispiel 134 |
| 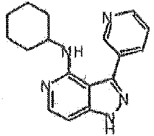 | Beispiel 135 |
| 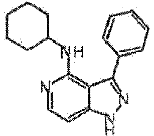 | Beispiel 136 |
| 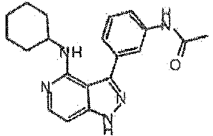 | Beispiel 137 |
| 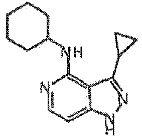 | Beispiel 138 |
| 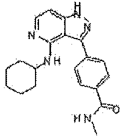 | Beispiel 139 |
| 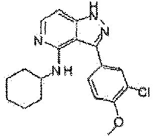 | Beispiel 140 |
| 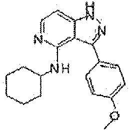 | Beispiel 141 |
| 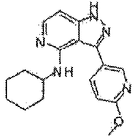 | Beispiel 142 |
| 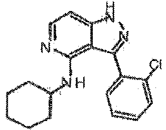 | Beispiel 143 |
| 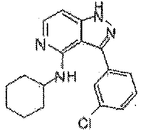 | Beispiel 144 |
| 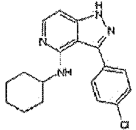 | Beispiel 145 |
| 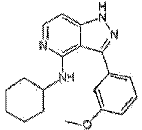 | Beispiel 146 |
| 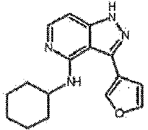 | Beispiel 147 |
| 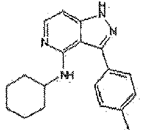 | Beispiel 148 |
| 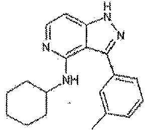 | Beispiel 149 |
| 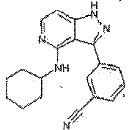 | Beispiel 150 |
| 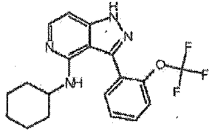 | Beispiel 151 |
| 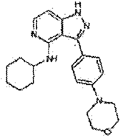 | Beispiel 152 |
| 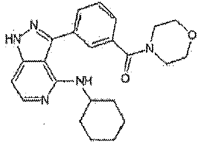 | Beispiel 153 |
| 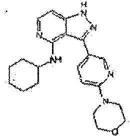 | Beispiel 154 |
| 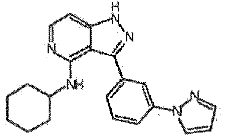 | Beispiel 155 |
| 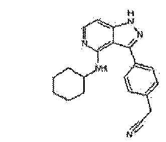 | Beispiel 156 |
| 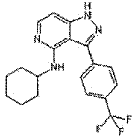 | Beispiel 157 |
| 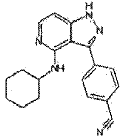 | Beispiel 158 |
| 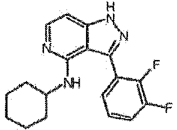 | Beispiel 159 |
| 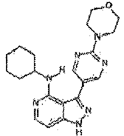 | Beispiel 160 |
| 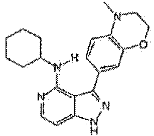 | Beispiel 161 |
| 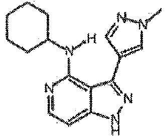 | Beispiel 162 |
| 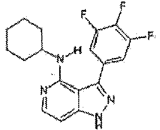 | Beispiel 163 |
| 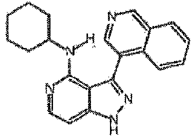 | Beispiel 164 |
| 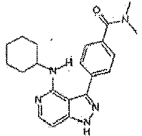 | Beispiel 165 |
| 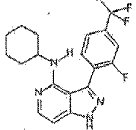 | Beispiel 166 |
| 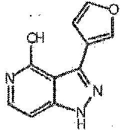 | Beispiel 167 |
| 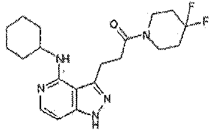 | Beispiel 168 |
| 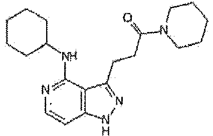 | Beispiel 169 |
| 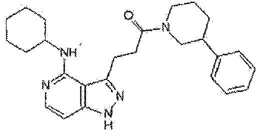 | Beispiel 170 |
| 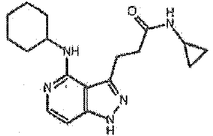 | Beispiel 171 |
| 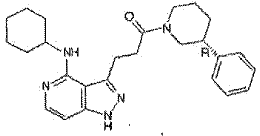 | Beispiel 172 |
| 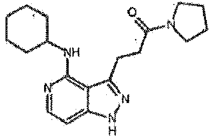 | Beispiel 173 |
| 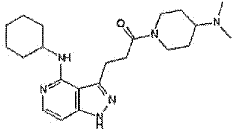 | Beispiel 174 |
| 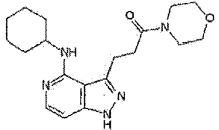 | Beispiel 175 |
| 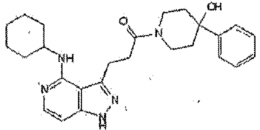 | Beispiel 176 |
| 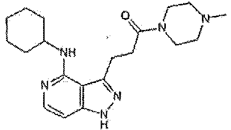 | Beispiel 177 |
| 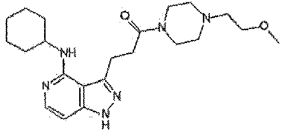 | Beispiel 178 |
| 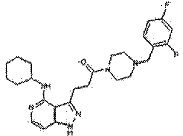 | Beispiel 179 |
| 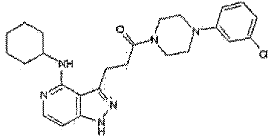 | Beispiel 180 |
| 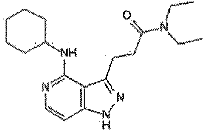 | Beispiel 181 |
| 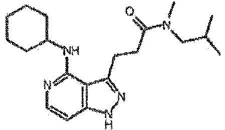 | Beispiel 182 |
| 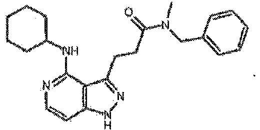 | Beispiel 183 |
| 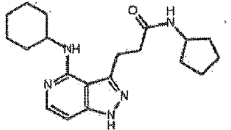 | Beispiel 184 |
| 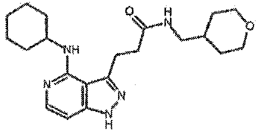 | Beispiel 185 |
| 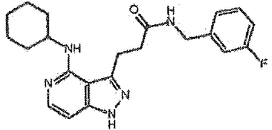 | Beispiel 186 |
| 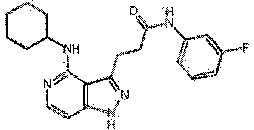 | Beispiel 187 |
| 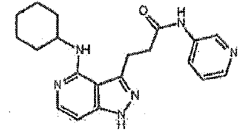 | Beispiel 188 |
| 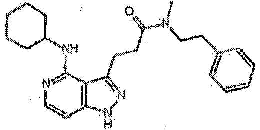 | Beispiel 189 |
| 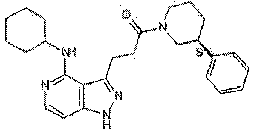 | Beispiel 190 |
| 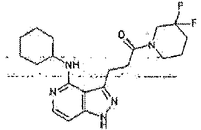 | Beispiel 191 |
| 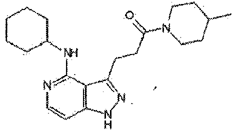 | Beispiel 192 |
| 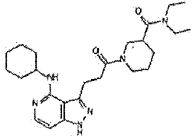 | Beispiel 193 |
| 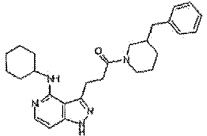 | Beispiel 194 |
| 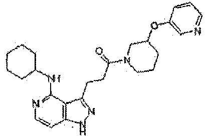 | Beispiel 195 |
| 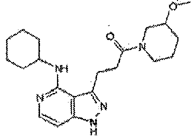 | Beispiel 196 |
| 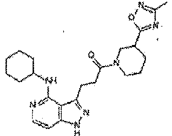 | Beispiel 197 |
| 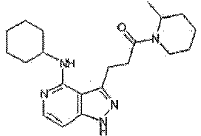 | Beispiel 198 |
| 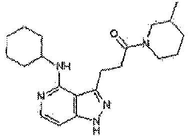 | Beispiel 199 |
| 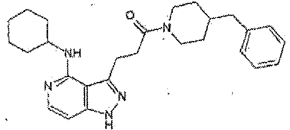 | Beispiel 200 |
| 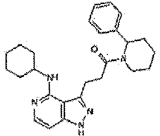 | Beispiel 201 |
| 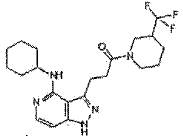 | Beispiel 202 |
| 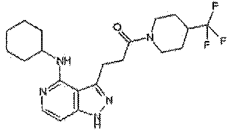 | Beispiel 203 |
| 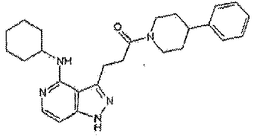 | Beispiel 204 |
| 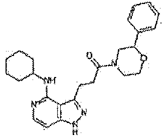 | Beispiel 205 |
| 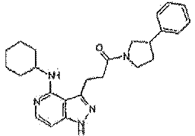 | Beispiel 206 |
| 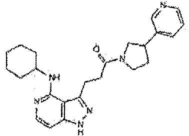 | Beispiel 207 |
| 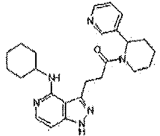 | Beispiel 208 |
| 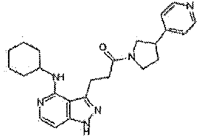 | Beispiel 209 |
| 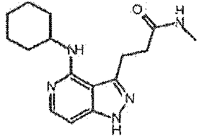 | Beispiel 210 |
| 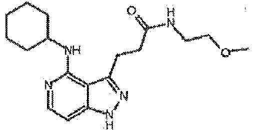 | Beispiel 211 |
| 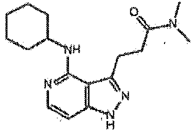 | Beispiel 212 |
| 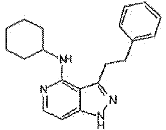 | Beispiel 213 |
| 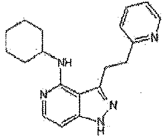 | Beispiel 214 |
| 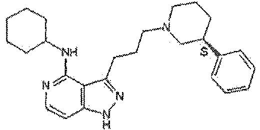 | Beispiel 215 |
| 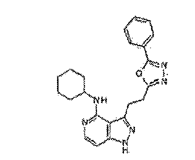 | Beispiel 216 |
| 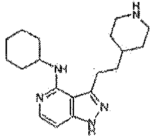 | Beispiel 217 |
| 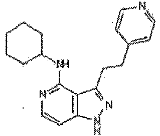 | Beispiel 218 |
| 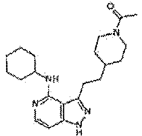 | Beispiel 219 |
| 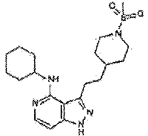 | Beispiel 220 |
| 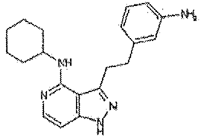 | Beispiel 221 |
| 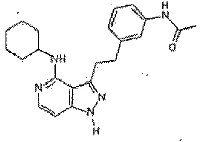 | Beispiel 222 |
| 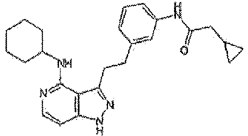 | Beispiel 223 |
| 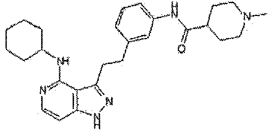 | Beispiel 224 |
| 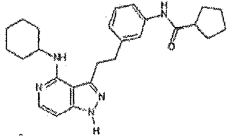 | Beispiel 225 |
| 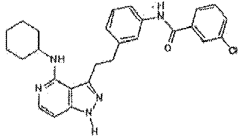 | Beispiel 226 |
| 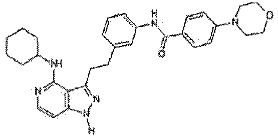 | Beispiel 227 |
| 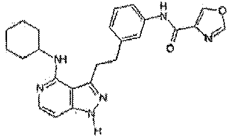 | Beispiel 228 |
| 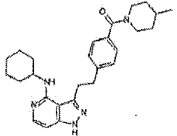 | Beispiel 229 |
| 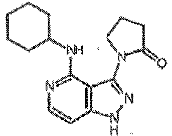 | Beispiel 230 |
| 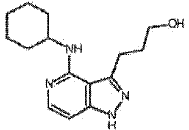 | Beispiel 231 |
| 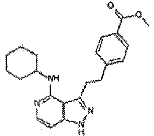 | Beispiel 232 |
| 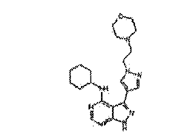 | Beispiel 233 |
| 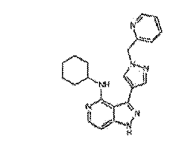 | Beispiel 234 |
| 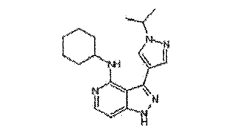 | Beispiel 235 |
| 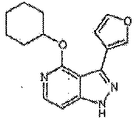 | Beispiel 236 |
| 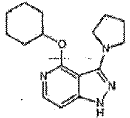 | Beispiel 237 |
| 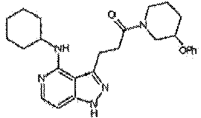 | Beispiel 238 |
| 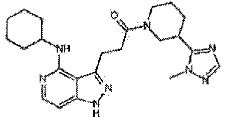 | Beispiel 239 |
| 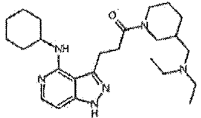 | Beispiel 240 |
| 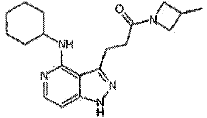 | Beispiel 241 |
| 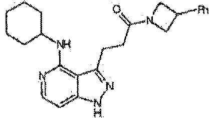 | Beispiel 242 |
| 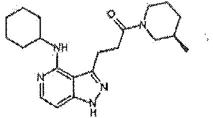 | Beispiel 243 |
| 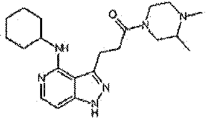 | Beispiel 244 |
| 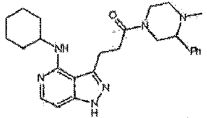 | Beispiel 245 |
| 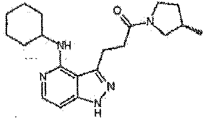 | Beispiel 246 |
| 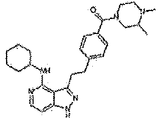 | Beispiel 247 |
| 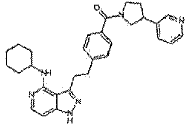 | Beispiel 248 |
| 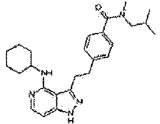 | Beispiel 249 |
| 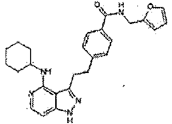 | Beispiel 250 |
| 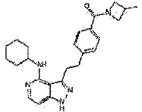 | Beispiel 251 |
| 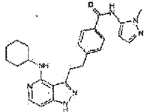 | Beispiel 252 |
| 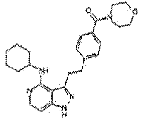 | Beispiel 253 |
| 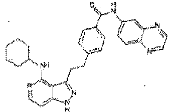 | Beispiel 254 |
| 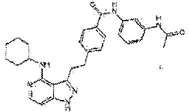 | Beispiel 255 |
| 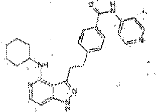 | Beispiel 256 |
| 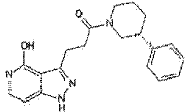 | Beispiel 257 |
| 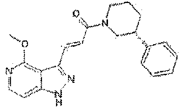 | Beispiel 258 |
| 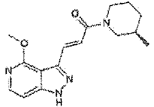 | Beispiel 259 |
| 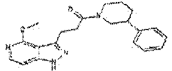 | Beispiel 260 |
| 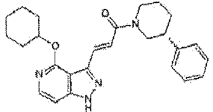 | Beispiel 261 |
| 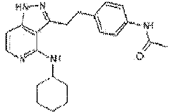 | Beispiel 262 |
| 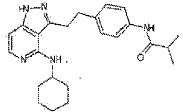 | Beispiel 263 |
| 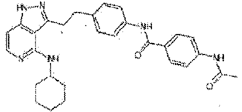 | Beispiel 264 |
| 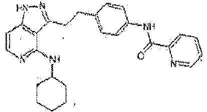 | Beispiel 265 |
| 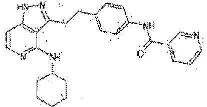 | Beispiel 266 |
| 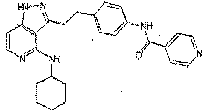 | Beispiel 267 |
| 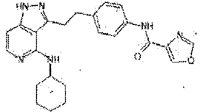 | Beispiel 268 |
| 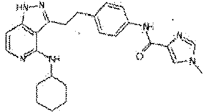 | Beispiel 269 |
| 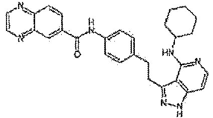 | Beispiel 270 |
| 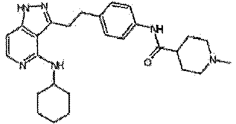 | Beispiel 271 |
| 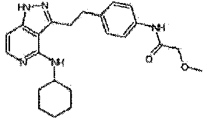 | Beispiel 272 |
| 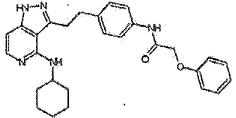 | Beispiel 273 |
| 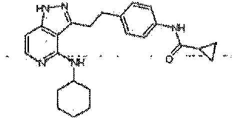 | Beispiel 274 |
| 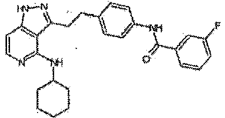 | Beispiel 275 |
| 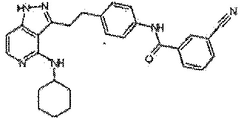 | Beispiel 276 |
| 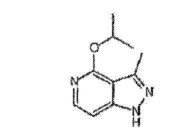 | Beispiel 277 |
| 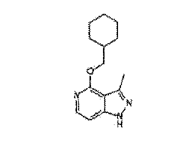 | Beispiel 278 |
| 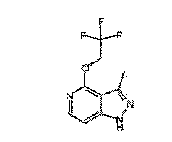 | Beispiel 279 |
| 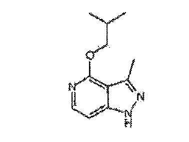 | Beispiel 280 |
| 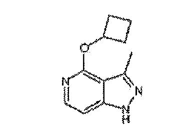 | Beispiel 281 |
| 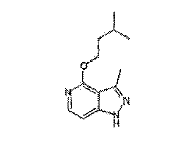 | Beispiel 282 |
| 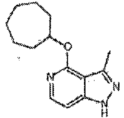 | Beispiel 283 |
| 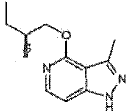 | Beispiel 284 |
| 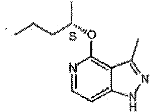 | Beispiel 285 |
| 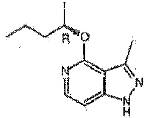 | Beispiel 286 |
| 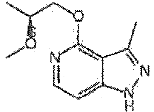 | Beispiel 287 |
| 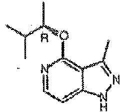 | Beispiel 288 |
| 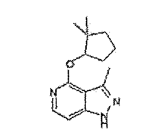 | Beispiel 289 |
| 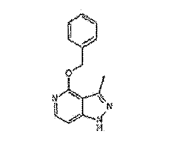 | Beispiel 290 |
| 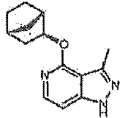 | Beispiel 291 |
| 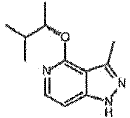 | Beispiel 292 |
| 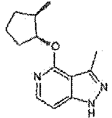 | Beispiel 293 |
| 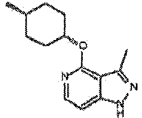 | Beispiel 294 |
| 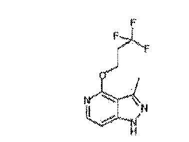 | Beispiel 295 |
| 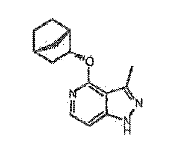 | Beispiel 296 |
| 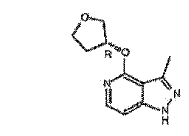 | Beispiel 297 |
| 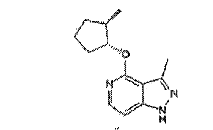 | Beispiel 298 |
| 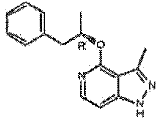 | Beispiel 299 |
| 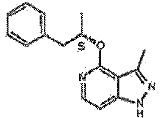 | Beispiel 300 |
| 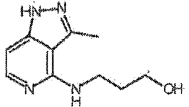 | Beispiel 301 |
| 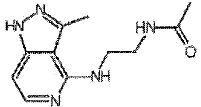 | Beispiel 302 |
| 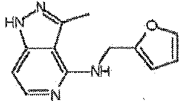 | Beispiel 303 |
| 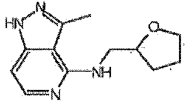 | Beispiel 304 |
| 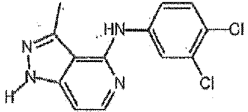 | Beispiel 305 |
| 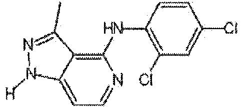 | Beispiel 306 |
| 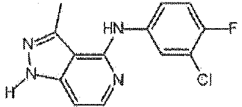 | Beispiel 307 |
| 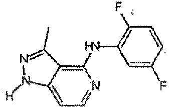 | Beispiel 308 |
| 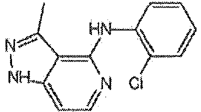 | Beispiel 309 |
| 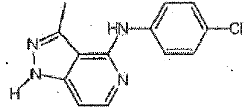 | Beispiel 310 |
| 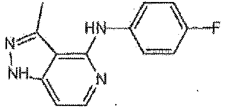 | Beispiel 311 |
| 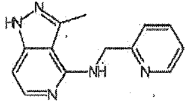 | Beispiel 312 |
| 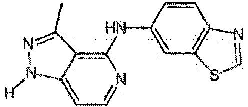 | Beispiel 313 |
| 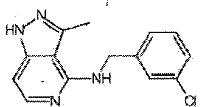 | Beispiel 314 |
| 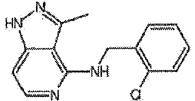 | Beispiel 315 |
| 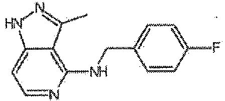 | Beispiel 316 |
| 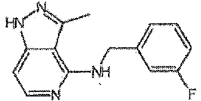 | Beispiel 317 |
| 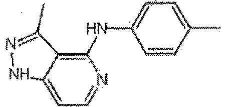 | Beispiel 318 |
| 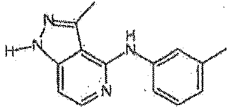 | Beispiel 319 |
| 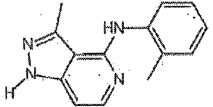 | Beispiel 320 |
| 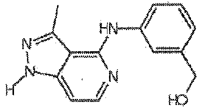 | Beispiel 321 |
| 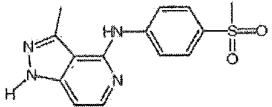 | Beispiel 322 |
| 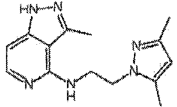 | Beispiel 323 |
| 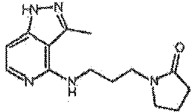 | Beispiel 324 |
| 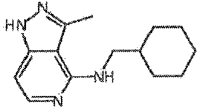 | Beispiel 325 |
| 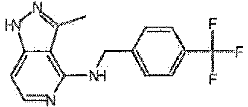 | Beispiel 326 |
| 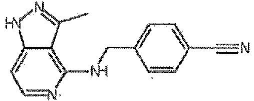 | Beispiel 327 |
| 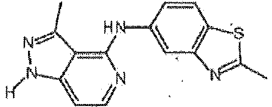 | Beispiel 328 |
| 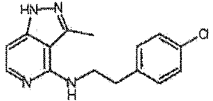 | Beispiel 329 |
| 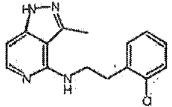 | Beispiel 330 |
| 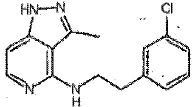 | Beispiel 331 |
| 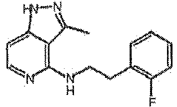 | Beispiel 332 |
| 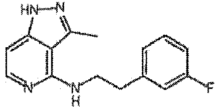 | Beispiel 333 |
| 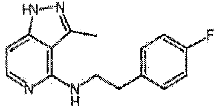 | Beispiel 334 |
| 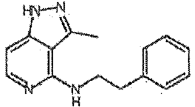 | Beispiel 335 |
| 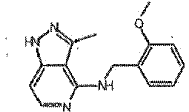 | Beispiel 336 |
| 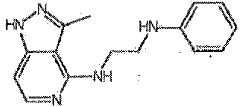 | Beispiel 337 |
| 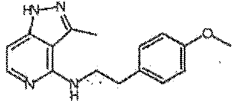 | Beispiel 338 |
| 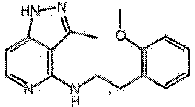 | Beispiel 339 |
| 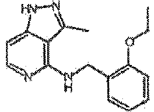 | Beispiel 340 |
| 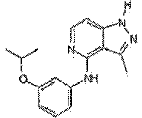 | Beispiel 341 |
| 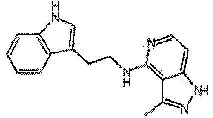 | Beispiel 342 |
| 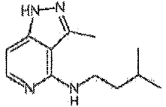 | Beispiel 343 |
| 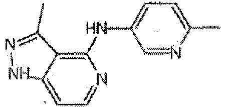 | Beispiel 344 |
| 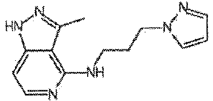 | Beispiel 345 |
| 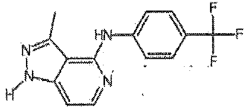 | Beispiel 346 |
| 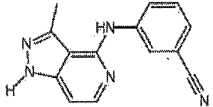 | Beispiel 347 |
| 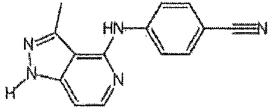 | Beispiel 348 |
| 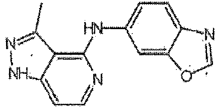 | Beispiel 349 |
| 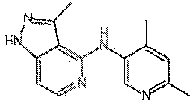 | Beispiel 350 |
| 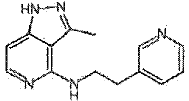 | Beispiel 351 |
| 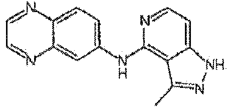 | Beispiel 352 |
| 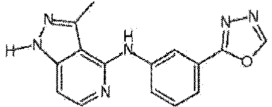 | Beispiel 353 |
| 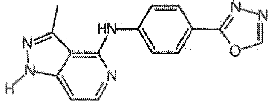 | Beispiel 354 |
| 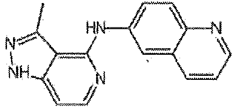 | Beispiel 355 |
| 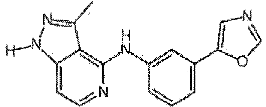 | Beispiel 356 |
| 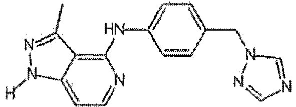 | Beispiel 357 |
| 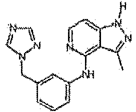 | Beispiel 358 |
| 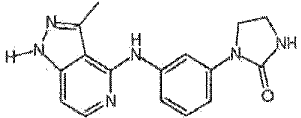 | Beispiel 359 |
| 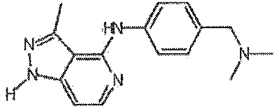 | Beispiel 360 |
| 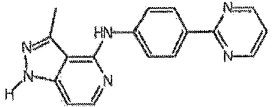 | Beispiel 361 |
| 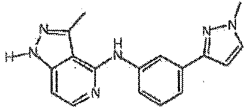 | Beispiel 362 |
| 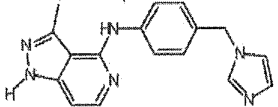 | Beispiel 363 |
| 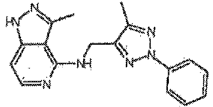 | Beispiel 364 |
| 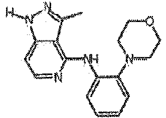 | Beispiel 365 |
| 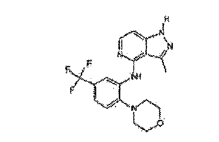 | Beispiel 366 |
| 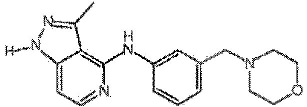 | Beispiel 367 |
| 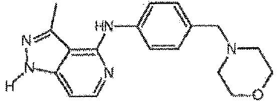 | Beispiel 368 |
| 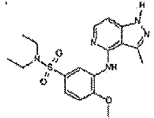 | Beispiel 369 |
| 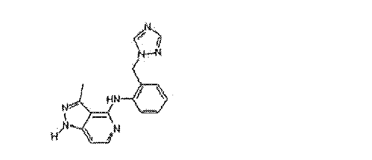 | Beispiel 370 |
| 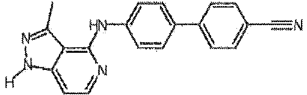 | Beispiel 371 |
| 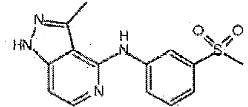 | Beispiel 372 |
| 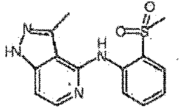 | Beispiel 373 |
| 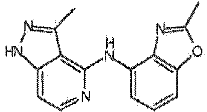 | Beispiel 374 |
| 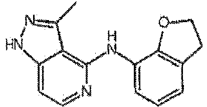 | Beispiel 375 |
| 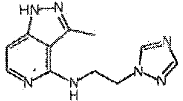 | Beispiel 376 |
| 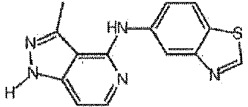 | Beispiel 377 |
| 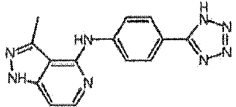 | Beispiel 378 |
| 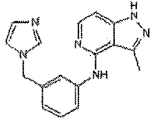 | Beispiel 379 |
| 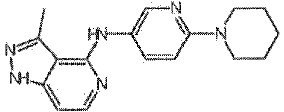 | Beispiel 380 |
| 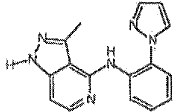 | Beispiel 381 |
| 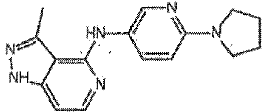 | Beispiel 382 |
| 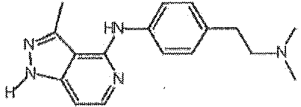 | Beispiel 383 |
| 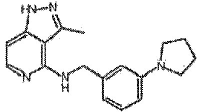 | Beispiel 384 |
| 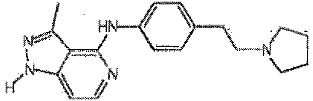 | Beispiel 385 |
| 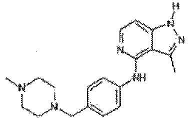 | Beispiel 386 |
| 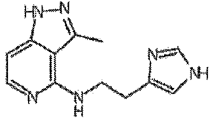 | Beispiel 387 |
| 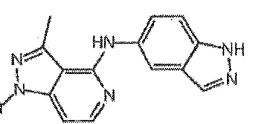 | Beispiel 388 |
| 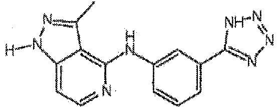 | Beispiel 389 |
| 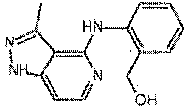 | Beispiel 390 |
| 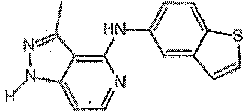 | Beispiel 391 |
| 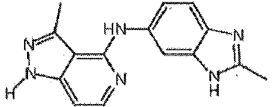 | Beispiel 392 |

23. Pharmazeutische Zusammensetzung, umfassend eine Verbindung nach einem der Ansprüche 1 bis 22 und einen pharmazeutisch unbedenklichen Träger, ein pharmazeutisch unbedenkliches Verdünnungsmittel oder einen pharmazeutisch unbedenklichen Hilfsstoff, wobei die pharmazeutische Zusammensetzung gegebenenfalls ferner ein zweites therapeutisches Mittel umfasst.

24. Verbindung nach einem der Ansprüche 1 bis 22 zur Verwendung in der Medizin.

25. Verbindung nach einem der Ansprüche 1 bis 22 zur Verwendung bei der Behandlung einer Störung, die aus Krebs und neurodegenerativen Erkrankungen ausgewählt ist.

26. Verwendung einer Verbindung nach einem der Ansprüche 1 bis 22 bei der Herstellung eines Medikaments zur Behandlung oder Prävention einer Störung, die aus Krebs und neurodegenerativen Erkrankungen ausgewählt ist, oder zur Prävention oder
Behandlung einer Störung, die durch abnormale Kinaseaktivität, vorzugsweise abnormale LRRK2-Aktivität, verursacht wird, damit assoziiert ist oder damit einhergeht.

27. Verwendung einer Verbindung nach einem der Ansprüche 1 bis 22 bei einem Assay zur Identifizierung weiterer Kandidatenverbindungen, die zur Inhibierung von LRRK, weiter bevorzugt LRRK2, befähigt sind.

28. Verfahren zur Herstellung einer Verbindung der Formel I gemäß Anspruch 1, bei dem man eine Verbindung der Formel II in eine Verbindung der Formel I umwandelt: 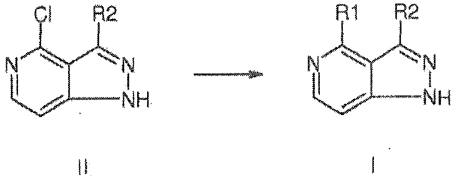 wobei das Verfahren gegebenenfalls ferner den Schritt der Herstellung der Verbindung der Formel II durch Behandeln einer Verbindung der Formel III mit Hydrazinmonohydrat umfasst: 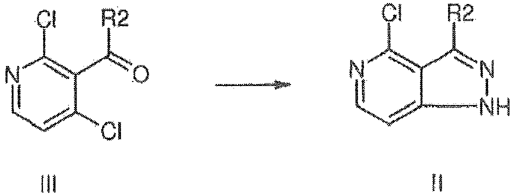 wobei das Verfahren gegebenenfalls ferner den Schritt der Herstellung der Verbindung der Formel III durch Behandeln einer Verbindung der Formel IV mit einem Oxidationsmittel umfasst: 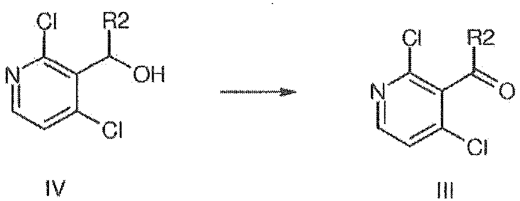 wobei das Verfahren gegebenenfalls ferner den Schritt der Herstellung der Verbindung der Formel IV durch Behandeln einer Verbindung der Formel V mit R²-Mg-Cl umfasst:

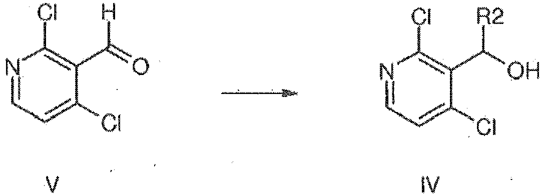

29. Verfahren nach Anspruch 28, wobei R¹ für -NHR³ steht und das Verfahren die Umsetzung einer Verbindung der Formel II mit einem Amin der Formel NH₂R³ umfasst.

30. Verfahren nach Anspruch 28, wobei R¹ für ein NH-haltiges C₄₋₇-Heterocycloalkyl oder ein NH-haltiges anelliertes Aryl-C₄₋₇-heterocycloalkyl steht und das Verfahren die Umsetzung einer Verbindung der Formel II mit der NH-Gruppe des C₄₋₇-Heterocycloalkyls oder des anellierten Aryl-C₄₋₇-heterocycloalkyls umfasst.

31. Verfahren nach Anspruch 28, wobei R¹ aus Aryl, Heteroaryl, C₄₋₇-Heterocycloalkyl, anelliertem Aryl-C₄₋₇-heterocycloalkyl, -C₃₋₇-Cycloalkyl und -C₁₋₆-Alkyl ausgewählt ist und das Verfahren die Umsetzung einer Verbindung der Formel II mit X-R¹, wobei X für eine 4,4,5,5-Tetramethyl-1,3,2-dioxaborolan-2-ylgruppe steht, in Gegenwart eines Kupplungsmittels umfasst.

32. Kombination, umfassend eine Verbindung nach einem der Ansprüche 1 bis 22 und ein weiteres Therapeutikum.

## Revendications

1. Composé de formule I, ou l'un des sels pharmaceutiquement acceptables de celui-ci, 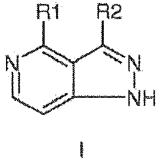 où :
R¹ est choisi parmi les groupements :
aryle ;
hétéroaryle ;
-NHR³ ;
aryl-(hétérocycloalkyle en C₄₋₇) fusionné ;
-CONR⁴R⁵ ;
-NHCOR⁶ ;
- (cycloalkyle en C₃₋₇) ;
-NR³R⁶ ;
OR³ ;
OH ;
NR⁴R⁵ ; et
-(alkyle en C₁₋₆) éventuellement substitué par un substituant choisi parmi R¹¹ et un groupement A ;
où chacun desdits groupements aryle, hétéroaryle, aryl-(hétérocycloalkyle en C₄₋₇) fusionné et hétérocycloalkyle en C₄₋₇ est éventuellement substitué par un ou plusieurs substituants choisis parmi les groupements alkyle en C₁₋₆, cycloalkyle en C₃₋₇, hétéroaryle, hétérocycloalkyle en C₄₋₇, aryle et un groupement A, et chacun desdits substituants alkyle en C₁₋₆, cycloalkyle en C₃₋₇, hétéroaryle, hétérocycloalkyle en C₄₋₇, et aryle est à son tour éventuellement substitué par un ou plusieurs groupements choisis parmi R¹¹ et un groupement A ;
R² est choisi parmi les groupements aryle, alkyle en C₁₋₆, alcényle en C₂₋₆, cycloalkyle en C₃₋₇, hétéroaryle, hétérocycloalkyle en C₄₋₇, aryl-(hétérocycloalkyle en C₄₋₇) fusionné et halogène, où chacun desdits substituants alkyle en C₁₋₆, alcényle en C₂₋₆, aryle, hétéroaryle, aryl-(hétérocycloalkyle en C₄₋₇) fusionné et hétérocycloalkyle en C₄₋₇ est éventuellement substitué par un ou plusieurs substituants choisis parmi R¹¹ et A ;
chacun des radicaux R³ est choisi parmi les groupements aryle, hétéroaryle, hétérocycloalkyle en C₄₋₇, cycloalkyle en C₃₋₇, aryl-(hétérocycloalkyle en C₄₋₇) fusionné et alkyle en C₁₋₆, chacun de ceux-ci étant éventuellement substitué par un ou plusieurs substituants choisis parmi R¹¹ et A ;
chacun desdits radicaux R⁴ et R⁵ est indépendamment choisi parmi l'atome d'hydrogène et les groupements cycloalkyle en C₃₋₇, (alkyle en C₁₋₆)-(cycloalkyle en C₃₋₇), aryle, hétéroaryle, alkyle en C₁₋₆ et cycle hétérocycloalkyle en C₃₋₆ comportant en outre éventuellement un ou plusieurs groupements choisis parmi l'oxygène, le soufre, l'azote et CO, et éventuellement substitué par un ou plusieurs groupements R¹⁰, où chacun desdits groupements alkyle en C₁₋₆, hétéroaryle et aryle est éventuellement substitué par un ou plusieurs substituants choisis parmi les groupements alkyle en C₁₋₆, halogène, cyano, hydroxyl, aryle, aryle halogéné, hétéroaryle, -NR⁸R⁹, -NR⁶R⁷, NR⁷(CO)R⁶, -NR⁷COOR⁶, -NR⁷(SO₂)R⁶, -COOR⁶, -CONR⁸R⁹, OR⁶,-SO₂R⁶ et cycle hétérocycloalkyle en C₃₋₆ comportant en outre éventuellement un ou plusieurs groupements choisis parmi l'oxygène, le soufre, l'azote et CO et éventuellement substitué par un ou plusieurs groupements R¹⁰ ; ou
R⁴ et R⁵ forment ensemble et avec l'atome N auquel ils sont liés un cycle hétérocycloalkyle en C₃₋₆ comportant en outre éventuellement un ou plusieurs groupements choisis parmi l'oxygène, le soufre, l'azote et CO, où ledit cycle hétérocycloalkyle en C₃₋₆ est saturé ou insaturé et est éventuellement substitué par un ou plusieurs groupements choisis parmi A, NR⁸R⁹ et R¹⁰ ;
chacun des radicaux R⁶ est indépendamment choisi parmi les groupements alkyle en C₁₋₆, cycloalkyle en C₃₋₇, hétérocycloalkyle en C₄₋₇, aryle et hétéroaryle, chacun de ceux-ci étant éventuellement substitué par un ou plusieurs substituants choisis parmi R¹⁰, R¹¹ et A ;
chacun des radicaux R⁷ est choisi parmi l'atome d'hydrogène et les groupements alkyle en C₁₋₆ et cycloalkyle en C₃₋₇, où chacun desdits groupements alkyle en C₁₋₆ est éventuellement substitué par un ou plusieurs atomes d'halogène ;
chacun des radicaux R⁸ et R⁹ est indépendamment choisi parmi l'atome d'hydrogène et les groupements alkyle en C₁₋₆, où chacun desdits groupements alkyle en C₁₋₆ est éventuellement substitué par un ou plusieurs atomes d'halogène ; ou
R⁸ et R⁹ forment ensemble et avec l'atome N auquel ils sont liés un cycle hétérocycloalkyle en C₄₋₆ comportant en outre éventuellement un ou plusieurs hétéroatomes choisis parmi l'oxygène et le soufre, où ledit cycle hétérocycloalkyle en C₄₋₆ est éventuellement substitué par un ou plusieurs groupements R¹⁰ ; et
chacun desdits radicaux R¹⁰ est choisi parmi les groupements cycloalkyle en C₃₋₇, aryle, hétéroaryle, O-hétéroaryle, arylalkyle et alkyle en C₁₋₆, chacun de ceux-ci étant éventuellement substitué par un ou plusieurs groupements A, où lorsque R¹⁰ représente un groupement alkyle en C₁₋₆ et deux groupements R¹⁰ ou plus sont liés au même atome de carbone, les groupements R¹⁰ peuvent être liés pour former un groupement spiroalkyle ; et
chacun des radicaux R¹¹ est indépendamment choisi parmi les groupements alkyle en C₁₋₆, cycloalkyle en C₃₋₇, (alkyle en C₁₋₆)-(cycloalkyle en C₃₋₇), (alkyle en C₁₋₆)-hétéroaryle, hétérocycloalkyle en C₄₋₇, aryle et hétéroaryle, chacun de ceux-ci étant éventuellement substitué par un ou plusieurs substituants choisis parmi A ; et
A est choisi parmi les atomes d'halogène et les groupements -NR⁴SO₂R⁵, -CN, -OR⁶, -NR⁴R⁵, -NR⁷R¹¹, hydroxyle, -CF₃, -CONR⁴R⁵, -NR⁴COR⁵, -NR⁷ (CO) NR⁴R⁵, -NO₂, -CO₂H, -CO₂R⁶, -SO₂R⁶, -SO₂NR⁴R⁵, -NR⁴COR⁵, -NR⁴COOR⁵, alkyle en C₁₋₆, aryle et -COR⁶.

2. Composé selon la revendication 1, où R² est choisi parmi les groupements suivants :
halogène, plus préférentiellement brome ;
aryle éventuellement substitué par un ou plusieurs substituants choisis parmi R¹¹ et A ;
alkyle en C₁₋₆ éventuellement substitué par un ou plusieurs substituants choisis parmi R¹¹ et A ;
alcényle en C₂₋₆ éventuellement substitué par un ou plusieurs substituants A ;
cycloalkyle en C₃₋₇ ;
hétéroaryle éventuellement substitué par un ou plusieurs substituants choisis parmi R¹¹ et A ;
hétérocycloalkyle en C₄₋₇ ; et
aryl-(hétérocycloalkyle en C₄₋₇) fusionné.

3. Composé selon la revendication 1 ou la revendication 2, où R² est choisi parmi les groupements suivants :
aryle éventuellement substitué par un ou plusieurs substituants choisis parmi les groupements -NR⁴R⁵, - NR⁴COR⁵, -CONR⁴R⁵, OR⁶, halogène, alkyle en C₁₋₆ éventuellement substitué, CN, hétérocycloalkyle en C₄₋₇ et hétéroaryle ;
alkyle en C₁₋₆ éventuellement substitué par un ou plusieurs substituants choisis parmi les groupements-NR⁴COR⁵, -CONR⁴R⁵, -NR⁴R⁵, OR⁶, aryle éventuellement substitué, hétéroaryle éventuellement substitué et hétérocycloalkyle en C₄₋₇ ;
alcényle en C₂₋₆ éventuellement substitué par un ou plusieurs substituants -CONR⁴R⁵ ;
cycloalkyle en C₃₋₇, plus préférentiellement cyclopropyle ;
hétéroaryle éventuellement substitué par un ou plusieurs substituants choisis parmi les groupements-NR⁴R⁵, hétérocycloalkyle en C₄₋₇, alkyle en C₁₋₆, cycloalkyle en C₃₋₇, (alkyle en C₁₋₆) - (cycloalkyle en C₃₋₇) et OR⁶ ;
hétérocycloalkyle en C₄₋₇ ; et
aryl-(hétérocycloalkyle en C₄₋₇) fusionné.

4. Composé selon l'une quelconque des revendications précédentes, où R² est choisi parmi les groupements suivants :
un groupement phényle éventuellement substitué par un ou plusieurs substituants choisis parmi les groupements -NHCO-(alkyle en C₁₋₆), -CONH-(alkyle en C₁₋₆), CO-(N-morpholinyle), Cl, F, -O-(alkyle en C₁₋₆), -CONMe₂, OCF₃, CN, CF₃, (alkyle en C₁₋₆)-(A), N-morpholinyle et pyrazolyle ;
un groupement hétéroaryle choisi parmi les groupements pyridinyle, quinoléinyle, pyrazoyle, furanyle et
pyrimidinyle, chacun étant éventuellement substitué par un ou plusieurs substituants choisis parmi les groupements alkyle en C₁₋₆, arylalkyle, O-(alkyle en C₁₋₆), N-morpholinyle ;
un groupement alkyle en C₁₋₆ éventuellement substitué par un ou plusieurs substituants choisis parmi les groupements -CONR⁴R⁵, phényle, pyridinyle, oxadiazolyle et pipéridinyle, où chacun desdits groupements phényle, pyridinyle, oxadiazolyle et pipéridinyle est en outre éventuellement substitué par un ou plusieurs groupements -NR⁴COR⁵, -CONR⁴R⁵, COR⁶, SO₂R⁶ ou aryle.

5. Composé selon la revendication 4, où chacun des groupements -CONR⁴R⁵ est indépendamment choisi parmi les suivants :
-CO(N-morpholinyle), -CO(N-pipéridinyle), -CO(N-pyrrolidinyle), -CO-(N-pipérazinyle), chacun étant en outre éventuellement substitué par un ou plusieurs substituants choisis parmi les groupements aryle, hétéroaryle, -OR⁶, CF₃, arylalkyle, -NR⁴COR⁵-CONR⁴R⁵,-NR⁴R⁵, halogène, alkyle en C₁₋₆ ; et
-CON (alkyle en C₁₋₆)₂, CONH (alkyle en C₁₋₆), CON(alkyle en C₁₋₆)(arylalkyle), CONH(cycloalkyle en C₃₋₇),-CONH(aryle), -CONH(hétéroaryle), où chacun desdits groupements alkyle en C₁₋₆, arylalkyle, aryle et hétéroaryle est en outre éventuellement substitué par un ou plusieurs groupements R¹¹ ou A.

6. Composé selon l'une quelconque des revendications précédentes, où R² représente un groupement alkyle en C₁₋₆ éventuellement substitué par un ou plusieurs substituants choisis parmi les groupements -NR⁴COR⁵, -CONR⁴R⁵, - NR⁴R⁵, OR⁶, hétérocycloalkyle en C₄₋₇, hétéroaryle et aryle, où ledit groupement aryle est éventuellement substitué par un ou plusieurs substituants choisis parmi les groupements -NR⁴COR⁵ et -CONR⁴R⁵.

7. Composé selon l'une quelconque des revendications 1 à 3, où R² est choisi parmi les groupements -CH₂CH₂CO-NR⁴R⁵, alkyle en C₁₋₆, cycloalkyle en C₃₋₇ et hétéroaryle choisi parmi les groupements furanyle et pyrazolyle, où lesdits groupements furanyle et pyrazolyle sont éventuellement substitués par un ou plusieurs substituants choisis parmi les groupements alkyle en C₁₋₆, cycloalkyle en C₃₋₇ et (alkyle en C₁₋₆)-(cycloalkyle en C₃₋₇).

8. Composé selon la revendication 7, où R² est choisi parmi les groupements Me, 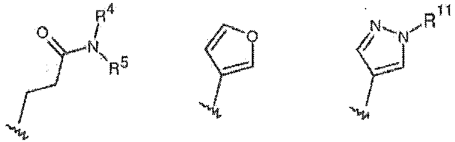 où R⁴ et R⁵ forment ensemble et avec l'atome N auquel ils sont liés un cycle hétérocycloalkyle en C₃₋₆ comportant en outre éventuellement un ou plusieurs groupements choisis parmi l'oxygène, le soufre, l'azote et CO, où ledit cycle hétérocycloalkyle en C₃₋₆ est saturé ou insaturé et est éventuellement substitué par un ou plusieurs groupements choisis parmi A, NR⁸R⁹ et R¹⁰.

9. Composé selon l'une quelconque des revendications 1 à 5, où R² représente un groupement alkyle en C₁₋₆ non substitué, plus préférentiellement méthyle.

10. Composé selon l'une quelconque des revendications précédentes, où R¹ est choisi parmi les groupements suivants :
-NHR³ ;
aryle ;
hétéroaryle ;
hétérocycloalkyle en C₄₋₇ ;
aryl-(hétérocycloalkyle en C₄₋₇) fusionné ;
- (cycloalkyle en C₃₋₇);
-NR³R⁶ ;
OR³ ;
NR⁴R⁵ ; et
-(alkyle en C₁₋₆) éventuellement substitué par un substituant choisi parmi R¹¹ et un groupement A ;
où chacun desdits groupements aryle, hétéroaryle, aryl-(hétérocycloalkyle en C₄₋₇) fusionné et hétérocycloalkyle en C₄₋₇ est éventuellement substitué par un ou plusieurs substituants choisis parmi les groupements alkyle en C₁₋₆, cycloalkyle en C₃₋₇, hétéroaryle, hétérocycloalkyle en C₄₋₇, aryle et un groupement A, et chacun desdits substituants alkyle en C₁₋₆, cycloalkyle en C₃₋₇, hétéroaryle, hétérocycloalkyle en C₄₋₇, et aryle est à son tour éventuellement substitué par un ou plusieurs groupements choisis parmi R¹¹ et un groupement A.

11. Composé selon l'une quelconque des revendications précédentes, où R¹ représente -NHR³, où R³ est choisi parmi les groupements alkyle en C₁₋₆, cycloalkyle en C₃₋₇, hétérocycloalkyle en C₄₋₇ et aryle, chacun étant éventuellement substitué par un ou plusieurs substituants choisis parmi R¹¹ et A.

12. Composé selon la revendication 11, où R¹ représente -NHR³ et R³ est choisi parmi les groupements suivants :
alkyle en C₁₋₆, éventuellement substitué par un ou plusieurs -OR⁶, NR⁴COR⁵, hétéroaryle, aryle, hétérocycloalkyle en C₄₋₇, et cycloalkyle en C₃₋₇, où chacun desdits groupements aryle et hétéroaryle est indépendamment en outre éventuellement substitué par un ou plusieurs groupements choisis parmi les groupements CF₃, halogène, alkyle en C₁₋₆, -OR⁶ et -NR⁴R⁵ ;
un groupement phényle éventuellement substitué par un ou plusieurs substituants choisis parmi les groupements -OR⁶, NR⁴COR⁵, -CONR⁴R⁵, aryl, -NR⁴R⁵, (alkyle en C₁₋₆)-hétéroaryle, hétéroaryle, halogène, -SO₂R⁶, CN, CF₃, alkyle en C₁₋₆, -SO₂NR⁴R⁵, -NR⁴SO₂R⁵, où chacun desdits groupements alkyle en C₁₋₆, hétéroaryle et aryle est indépendamment en outre éventuellement substitué par un ou plusieurs groupements choisis parmi les groupements CN, CF₃, halogène, alkyle en C₁₋₆, -OR⁶ et -NR⁴R⁵ ;
un groupement hétéroaryle éventuellement substitué par un ou plusieurs substituants choisis parmi les groupements aryle, alkyle en C₁₋₆ et -NR⁴R⁵, où ledit groupement aryle est en outre éventuellement substitué par un ou plusieurs groupements A ;
un groupement hétérocycloalkyle en C₄₋₇ éventuellement substitué par un ou plusieurs groupements -COR⁶ ;
un groupement cycloalkyle en C₃₋₇ éventuellement substitué par un ou plusieurs atomes d'halogène ou groupements alkyle en C₁₋₆.

13. Composé selon l'une quelconque des revendications 1 à 10, où R¹ représente -OR³, où R³ est choisi parmi les groupements alkyle en C₁₋₆, cycloalkyle en C₃₋₇, hétérocycloalkyle en C₄₋₇ et aryle, chacun étant éventuellement substitué par un ou plusieurs substituants choisis parmi R¹¹ et A.

14. Composé selon l'une quelconque des revendications 1 à 10, où R¹ représente un groupement aryle ou hétéroaryle, chacun étant éventuellement substitué par un ou plusieurs substituants choisis parmi R¹¹ et A.

15. Composé selon l'une quelconque des revendications 1 à 10, où R¹ représente un groupement - NH-(cycloalkyle en C₃₋₇) ou NH-(hétérocycloalkyle en C₄₋₇), chacun étant éventuellement substitué par un ou plusieurs substituants A.

16. Composé selon l'une quelconque des revendications précédentes, où R³ représente un groupement cyclohexyle ou tétrahydropyranyle, chacun étant éventuellement substitué par un ou plusieurs substituants A.

17. Composé selon l'une quelconque des revendications précédentes, où R¹ est choisi parmi les groupements suivants : 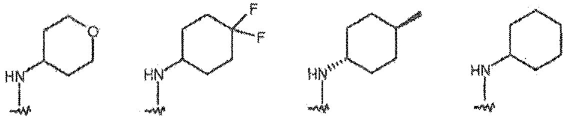 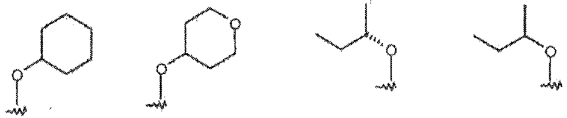 et où R¹ représente préférentiellement un groupement - NH-cyclohexyle.

18. Composé selon la revendication 1, où R¹ représente -NHR³ et R² représente un groupement alkyle en C₁₋₆ non substitué, plus préférentiellement méthyle.

19. Composé selon la revendication 1, où R¹ représente -NHR³ et R² représente un groupement alkyle en C₁₋₆ substitué par un ou plusieurs groupements - CONR⁴R⁵.

20. Composé selon la revendication 1, où R¹ représente -NHR³ et R² représente un groupement aryle ou hétéroaryle, chacun étant éventuellement substitué par un ou plusieurs substituants choisis parmi les groupements hétérocycloalkyle en C₄₋₇, alkyle en C₁₋₆, cycloalkyle en C₃₋₇, (alkyle en C₁₋₆)-(cycloalkyle en C₃₋₇) et OR⁶.

21. Composé selon la revendication 1, où R¹ est choisi parmi les groupements suivants : 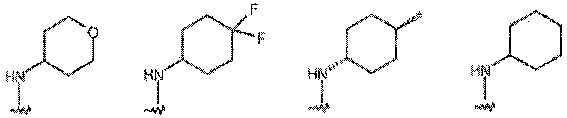 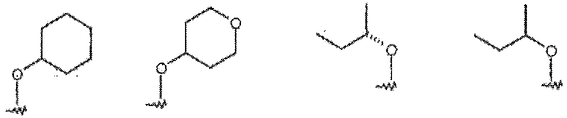 et R² est choisi parmi Me, 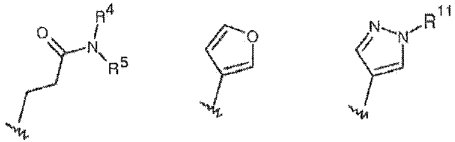 où R⁴ et R⁵ forment ensemble et avec l'atome N auquel ils sont liés un cycle hétérocycloalkyle en C₃₋₆ comportant en outre éventuellement un ou plusieurs groupements choisis parmi l'oxygène, le soufre, l'azote et CO, où ledit cycle hétérocycloalkyle en C₃₋₆ est saturé ou insaturé et est éventuellement substitué par un ou plusieurs groupements choisis parmi A, NR⁸R⁹ et R¹⁰.

22. Composé qui est choisi parmi les suivants :
| | |
|---|---|
| 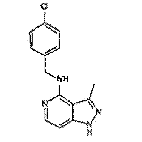 | Exemple 1 |
| 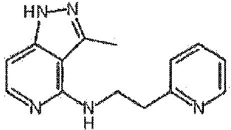 | Exemple 2 |
| 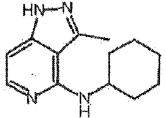 | Exemple 3 |
| 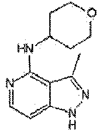 | Exemple 4 |
| 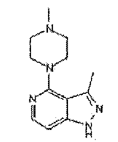 | Exemple 5 |
| 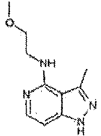 | Exemple 6 |
| 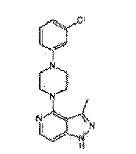 | Exemple 7 |
| 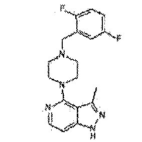 | Exemple 8 |
| 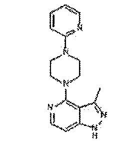 | Exemple 9 |
| 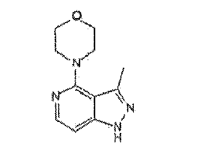 | Exemple 10 |
| 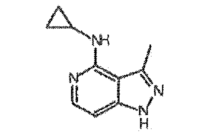 | Exemple 11 |
| 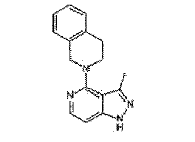 | Exemple 12 |
| 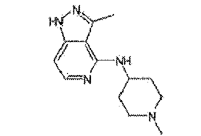 | Exemple 13 |
| 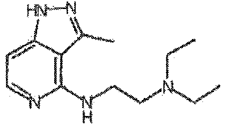 | Exemple 14 |
| 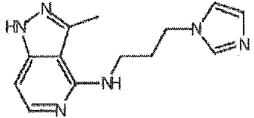 | Exemple 15 |
| 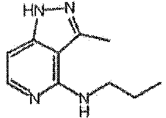 | Exemple 16 |
| 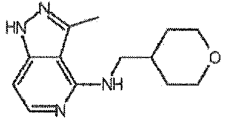 | Exemple 17 |
| 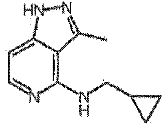 | Exemple 18 |
| 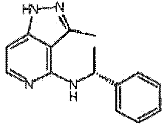 | Exemple 19 |
| 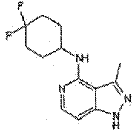 | Exemple 20 |
| 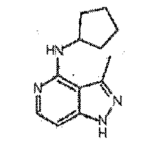 | Exemple 21 |
| 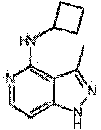 | Exemple 22 |
| 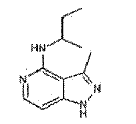 | Exemple 23 |
| 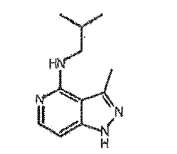 | Exemple 24 |
| 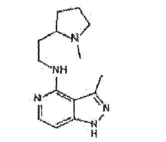 | Exemple 25 |
| 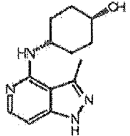 | Exemple 26 |
| 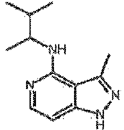 | Exemple 27 |
| 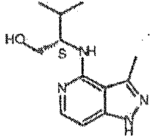 | Exemple 28 |
| 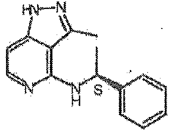 | Exemple 29 |
| 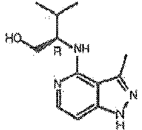 | Exemple 30 |
| 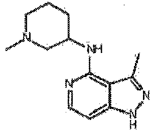 | Exemple 31 |
| 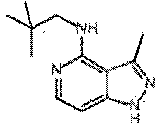 | Exemple 32 |
| 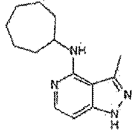 | Exemple 33 |
| 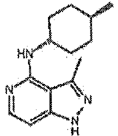 | Exemple 34 |
| 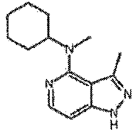 | Exemple 35 |
| 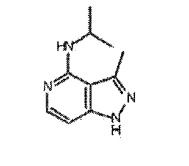 | Exemple 36 |
| 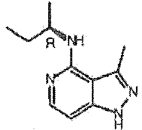 | Exemple 37 |
| 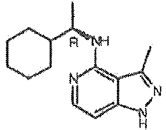 | Exemple 38 |
| 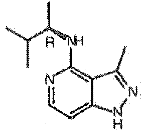 | Exemple 39 |
| 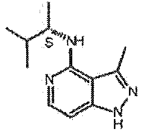 | Exemple 40 |
| 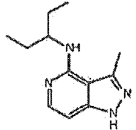 | Exemple 41 |
| 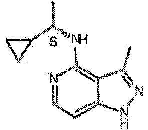 | Exemple 42 |
| 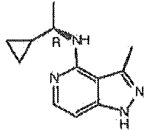 | Exemple 43 |
| 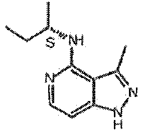 | Exemple 44 |
| 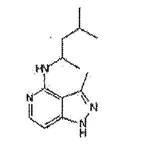 | Exemple 45 |
| 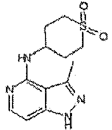 | Exemple 46 |
| 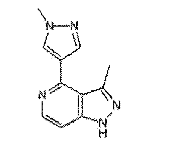 | Exemple 47 |
| 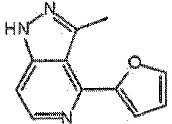 | Exemple 48 |
| 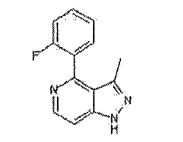 | Exemple 49 |
| 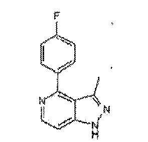 | Exemple 50 |
| 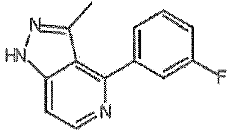 | Exemple 51 |
| 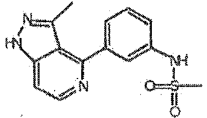 | Exemple 52 |
| 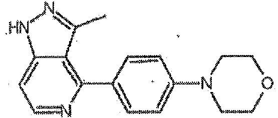 | Exemple 53 |
| 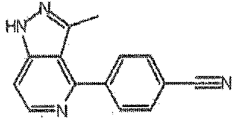 | Exemple 54 |
| 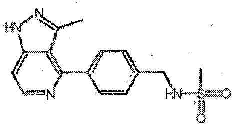 | Exemple 55 |
| 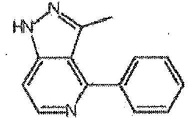 | Exemple 56 |
| 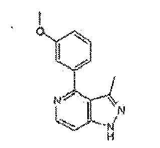 | Exemple 57 |
| 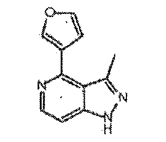 | Exemple 58 |
| 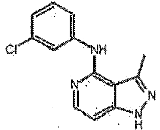 | Exemple 59 |
| 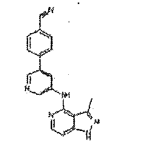 | Exemple 60 |
| 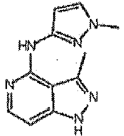 | Exemple 61 |
| 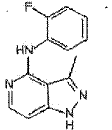 | Exemple 62 |
| 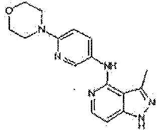 | Exemple 63 |
| 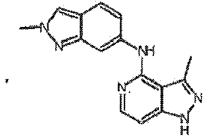 | Exemple 64 |
| 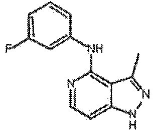 | Exemple 65 |
| 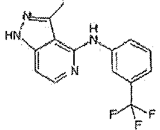 | Exemple 66 |
| 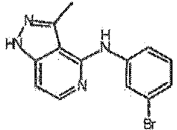 | Exemple 67 |
| 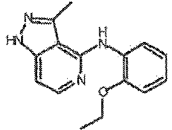 | Exemple 68 |
| 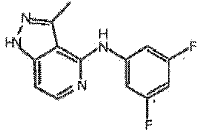 | Exemple 69 |
| 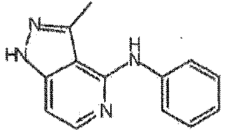 | Exemple 70 |
| 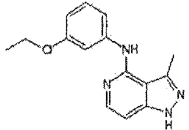 | Exemple 71 |
| 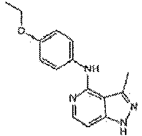 | Exemple 72 |
| 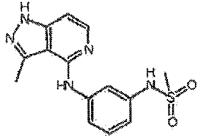 | Exemple 73 |
| 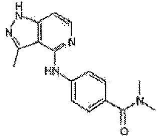 | Exemple 74 |
| 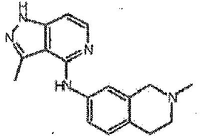 | Exemple 75 |
| 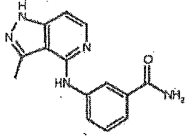 | Exemple 76 |
| 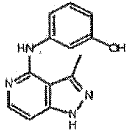 | Exemple 77 |
| 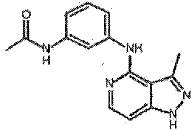 | Exemple 78 |
| 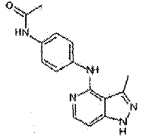 | Exemple 79 |
| 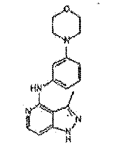 | Exemple 80 |
| 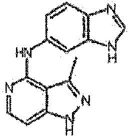 | Exemple 81 |
| 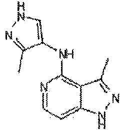 | Exemple 82 |
| 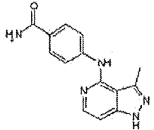 | Exemple 83 |
| 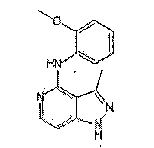 | Exemple 84 |
| 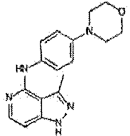 | Exemple 85 |
| 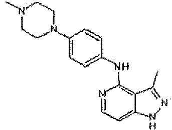 | Exemple 86 |
| 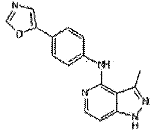 | Exemple 87 |
| 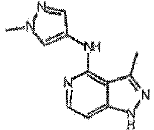 | Exemple 88 |
| 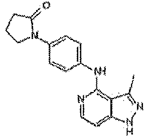 | Exemple 89 |
| 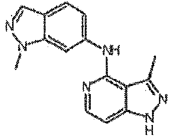 | Exemple 90 |
| 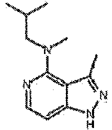 | Exemple 91 |
| 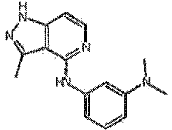 | Exemple 92 |
| 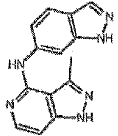 | Exemple 93 |
| 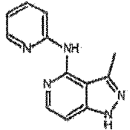 | Exemple 94 |
| 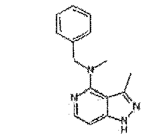 | Exemple 95 |
| 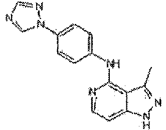 | Exemple 96 |
| 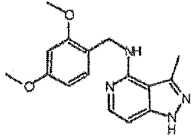 | Exemple 97 |
| 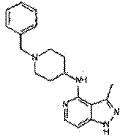 | Exemple 98 |
| 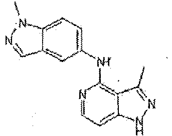 | Exemple 99 |
| 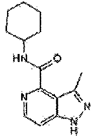 | Exemple 100 |
| 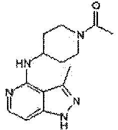 | Exemple 101 |
| 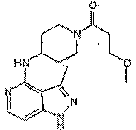 | Exemple 102 |
| 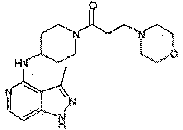 | Exemple 103 |
| 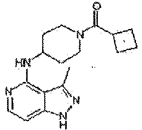 | Exemple 104 |
| 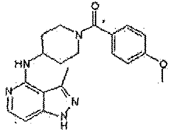 | Exemple 105 |
| 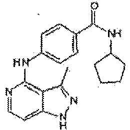 | Exemple 106 |
| 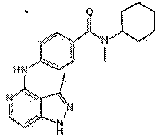 | Exemple 107 |
| 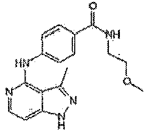 | Exemple 108 |
| 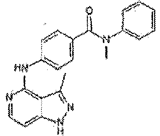 | Exemple 109 |
| 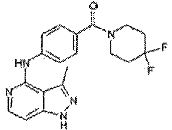 | Exemple 110 |
| 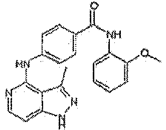 | Exemple 111 |
| 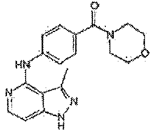 | Exemple 112 |
| 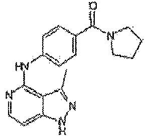 | Exemple 113 |
| 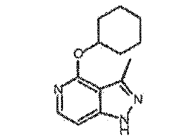 | Exemple 114 |
| 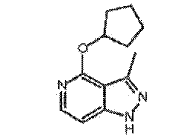 | Exemple 115 |
| 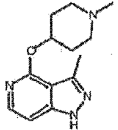 | Exemple 116 |
| 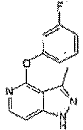 | Exemple 117 |
| 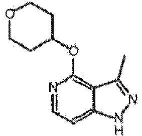 | Exemple 118 |
| 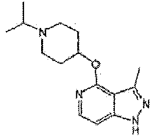 | Exemple 119 |
| 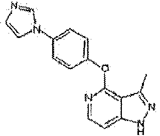 | Exemple 120 |
| 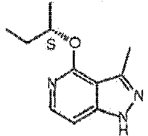 | Exemple 121 |
| 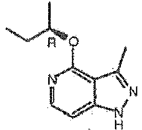 | Exemple 122 |
| 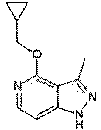 | Exemple 123 |
| 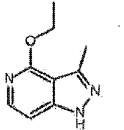 | Exemple 124 |
| 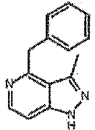 | Exemple 125 |
| 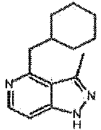 | Exemple 126 |
| 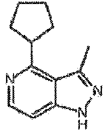 | Exemple 127 |
| 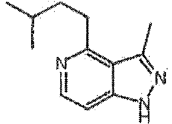 | Exemple 128 |
| 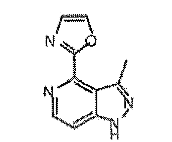 | Exemple 129 |
| 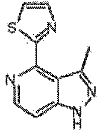 | Exemple 130 |
| 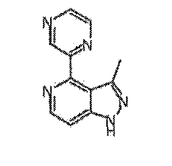 | Exemple 131 |
| | |
| 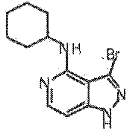 | Exemple 134 |
| 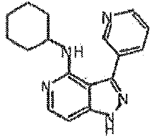 | Exemple 135 |
| 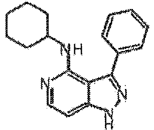 | Exemple 136 |
| 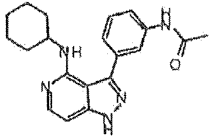 | Exemple 137 |
| 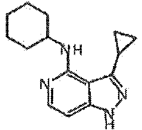 | Exemple 138 |
| 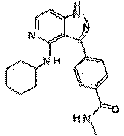 | Exemple 139 |
| 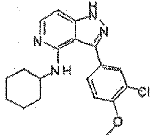 | Exemple 140 |
| 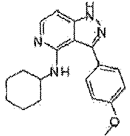 | Exemple 141 |
| 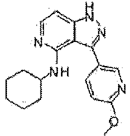 | Exemple 142 |
| 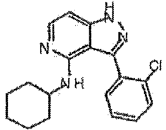 | Exemple 143 |
| 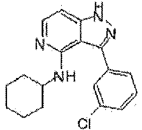 | Exemple 144 |
| 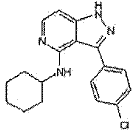 | Exemple 145 |
| 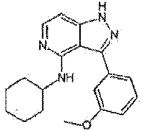 | Exemple 146 |
| 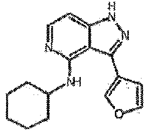 | Exemple 147 |
| 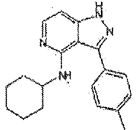 | Exemple 148 |
| 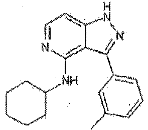 | Exemple 149 |
| 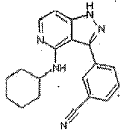 | Exemple 150 |
| 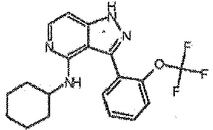 | Exemple 151 |
| 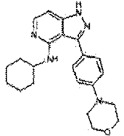 | Exemple 152 |
| 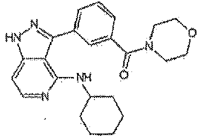 | Exemple 153 |
| 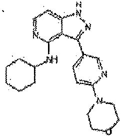 | Exemple 154 |
| 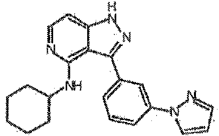 | Exemple 155 |
| 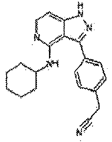 | Exemple 156 |
| 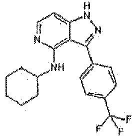 | Exemple 157 |
| 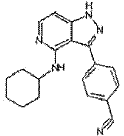 | Exemple 158 |
| 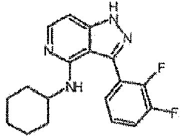 | Exemple 159 |
| 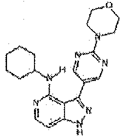 | Exemple 160 |
| 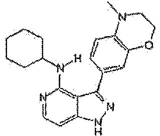 | Exemple 161 |
| 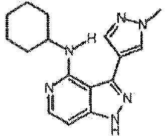 | Exemple 162 |
| 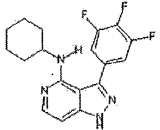 | Exemple 163 |
| 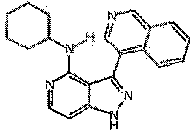 | Exemple 164 |
| 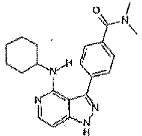 | Exemple 165 |
| 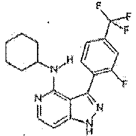 | Exemple 166 |
| 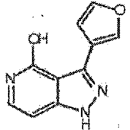 | Exemple 167 |
| 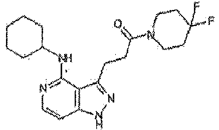 | Exemple 168 |
| 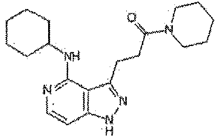 | Exemple 169 |
| 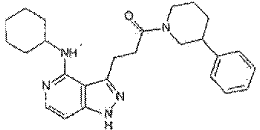 | Exemple 170 |
| 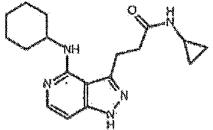 | Exemple 171 |
| 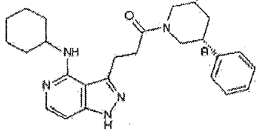 | Exemple 172 |
| 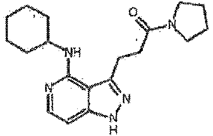 | Exemple 173 |
| 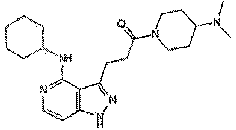 | Exemple 174 |
| 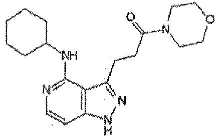 | Exemple 175 |
| 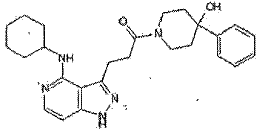 | Exemple 176 |
| 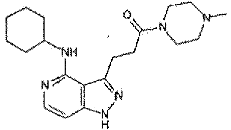 | Exemple 177 |
| 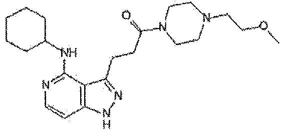 | Exemple 178 |
| 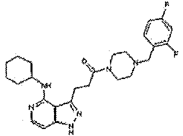 | Exemple 179 |
| 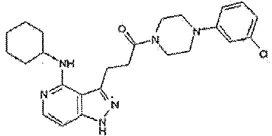 | Exemple 180 |
| 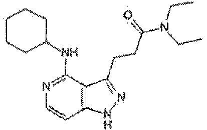 | Exemple 181 |
| 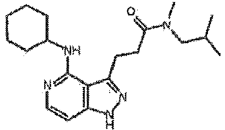 | Exemple 182 |
| 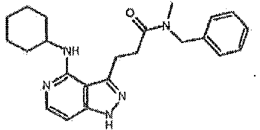 | Exemple 183 |
| 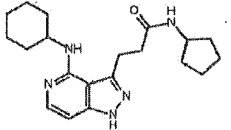 | Exemple 184 |
| 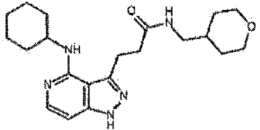 | Exemple 185 |
| 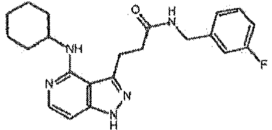 | Exemple 186 |
| 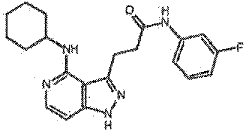 | Exemple 187 |
| 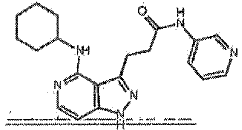 | Exemple 188 |
| 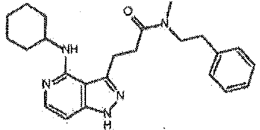 | Exemple 189 |
| 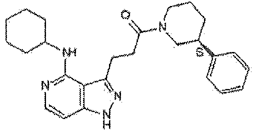 | Exemple 190 |
| 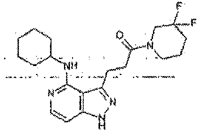 | Exemple 191 |
| 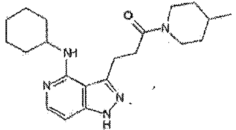 | Exemple 192 |
| 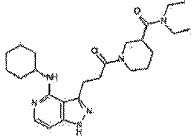 | Exemple 193 |
| 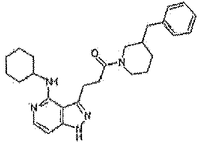 | Exemple 194 |
| 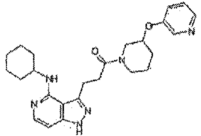 | Exemple 195 |
| 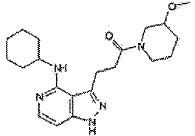 | Exemple 196 |
| 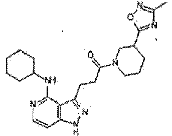 | Exemple 197 |
| 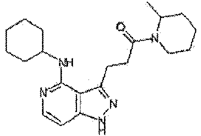 | Exemple 198 |
| 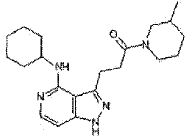 | Exemple 199 |
| 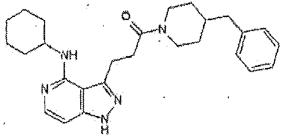 | Exemple 200 |
| 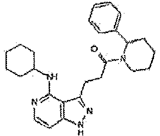 | Exemple 201 |
| 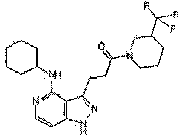 | Exemple 202 |
| 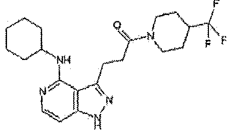 | Exemple 203 |
| 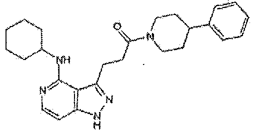 | Exemple 204 |
| 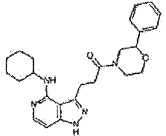 | Exemple 205 |
| 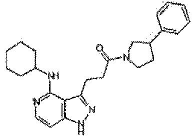 | Exemple 206 |
| 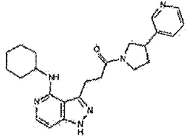 | Exemple 207 |
| 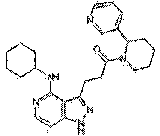 | Exemple 208 |
| 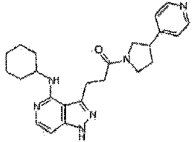 | Exemple 209 |
| 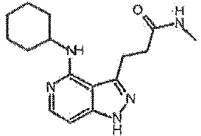 | Exemple 210 |
| 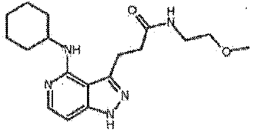 | Exemple 211 |
| 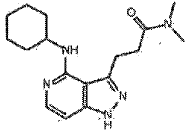 | Exemple 212 |
| 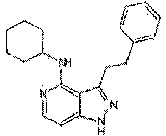 | Exemple 213 |
| 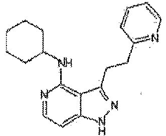 | Exemple 214 |
| 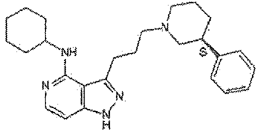 | Exemple 215 |
| 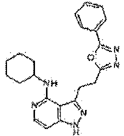 | Exemple 216 |
| 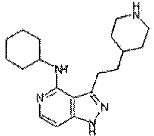 | Exemple 217 |
| 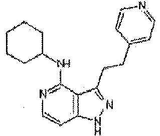 | Exemple 218 |
| 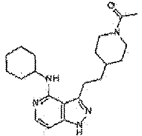 | Exemple 219 |
| 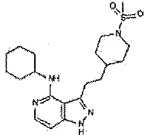 | Exemple 220 |
| 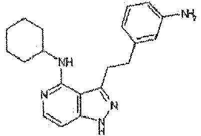 | Exemple 221 |
| 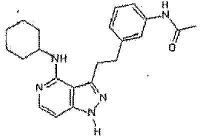 | Exemple 222 |
| 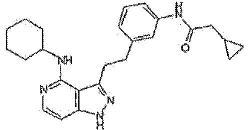 | Exemple 223 |
| 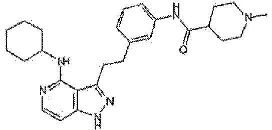 | Exemple 224 |
| 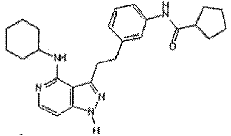 | Exemple 225 |
| 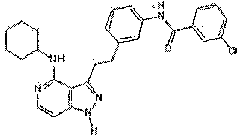 | Exemple 226 |
| 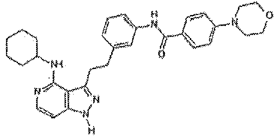 | Exemple 227 |
| 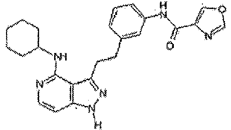 | Exemple 228 |
| 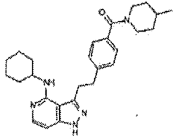 | Exemple 229 |
| 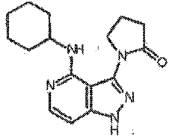 | Exemple 230 |
| 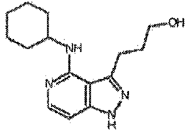 | Exemple 231 |
| 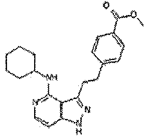 | Exemple 232 |
| 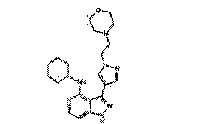 | Exemple 233 |
| 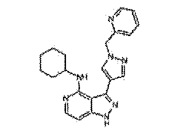 | Exemple 234 |
| 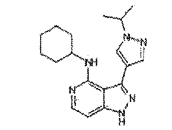 | Exemple 235 |
| 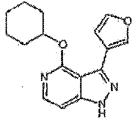 | Exemple 236 |
| 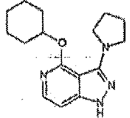 | Exemple 237 |
| 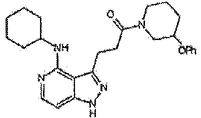 | Exemple 238 |
| 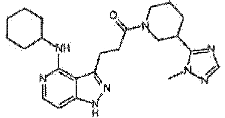 | Exemple 239 |
| 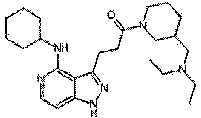 | Exemple 240 |
| 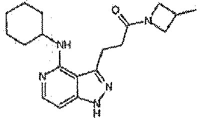 | Exemple 241 |
| 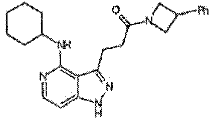 | Exemple 242 |
| 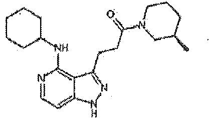 | Exemple 243 |
| 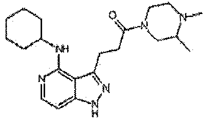 | Exemple 244 |
| 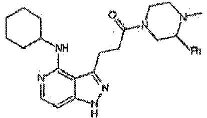 | Exemple 245 |
| 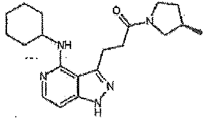 | Exemple 246 |
| 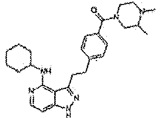 | Exemple 247 |
| 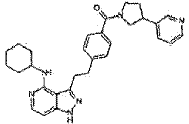 | Exemple 248 |
| 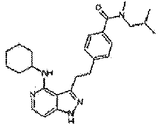 | Exemple 249 |
| 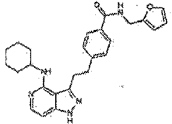 | Exemple 250 |
| 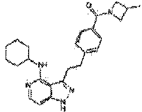 | Exemple 251 |
| 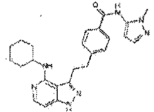 | Exemple 252 |
| 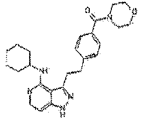 | Exemple 253 |
| 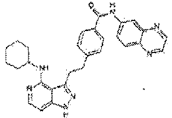 | Exemple 254 |
| 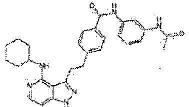 | Exemple 255 |
| 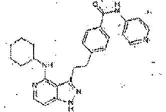 | Exemple 256 |
| 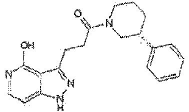 | Exemple 257 |
| 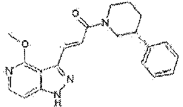 | Exemple 258 |
| 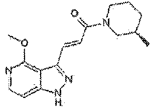 | Exemple 259 |
| 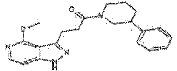 | Exemple 260 |
| 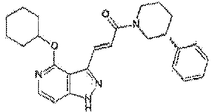 | Exemple 261 |
| 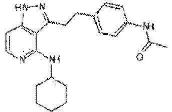 | Exemple 262 |
| 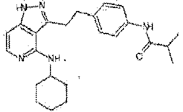 | Exemple 263 |
| 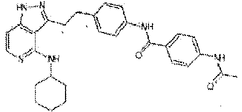 | Exemple 264 |
| 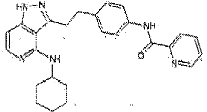 | Exemple 265 |
| 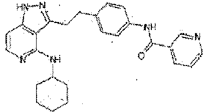 | Exemple 266 |
| 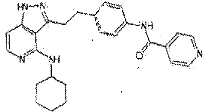 | Exemple 267 |
| 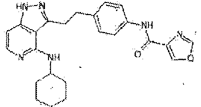 | Exemple 268 |
| 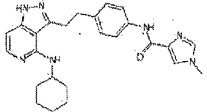 | Exemple 269 |
| 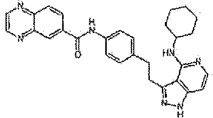 | Exemple 270 |
| 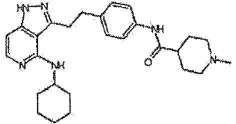 | Exemple 271 |
| 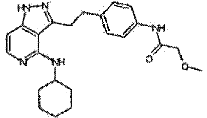 | Exemple 272 |
| 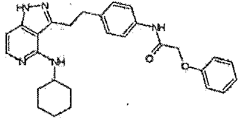 | Exemple 273 |
| 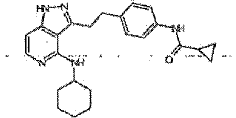 | Exemple 274 |
| 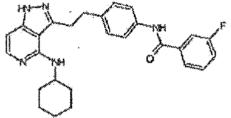 | Exemple 275 |
| 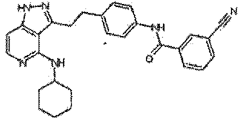 | Exemple 276 |
| 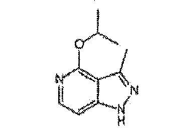 | Exemple 277 |
| 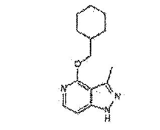 | Exemple 278 |
| 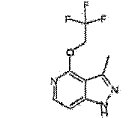 | Exemple 279 |
| 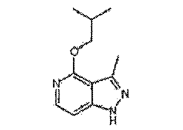 | Exemple 280 |
| 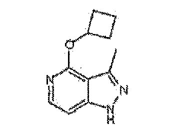 | Exemple 281 |
| 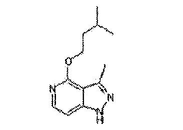 | Exemple 282 |
| 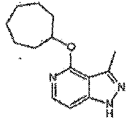 | Exemple 283 |
| 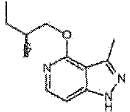 | Exemple 284 |
| 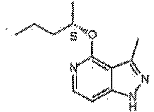 | Exemple 285 |
| 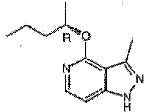 | Exemple 286 |
| 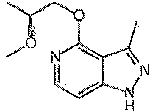 | Exemple 287 |
| 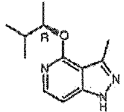 | Exemple 288 |
| 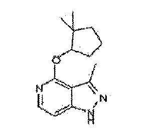 | Exemple 289 |
| 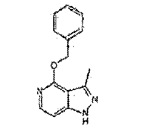 | Exemple 290 |
| 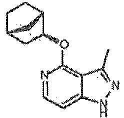 | Exemple 291 |
| 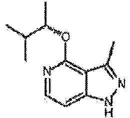 | Exemple 292 |
| 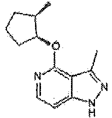 | Exemple 293 |
| 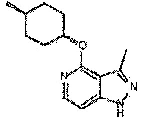 | Exemple 294 |
| 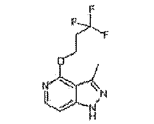 | Exemple 295 |
| 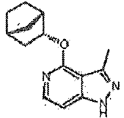 | Exemple 296 |
| 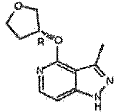 | Exemple 297 |
| 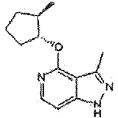 | Exemple 298 |
| 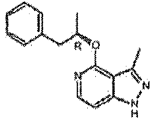 | Exemple 299 |
| 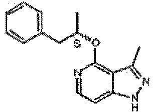 | Exemple 300 |
| 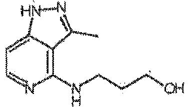 | Exemple 301 |
| 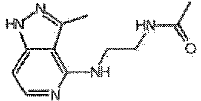 | Exemple 302 |
| 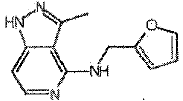 | Exemple 303 |
| 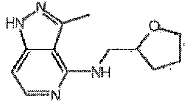 | Exemple 304 |
| 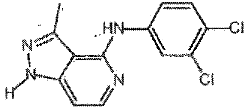 | Exemple 305 |
| 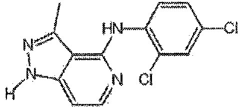 | Exemple 306 |
| 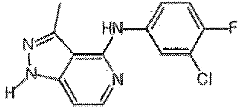 | Exemple 307 |
| 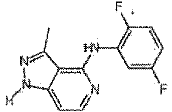 | Exemple 308 |
| 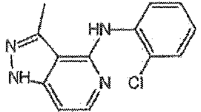 | Exemple 309 |
| 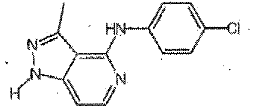 | Exemple 310 |
| 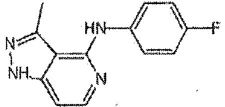 | Exemple 311 |
| 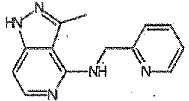 | Exemple 312 |
| 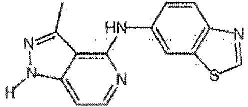 | Exemple 313 |
| 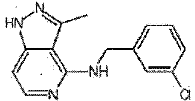 | Exemple 314 |
| 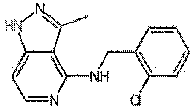 | Exemple 315 |
| 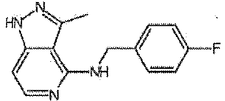 | Exemple 316 |
| 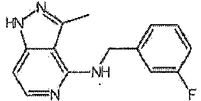 | Exemple 317 |
| 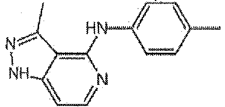 | Exemple 318 |
| 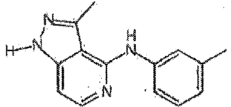 | Exemple 319 |
| 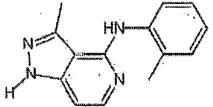 | Exemple 320 |
| 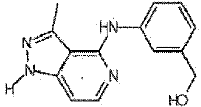 | Exemple 321 |
| 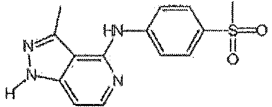 | Exemple 322 |
| 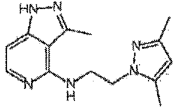 | Exemple 323 |
| 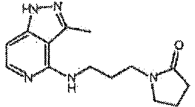 | Exemple 324 |
| 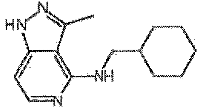 | Exemple 325 |
| 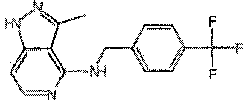 | Exemple 326 |
| 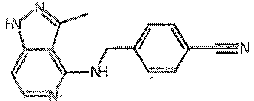 | Exemple 327 |
| 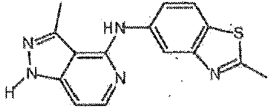 | Exemple 328 |
| 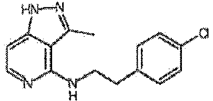 | Exemple 329 |
| 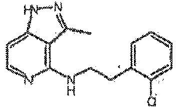 | Exemple 330 |
| 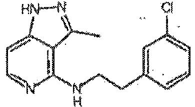 | Exemple 331 |
| 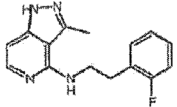 | Exemple 332 |
| 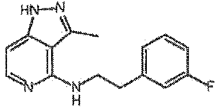 | Exemple 333 |
| 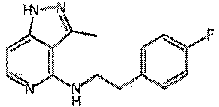 | Exemple 334 |
| 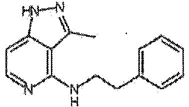 | Exemple 335 |
| 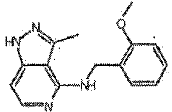 | Exemple 336 |
| 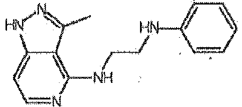 | Exemple 337 |
| 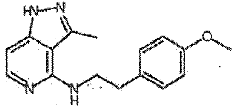 | Exemple 338 |
| 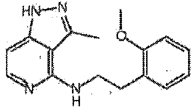 | Exemple 339 |
| 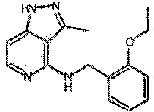 | Exemple 340 |
| 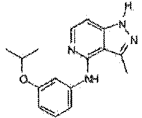 | Exemple 341 |
| 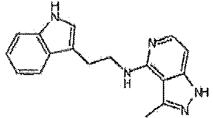 | Exemple 342 |
| 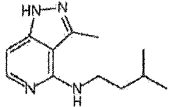 | Exemple 343 |
| 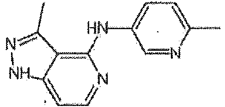 | Exemple 344 |
| 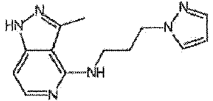 | Exemple 345 |
| 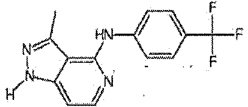 | Exemple 346 |
| 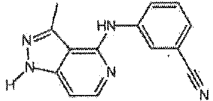 | Exemple 347 |
| 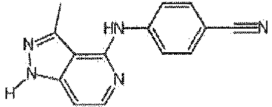 | Exemple 348 |
| 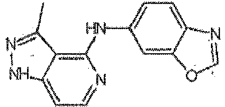 | Exemple 349 |
| 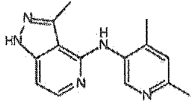 | Exemple 350 |
| 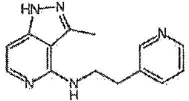 | Exemple 351 |
| 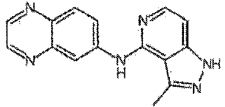 | Exemple 352 |
| 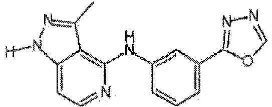 | Exemple 353 |
| 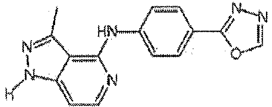 | Exemple 354 |
| 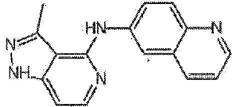 | Exemple 355 |
| 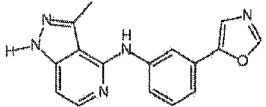 | Exemple 356 |
| 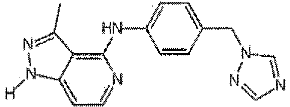 | Exemple 357 |
| 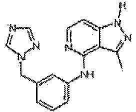 | Exemple 358 |
| 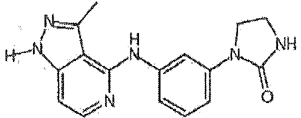 | Exemple 359 |
| 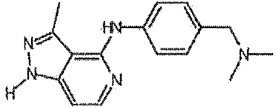 | Exemple 360 |
| 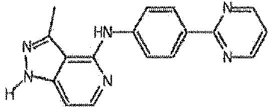 | Exemple 361 |
| 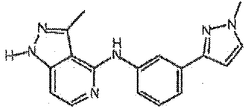 | Exemple 362 |
| 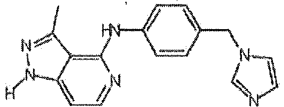 | Exemple 363 |
| 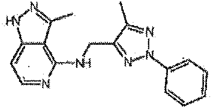 | Exemple 364 |
| 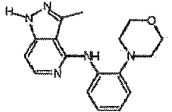 | Exemple 365 |
| 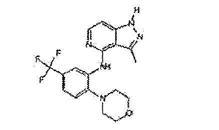 | Exemple 366 |
| 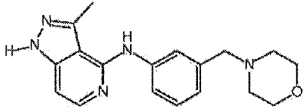 | Exemple 367 |
| 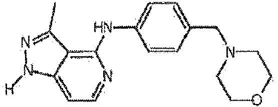 | Exemple 368 |
| 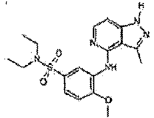 | Exemple 369 |
| 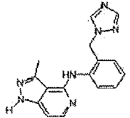 | Exemple 370 |
| 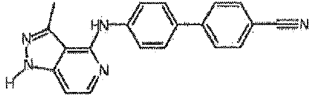 | Exemple 371 |
| 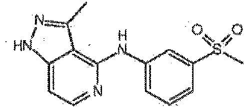 | Exemple 372 |
| 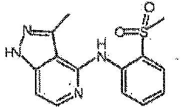 | Exemple 373 |
| 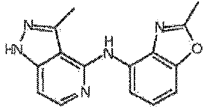 | Exemple 374 |
| 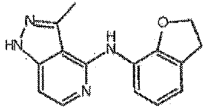 | Exemple 375 |
| 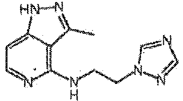 | Exemple 376 |
| 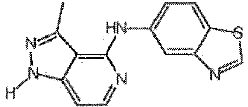 | Exemple 377 |
| 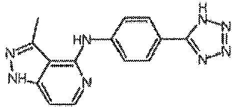 | Exemple 378 |
| 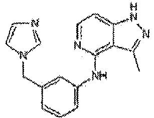 | Exemple 379 |
| 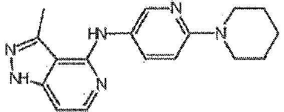 | Exemple 380 |
| 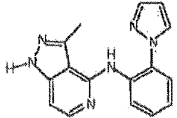 | Exemple 381 |
| 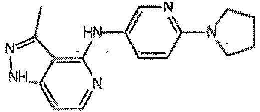 | Exemple 382 |
| 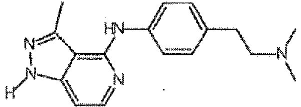 | Exemple 383 |
| 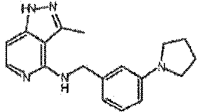 | Exemple 384 |
| 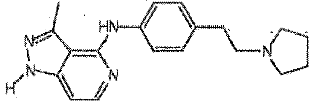 | Exemple 385 |
| 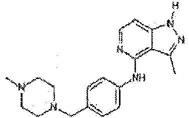 | Exemple 386 |
| 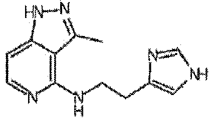 | Exemple 387 |
| 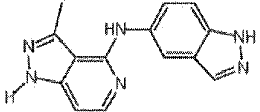 | Exemple 388 |
| 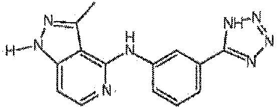 | Exemple 389 |
| 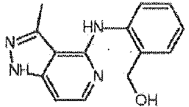 | Exemple 390 |
| 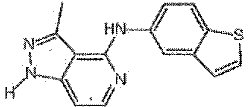 | Exemple 391 |
| 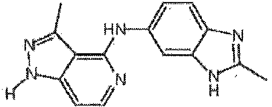 | Exemple 392 |

23. Composition pharmaceutique comprenant un composé selon l'une quelconque des revendications 1 à 22 et un vecteur, diluant ou excipient pharmaceutiquement acceptable, où ladite composition pharmaceutique comprend en outre éventuellement un deuxième agent thérapeutique.

24. Composé selon l'une quelconque des revendications 1 à 22 pour utilisation en médecine.

25. Composé selon l'une quelconque des revendications 1 à 22 pour utilisation dans le traitement d'un trouble choisi parmi le cancer et les maladies neurodégénératives.

26. Utilisation d'un composé selon l'une quelconque des revendications 1 à 22 dans l'élaboration d'un médicament destiné au traitement prophylactique ou thérapeutique d'un trouble choisi parmi le cancer et les maladies neurodégénératives, ou au traitement prophylactique ou thérapeutique d'un trouble provoqué, associé ou accompagné d'une activité anormale des kinases, préférentiellement d'une activité anormale de LRRK2.

27. Utilisation d'un composé selon l'une quelconque des revendications 1 à 22 dans un dosage d'identification de composés candidats supplémentaires capables d'inhiber la LRRK, plus préférentiellement la LRRK2.

28. Procédé de synthèse d'un composé de formule I selon la revendication 1, ledit procédé comprenant la conversion d'un composé de formule II en un composé de formule I : 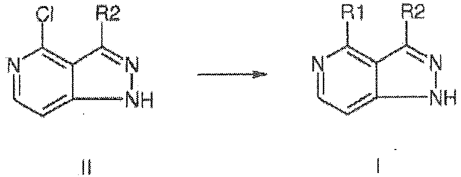 où ledit procédé comprend en outre éventuellement l'étape de synthèse dudit composé de formule II par traitement d'un composé de formule III par du monohydrate d'hydrazine : 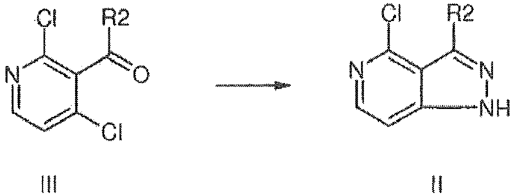 où ledit procédé comprend en outre éventuellement l'étape de synthèse dudit composé de formule III par traitement d'un composé de formule IV par un agent oxydant : 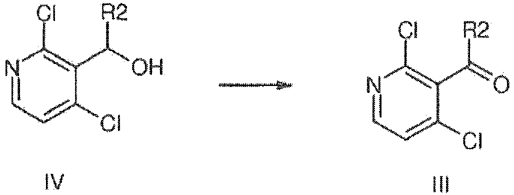 où ledit procédé comprend en outre éventuellement l'étape de synthèse dudit composé de formule IV par traitement d'un composé de formule V par R²-Mg-Cl :

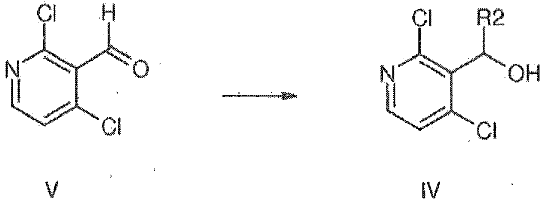

29. Procédé selon la revendication 28, où R¹ représente -NHR³, et ledit procédé comprend la réaction d'un composé de formule II avec une amine de formule NH₂R³.

30. Procédé selon la revendication 28, où R¹ représente un groupement hétérocycloalkyle en C₄₋₇ contenant NH ou aryl-(hétérocycloalkyle en C₄₋₇) fusionné contenant NH, et ledit procédé comprend la réaction d'un composé de formule II avec le groupement NH dudit groupement hétérocycloalkyle en C₄₋₇ ou aryl-(hétérocycloalkyle en C₄₋₇) fusionné.

31. Procédé selon la revendication 28, où R¹ est choisi parmi les groupements aryle, hétéroaryle, hétérocycloalkyle en C₄₋₇, aryl-(hétérocycloalkyle en C₄₋₇) fusionné, - (cycloalkyle en C₃₋₇) et -(alkyle en C₁₋₆), et ledit procédé comprend la réaction d'un composé de formule II avec X-R¹, où X représente un groupement 4,4,5,5-tétraméthyl-1,3,2-dioxaborolan-2-yle, en présence d'un agent de couplage.

32. Combinaison comprenant un composé selon l'une quelconque des revendications 1 à 22 et un agent thérapeutique supplémentaire.
